# FASCICULE DE BREVET EUROPEEN

(11) **EP 1 286 966 B1**
(45) Date de publication et mention de la délivrance du brevet: **16.07.2008**
(21) Numéro de dépôt: 00993405.0
(22) Date de dépôt: 13.12.2000
(51) Int. Cl.: C07D 211/58, C07D 409/14, C07D 417/14, C07D 401/14, C07D 405/14, C07D 401/12, A61P 5/00, A61K 31/445

(54) **DERIVES DE 4-AMINOPIPERIDINE ET LEUR UTILISATION EN TANT QUE MEDICAMENT**
4-AMINOPIPERIDIN-DERIVATE UND IHRE VERWENDUNG ALS ARZNEIMITTEL
4-AMINOPIPERIDINE DERIVATIVES AND THEIR USE AS MEDICINE

(30) Priorité: 14.12.1999 FR 9915724
(43) Date de publication de la demande: 05.03.2003
(73) Titulaire: SOCIETE DE CONSEILS DE RECHERCHES ET D'APPLICATIONS SCIENTIFIQUES (S.C.R.A.S.), 75016 Paris (FR)
(72) Inventeur: THURIEAU, Christophe, F-75016 Paris (FR); GONZALEZ, Jérôme, F-74100 Annemasse (FR); MOINET, Christophe, MONTREAL, QUEBEC H2M 2A8 (CA)
(74) Mandataire: Bourgouin, André
(86) Numéro de dépôt international: PCT/FR2000/003497
(87) Numéro de publication internationale: WO 2001/044191

(56) Documents cités:
- WO-A-98/44921
- WO-A-99/22735
- DE-A- 2 530 894
- DE-A- 2 751 138
- J. MED. CHEM.,, vol. 7, 1964, pages 729-732,
- DATABASE CA [en ligne] CHEMICAL ABSTRACTS SERVICE, COLUMBUS, OHIO, US 73:25305 & HU 157325 (08.04.1970)

## Description

La présente demande a pour objet de nouveaux dérivés de 4-aminopipéridines et leurs procédés de préparation par des méthodes de synthèse en parallèle en phase liquide et solide. Ces produits ayant une bonne affinité avec certains sous-types de récepteurs de la somatostatine, ils sont particulièrement intéressants pour traiter les états pathologiques ou les maladies dans lesquels un (ou plusieurs) des récepteurs de la somatostatine est (sont) impliqué(s).

La somatostatine (SST) est un tétradécapeptide cyclique qui a été isolé pour la première fois de l'hypothalamus en tant que substance inhibitrice de l'hormone de croissance (Brazeau P. et al., Science 1973, 179, 77-79). Elle intervient également en tant que neurotransmetteur dans le cerveau (Reisine T. et al., Neuroscience 1995, 67, 777-790 ; Reisine et al., Endocrinology 1995, 16, 427-442). Le clonage moléculaire a permis de montrer que la bioactivité de la somatostatine dépend directement d'une famille de cinq récepteurs liés à la membrane.

L'hétérogénéité des fonctions biologiques de la somatostatine a conduit à des études pour essayer d'identifier les relations structure-activité des analogues peptidiques sur les récepteurs de la somatostatine, ce qui a amené la découverte de 5 sous-types de récepteurs (Yamada et al., Proc. Natl. Acad. Sci. U.S.A, 89, 251-255, 1992 ; Raynor, K. et al, Mol. Pharmacol., 44, 385-392, 1993). Les rôles fonctionnels de ces récepteurs sont actuellement activement étudiés. Les affinités avec les différents sous-types de récepteurs de la somatostatine ont été associés au traitement des désordres / maladies suivants. L'activation des sous-types 2 et 5 a été associée à la suppression de l'hormone de croissance (GH) et plus particulièrement à celle des adénomes sécrétant GH (acromégalie) et de ceux sécrétant l'hormone TSH. L'activation du sous-type 2 mais pas du sous-type 5 a été associée au traitement des adénomes sécrétant la prolactine. D'autres indications associées avec l'activation des sous-types de récepteurs de la somatostatine sont la resténose, l'inhibition de la sécrétion d'insuline et/ou de glucagon et en particulier le diabète mellitus, l'hyperlipidémie, l'insensiblité à l'insuline, le Syndrome X, l'angiopathie, la rétinopathie proliférative, le phénomène de Dawn et la néphropathie ; l'inhibition de la sécrétion d'acide gastrique et en particulier les ulcères peptiques, les fistules entérocutanées et pancréaticocutanées, le syndrome du colon irritable, le syndrome de Dumping, le syndrome des diarrhées aqueuses, les diarrhées reliées au SIDA, les diarrhées induites par la chimiothérapie, la pancréatite aiguë ou chronique et les tumeurs gastrointestinales sécrétrices ; le traitement du cancer comme les hépatomes ; l'inhibition de l'angiogénèse, le traitement des désordres inflammatoires comme l'arthrite ; le rejet chronique des allogreffes ; l'angioplastie ; la prévention des saignements des vaisseaux greffés et des saignements gastrointestinaux. Les agonistes de la somatostatine peuvent aussi être utilisés pour diminuer le poids d'un patient.

Parmi les désordres pathologiques associés à la somatostatine (Moreau J.P. et al., Life Sciences, 1987, 40, 419 ; Harris A.G. et al., The European Journal of Medicine, 1993, 2, 97-105), on peut donc citer par exemple : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que la maladie d'Alzheimer. On peut également citer l'ostéoporose.

Les déposants ont trouvé que les composés de formule générale décrits ci-après présentaient une affinité et une sélectivité pour les récepteurs de la somatostatine. Comme la somatostatine et ses analogues peptidiques ont souvent une mauvaise biodisponibilité par voie orale et une faible sélectivité (Robinson, C., Drugs of the Future, 1994, 19, 992; Reubi, J.C. et al., TIPS, 1995, 16, 110), lesdits composés, agonistes ou antagonistes non-peptidiques de la somatostatine, peuvent être avantageusement utilisés pour traiter les états pathologiques ou les maladies tels que présentés ci-dessus et dans lesquels un (ou plusieurs) des récepteurs de la somatostatine est (sont) impliqué(s). De manière préférentielle, lesdits composés peuvent être utilisés pour le traitement de l'acromégalie, des adénomes hypophysaires ou des tumeurs gastroentéropancréatiques endocriniennes dont le syndrome carcinoïde.

Des dérivés de la pipéridine, agonistes de la somatostatine, sont décrits dans WO 99/22735 et WO 98/44921. Ces dérivés présentent des radicaux en position 1 et 4 du cycle pipéridine bien différents des radicaux des composés de la présente invention.

La présente invention a donc pour objet des composés de formule générale 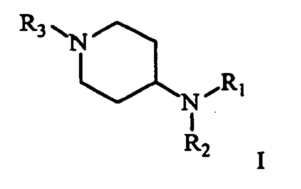 sous forme racémique, d'énantiomère ou toutes combinaisons de ces formes, dans laquelle :
**soit**
R₁ représente un radical (C₁-C₆)alkyle linéaire ou ramifié, le radical-(CH₂)ₘ-Y-Z₁₁ ou-(CH₂)ₘ-Z₁₂ dans lequel
Z₁₁ représente un (C₁-C₆)alkyle,
Z₁₂ représente *bis*-phényle, (C₃-C₇)cycloalkyle, (C₃-C₇)hétérocycloalkyle éventuellement substitué par un ou plusieurs substituants choisis indépendamment parmi les radicaux oxy et alkyle, ou aryle ou hétéroaryle éventuellement substitué par un ou plusieurs substituants choisis indépendamment parmi les radicaux fluoro, chloro, bromo, iodo, alkyle, alkoxy,
ou bien Z₁₂ représente 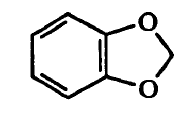 Y représente l'atome d'oxygène,
ou bien R₁ représente un radical de formule 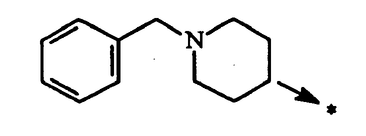 R₂ représente un radical de formule -C(Y)NHX₁, -C(O)X₂ ou SO₂X₃ dans laquelle
Y représente un atome d'oxygène ou de soufre ;
X₁ représente un radical (C₁-C₁₅)alkyle linéaire ou ramifié, ou -(CH₂)ₚZ₂₂ dans lequel
Z₂₂ représente cyclohexényle, *bis*-phényle, (C₃-C₇)cycloalkyle, (C₃-C₇)hétérocycloalkyle, mono- ou di-alkylamino, -C(O)-O-alkyle, ou aryle ou hétéroaryle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle, ou phényle,
ou bien Z₂₂ représente un radical de formule 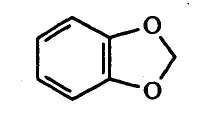 X₂ représente un radical alkynyle, -(CH₂)ₘ-W-(CH₂)_{q}-Z₂₃ ou -(CH₂)ₚ-U-Z₂₄ dans lequel
W représente SO₂,
U représente une liaison covalente,
Z₂₃ représente un radical aryle ;
Z₂₄ représente cyclohexényle, *bis*-phényle, (C₃-C₇)cycloalkyle éventuellement substitué par un radical aminoalkyle, ou aryle ou hétéroaryle éventuellement substitué par un ou plusieurs radicaux choisis parmi fluoro, chloro, bromo, iodo, alkyle, alkoxy, -CF₃, -OCF₃, -SCF₃, hydroxy, -O-C(O)-allcyle, mono- ou di-alkylamino, amino
ou Z₂₄ représente un radical de formule 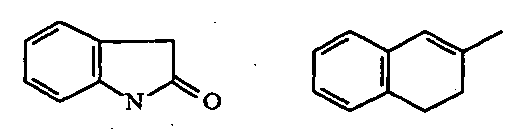 ou bien X₂ représente 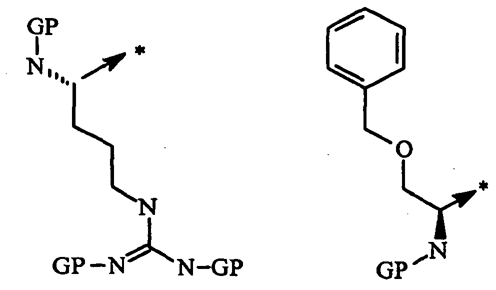 X₃ représente un radical-(CH₂)ₚZ₂₅ dans lequel Z₂₅ représente un radical aryle éventuellement substitué par un ou plusieurs radicaux identiques ou différents choisis parmi alkoxy et CF₃,
R₃ représente l'atome d'hydrogène, un radical alkyle, alkényle, hétéroarylalkyle éventuellement substitué ou un radical de formule -C(Y)-NHX₁, -C(O)X₂ ou SO₂X₃ dans laquelle
Y représente un atome d'oxygène ou de soufre;
X₁ représente un radical -(CH₂)ₚZ₂₂ dans lequel
Z₂₂ représente un radical aryle éventuellement substitué par un ou plusieurs radicaux choisis indépendamment parmi les radicaux fluoro, chloro, bromo, iodo, alkyle, alkoxy, CF₃, nitro, phénoxy ;
X₂ représente le radical vinyle substitué par un phényle, le radical phényle étant lui-même éventuellement substitué par un ou plusieurs radicaux halo, ou -(CH₂)ₚ-U-Z₂₄ dans lequel
U représente une liaison covalente ou l'atome d'oxygène ;
Z₂₄ représente alkyle, (C₃-C₇)cycloalkyle, (C₃-C₇)hétérocycloalkyle, *bis*-phényle, amino, mono ou di-alkylamino, ou aryle ou hétéroaryle éventuellement substitué par un ou plusieurs radicaux choisis parmi alkoxy, bromo, chloro, fluoro, hydroxy, CF₃, nitro, amino, mono- et di-alkylamino, pyrrolyle,
ou bien X₂ représente un radical de formule 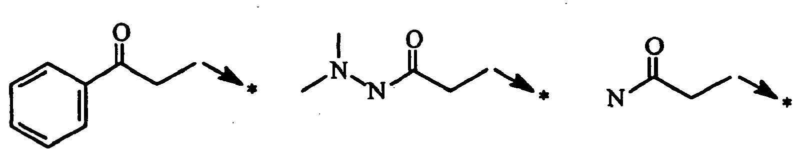 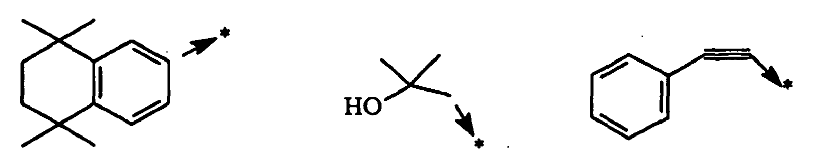 X₃ représente un radical (C₁-C₁₀)alkyle linéaire ou ramifié, le radical vinyle substitué par un radical (le radical phényle étant lui-même éventuellement substitué), CF₃, ou -(CH₂)ₚZ₂₅ dans lequel
Z₂₅ représente aryle ou hétéroaryle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino ;
n est un entier de 0 à 4 ;
m est un entier de 1 à 6 ;
p est un entier de 0 à 6 ;
q est un entier de 0 à 2,
soit
les radicaux R₁, R₂ et R₃ représentent respectivement les radicaux suivants :

| R1 | R2 | R3 |
|---|---|---|
| 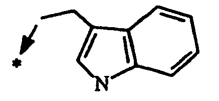 | 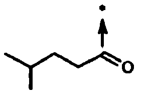 | 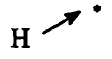 |
| 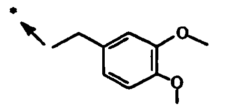 | 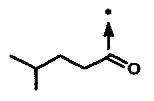 | 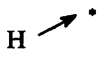 |
| 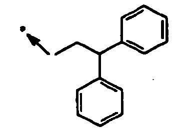 | 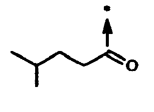 | 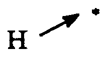 |
| 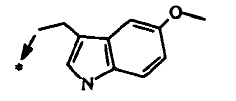 | 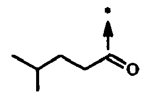 | 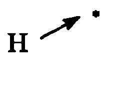 |
| 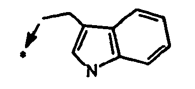 | 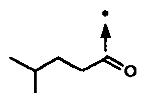 | 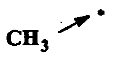 |
| 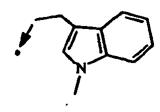 | 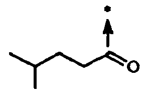 | 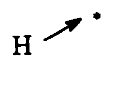 |
| 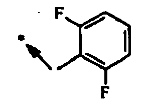 | 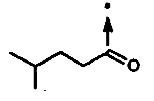 | 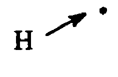 |
| 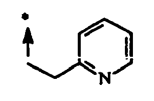 | 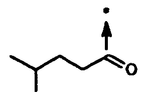 | 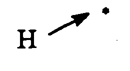 |

ou leurs sels d'addition avec les acides minéraux ou organiques pharmaceutiquement acceptables.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, *sec*-butyle et *tert*-butyle, pentyle ou amyle, isopentyle, neopentyle, hexyle ou isohexyle. Parmi les radicaux alkyle contenant de 1 à 15 atomes de carbone, on peut citer les alkyles tels que définis ci-dessus mais également les radicaux heptyle, octyle, nonyle, décyle, dodécyle, tridécyle ou pentadécyle.

Par alkényle, lorsqu'il n'est pas donné plus de précision, on entend un radical alkyle linéaire ou ramifié comptant de 1 à 6 atomes de carbone et présentant au moins une insaturation (double liaison), comme par exemple vinyle, allyle, propényle, butènyle ou pentènyle. Par alkynyle, lorsqu'il n'est pas donné plus de précision, on entend un radical alkyle linéaire ou ramifié comptant de 1 à 6 atomes de carbone et présentant au moins une double insaturation (triple liaison) comme par exemple un radical éthynyle, propargyle, butynyle ou pentynyle.

Le terme cycloalkyle désigne un système monocyclique carboné comprenant de 3 à 7 atomes de carbone, et de préférence les cycles cyclopropyle, cyclobutyle, cyclopentyle ou cyclohexyle. L'expression hétérocycloalkyle désigne un cycloalkyle saturé contenant de 2 à 7 atomes de carbones et au moins un hétéroatome. Ce radical peut contenir plusieurs hétéroatomes identiques ou différents. De préférence, les hétéroatomes sont choisis parmi l'oxygène, le soufre ou l'azote. Comme exemples d'hétérocycloalkyle, on peut citer le cycle pyrrolidine, pyrrolidinone, imidazolidine, pyrrazolidine, isothiazolidine, thiazolidine, isoxazolidine, pipéridine, pipérazine ou morpholine.

Les radicaux alkoxy peuvent correspondre aux radicaux alkyle indiqués ci-dessus comme par exemple les radicaux méthoxy, éthoxy, propyloxy ou isopropyloxy mais également butoxy linéaire, secondaire ou tertiaire, pentyloxy. Le terme alkylthio inférieur désigne de préférence les radicaux dans lesquels le radical alkyle est tel que défini ci-dessus comme par exemple méthylthio, éthylthio. Le terme alkylsulfonyle désigne de préférence les radicaux dans lesquels le radical alkyle est tel que défini ci-dessus.

L'expression aryle représente un radical aromatique, constitué d'un cycle ou de cycles condensés, comme par exemple le radical phényle ou naphtyle. L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux thiényle, furyle, pyrrolyle, imidazolyle, pyrazolyle, isothiazolyle, thiazolyle, isoxazolyle, oxazolyle, triazolyle, pyridyle, pyrazinyle, pyrimidyle, quinolyle, isoquinolyle, quinoxalinyle, benzothiényle, benzofuryle, indolyle, benzoxadiazoyle.

Les termes mono- et di-alkylamino désignent de préférence les radicaux dans lesquels les radicaux alkyle sont tels que définis ci-dessus, comme par exemple méthylamino, éthylamino, diméthylamino, diéthylamino ou (méthyl)(éthyl)amino.

Le symbole -> * correspond au point de rattachement du radical. Lorsque le site de rattachement n'est pas précisé sur le radical, cela signifie que le rattachement s'effectue sur un des sites disponibles de ce radical pour un tel rattachement.

De manière préférentielle, R₁ représente un radical (C₁-C₆)alkyle linéaire ou ramifié, le radical-(CH₂)ₘ-Y-Z₁₁ ou-(CH₂)ₘ-Z₁₂ dans lequel
Z₁₁ représente un (C₁-C₆)alkyle,
Z₁₂ représente naphtyle, morpholino, *bis*-phényle, pyrrolidinyle substitué par le radical oxy, ou bien les radicaux phényle, pipérazinyle, pyridinyle et indolyle qui sont éventuellement substitués par un ou plusieurs substituants choisis indépendamment parmi les radicaux bromo, fluoro, chloro, alkyle, alkoxy, -CF₃, -OCF₃ ;
ou bien Z₁₂ représente 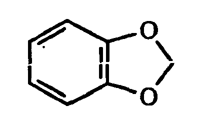 Y représente l'atome d'oxygène,
ou bien R₁ représente un radical de formule ci-dessous : 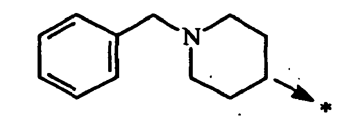 De manière préférentielle, R₂ représente un radical de formule-C(Y)NHX₁, -C(O)X₂ ou SO₂X₃ dans laquelle
X₁ représente un radical (C₁-C₁₀)alkyle linéaire ou ramifié, ou-(CH₂)ₚZ₂₂ dans lequel
Z₂₂ représente cyclohexyle, cyclohexényle, *bis*-phényle, morpholino, pipéridino, mono- ou di-alkylamino, -C(O)-O-alkyle, ou phényle, naphtyle ou furyle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle,-C(O)-O-alkyle, -C(O)-alkyle ou phényle,
ou bien Z₂₂ représente un radical de formule 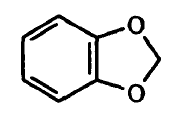 X₂ représente un radical alkynyle, -(CH₂)ₘ-W-(CH₂)_{q}-Z₂₃ ou -(CH₂)ₚZ₂₄ dans lequel
W représente SO₂ ;
Z₂₃ représente le radical phényle ;
Z₂₄ représente cyclohexényle, *bis*-phényle, cyclohexyle éventuellement substitué par un radical aminoalkyle, ou phényle, naphtyle, benzothiényle, thiényle ou indolyle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, -NH-C(O)-allcyle, mono- ou di-alkylamino, amino, ou
Z₂₄ représente un radical de formule 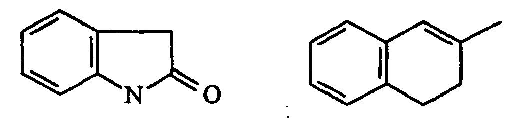 ou bien X₂ représente 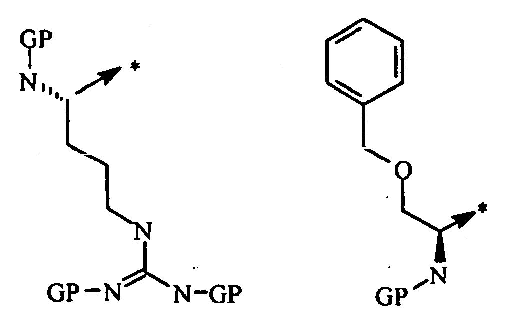 X₃ représente un radical -(CH₂)ₚZ₂₅ dans lequel Z₂₅ représente le radical phényle éventuellement substitué par un ou plusieurs radicaux identiques ou différents choisis parmie alkoxy et CF₃,

De manière préférentielle, R₃ représente l'atome d'hydrogène, un radical alkyle, alkényle ou furyl-méthyl susbtitué par un ou plusieurs radicaux nitro, ou un radical de formule -C(Y)-NHXt, -C(O)X₂ ou SO₂X₃ dans laquelle
X₁ représente un radical -(CH₂)ₚZ₂₂ dans lequel
Z₂₂ représente le radical phényle ou naphtyle éventuellement substitué par un ou plusieurs radicaux choisis indépendamment parmi les radicaux fluoro, chloro, bromo, iodo, alkyle, alkoxy, CF₃, nitro, phénoxy,
X₂ représente le radical vinyle substitué par un radical phényle lui-même éventuellement substitué par un ou plusieurs radicaux halo, ou -(CH₂)ₚ-U-Z₂₄ dans lequel
Z₂₄ représente alkyle, cyclohexyle, tétrahydrofuryle, *bis*-phényle, amino, mono ou di-alkylamino, ou phényle, indolyle, thiényle, pyridinyle, benzothiényle et furyle éventuellement substitué par un ou plusieurs radicaux choisis parmi alkoxy, bromo, chloro, fluoro, amino, mono- et di-alkylamino, nitro, hydroxy, pyrrolyle
ou bien X₂ représente un radical de formule 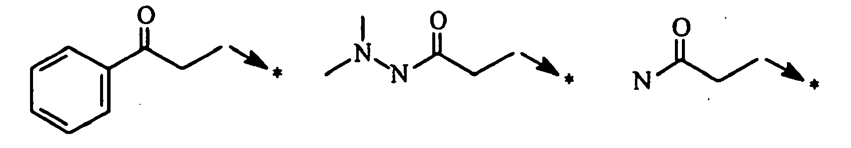 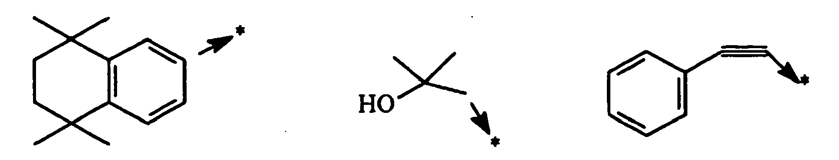 X₃ représente un radical (C₁-C₁₀)alkyle linéaire ou ramifié, le radical vinyle substitué par un radical phényle, CF₃, ou -(CH₂)ₚZ₂₅ dans lequel
Z₂₅ représente un radical phényle, napthyle, thiényle, pyrazolyle ou thiazolyle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino ;

De manière très préférentielle, R₁ représente le radical -(CH₂)ₘZ₁₂ dans lequel m = 2 et Z₁₂ représente *bis*-phényle ou bien le radical indolyle substitués par un ou plusieurs substituants choisis indépendamment parmi les radicaux alkyle et alkoxy.

De manière très préférentielle, R₂ représente les radicaux de formule -C(Y)NHX₁ et -C(O)X₂ dans laquelle
Y représente S ;
X₁ représente un radical phényle éventuellement substitué par un ou plusieurs radicaux azido,
X₂ représente -(CH₂)ₚZ₂₄ dans lequel
p est égal à 1, 2 ou 3,
Z₂₄ représente cyclohexyle, ou phényle ou benzothiényle éventuellement substitué par un ou plusieurs radicaux choisis parmi fluoro, chloro, bromo, iodo ou -CF₃.

De manière très préférentielle, R₃ représente l'atome d'hydrogène ou le radical méthyle.

Les composés selon l'invention peuvent être préparés en phase solide ou liquide.

### A) Synthèses en phase liquide via la pipéridone N-substituée

### A1) Amination réductrice

Elle s'effectue selon l'étape suivante : 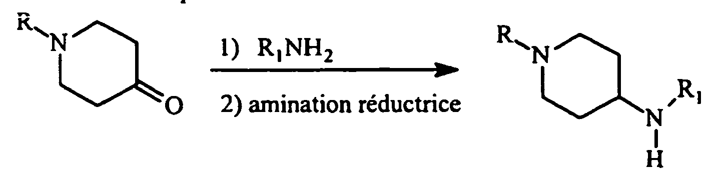 dans laquelle R représente méthyle ou Boc et R₁ a la signification indiquée ci-dessus.

La procédure générale est la suivante : l'amination réductrice (Abdel-Magid, A.F. ; Maryanoff, C.A. ; Carson, K.G. Tetrahedron Lett. 1990, 31, 5595-5598 ; Abdel-Magid, A.F. ; Carson, K.G. ; Harris, B.D. ; Maryanoff, C.A. ; Shah, R.D., J. Org. Chem. 1996, 61, 3849-3862) de la pipéridone N-substituée est réalisée dans des solvants anhydres chlorés tel que le dichloroéthane en présence d'une amine primaire (1,1 à 1,5 éq.), d'un agent réducteur tel que le triacétoxyborohydrure de sodium (1,1 à 1,5 éq.) et d'acide acétique (10 % en masse relatif à la pipéridone N-substituée). Le mélange réactionnel est agité pendant 1 à 4 heures à température ambiante. Dans certains cas, une solution de soude (0.1 M) est ajoutée et le mélange agité pendant 20 à 90 minutes. Sinon, le mélange réactionnel est lavé avec une solution saturée de bicarbonate de sodium, chlorure de sodium, séché sur sulfate de magnésium, filtré et concentré. Le produit désiré est purifié par chromatographie flash sur gel de silice.

### Préparation 1 : Carboxylate de tert-butyl 4-[(3,3-diphénylpropyl)amino]-1-pipéridine (C₂₅H₃₄N₂O₂, M = 394,56)

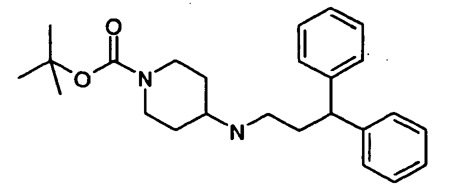

A 5 g (25 mmol) de N-Boc-pipéridone dans 100 ml de dichloroéthane sec est ajouté la 3,3-diphénylpropylamine (5,8 g, 27,5 mmol), le triacétoxyborohydrure de sodium (6,36 g, 30 mmol) et 0,5 ml d'acide acétique. La solution jaune trouble est agitée à température ambiante pendant 1 heure. 50 ml d'une solution de soude (0.1 M) sont alors ajoutés et le mélange agité pendant 30 minutes. La phase organique est lavée avec une solution saturée de bicarbonate de sodium, de chlorure de sodium, séchée sur sulfate de magnésium, filtrée et concentrée pour donner 10 g d'un solide jaune. Ce solide est purifié par chromatographie flash sur gel de silice en éluant avec un mélange heptane/acétate d'éthyle (4/1, 3 /1, 2/1 puis 1/1) puis à l'acétate d'éthyle pur. Les fractions sont concentrées sous vide pour donner 5,6 g (rdt = 57 %) d'un solide jaune pâle.

RMN ¹H (CD₃OD, 400 MHz) δ: 7,27 (m, 8H) ; 7,16 (m, 2H) ; 4 (dd, J = 6,4 et 14Hz, 3H) ; 2,73 (m, 2H) ; 2,55 (m, 3H) ; 2,26 (q, J = 7,6Hz, 2H) ; 1,78 (d, J = 12Hz, 2H); 1,45 (s, 9H) ; 1,15 (qd, J = 4,4 et 12,8Hz, 2H). SM/CL : m/z = 395,2 (M+H).

Une série de 4-aminosubstitué-1-pipéridine est préparée selon cette procédure avec les autres groupes R₁ suivants :

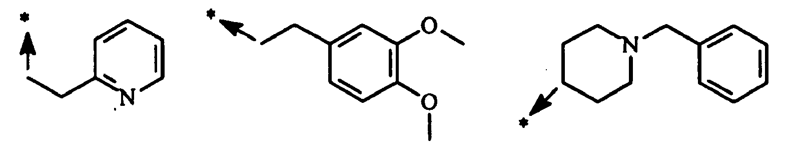

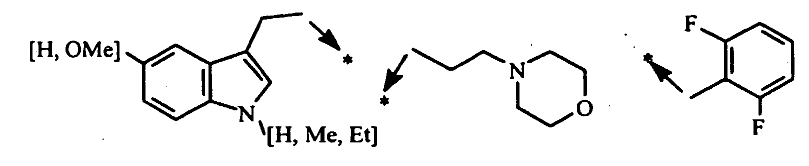

### A2) Fonctionnalisation des pipéridines

### A2a) Synthèses d'urées et de thiourées

Les synthèses d'urées et de thiourées sont mises en oeuvre selon la procédure décrite dans la littérature (Kaldor, S.W. ; Siegel, M.G. ; Fritz, J.E. ; Dressman, B.A. ; Hahn, P.J. Tetrahedron Lett. 1996, 37, 7193-7196 ; Kaldor, S.W. ; Fritz, J.E. ; Tang, J. ; McKinney, E.R. Bioorg. Med. Chem. Lett. 1996, 6, 3041-3044 ; Booth, R.J. ; Hodges, J.C. J. Am. Chem. Soc. 1997, 119, 4882-4886 ; Flynn, D.L. ; Crich, J.Z. ; Devraj, R.V. ; Hockerman, S.L. ; Parlow, J.J. ; South, M.S. ; Woodard, S. J. Am. Chem. Soc. 1997, 119, 4874-4881) suivant le schéma suivant : 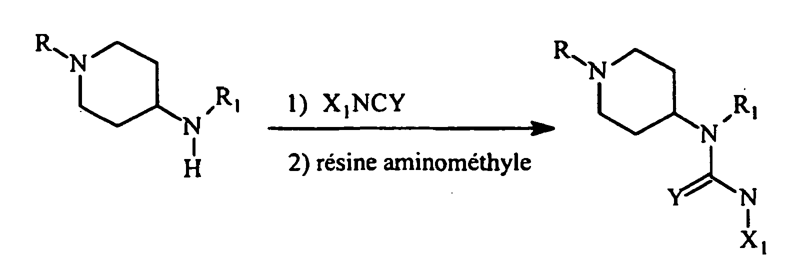 dans laquelle R représente méthyle ou Boc et X₁ et Y ont la signification indiquée ci-dessus. Il est à noter que dans le cas où R représente Boc, le produit ainsi obtenu est un produit final répondant à la formule 1 selon l'invention mais peut également être utilisé comme intermédiaire de synthèse.

La procédure générale est la suivante : l'isocyanate ou l'isothiocyanate (1,1 à 1,5 éq.) est ajouté à la 4-aminosubstitué-1-pipéridine dans des solvants aprotiques tels que le dichlorométhane, le tétrahydrofurane ou la diméthylformamide et le mélange est agité de 45 minutes à 18 heures à température ambiante. La résine aminométhyle (Novabiochem, 1,33 mmol/g, 0,2 à 1 éq.) est ajoutée et le mélange agité de 45 minutes à 18 heures. Dans certains cas, de la résine basique échangeuse d'ions telle que la IRA-68 (Gayo, L.M. ; Suto, M.J. Tetrahedron Lett. 1997, 38, 513-516) peut être ajoutée. Les résines sont filtrées et le filtrat concentré. D'autres purifications sur cartouche de gel de silice ou d'alumine basique (500 mg, Interchim) peuvent éventuellement être réalisées.

### Exemple A2a : Carboxylate de tert-butyl-4-((3,3-diphénylpropyl) {[3-(trifluoro méthyl) anilino] carbonyl}amino)-1-pipéridine (C₃₃H₃₈F₃N₃O₃, M = 581,68)

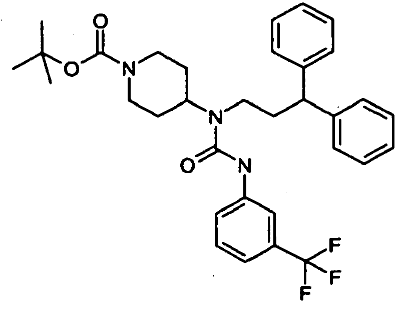

A une solution de carboxylate de *tert*-butyl 4-[(3,3-diphénylpropyl)amino]-1-pipéridine (470 mg, 1,2 mmol) dans 5 ml de dichlorométhane est ajouté 246 mg (1,32 mmol) d'isocyanate de 3-(trifluorométhyl)phényle. La solution est agitée 45 minutes, et la résine aminométhyle (180 mg, 0,36 mmol) est ajoutée et la réaction remise sur l'agitateur orbital pendant 45 minutes. La résine est filtrée et lavée au dichlorométhane. Le filtrat est concentré *in vacuo* pour donner 610 mg (rdt = 87 %) d'une mousse blanche.
RMN ¹H (CD₃OD, 400 MHz) δ : 7,71 (s, 1H) ; 7,57 (d, 1H) ; 7,43 (t, 1H) ; 7,26 (m, 10H) ; 7,15 (m, 1H) ; 4,1 (m, 3H) ; 3,97 (dd, J = 7,6 et 10Hz, 1H) ; 3,17 (m, 2H) ; 2,75 (m, 2H) ; 2,35 (m, 2H) ; 1,65 (d, J = 12Hz, 2H) ; 1,46 (s, 9H, groupe *t*butyle) ; 1,39 (dd, J = 2,4 et 10,8Hz, 2H) ; 1,29 ( s, 1H). SM/CL : m/z = 582 (M+H).

Pour les groupes R₁ tels qu'illustrés au point A1 ci-dessus, les groupes X₁ que l'on peut envisager pour la synthèse d'urées (Y = O) selon la procédure ci-dessus, sont les suivants :

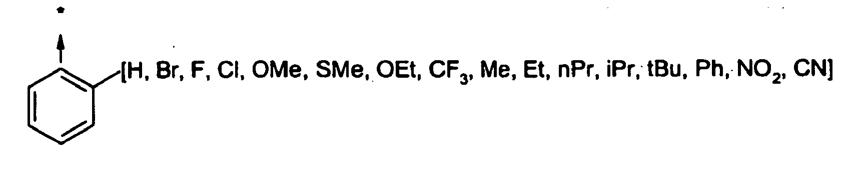

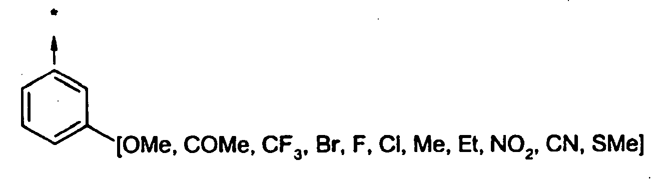

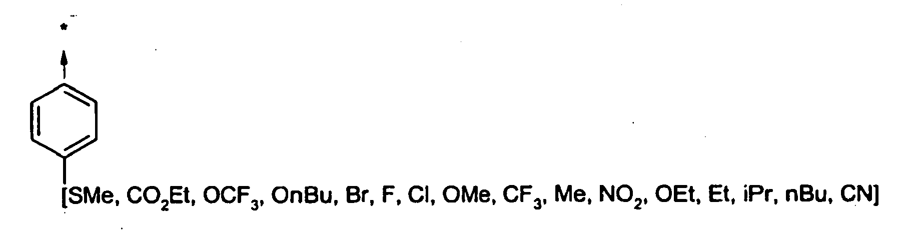

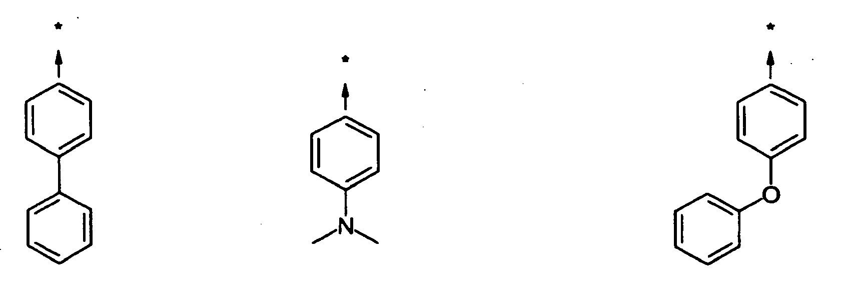

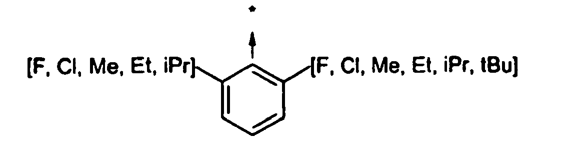

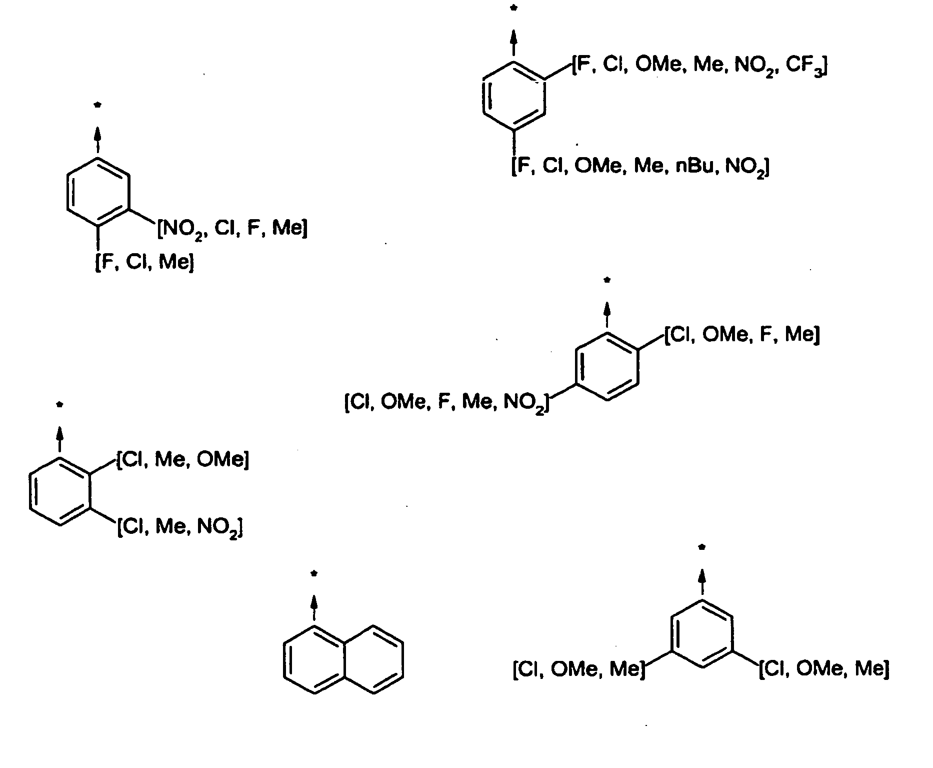

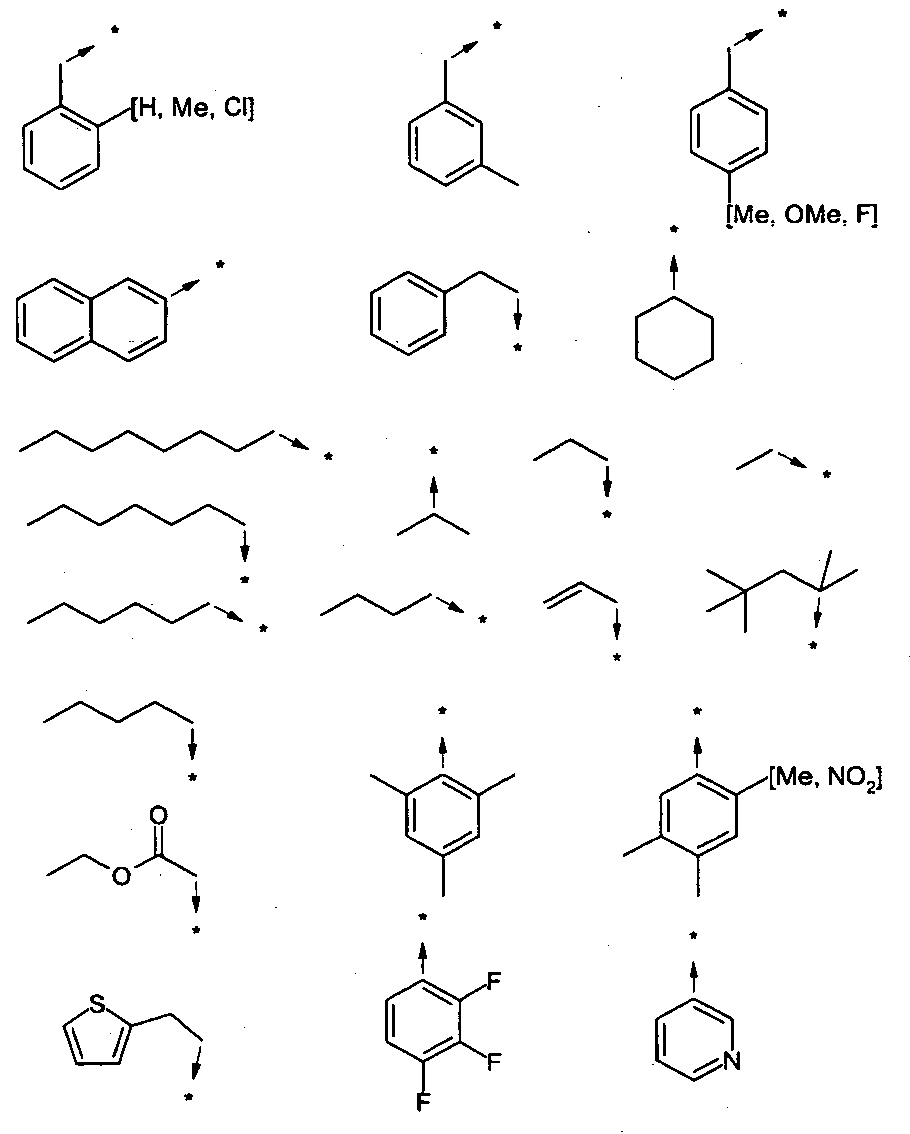

Pour les groupes R₁ tels qu'illustrés au point A1 ci-dessus, les groupes X₁ que l'on peut envisager pour la synthèse de thiourées (Y = S) selon la procédure ci-dessus, sont les suivants :

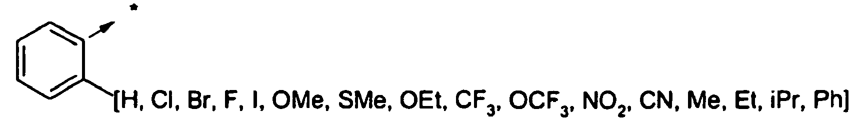

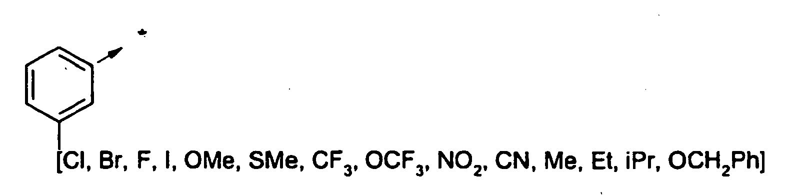

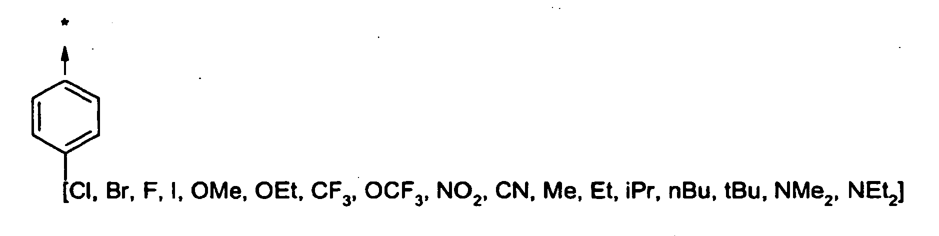

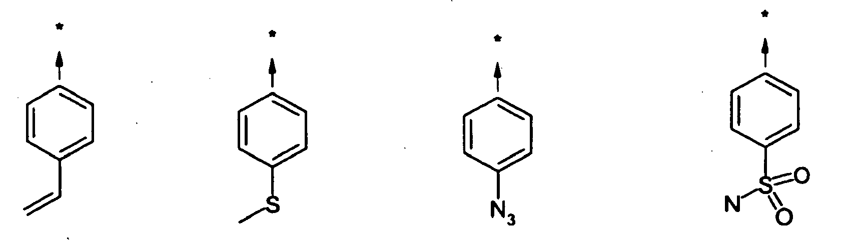

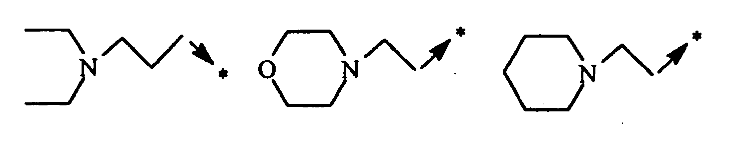

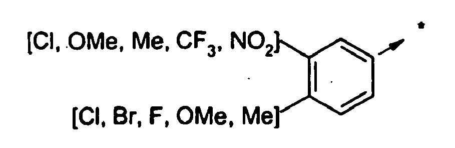

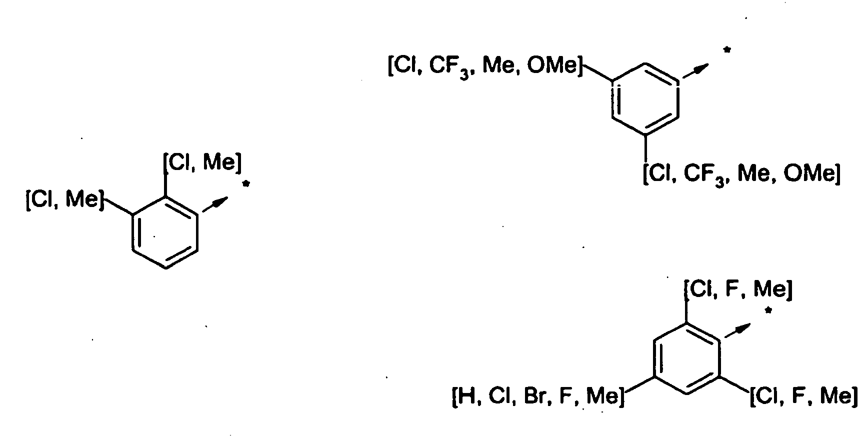

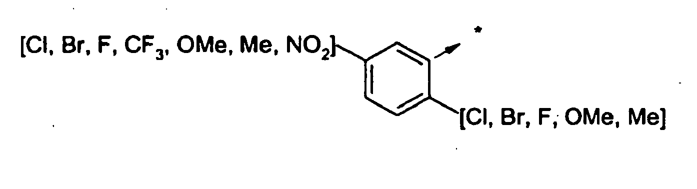

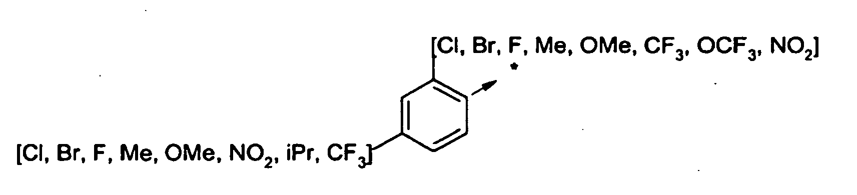

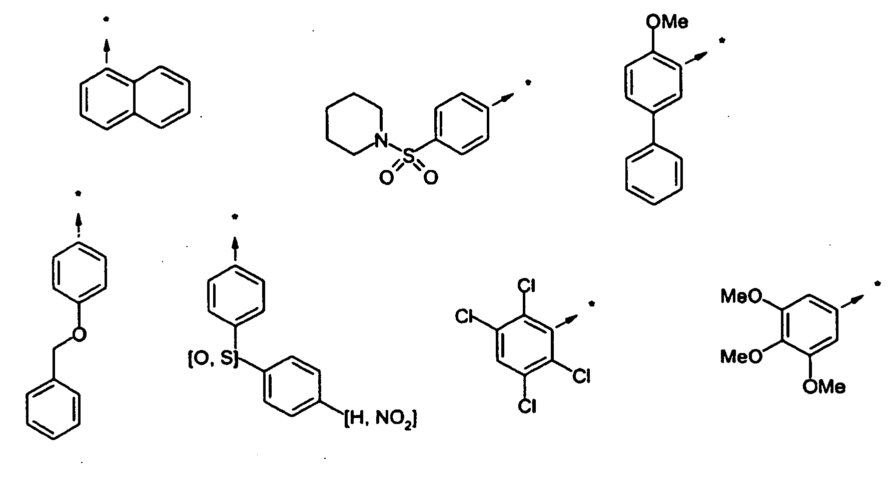

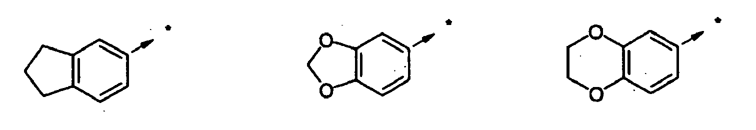

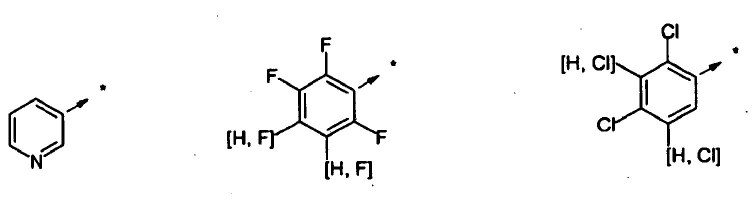

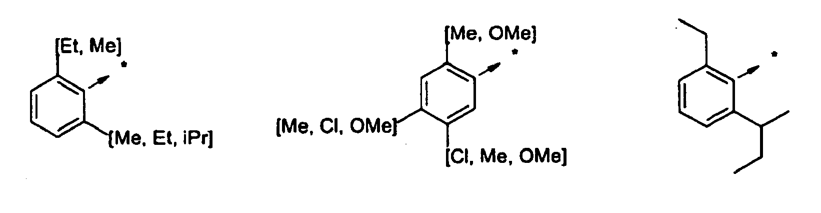

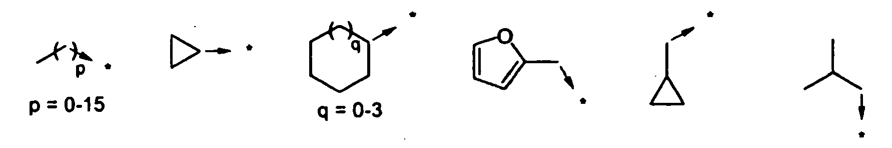

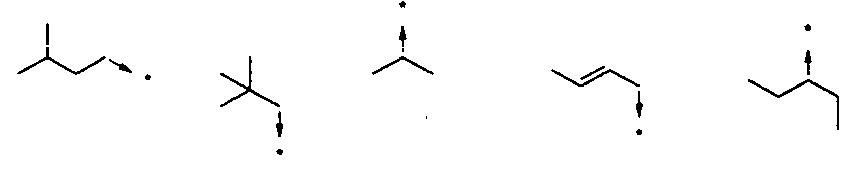

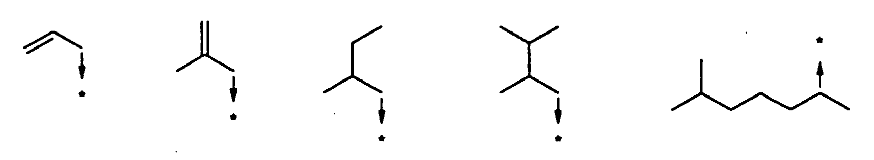

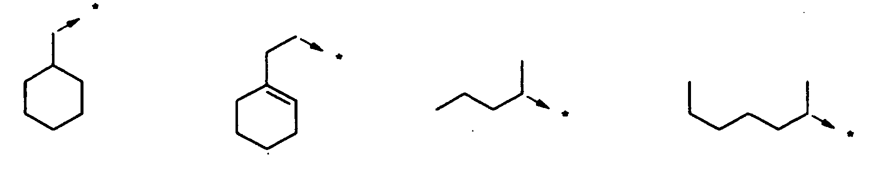

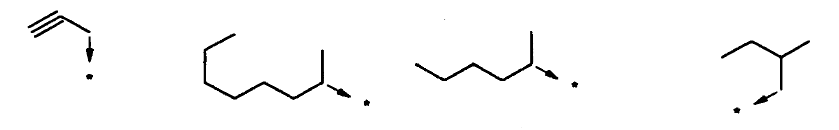

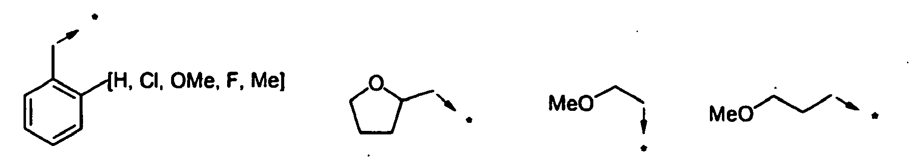

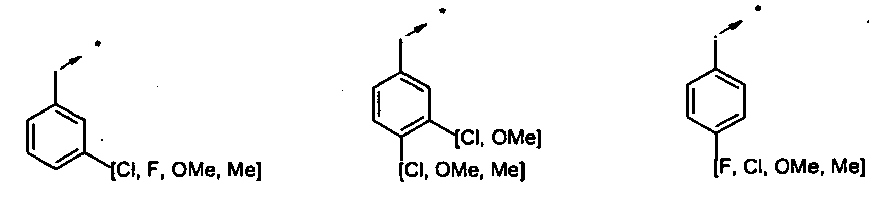

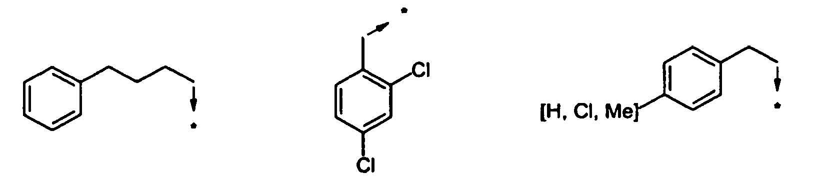

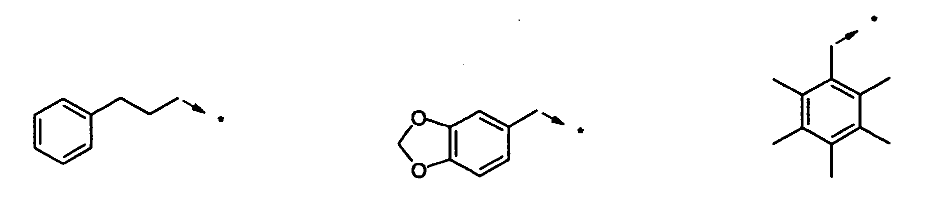

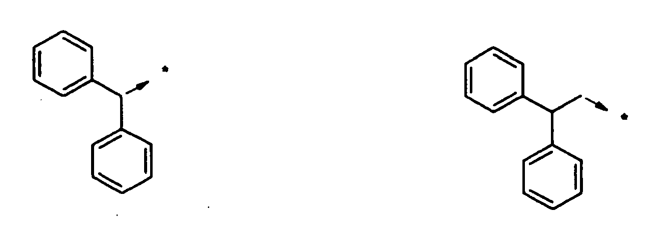

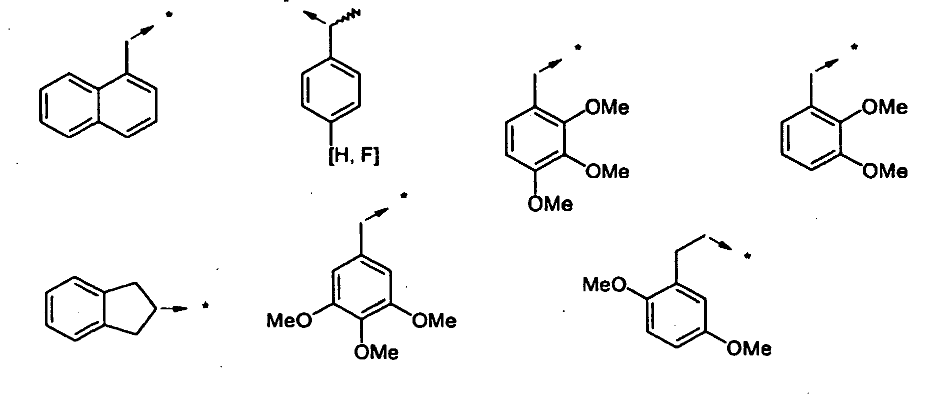

### A2b) Synthèse d'amides à partir d'acides carboxyliques

Les synthèses d'amides à partir d'acides carboxyliques sont mises en oeuvre selon le schéma réactionnel suivant : 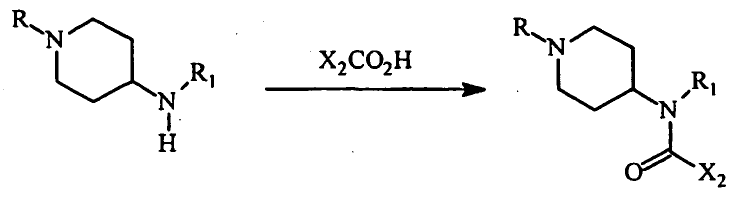 dans laquelle R représente méthyle ou Boc et X₂ a la signification indiquée ci-dessus. Il est à noter que dans le cas où R représente Boc, le produit ainsi obtenu est un produit final répondant à la formule I selon l'invention mais peut également être utilisé comme intermédiaire de synthèse.

La procédure générale est la suivante : l'acide carboxylique (1,1 à 2,5 éq.) dissout dans un solvant aprotique anhydre tel que le dichlorométhane, la diméthylformamide ou le tétrahydrofurane est activé avec du 1-éthyl-3-(3-diméthylaminopropyl) carbodiimide greffé sur résine (P-EDC, Novabiochem, 2,33 mmol/g, 1,3 à 3 éq.) (Desai, M.C. ; Stephens Stramiello, L.M. Tetrahedron Lett. 1993, 34, 7685-7688). Ce mélange est agité 5 à 30 minutes à température ambiante. La 4-aminosubstitué-1-pipéridine prédissoute dans un solvant aprotique anhydre tel que le dichlorométhane, la diméthylformamide ou le tétrahydrofurane est alors ajouté et le mélange réactionnel agité à température ambiante 1 à 18 heures. Dans certains cas, de la résine basique échangeuse d'ions (IRA-68, SAX) est ajoutée et le mélange à nouveau agité à température ambiante 1 à 18 heures. Les résines sont filtrées sur fritté ou sur cartouche de résine basique échangeuse d'ions (IRA-68, SAX) ou sur cartouche d'alumine basique (500 mg, Interchim).

### Exemple A2b : Carboxylate de tert-butyl 4-{(3,4-diméthoxyphénéthyl)[2-(1H-indol-3-yl)acétyl]amino}-1-pipéridine (C₃₅H₄₁N₃O₃, M = 551,74)

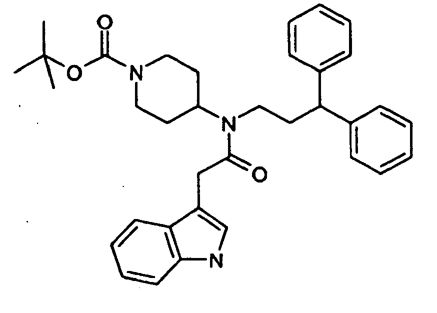

512 mg (1,12 mmol, 1,4 éq.) de résine P-EDC est prégonflée dans le dichlorométhane. L'acide 2-(1*H*-indol-3-yl)acétique (153 mg, 0,875 mmol, 1,1 éq.) est ajouté et le mélange agité 10 minutes. Le carboxylate de *tert*-butyl 4-[(3,3-diphénylpropyl)amino]-1-pipéridine (292 mg, 0,8 mmol) dans le tétrahydrofurane est ajouté et la réaction agitée toute la nuit. 2 spatules de résine basique échangeuse d'ions IRA-68 sont ajoutées et la réaction agitée à nouveau toute la nuit. Les résines sont filtrées et le filtrat est concentré sous vide pour donner 250 mg (rdt = 86 %) d'une mousse jaune pâle.
RMN H (CD₃OD, 400 MHz) δ : 7,63 (d, J = 8Hz, 1H) ; 7,44 (d, J = 8Hz, 1H) ; 7,36 (d, J = 8Hz, 1H) ; 7,26 (d, J = 8Hz, 1H) ; 7,2 (m, 6H) ; 7,13 (m, 3H) ; 7,1 (m, 2H) ; 6,68 (s, 1H); 4-3,75 (m, 4H) ; 3,65 (s, 1H) ; 3,2 (m, 1H) ; 3 (m, 1H) ; 2,75 (m, 1H) ; 2,26 (m, 3H) ; 1,6 (m, 2H) ; 1,44 (s, 9H) ; 1,13 (m, 2H). SM/CL: m/z = 552.4 (M+H).

Une série d'amides a été synthétisée selon cette procédure. Les radicaux X₂ que l'on peut envisager sont les suivants : 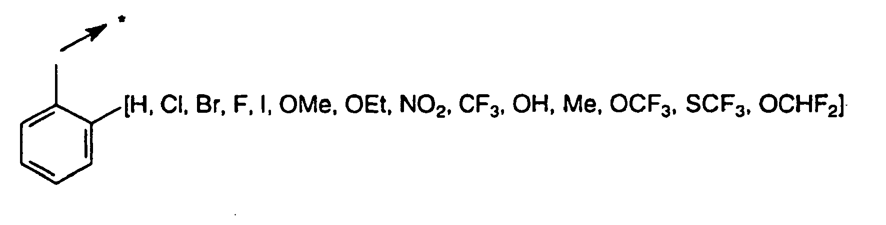 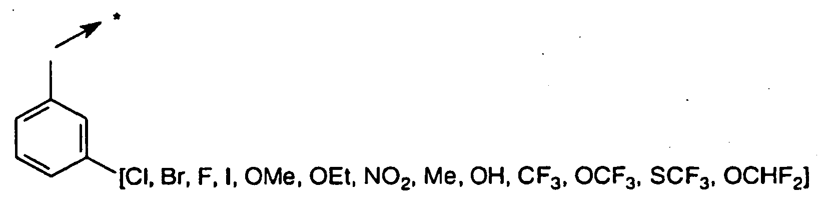 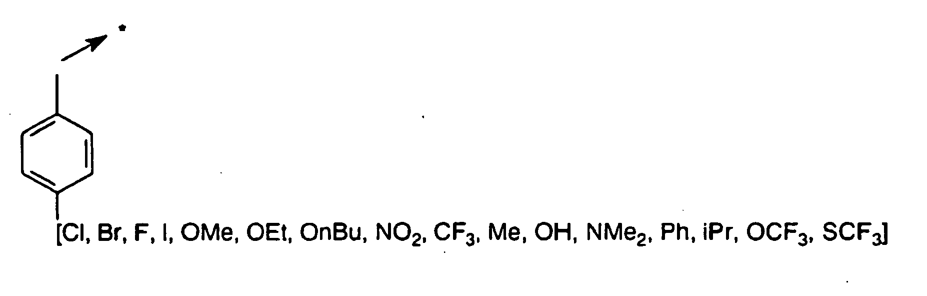 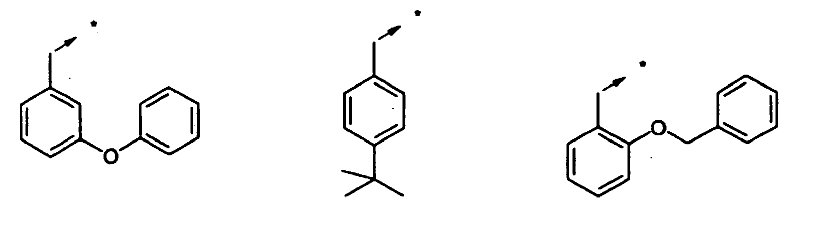 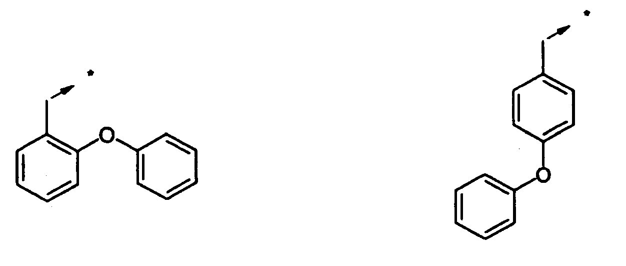 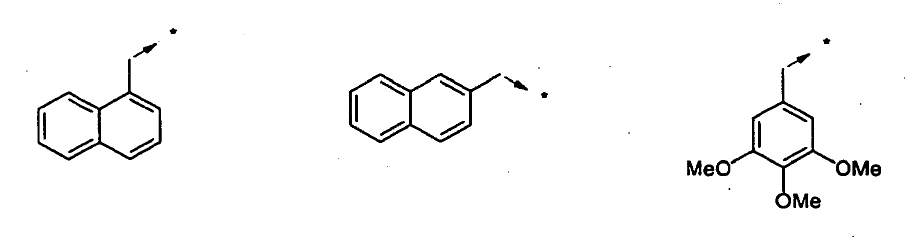 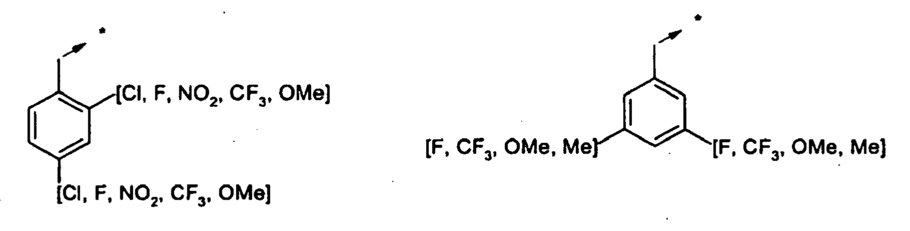 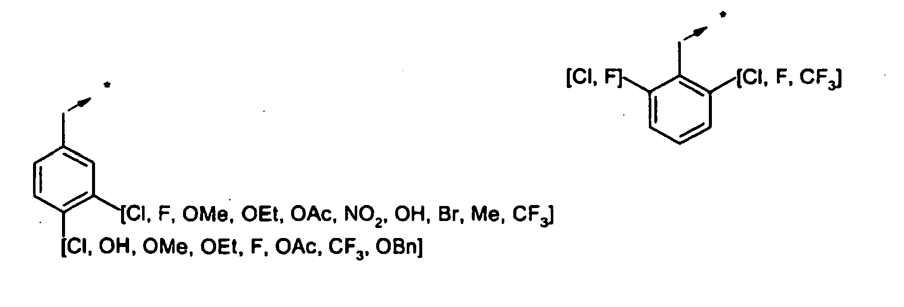 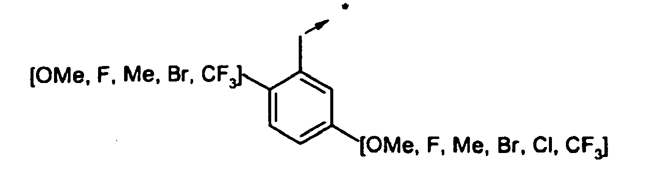 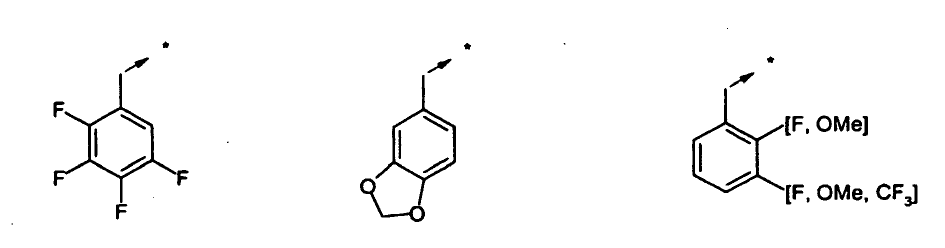 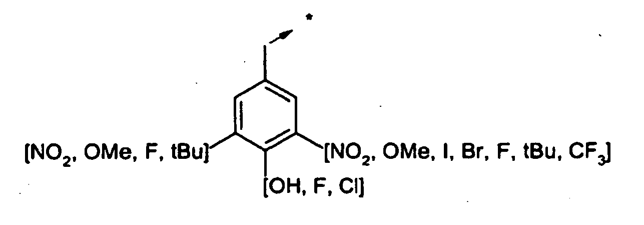 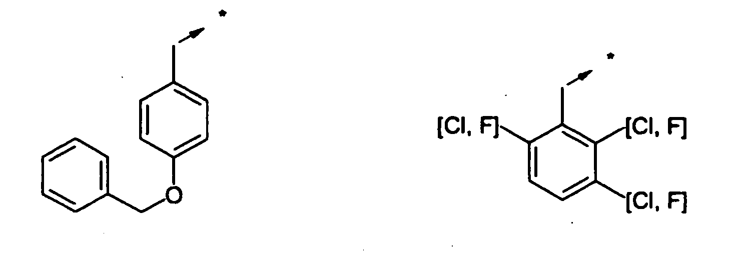 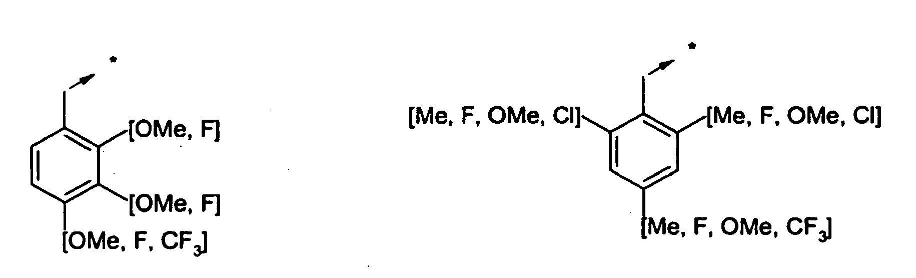 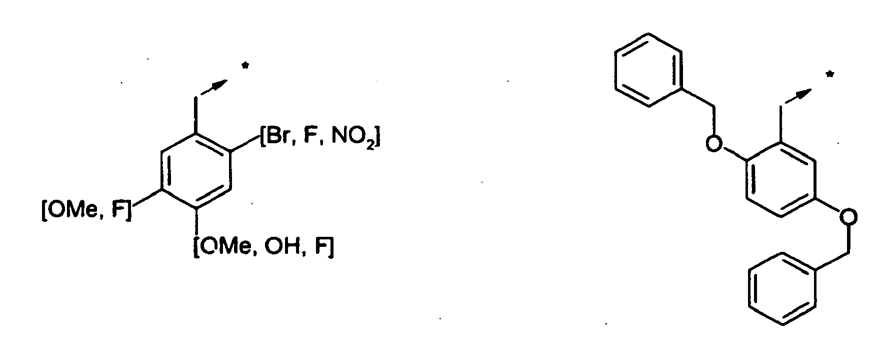 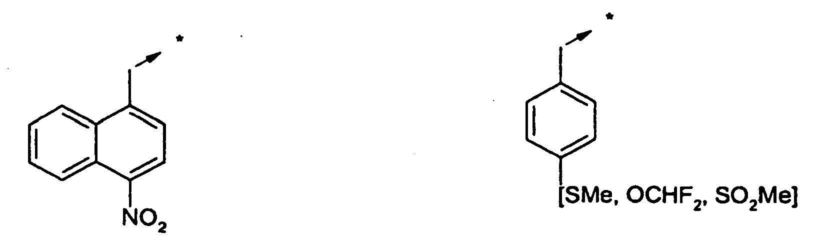 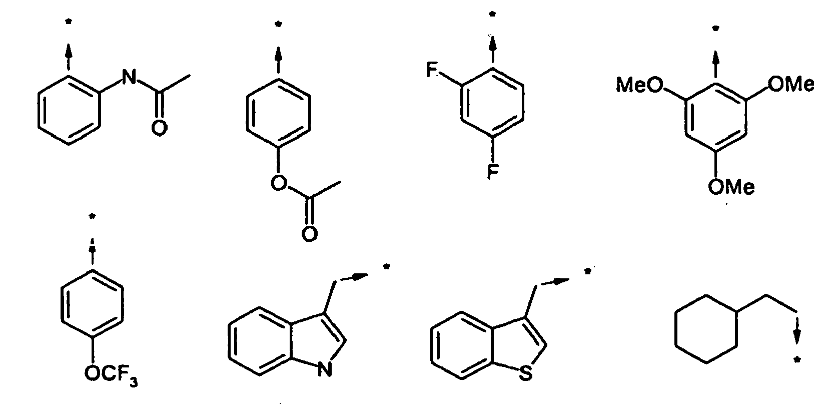 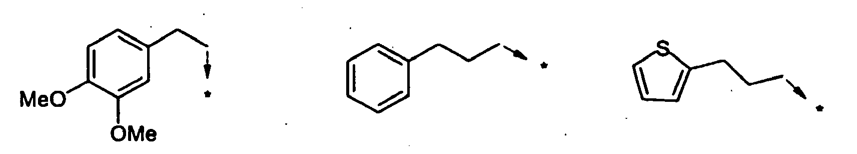 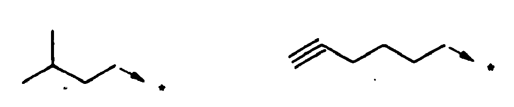 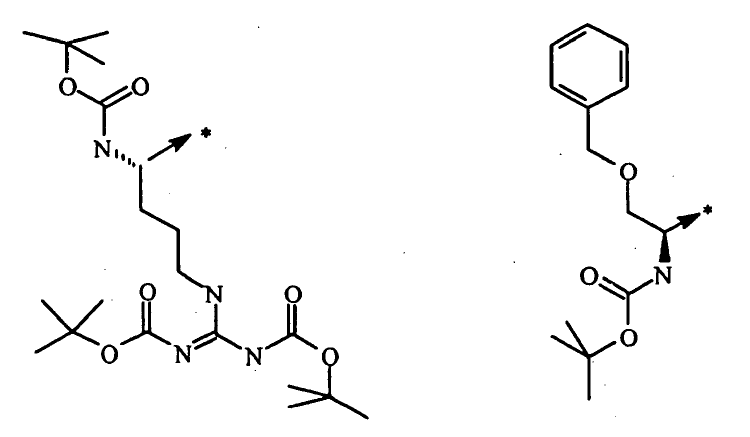 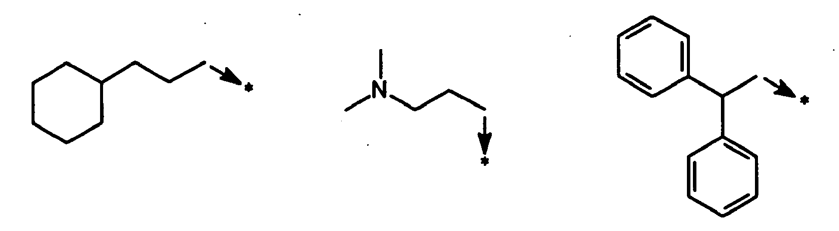 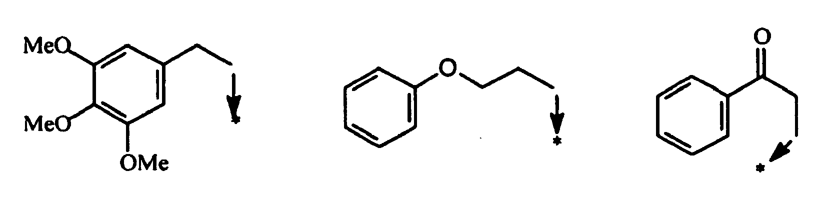 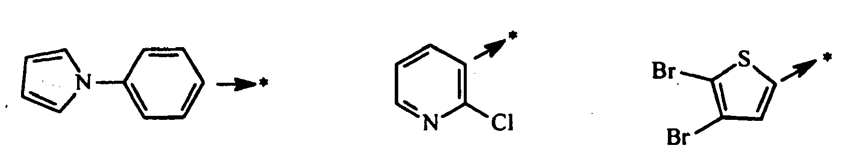 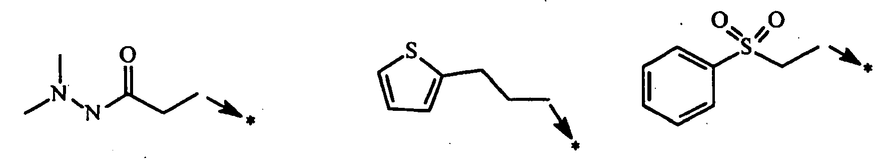 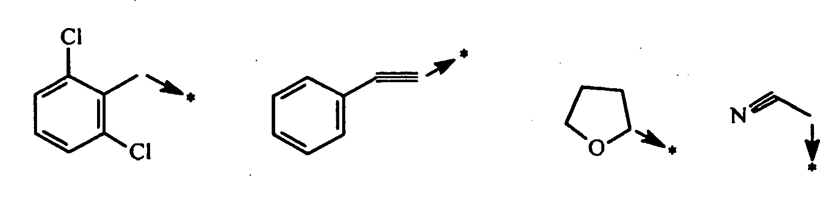 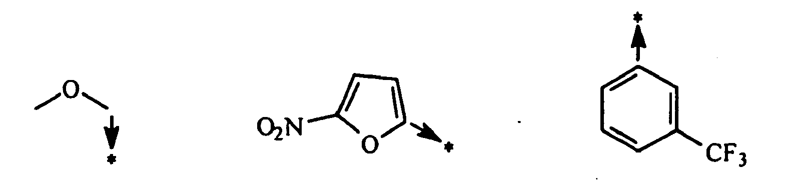 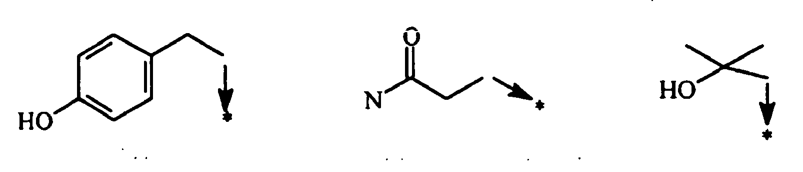 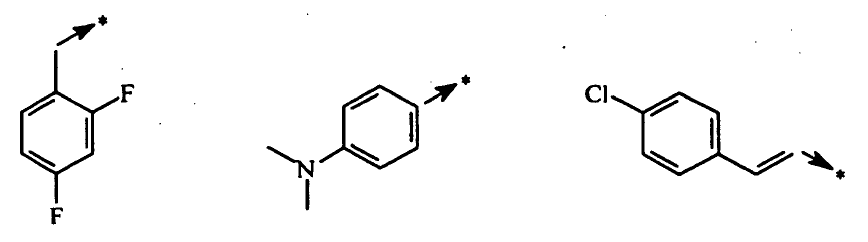 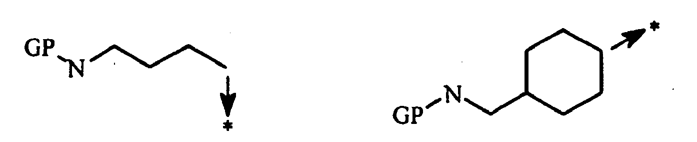 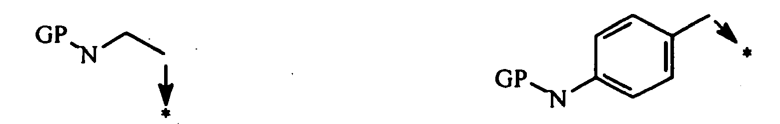 où le groupe protecteur (GP) représente H ou *tert*-butyloxycarbonyle.

### A3) Synthèses de pipéridines 4-aminodisubstituées

La synthèse des pipéridines 4-aminodisubstituées selon l'invention, peut s'effectuer par traitement acide des composés N-Boc précédemment décrits, suivant le schéma réactionnel suivant :

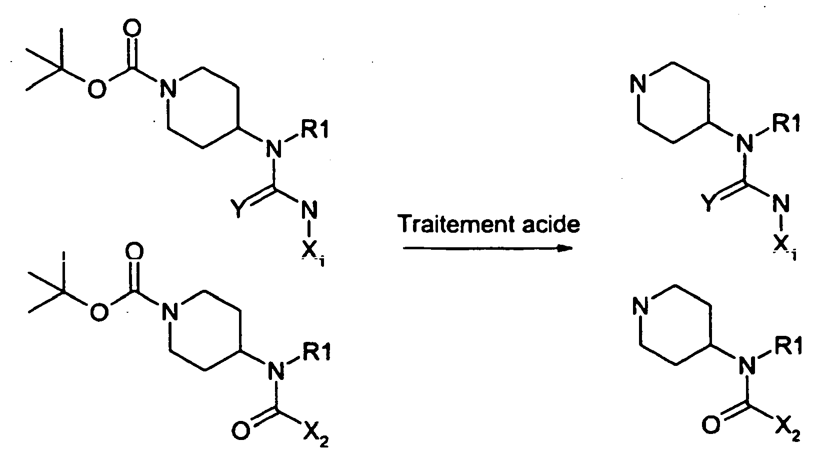

Procédure générale : deux méthodes ont été utilisées pour effectuer la déprotection en milieu acides des urées, thiourées et amides précédemment décrits. La première consiste à dissoudre le composé dans du dichlorométhane et additionner l'acide trifluoroacétique (5 à 20 éq.) tandis que dans la seconde une solution d'acide chlorhydrique dilué dans des solvants tels que l'acétate d'éthyle, le dioxane ou le diéthyléther (5 à 20 éq.) est utilisée. La réaction est agitée 1 à 4 heures à température ambiante. Dans certains cas, du dichlorométhane est ajouté et la phase organique est lavée avec une solution saturée de bicarbonate de sodium, séchée sur sulfate de magnésium, filtrée et concentrée sous vide pour isoler la base libre.

### Exemple A3 : Urée de N-(3,3-diphénylpropyl)-N-(4-pipéridinyl)-N'-[3-(trifluoro méthyl)phényl] (C₂₈H₃₀F₃N₃O, M = 481,57)

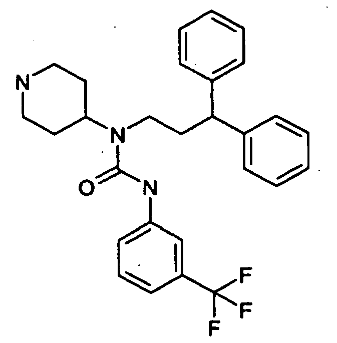

A une solution de carboxylate de *tert-*butyl 4-((3,3-diphénylpropyl) {[3-(trifluorométhyl)anilino]carbonyl}amino)-1-pipéridine (600 mg, 1,04 mmol) dans le dichlorométhane est ajouté 1,6 ml (21 mmol, 20 éq.) d'acide trifluoroacétique. La réaction est agitée 90 minutes puis concentrée. Du dichlorométhane est ajouté et la phase organique est lavée avec une solution saturée de bicarbonate de sodium, séchée sur sulfate de magnésium, filtrée et concentrée sous vide pour isoler 490 mg (rdt = 98 %) d'une mousse blanche.

RMN ¹H (CD₃OD, 400 MHz) δ : 7,7 (s, 1H); 7,55 (d, 1H); 7,44 (t, 1H); 7,28 (m, 9H) ; 7,18 (m, 2H) ; 4,05 (m, 2H); 3,26 (m, 2H) ; 3,11 (d, J = 10,8Hz, 2H) ; 2,7 (td, J = 2,4 et 12,4Hz, 2H) ; 2,38 (q, J = 8Hz, 2H) ; 1,76 (d, J = 10Hz, 2H) ; 1,63 (qd, J = 4 et 12,4Hz, 2H). SM/CL : m/z = 482,2 (M+H).

Une série de 4-aminopipéridines a été synthétisée selon cette procédure. Les radicaux R₁, X₁ et X₂ que l'on peut envisager sont ceux déjà illustrés aux points A1 et A2 ci-dessus.

### B) Synthèse en phase solide de 4-aminopipéridines

Des 4-aminopipéridines ont été préparées par synthèse en phase solide en partant de la résine de Wang.

### B1) Préparation de la résine

### B1a) Préparation de la résine carbonate de p-nitrophényle de Wang

Elle s'effectue selon le schéma suivant

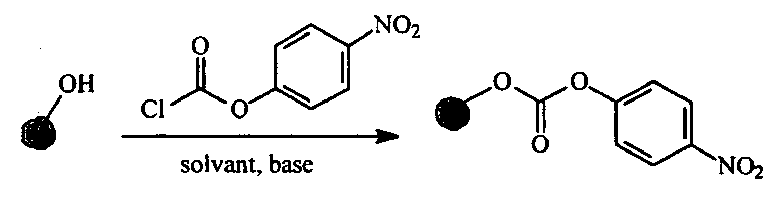

Cette résine a été préparée à partir de la résine de Wang (fournie par Bachem ou Novabiochem) avec des taux de charge supérieurs à 0,89 mmol/g, suivant la procédure décrite dans la littérature (Bunin, B.A. The Combinatorial Index, Academic Press, 1998, p. 62-63; Dressman, B.A. ; Spangle, L.A.; Kaldor, S.W. Tetrahedron Lett. 1996, 37, 937-940 ; Hauske, J.R. ; Dorff, P. Tetrahedron Lett. 1995, 36, 1589-1592; Cao, J. ; Cuny, G.D. ; Hauske, J.R. Molecular Diversity 1998, 3, 173-179) : de la N-méthylmorpholine ou de la pyridine et du chloroformiate de 4-nitrophényle sont successivement additionnés à la résine de Wang prégonflée dans du dichlorométhane ou du tétrahydrofurane à température ambiante. Le mélange est agité toute la nuit. La résine est lavée avec du tétrahydrofurane, du diéthyléther et du dichlorométhane puis séchée *in vacuo* à 50° C toute la nuit.

### B1b) Préparation de la résine carbamate de pipéridone

Elle s'effectue selon le schéma suivant

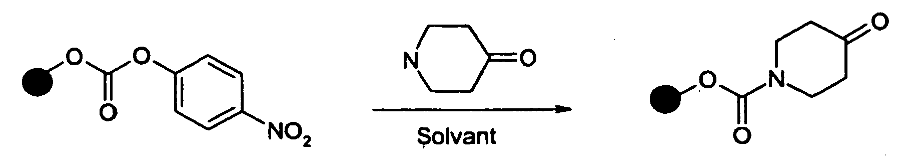

Au chlorhydrate hydraté de pipéridone dilué dans de la diméthylformamide est ajoutée de la triéthylamine (1 éq.) et du tamis moléculaire. Le mélange est chauffé jusqu'à dissolution complète de la cétone. Cette solution est ajoutée à la résine carbonate de *p*-nitrophényle de Wang (0,05 éq.) prégonflée dans la diméthylformamide. Après 24 à 72 heures d'agitation à température ambiante, la résine est filtrée puis lavée plusieurs fois avec de la diméthylformamide, du tétrahydrofurane, du diéthyléther et du dichlorométhane.

### Préparation 2

2,5 g de la résine carbonate de *p*-nitrophényle de Wang (taux de charge de 0,88 mmol/g, 2,2 mmol) est prégonflée dans 100 ml de diméthylformamide. Dans le même temps, 6,7 g (44 mmol, 20 éq.) de chlorhydrate hydraté de pipéridone, 4,45 g (44 mmol, 20 éq.) de triéthylamine et trois spatules de tamis moléculaire sont chauffés dans 100 ml de diméthylformamide jusqu'à dissolution complète. La solution jaunâtre est versée chaude sur la résine et le mélange agité 40 heures à température ambiante. La résine est filtrée puis lavée avec de la diméthylformamide, du tétrahydrofurane, du diéthyléther et du dichlorométhane (3 fois chaque solvant) puis séchée sous vide. 2,4 g de résine jaune pâle sont isolés avec un taux de charge de 0,88 mmol/g calculé d'après l'analyse élémentaire de l'azote.

### B2) Amination réductrice sur support solide

Elle s'effectue selon le schéma suivant :

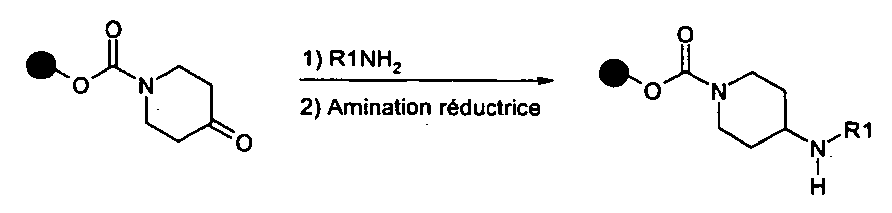

La procédure générale est la suivante : à la résine cétonique prégonflée dans le triméthylorthoformiate (TMOF) est ajoutée l'amine primaire (5 à 10 éq.) puis le mélange est soniqué. On ajoute ensuite le complexe de borane pyridine (8 M, 5 à 10 éq.) et le mélange est agité 12 à 72 heures. La résine est filtrée, lavée avec des solvants tels que le dichlorométhane, la diméthylformamide et le tétrahydrofurane puis séchée sous vide (Pelter, A.; Rosser, R.M. J. Chem. Soc. Perkin Trans I 1984, 717-720 ; Bomann, M.D.; Guch, LC.; DiMare, M. J. Org. Chem. 1995, 60, 5995-5996 ; Khan, N.M.; Arumugam, V.; Balasubramanian, S. Tetrahedron Lett. 1996, 37, 4819-4822).

### Préparation 3

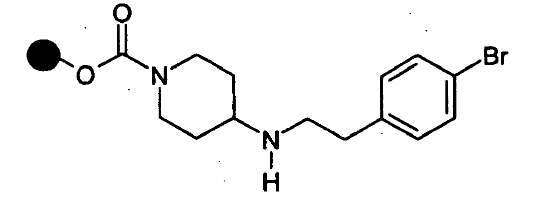

300 mg (taux de charge de 0,88 mmol/g, 0,27 mmol) de résine cétonique sont prégonflés dans le TMOF. On ajoute ensuite la 4-bromophénéthylamine (540 mg, 420 µl, 2,7 mmol, 10 éq.) puis le complexe de borane pyridine (8 M, 338 µl, 2,7 mmol, 10 éq.). Le mélange est agité 56 heures à température ambiante. La résine est filtrée, rincée successivement avec du dichlorométhane, du diméthylformamide, du tétrahydrofurane et du dichlorométhane puis séchée sous vide. 340 mg de résine jaune pâle sont ainsi obtenus avec un taux de charge de 0,81 mmol/g calculé d'après l'analyse élémentaire de l'azote.

### B3) Fonctionnalisation

### B3a) Fonctionnalisation avec des isocyanates ou des isothiocyanates

Elle s'effectue selon le schéma

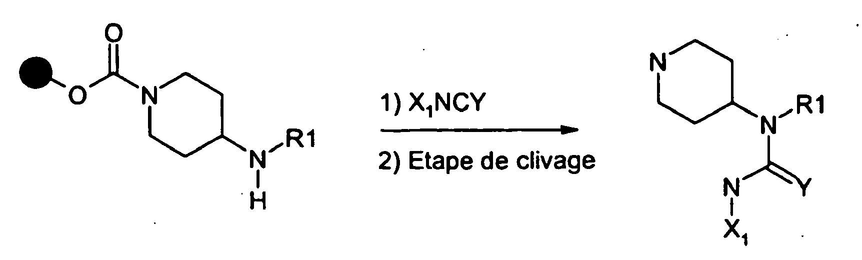

La procédure générale est la suivante : la résine "amine secondaire" est prégonflée dans un solvant tel que le dichlorométhane ou le diméthylformamide avant l'addition d'isocyanate ou d'isothiocyanate (3 à 10 éq.). Le mélange est agité 1 à 24 heures à température ambiante. La résine est alors filtrée, lavée avec des solvants tels que le dichlorométhane, le diméthylformamide et le tétrahydrofurane puis séchée sous vide. Le clivage de la résine s'effectue en présence d'un mélange équimolaire de dichlorométhane et d'acide trifluoroacétique et une agitation de 30 minutes à 4 heures. La résine est rincée au dichlorométhane puis le filtrat est concentré sous vide. Dans certains cas le filtrat est redissout dans du dichlorométhane puis désalifié avec une solution saturée de carbonate de sodium. La phase organique est évaporée sous vide pour donner la base libre.

### Exemple B3a : Urée de N-(4-bromophénéthyl)-N-(4-pipéridinyl)-N'-[4-(trifluorométhyl) phényl] (C₂₁H₂₃BrF₃N₃O, M = 470,3)

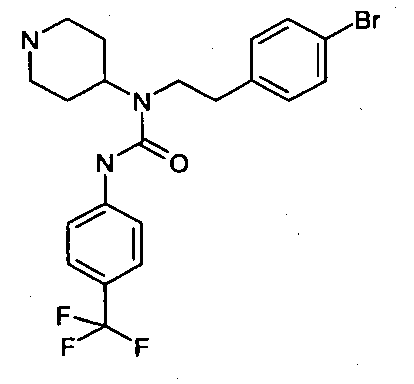

55 mg (50 µmol) de la résine (voir préparation 3) sont prégonflés dans du dichlorométhane anhydre. On ajoute ensuite le 4-trifluorophénylisocyanate (28 mg, 150 µmol, 3 éq.) et le tout est agité toute la nuit. La résine est filtrée, rincée au tétrahydrofurane, au diméthylformamide, au tétrahydrofurane puis au dichlorométhane avant d'être séchée sous vide. On agite ensuite 1,5 heure en présence de 800 µl d'un mélange équimolaire de dichlorométhane et d'acide trifluoroacétique. La résine est filtrée et rincée au dichlorométhane, le filtrat est concentré, redilué dans du dichlorométhane et lavé avec une solution saturée de bicarbonate de sodium. 6 mg d'une huile brune (rdt = 25 %) sont ainsi isolés.

RMN ¹H (CD₃OD, 400 MHz) δ: 7,53 (m, 4H); 7,44 (d, J = 6,8Hz, 2H); 7,21 (d, J = 8,4Hz, 2H); 4,1 (m, 1H); 3,53 (t, J = 7,2Hz, 2H); 3,12 (d, J = 12,8Hz, 2H); 2,89 (t, J = 8Hz, 2H); 2,7 (m, 2H); 1,73 (m, 4H). SM/CL : m/z = 472,2 (M+H).

Une série d'urées (Y = O) et de thiourées (Y = S) a été synthétisée selon cette procédure. Les radicaux R₁ que l'on peut envisager sont les suivants :

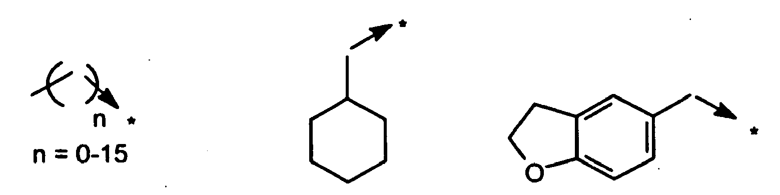

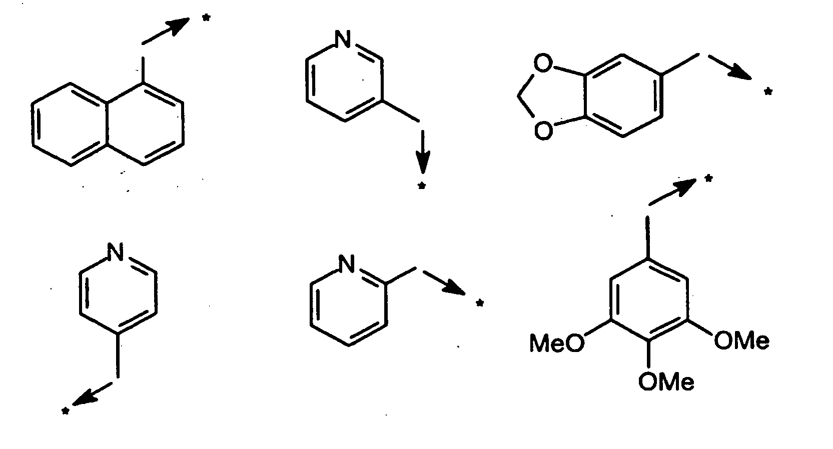

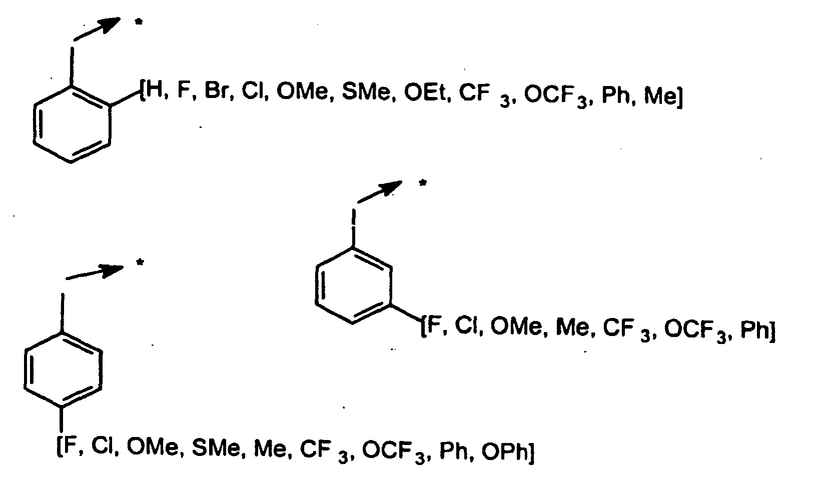

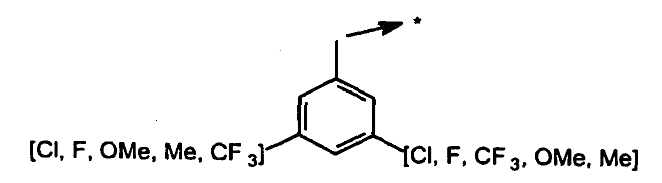

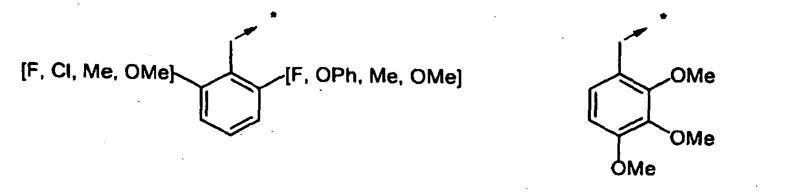

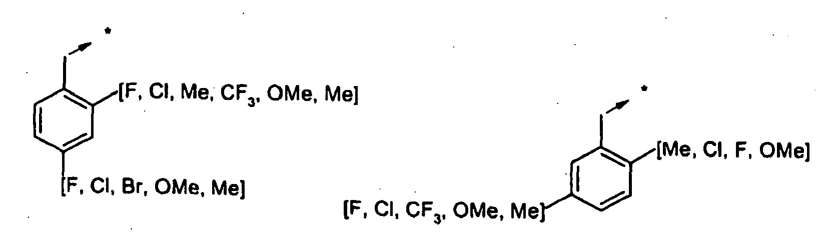

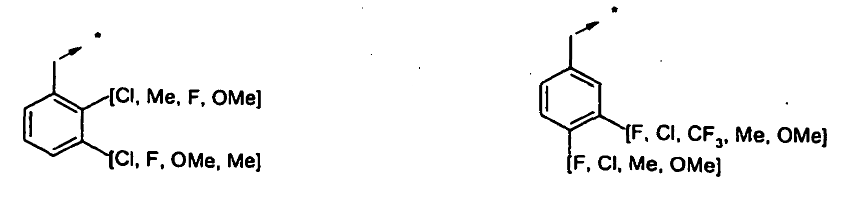

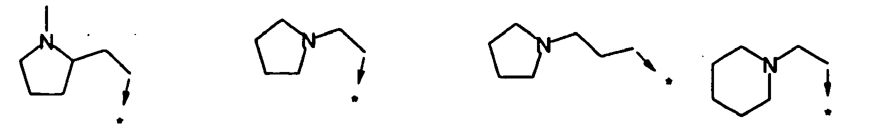

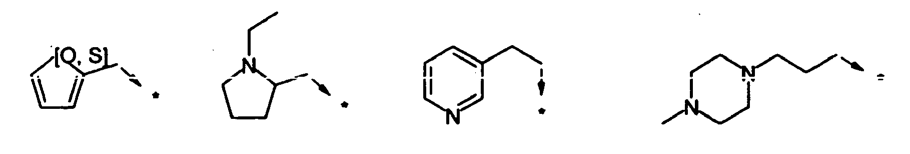

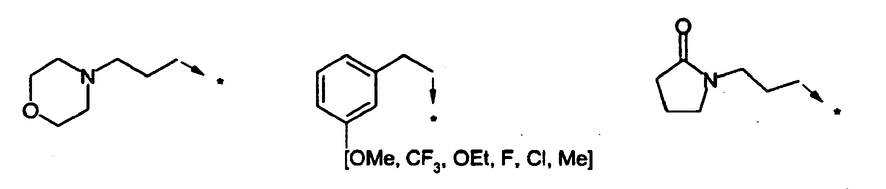

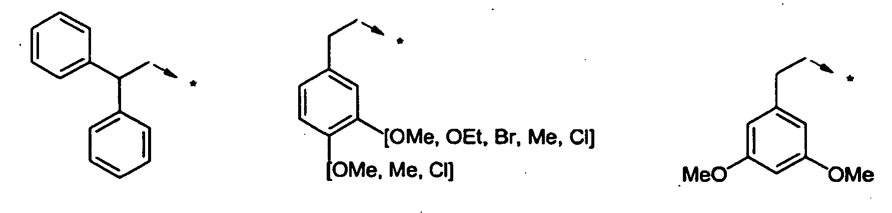

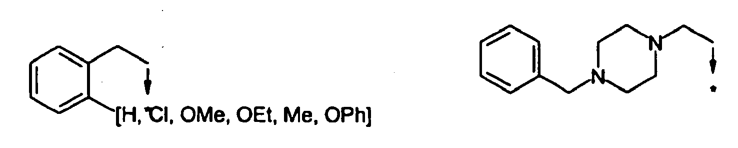

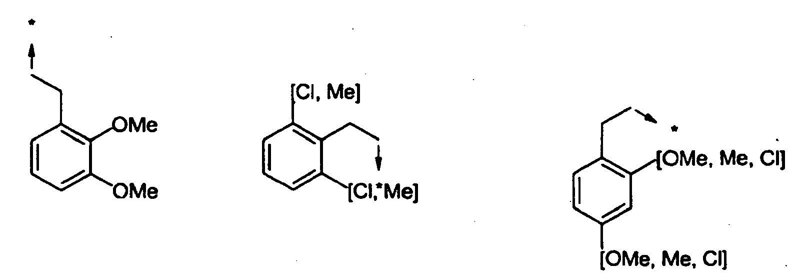

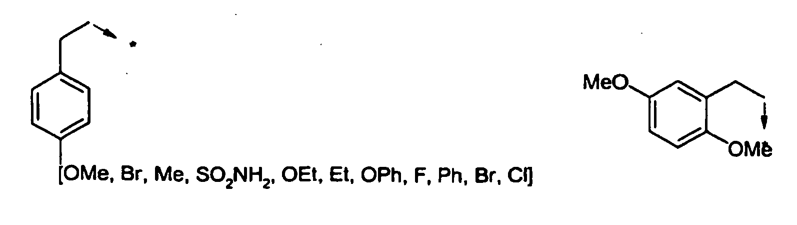

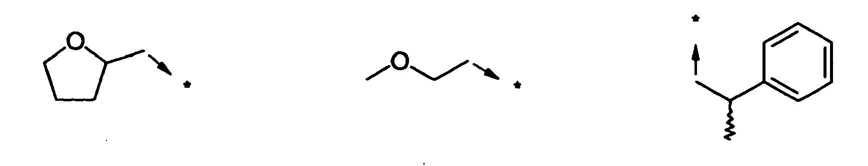

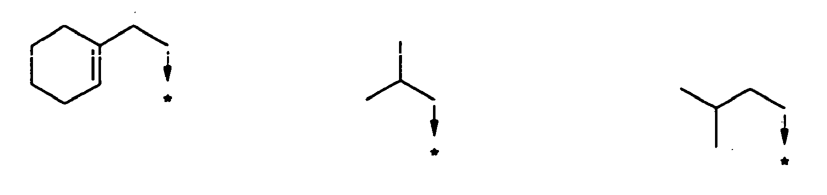

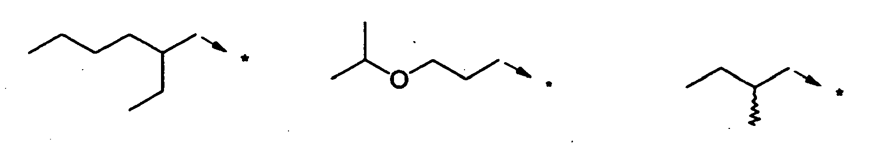

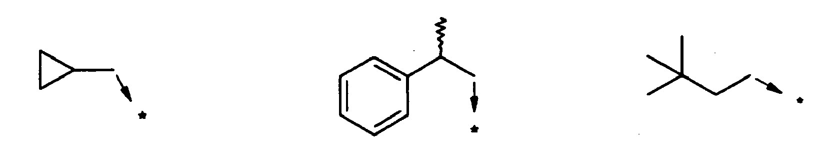

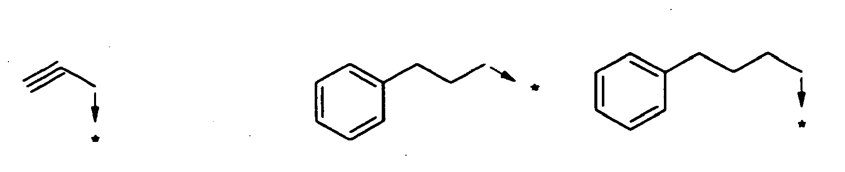

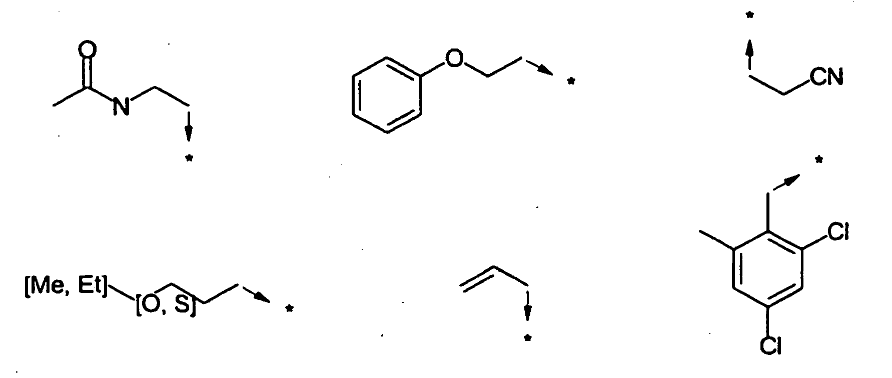

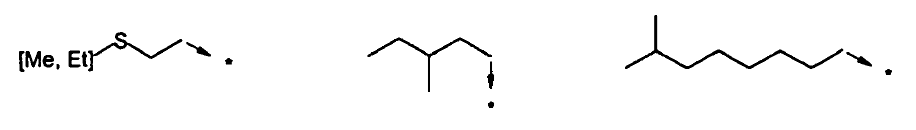

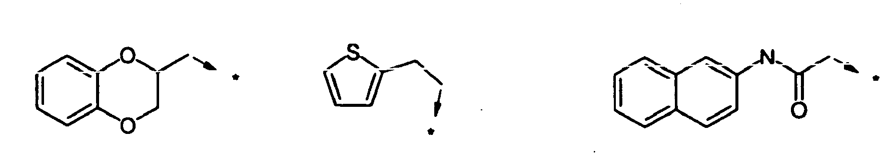

Les radicaux X₁ que l'on peut envisager sont ceux illustrés au point A ci-dessus.

### B3b) Fonctionnalisation avec des chlorures de sulfonyles

Elle s'effectue selon le schéma suivant

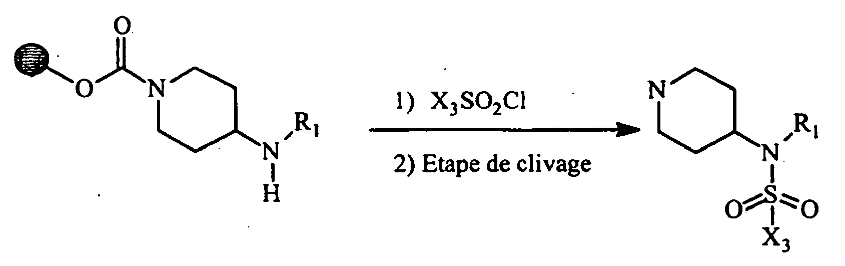

Procédure générale : la résine "amine secondaire" est prégonflée dans des solvants comme le dichlorométhane, le diméthylformamide ou le tétrahydrofurane. Puis le chlorure de sulfonyle (5 à 10 éq.) et la triéthylamine (6 à 12 éq.) sont ajoutés et le mélange agité de 12 à 24 heures à température ambiante. La résine est filtrée, lavée avec des solvants tels que le dichlorométhane, le diméthylformamide et le tétrahydrofurane, puis séchée sous vide. On agite ensuite la résine de 1 à 4 heures en présence d'un mélange équimolaire de dichlorométhane et d'acide trifluoroacétique. La résine est rincée au dichlorométhane puis le filtrat est concentré sous vide. Dans certains cas le filtrat est redissout dans du dichlorométhane puis désalifié avec une solution saturée de carbonate de sodium. La phase organique est évaporée sous vide pour donner la base libre.

### Exemple B3b : Sulfonamide de N-(4-bromophénéthyl)-4-méthoxy-N-(4-pipéridinyl) phényle (C₂₀H₂₅BrN₂O₃S, M = 453,4)

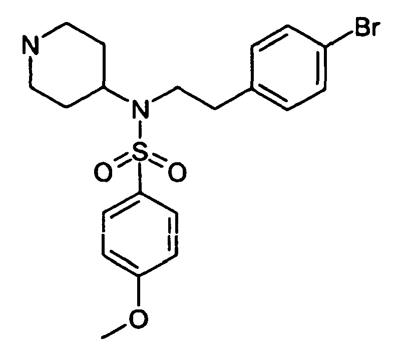

55 mg (50 µmol) de résine (voir préparation 3) sont prégonflés dans du dichlorométhane anhydre. On ajoute ensuite la triéthylamine (42 µl, 300 µmol, 6 éq.) puis le chlorure de 4-méthoxybenzène sulfonyle (51,5 mg, 250 µmol, 5 éq.) et le tout est agité toute la nuit. La résine est filtrée, rincée au tétrahydrofurane, au diméthylformamide, au tétrahydrofurane puis au dichlorométhane avant d'être séchée sous vide. On répète la réaction une seconde fois pour avoir une substitution complète. On ajoute 800 µl d'un mélange équimolaire de dichlorométhane et d'acide trifluoroacétique et on agite 1,5 heure à température ambiante. La résine est filtrée et rincée au dichlorométhane. Le filtrat est concentré, redilué dans du dichlorométhane et lavé avec une solution saturée de bicarbonate de sodium. 14 mg d'une huile brune (rdt = 63 %) ont ainsi été isolés.

RMN ¹H (CD₃OD, 400 MHz) δ : 7,8 (dd, J = 2,8 et 10Hz, 2H); 7,44 (dd, J = 1,2 et 6,8Hz, 2H); 7,17 (d, J = 8,4Hz, 2H); 7,07 (dd, J = 3,2 et 10Hz, 2H); 3,87 (s, 3H, OC*H*₃); 3,72 (m, 1H); 3,3 (m, 2H); 3,04 (d, J = 12,8Hz, 2H); 2,92 (t, J = 8,4Hz, 2H); 2,6 (t, J = 12,4Hz, 2H); 1,58 (m, 2H); 1,47 (d large, J = 10Hz, 2H). SM/CL : m/z = 455 (M+H).

Une série de sulfonamides a été synthétisée selon cette procédure. Les radicaux R₁ que l'on peut envisager sont ceux illustrés aux points A et B3a ci-dessus. Les radicaux X₃ que l'on peut envisager sont les suivants :

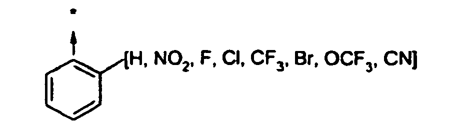

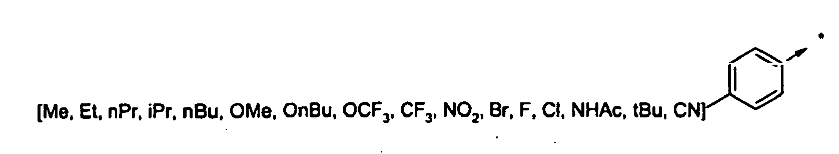

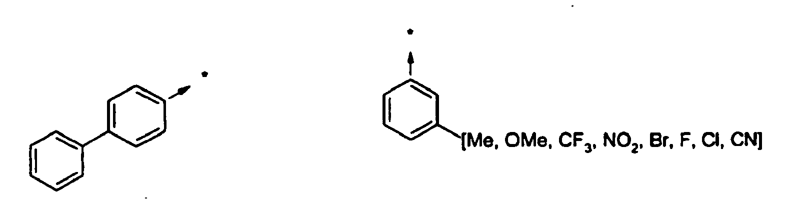

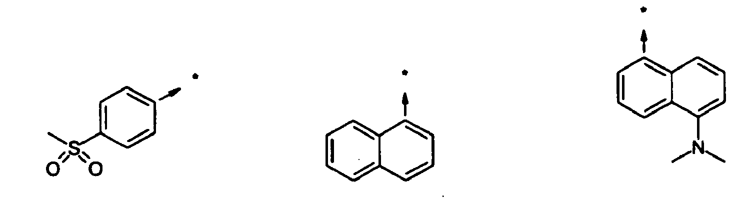

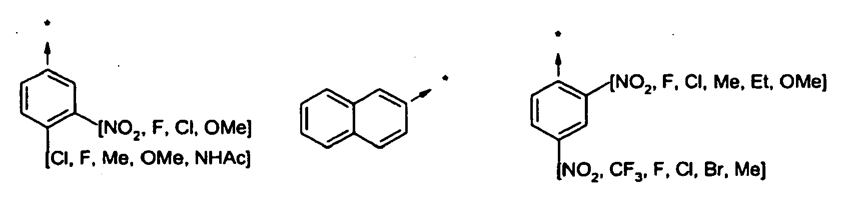

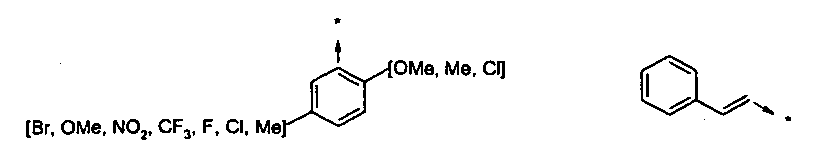

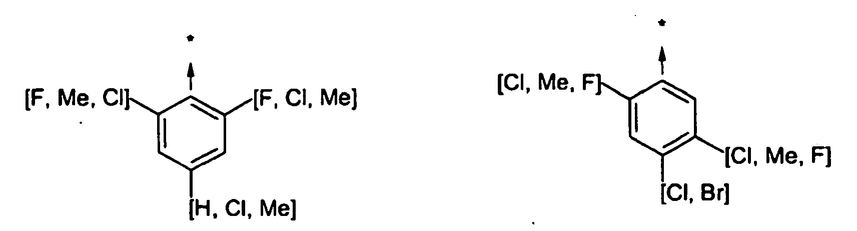

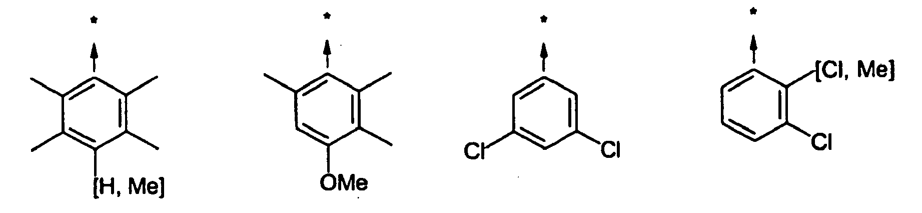

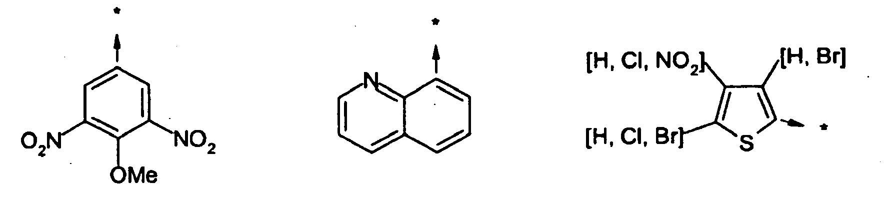

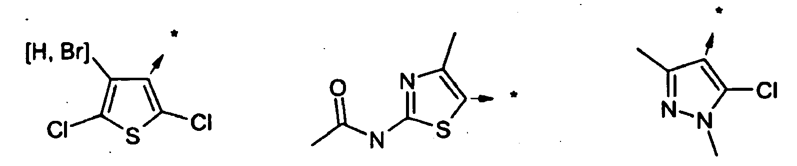

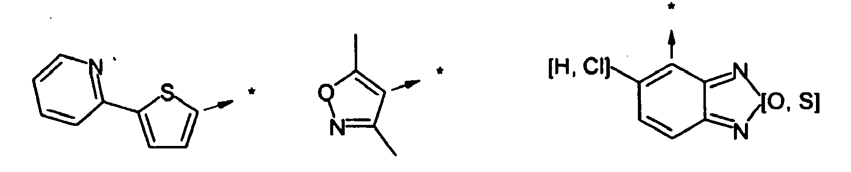

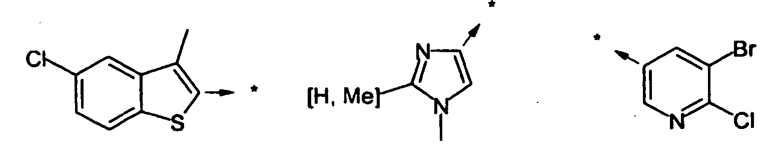

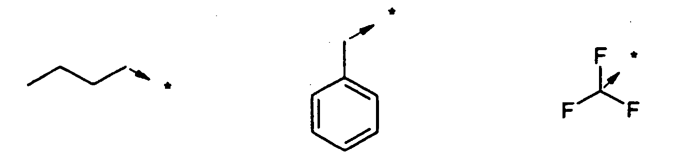

### B3c) Fonctionnalisation avec des chlorures d'acides

Elle s'effectue selon le schéma suivant

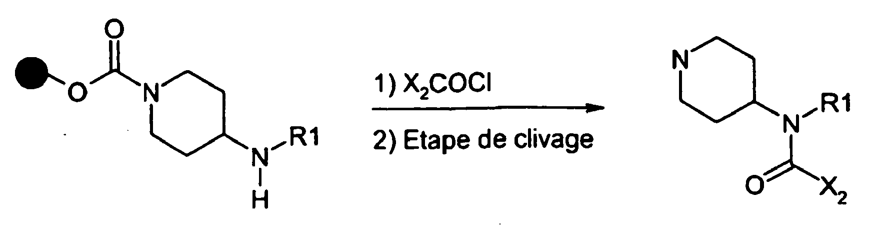

Procédure générale : la résine "amine secondaire" est prégonflée dans des solvants comme le dichlorométhane, le diméthylformamide ou le tétrahydrofurane. Puis le chlorure d'acide (5 à 10 éq.) et la triéthylamine (6 à 12 éq.) sont ajoutés et le mélange agité de 12 à 24 heures à température ambiante. La résine est filtrée, lavée avec des solvants tels que le dichloromethane, le diméthylformamide et le tétrahydrofurane, puis séchée sous vide. On agite ensuite la résine de 1 à 4 heures en présence d'un mélange équimolaire de dichlorométhane et d'acide trifluoroacétique. La résine est rincée au dichlorométhane puis le filtrat est concentré sous vide. Dans certains cas le filtrat est redissout dans du dichlorométhane puis désalifié avec une solution saturée de carbonate de sodium. La phase organique est évaporée sous vide pour donner la base libre.

### Exemple B3c : Carboxamide de N-(4-bromophénéthyl)-N-(4-pipéridinyl)-2-thiophène (C₁₈H₂₁BrN₂OS, M = 393,3)

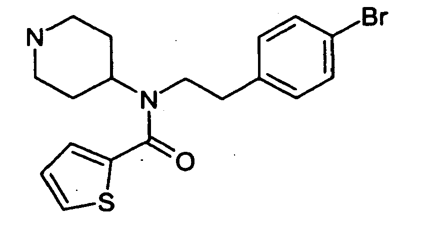

55 mg (50 µmol) de la résine (voir préparation 3) sont prégonflés dans du tétrahydrofurane anhydre. On ajoute ensuite la triéthylamine (42 µl, 300 µmol, 6 éq.) puis le chlorure de 2-thiophène carbonyle (37 mg, 250 µmol, 5 éq.) et le tout est agité toute la nuit. La résine est filtrée, rincée au tétrahydrofurane, à la diméthylformamide, au tétrahydrofurane puis au dichlorométhane avant d'être séchée sous vide. On ajoute 800 µl d'un mélange équimolaire de dichlorométhane et d'acide trifluoroacétique et on agite 1,5 heure à température ambiante. La résine est filtrée et rincée au dichlorométhane. Le filtrat est concentré, redilué dans du dichlorométhane et lavé avec une solution saturée de bicarbonate de sodium. pour obtenir 10 mg d'une huile brune (rdt = 50 %).

RMN ¹H (CD₃OD, 400 MHz) δ : 7,64 (dd, J = 0,8 et 4,8Hz, 1H); 7,44 (d, J = 8,4Hz, 2H); 7,36 (d, J = 3,6Hz, 1H); 7,14 (m, 3H); 4,11 (m, 1H); 3,61 (t, J = 8Hz, 2H); 3,09 (d, J = 12Hz, 2H); 2,92 (m, 2H); 2,54 (m, 2H); 1,82 (m, 2H); 1,7 (m, 2H). SM/CL : m/z = 393,1 (M+H).

Une série d'amides a été synthétisée selon cette procédure. Les groupes R₁ envisagés sont ceux illustrés aux points A et B3a ci-dessus. Les groupes X₂ sont illustrés ci-dessous.

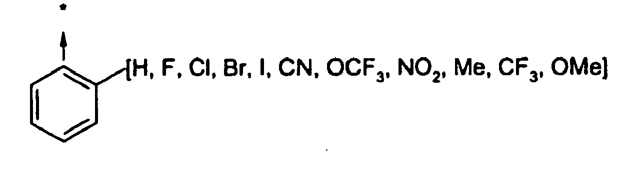

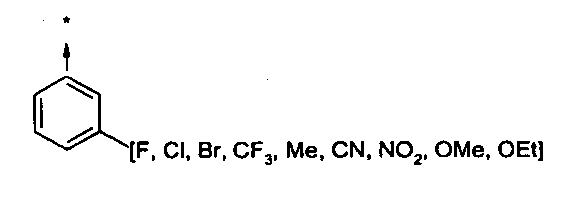

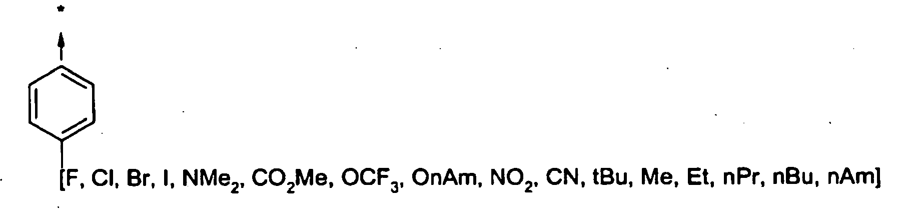

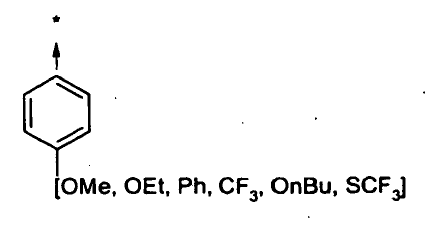

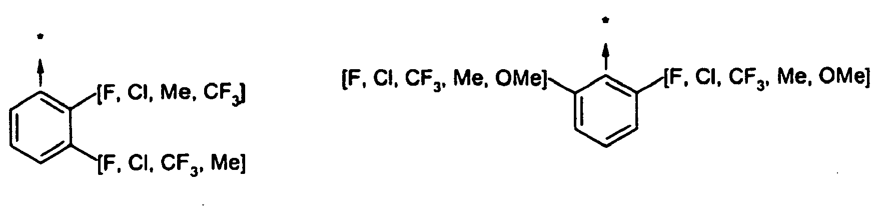

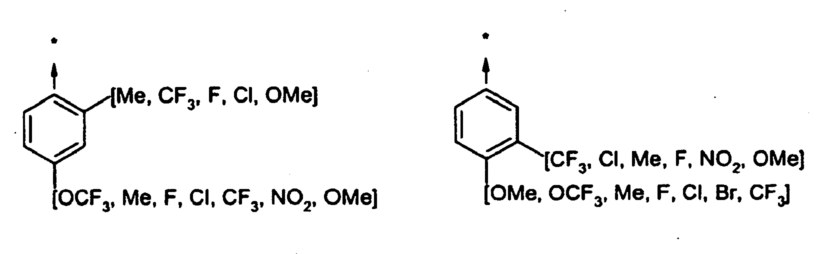

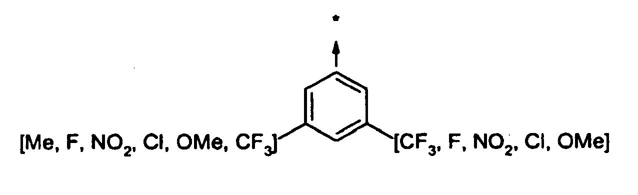

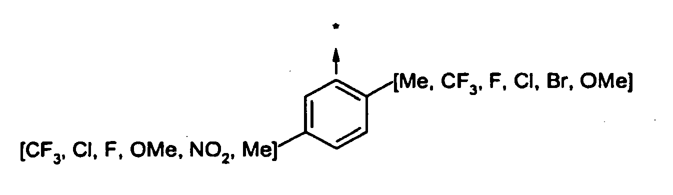

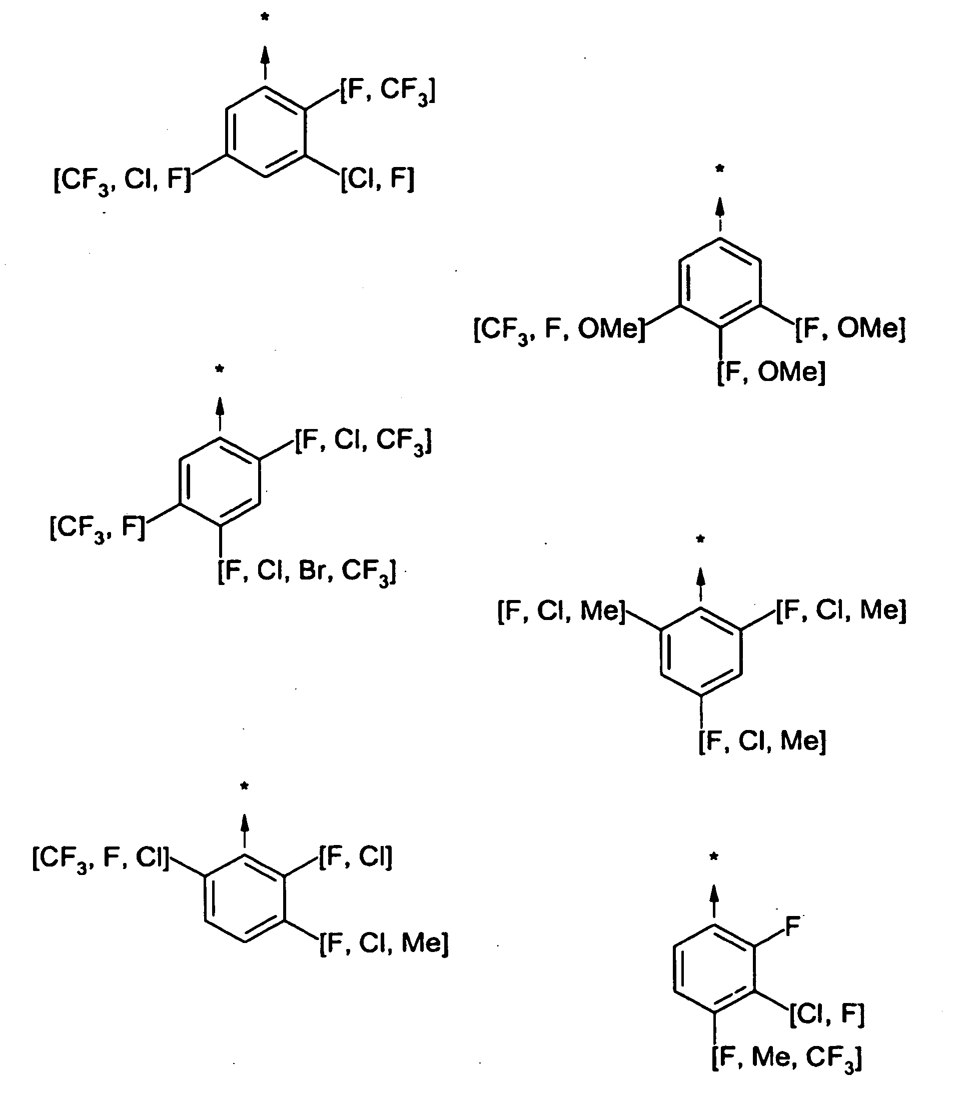

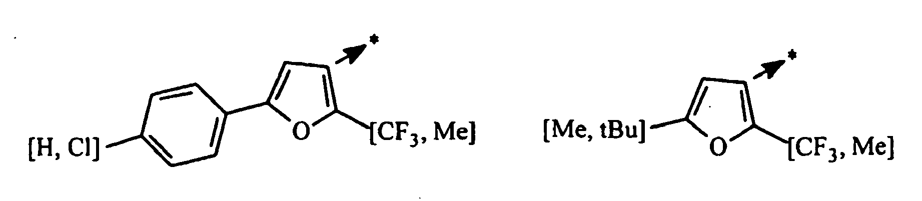

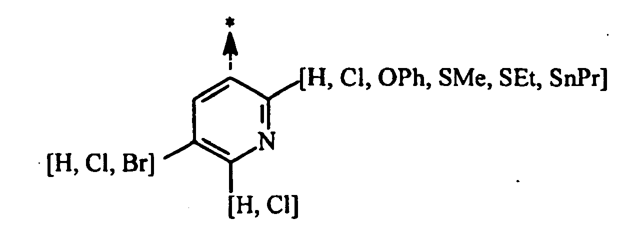

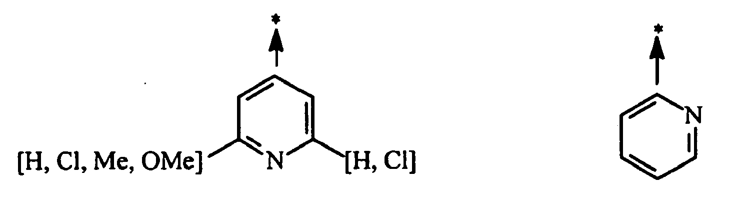

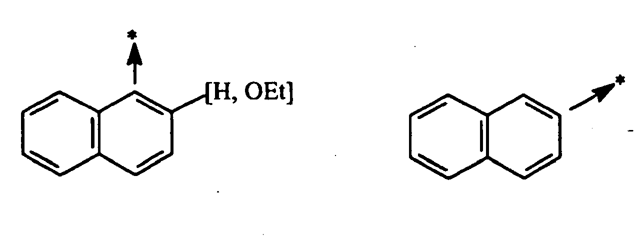

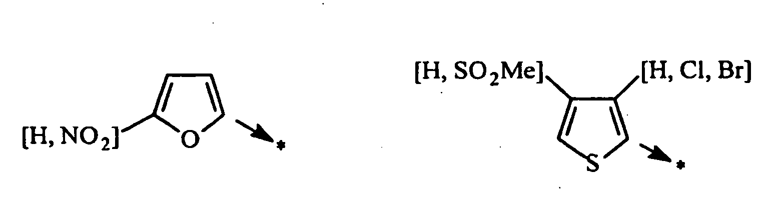

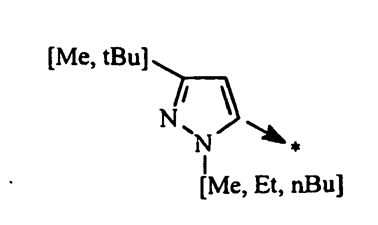

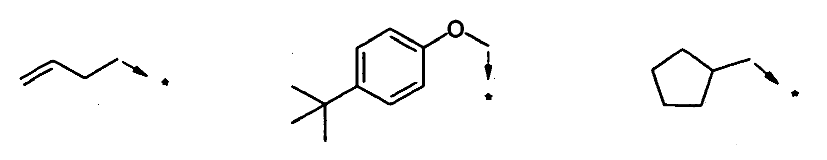

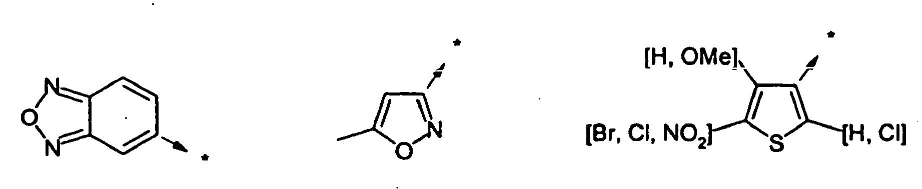

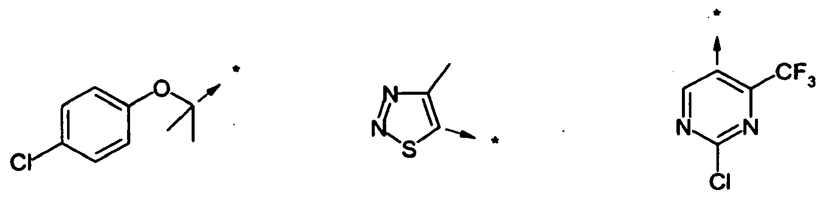

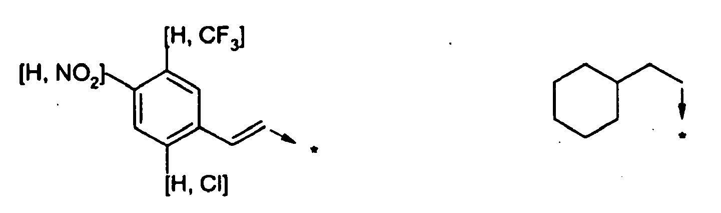

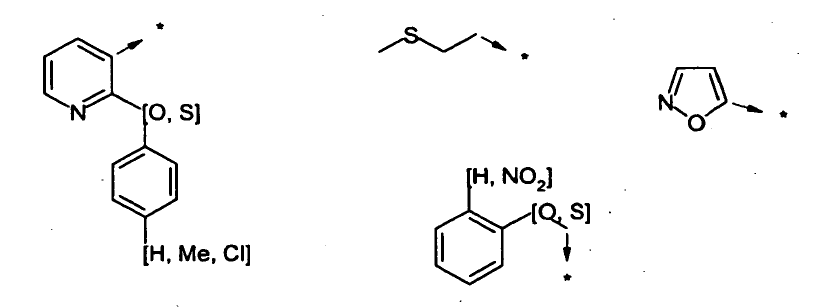

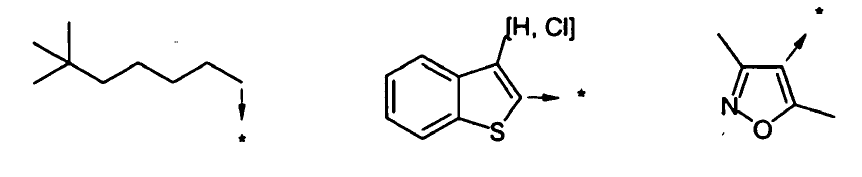

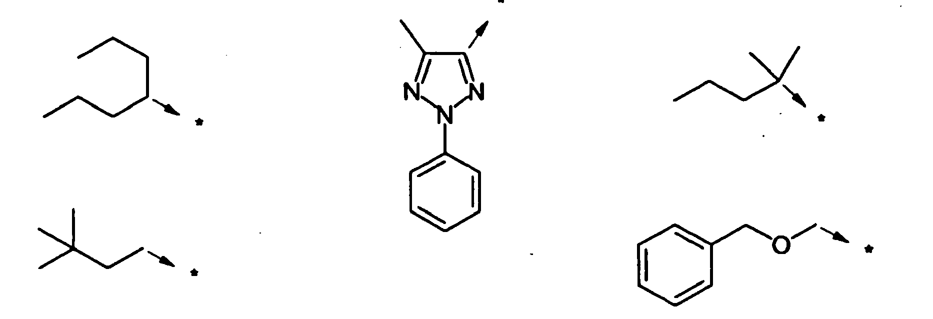

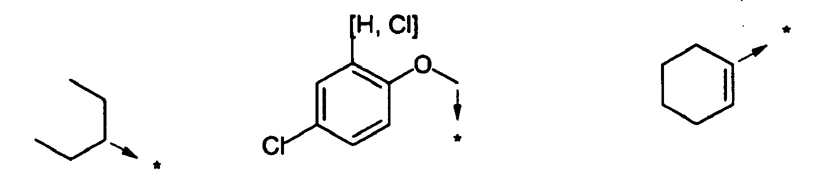

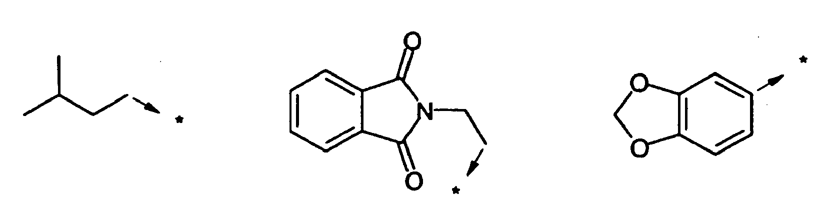

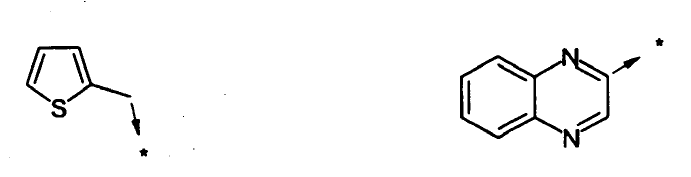

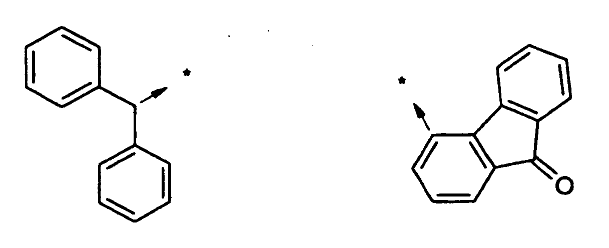

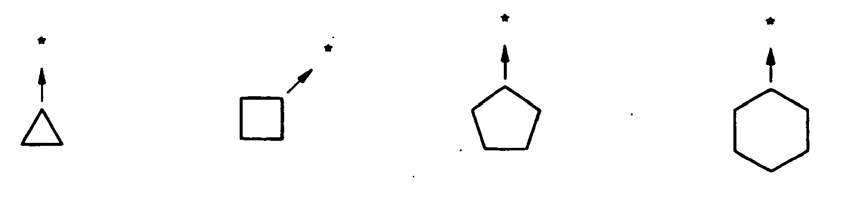

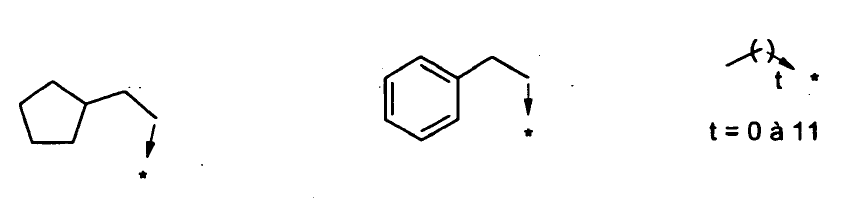

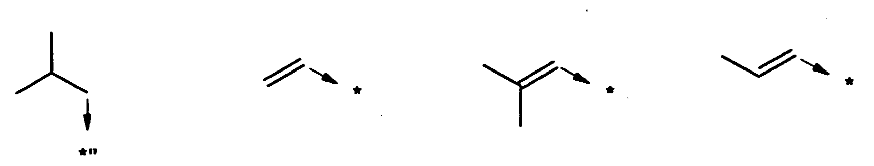

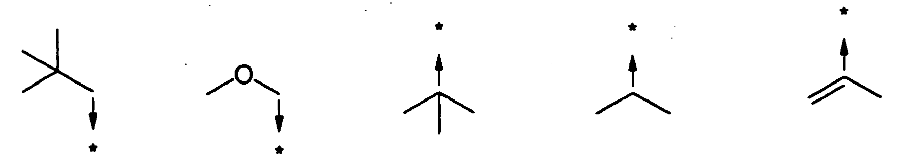

### B3d) Fonctionnalisation avec des acides carboxyliques

Elle s'effectue selon la procédure décrite dans la littérature (Kobayashi, S ; Aoki, Y., J. Comb. Chem. 1999,1,371-372) suivant le schéma suivant :

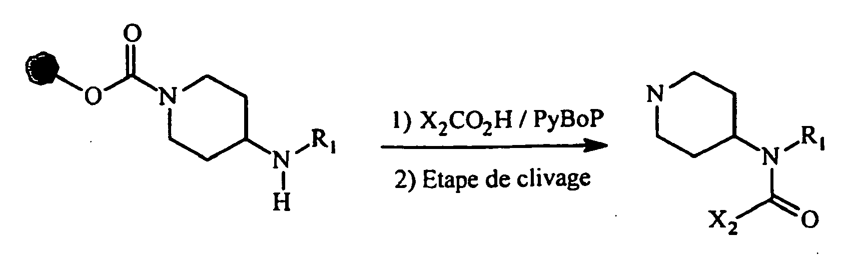

Procédure générale : la résine "amine secondaire" est prégonflée dans des solvants comme le dichlorométhane, le diméthylformamide ou le tétrahydrofurane. Puis l'acide carboxylique (3 à 5 éq.), l'hexafluorophosphate de benzo-triazol-1-yl-oxy-*tris*-pyrrolidino phosphonium (PyBoP, 3 à 5 éq.) et la diisopropyléthylamine (6 à 10 éq.) sont ajoutés et le mélange agité 24 heures à température ambiante. La résine est filtrée, lavée avec des solvants tels que le dichlorométhane, le diméthylformamide et le tétrahydrofurane, puis séchée sous vide. On agite ensuite la résine de 1 à 4 heures en présence d'un mélange équimolaire de dichlorométhane et d'acide trifluoroacétique. La résine est rincée au dichlorométhane puis le filtrat est concentré sous vide. Dans certains cas le filtrat est redissout dans du dichlorométhane puis désalifié avec une solution saturée de carbonate de sodium. La phase organique est évaporée sous vide pour donner la base libre.

### Exemple B3d : Acétamide de N-[2-(4-bromophényl)éthyl]-N-(4-pipéridinyl) (C₁₅H₂₁BrN₂O, M = 325,25)

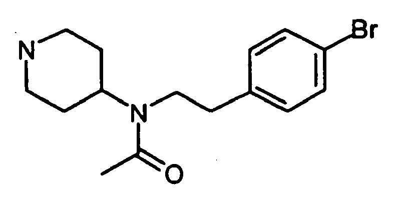

55 mg (50 µmol) de la résine (voir préparation 3) sont prégonflés dans de la diméthylformamide anhydre. On ajoute ensuite l'acide acétique (8,8 mg, 150 µmol, 3 éq.), le PyBoP (76 mg, 150 µmol, 3 éq.) puis la diisopropyléthylamine (38 mg, 300 µmol, 6 éq.) et le tout est agité toute la nuit. La résine est filtrée, rincée au diméthylformamide, au tétrahydrofurane puis au dichlorométhane avant d'être séchée sous vide. On ajoute 800 µl d'un mélange équimolaire de dichlorométhane et d'acide trifluoroacétique et on agite 1,5 heure à température ambiante. La résine est filtrée et rincée au dichlorométhane. Le filtrat est concentré, redilué dans du dichlorométhane et lavé avec une solution saturée de bicarbonate de sodium pour obtenir 11 mg d'une huile brune (rdt = 68 %).

RMN ¹H (CD₃OD, 400 MHz) δ : 7,44 (m, 2H); 7,20 (m, 2H); 4,05 (m,1H); 3,45 (m, 2H); 3,10 (m, 2H); 2,83 (m, 2H); 2,64 (m, 2H); 2,13 (s,3H) ; 1,73 (m, 4H). SM/CL: m/z = 325,2 (M+H).

Une série d'amides a été synthétisée selon cette procédure. Les groupes R₁ envisagés sont ceux illustrés aux points A et B3a ci-dessus. Les groupes X₂ sont illustrés au point A ci-dessus.

### C) Fonctionnalisation de la partie pipéridine en solution

### C1) Obtention de pipéridine avec R₃= -C(Y)NHX₁

Elle s'effectue selon le schéma

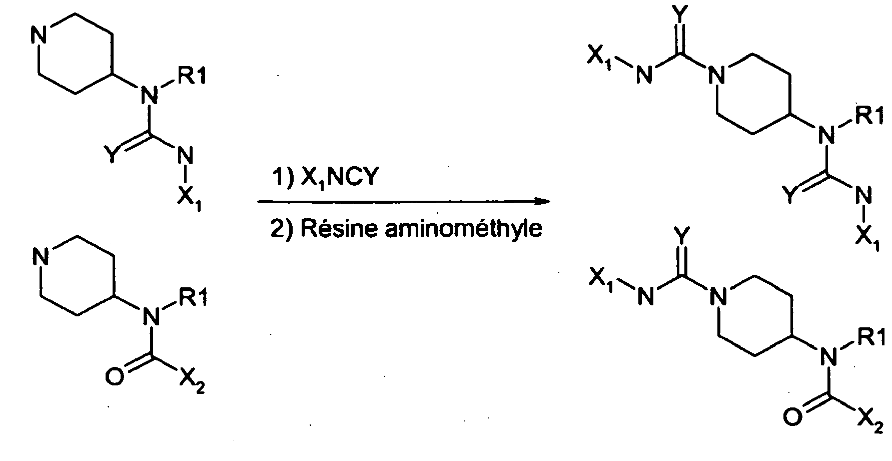

Procédure générale : à la pipéridine sous forme de base libre diluée dans du dichlorométhane, est ajouté un isocyanate ou isothiocyanate (1,1 à 1,5 éq.). Le mélange est agité d'une à 18 heures à température ambiante. La résine aminométhyle (0,2 à 1 éq.) est ajoutée et le mélange à nouveau agité de 2 à 18 heures. Dans certains cas, de la résine échangeuse d'ions telle que IRA68 ou SAX est ajoutée. Les résines sont filtrées et le filtrat concentré. Dans certains cas, le produit est dissout dans du dichlorométhane ou de l'acétate d'éthyle puis filtré sur une catouche de gel de silice ou d'alumine basique (500 mg, Interchim).

### Exemple C1 : Carboxamide de 4-((3,3-diphénylpropyl){[3-(trifluorométhyl)anilino] carbonyl}amino)-N-phényl-1-pipéridine (C₃₅H₃₅F₃N₄O₂, M = 600,68)

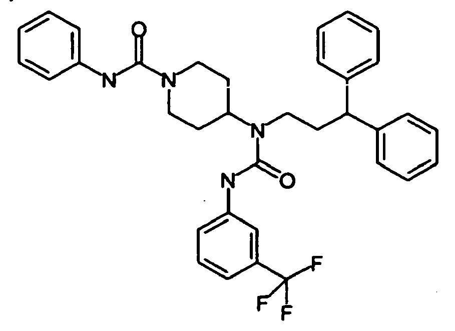

L'urée de N-(3,3-diphénylpropyl)-N-(4-pipéridinyl)-N'-[3-(trifluorométhyl)phényl] (24 mg, 0,05 mmol) est dissoute dans du dichlorométhane. Le phénylisocyanate (9 mg, 0,075 mmol, 1,5 éq.) est ajouté et le mélange agité pendant 2,5 heures. La résine aminométhyle (0,02 mmol) est ajoutée et la réaction à nouveau agitée toute la nuit. La résine est filtrée, rincée avec du dichlorométhane et le filtrat concentré. L'huile obtenue est passée sur une cartouche de gel de silice en éluant avec un mélange équimolaire d'heptane et d'acétate d'éthyle pour obtenir après concentration 12 mg (rdt = 40 %) d'une huile jaune.

RMN ¹H (CD₃OD, 400 MHz) δ : 7,72 (s, 1H) ; 7,58 (d, 1H) ; 7,44 (m, 1H) ; 7,38 (m, 2H) ; 7,29 (m, 12H) ; 7,12 (m, 2H) ; 7,07 (m, 1H) ; 4,2 (d, J = 12,4Hz, 3H) ; 3,21 (t, J = 8Hz, 2H) ; 2,9 (t, J = 12,4Hz, 2H) ; 2,38 (q, J = 8Hz, 2H) ; 1,73 (d, J = 10Hz, 2H) ; 1,54 (qd, J = 3,6 et 12Hz, 2H). SM/CL : m/z = 601,4 (M+H).

Une série d'urées (Y = O) et de thiourées (Y = S) ont été synthétisées selon cette procédure. Les groupes R₁, X₁ et X₂ que l'on peut envisager, sont ceux illustrés aux points ci-dessus (A et B3a), A, et (A et B3c) respectivement.

### C2) Fonctionnalisation avec des acides carboxyliques

Elle s'effectue selon le schéma suivant

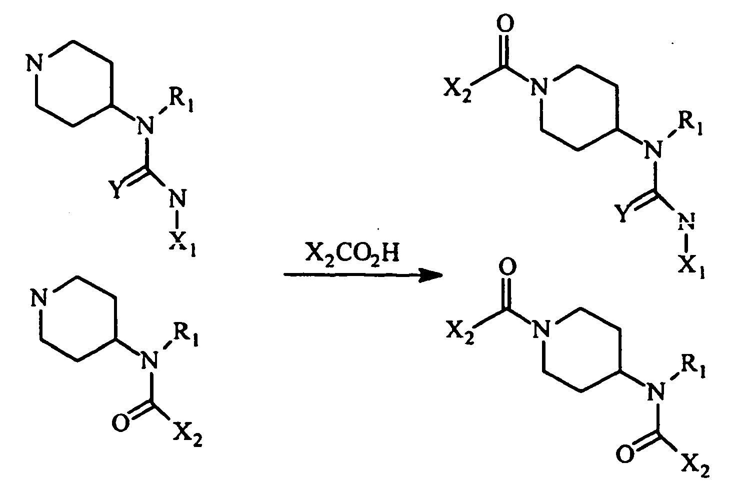

Procédure générale : la résine P-EDC (1,3 à 3 éq.) est prégonflée dans du dichlorométhane anhydre. L'acide carboxylique (1,1 à 2,5 éq.) est dissout dans un solvant anhydre tel que le dichlorométhane, la diméthylformamide ou le tétrahydrofurane et ajouté à la résine. Ce mélange est agité de 5 à 30 minutes à température ambiante. On ajoute alors à ce mélange la pipéridine 4-aminodisubstituée, sous forme de base libre, en solution dans un solvant anhydre tel que le dichlorométhane, le diméthylformamide ou le tétrahydrofurane et on agite le tout à température ambiante de 1 à 18 heures. Dans certains cas, de la résine échangeuse d'ions telle que IRA68 ou SAX est ajoutée et le mélange agité à nouveau à température ambiante de 1 à 18 heures. Les résines sont filtrées sur fritté, sur cartouche de résine échangeuse d'ions SAX (500 mg, Interchim) ou sur cartouche d'alumine basique (500 mg, Interchim).

### Exemple C2 : Urée de N-(1-acétyl-4-pipéridinyl)-N-(3,3-diphénylpropyl)-N'-[3-(trifluorométhyl) phényl] (C₃₀H₃₂F₃N₃O₂, M = 523,60)

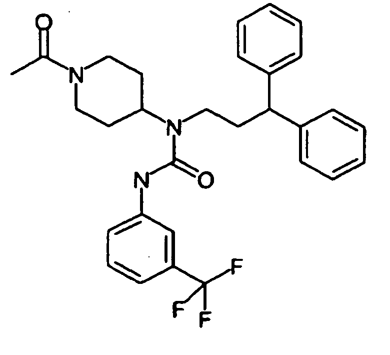

117 mg (175 µmol, 3,5 éq.) de résine P-EDC est prégonflée dans 1,5 ml de dichlorométhane anhydre. L'acide acétique (7,5 mg, 125 µmol, 2,5 éq.) est ajouté et le mélange agité 10 minutes. Puis l'urée de N-(3,3-diphénylpropyl)-N-(4-pipéridinyl)-N'-[3-(trifluorométhyl)phényl] (24,3 mg, 50 µmol) est ajoutée à son tour et le mélange agité toute la nuit. La résine est filtrée et le filtrat concentré. L'huile obtenue est passée sur une cartouche de gel de silice en éluant avec un mélange équimolaire d'heptane et d'acétate d'éthyle pour obtenir après concentration 16 mg (rdt = 62 %) d'une mousse blanche.

RMN ¹H (CD₃OD, 400 MHz) δ : 7,71 (s, 1H) ; 7,58 (d, J = 8,4Hz, 1H) ; 7,43 (t, J = 8Hz, 1H) ; 7,28 (m, 9H) ; 7,17 (m, 2H) ; 4,56 (dd, J = 2 et 11,2Hz, 1H) ; 4,17 (m, 1H) ; 3,96 (t, J = 7,6Hz, 1H) ; 3,88 (d, J = 12Hz, 1H) ; 3,19 (q, J = 4 et 8Hz, 2H) ; 3,1 (t, J = 12Hz, 1H) ; 2,58 (t, J = 12Hz, 1H); 2,37 (m, 2H); 2,06 (s, 3H, C*H₃*) ; 1,72 (t, J = 14,4Hz, 2H) ; 1,43 (qd, J = 4 et 12,4Hz, 2H). SM/CL : m/z = 524,3 (M+H).

Une série d'amides a été synthétisée selon cette procédure. Les groupes R₁, X₁ et X₂ que l'on peut envisager, sont ceux illustrés aux points (A et B3a), A, (A et B3c) respectivement.

### C3) Fonctionnalisation avec des chlorures de sulfonyles

Elle s'effectue selon le schéma suivant :

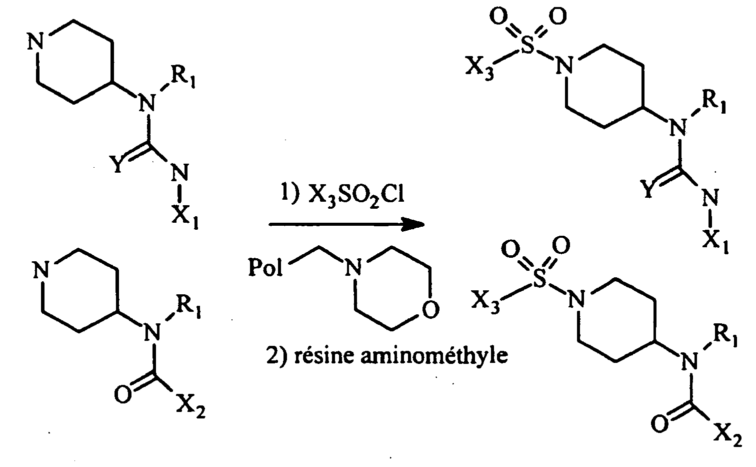

Procédure générale : la résine morpholinométhyle (Novabiochem, 2 à 3 éq.) est prégonflée dans des solvants anhydres tels que le dichlorométhane, le diméthylformamide ou le tétrahydrofurane. Le chlorure de sulfonyle (1,1 à 2 éq.) dissout dans des solvants anhydres tels que le dichlorométhane, le diméthylformamide ou le tétrahydrofurane est ajouté, suivi de la pipéridine 4-aminodisubstituée. Le mélange est agité de 16 à 48 heures. La résine aminométhyle (0,1 à 1,5 éq.) est ajoutée et la réaction agitée toute la nuit. Dans certains cas, de la résine échangeuse d'ions telle que IRA68 ou SAX est ajoutée et le mélange agité à température ambiante de 1 à 18 heures. Les résines sont filtrées sur fritté, sur cartouche de résine échangeuse d'ions SAX (500 mg, Interchim) ou sur cartouche d'alumine basique (500 mg, Interchim).

### Exemple C3 : Urée de N-(3,3-diphénylpropyl)-N-{1-[(4-méthoxyphényl)sulfonyl]-4-pipéridinyl}-N'-[3-(trifluorométhyl)phényl] (C₃₅H₃₆F₃N₃O₄S, M = 651,75)

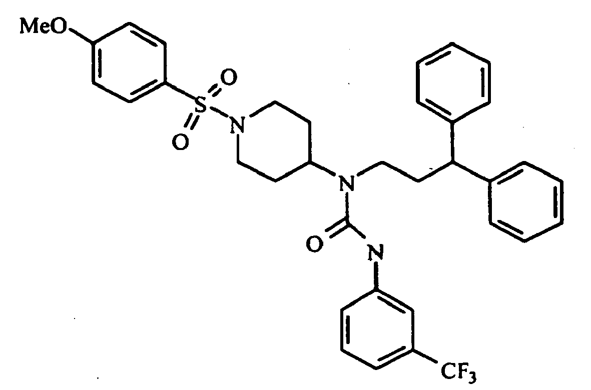

27,5 mg (100 µmol, 2 éq.) de résine morpholinométhyle est prégonflée dans du tétrahydrofurane anhydre, on ajoute alors le chlorure de 4-méthoxyphénylsulfonyle (15,5 mg, 0,075 mmol, 1,5 éq.) puis l'urée de N-(3,3-diphénylpropyl)-N-(4-pipéridinyl)-N'-[3-(trifluorométhyl)phényl] (24,3 mg, 0,05 mmol). Le mélange est agité toute la nuit. Les résines aminométhyle (20 mg) et échangeuse d'ions SAX sont ajoutées et le mélange est agité toute la nuit. Les résines sont filtrées et rincées au dichlorométhane. L'huile obtenue après évaporation est passée sur une cartouche de gel de silice (500 mg, Interchim) en éluant avec de l'acétate d'éthyle pour obtenir après concentration 18 mg (rdt = 56 %) d'un solide blanc.

RMN ¹H (CD₃OD, 400 MHz) δ : 7,71 (d, J = 9,2Hz, 2H) ; 7,65 (s, 1H) ; 7,51 (d, 1H); 7,41 (t, J = 7,6Hz, 1H); 7,29 (m, 9H); 7,20 (m, 2H) ; 7,11 (dd, J = 1,6 et 6,8Hz, 2H) ; 3,88 (s, 3H, OC*H₃*); 3,77 (d, J = 12,4Hz, 2H) ; 3,16 (t, J = 8Hz, 2H) ; 2,33 (m, 4H) ; 1,71 (d, J = 10Hz, 2H); 1,62 (qd, J = 4 et 12Hz, 2H) ; 1,3 (m, 2H). SM/CL : m/z = 652,4 (M+H).

Une série de sulfonamides a été synthetisée selon cette procédure. Les groupes R₁, X₁, X₂ et X₃ que l'on peut envisager, sont ceux illustrés aux points (A et B3a), A, (A et B3c) et B3b respectivement.

### D) Synthèse de pipéridines tri-substituées en phase solide

Elle s'effectue à partir de la résine vinyle sulfone (Kroll, F.E.K. ; Morphy, R. ; Rees, D. ; Gani, D. Tetrahedron Lett. 1997, 38, 8573-8576 ; Brown, A.R. J. Comb. Chem. 1999, 1, 283-285) selon le schéma suivant :

### D1) Préparation de la résine

Elle s'effectue selon le schéma suivant :

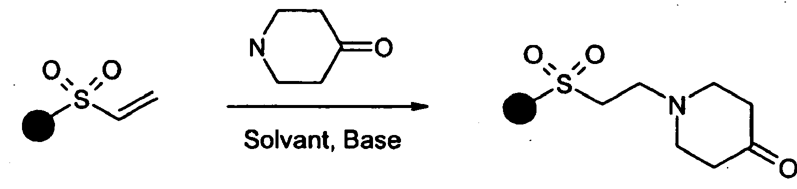

Au chlorhydrate hydraté de pipéridone dilué dans la diméthylformamide est ajoutée de la triéthylamine (1 éq.). Le mélange est chauffé jusqu'à dissolution complète de la cétone. Cette solution est ajoutée à la résine vinyle sulfone (0,05 éq.) prégonflée dans la diméthylformamide. Après 24 à 72 heures d'agitation à température ambiante, la résine est filtrée puis lavée plusieurs fois avec de la diméthylformamide, du tétrahydrofurane, du diéthyléther et du dichlorométhane.

### Préparation 4

1,5 g de la résine vinyle sulfone (Novabiochem, taux de charge de 1 mmol/g, 1,5 mmol) est prégonflée dans 50 ml de diméthylformamide. Dans le même temps, 2,3 g (15 mmol, 10 éq.) de chlorhydrate hydraté de pipéridone et 1,8 g (15 mmol, 10 éq.) de triéthylamine sont chauffés dans 100 ml de diméthylformamide jusqu'à dissolution complète. La solution jaunâtre est versée chaude sur la résine et le mélange agité 24 heures à température ambiante. La résine est filtrée puis lavée avec de la diméthylformamide, du tétrahydrofurane, du diéthyléther et du dichlorométhane (3 fois chaque solvant) puis séchée sous vide. 1,7 g de résine jaune pale sont isolés avec un taux de charge de 1 mmol/g calculé d'après l'analyse élémentaire de l'azote.

### D2) Amination réductrice sur support solide

Elle s'effectue selon la procédure décrite dans la littérature (Pelter, A. ; Rosser, R.M. J. Chem. Soc. Perkin Trans I 1984, 717-720 ; Bomann, M.D.; Guch, I.C. ; DiMare, M. J. Org. Chem. 1995, 60, 5995-5996; Khan, N.M. ; Arumugam, V. ; Balasubramanian, S. Tetrahedron Lett. 1996, 37, 4819-4822) suivant le schéma :

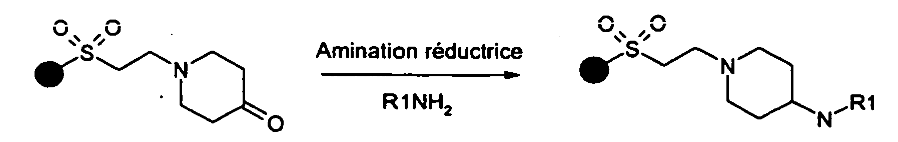

Procédure générale : à la résine cétonique prégonflée dans le triméthylorthofonniate (TMOF) est ajoutée l'amine primaire (5 à 10 éq.) puis le mélange est soniqué. On ajoute ensuite le complexe de borane pyridine (8 M, 5 à 10 éq.) et le mélange est agité 12 à 72 heures. La résine est filtrée, lavée avec des solvants tels que le dichlorométhane, la diméthylformamide, le méthanol et le tétrahydrofurane puis séchée sous vide.

### Préparation 5

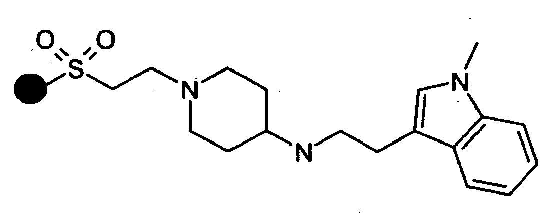

1 g (taux de charge de 1 mmol/g, 1 mmol) de résine cétonique sont prégonflés dans le TMOF. On ajoute ensuite la 2-(1-méthyl-1*H-*indol-3-yl)éthylamine (1,01 g, 10 mmol, 10 éq.) puis le complexe de borane pyridine (8M, 1,25 ml, 10 mmol, 10 éq.). Le mélange est agité 48 heures à température ambiante. La résine est filtrée, rincée successivement avec du dichlorométhane, du diméthylformamide, du méthanol, du tétrahydrofurane et du dichlorométhane puis séchée sous vide. 1,05 g de résine jaune pâle sont ainsi obtenus avec un taux de charge de 0,91 mmol/g calculé d'après l'analyse élémentaire de l'azote.

### D3) Fonctionnalisation de l'amine secondaire

D3a) Fonctionnalisation avec des isocyanates

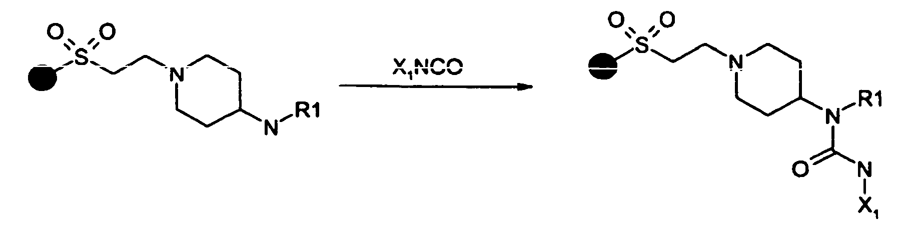

Procédure générale : la résine "amine secondaire" est prégonflée dans un solvant tel que le dichlorométhane ou la diméthylformamide avant l'addition de l'isocyanate (3 à 10 éq.). Le mélange est agité 1 à 24 heures à température ambiante. La résine est alors filtrée, lavée avec des solvants tels que le dichlorométhane, la diméthylformamide et le tétrahydrofurane puis séchée sous vide.

### Préparation 6

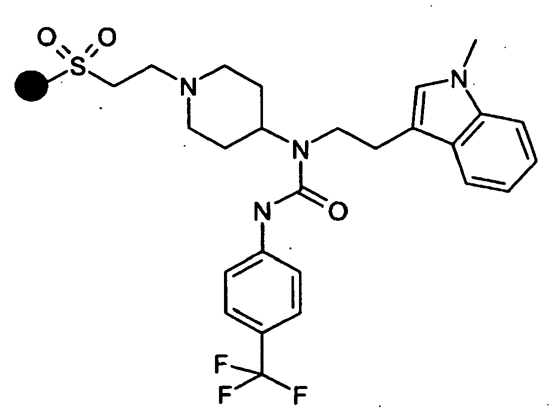

55 mg (50 µmol) de la résine (voir préparation 5) sont prégonflés dans du dichlorométhane anhydre. On ajoute ensuite le 4-trifluorophénylisocyanate (28 mg, 150 µmol, 3 éq.) et le tout est agité 2 heures à température ambiante. La résine est filtrée, rincée au tétrahydrofurane, au diméthylformamide, au tétrahydrofurane puis au dichlorométhane avant d'être séchée sous vide.

### D3b) Fonctionnalisation avec des chlorures de sulfonyles

Le mode opératoire de fonctionnalisation est identique à celui exposé au point B3b.

### D3c) Fonctionnalisation avec des chlorures d'acides

Le mode opératoire de fonctionnalisation est identique à celui exposé au point B3c.

### D3d) Fonctionnalisation avec des acides carboxyliques

Le mode opératoire de fonctionnalisation est identique à celui exposé au point B3d.

### D4) Etape de clivage

L'étape de clivage décrite ci-dessous est valable quelle que soit la fonctionnalisation opérée au préalable sur l'amine secondaire :

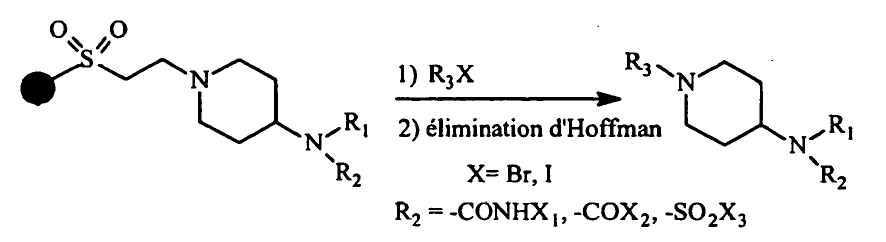

Procédure générale : la résine disubstituée est gonflée dans des solvants tels que le dichlorométhane, la diméthylformamide ou le tétrahydrofurane puis est ajouté l'halogénure R₃X dans lequel R₃ a la signification indiquée précédemment et X représente un atome d'halogène (5 éq.) et le mélange agité toute la nuit à une température comprise entre 20 et 60° C. La résine est filtrée, rincée avec des solvants tels que la diméthylformamide, le tétrahydrofurane, le méthanol et le dichlorométhane puis séchée sous vide. La résine est regonflée dans le dichlorométhane et de la résine échangeuse d'ions basiques (Ouyang, X. ; Armstrong, R.W. ; Murphy, M.M. J. Org. Chem. 1998, 63, 1027-1032) est ajoutée. Le tout est agité 48 heures à température ambiante. Les résines sont filtrées, rincées au dichlorométhane et le filtrat concentré sous vide.

### Exemple D4 : Urée de N-[2-(1-méthyl-1H-indol-3-yl)éthyl]-N-(1-méthyl-4-pipéridinyl)-N'-[4-(trifluorométhyl)phényl] (C₂₅H₂₉F₃N₄O, M = 458,5)

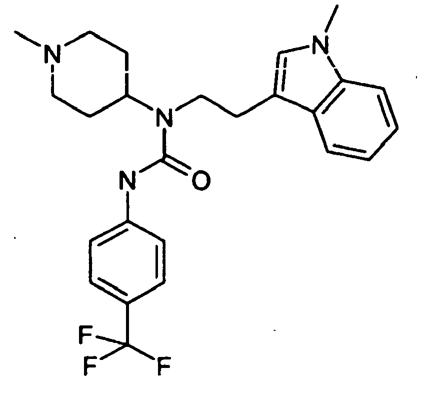

55 mg (50 µmol) de la résine urée sont gonflés dans de la diméthylformamide puis 35 mg (250 µmol, 5 éq.) de iodométhane sont ajoutés et le mélange agité 18 heures à température ambiante. La résine est filtrée, rincée avec de la diméthylformamide, du tétrahydrofurane, du méthanol et du dichlorométhane puis séchée sous vide. La résine est regonflée dans du dichlorométhane puis environ 100 mg de résine amberlite IRA68 sont ajoutés et le mélange agité 48 heures. Les résines sont filtrées, rincées avec du dichlorométhane et le filtrat concentré pour donner 18 mg (rdt = 78 %) d'une huile incolore.

RMN ¹H (CD₃OD, 400 MHz) δ: 7,65 (m, 2H); 7,40 (m, 2H); 7,31 (m, 1H); 7,20 (t, 1H) ; 7,10 (m, 1H); 7,06 (m, 2H) ; 4,04 (m, 1H); 3,68 (s, 3H) ; 3,60 (t, 2H); 3,04 (t, 2H); 2,94 (m, 2H); 2,29 (s, 3H); 2,14 (m, 2H) ; 1,91 (m, 2H) ; 1,76 (m, 2H). SM/CL : m/z = 459,3 (M+H).

Pour les groupes R₁, X₁, X₂ et X₃ tels qu'illustrés aux points A et B ci-dessus, les groupes R₃ que l'on peut envisager pour la synthèse de 4-aminopipéridines trisubstitutées selon la procédure ci-dessus, sont les suivants :

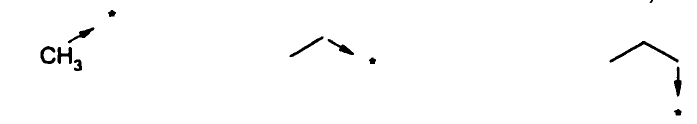

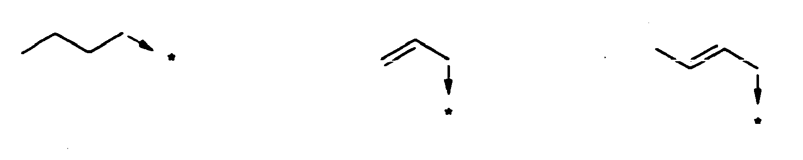

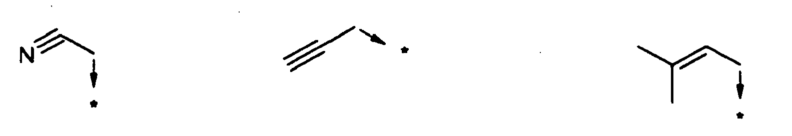

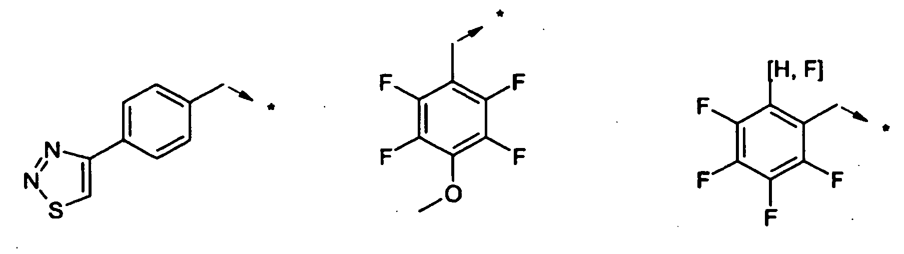

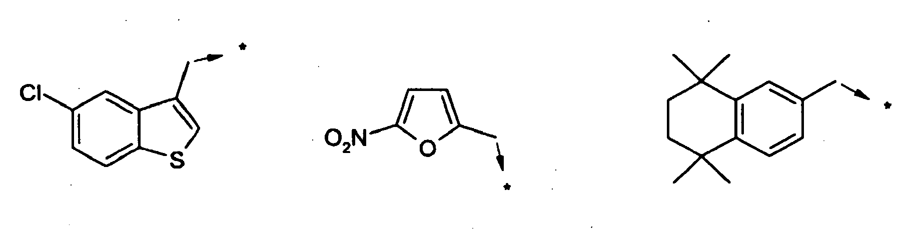

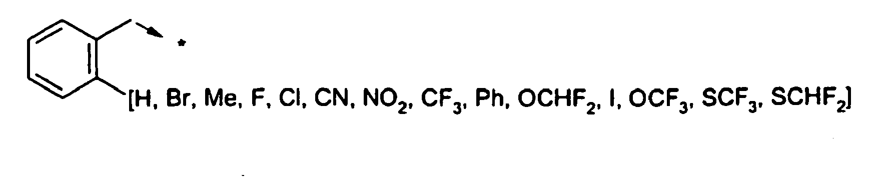

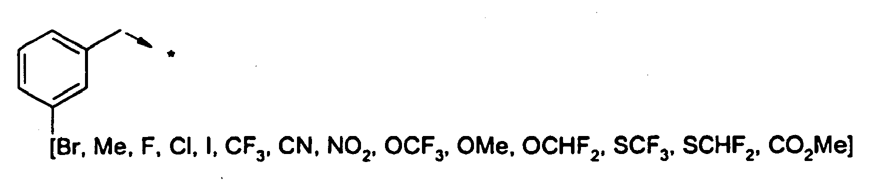

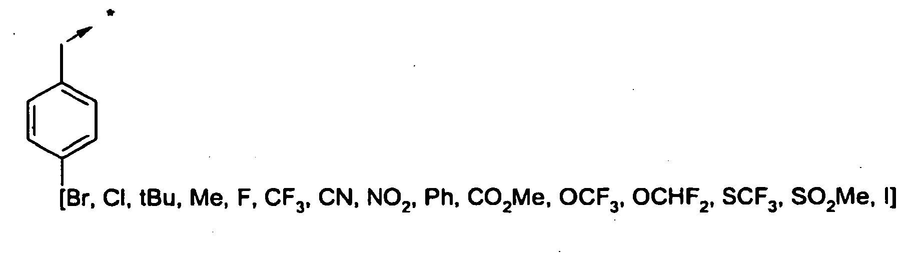

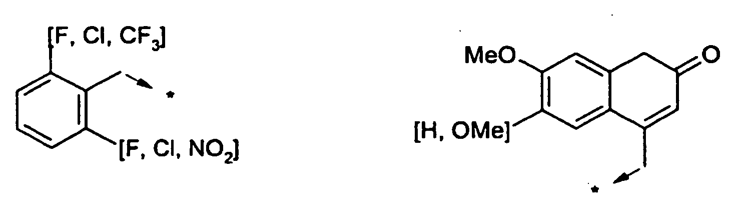

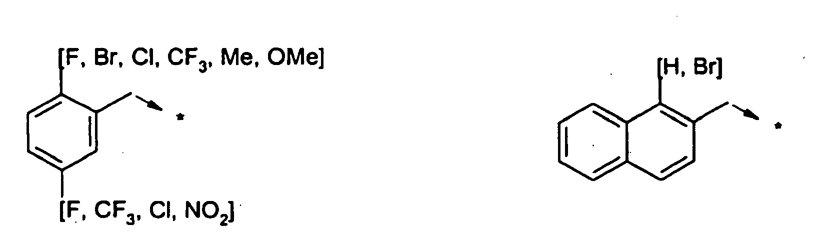

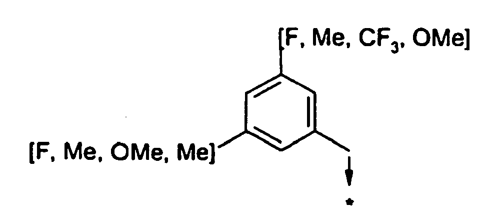

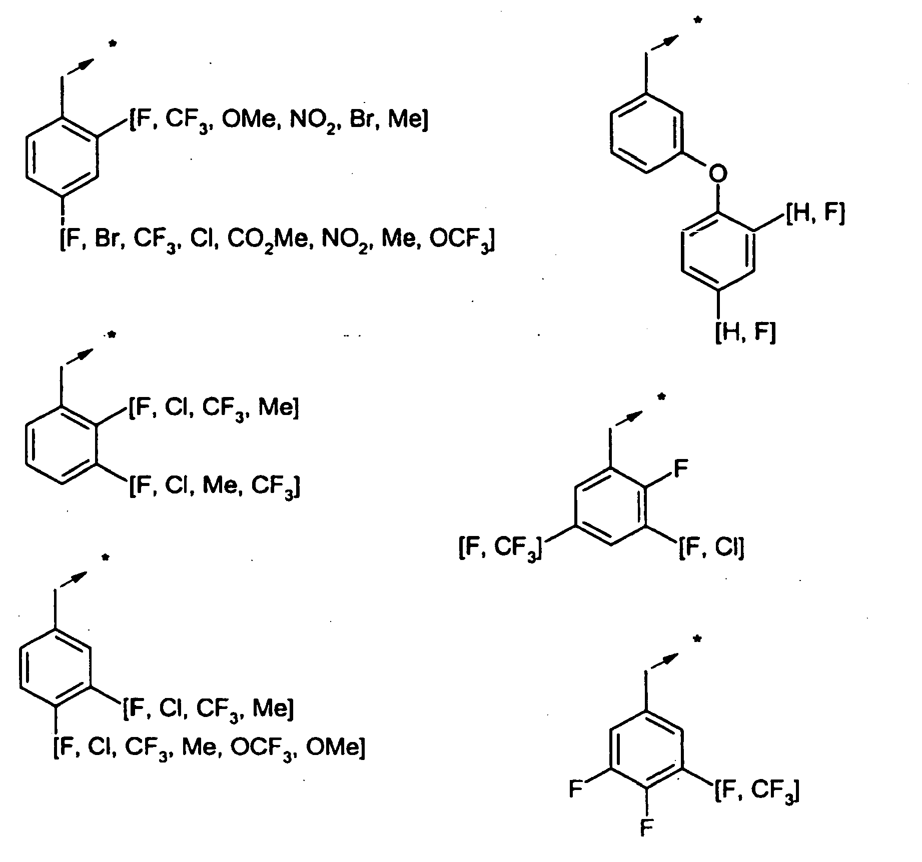

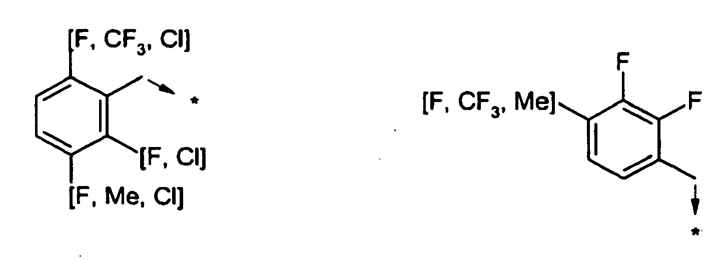

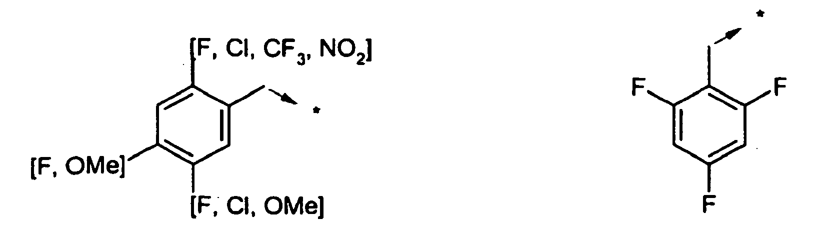

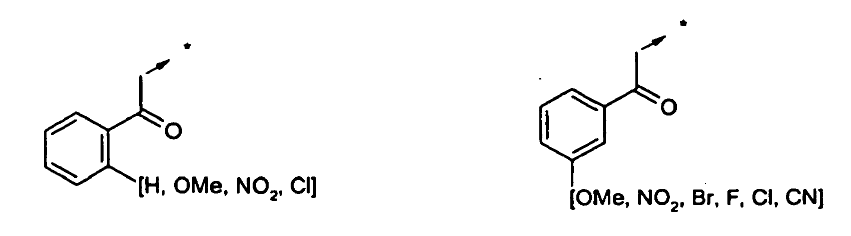

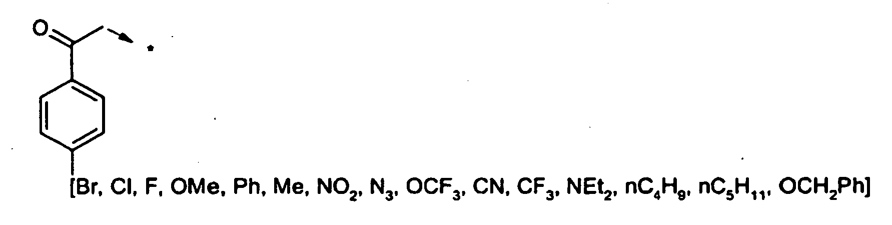

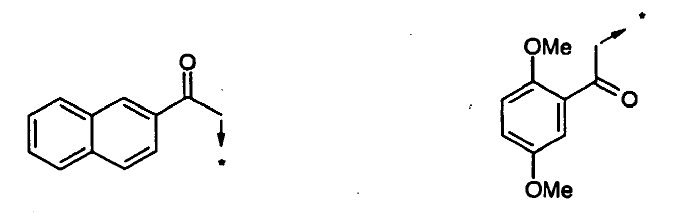

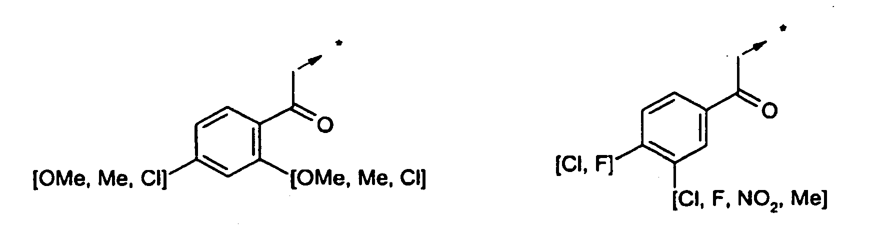

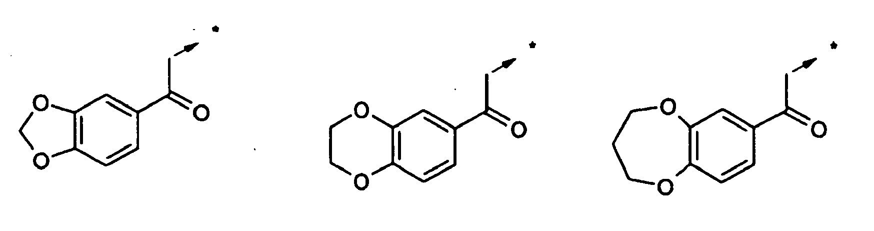

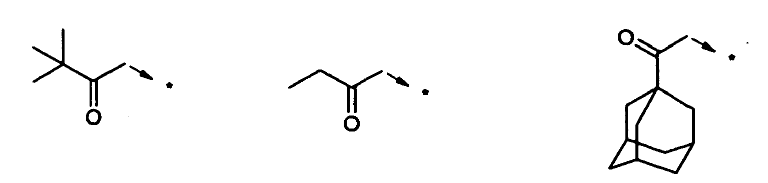

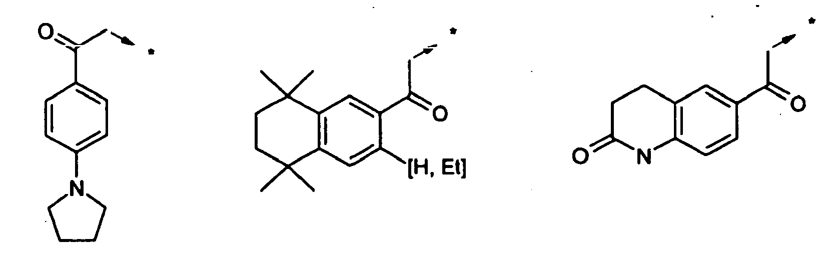

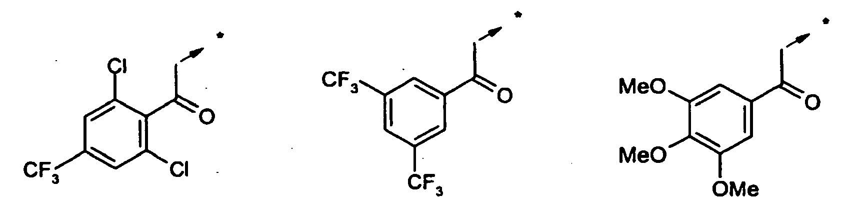

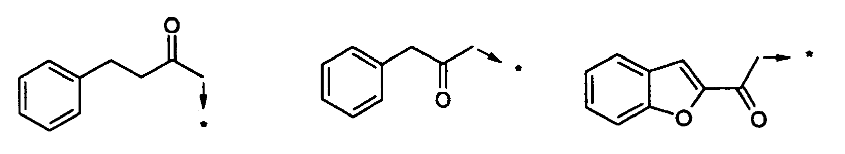

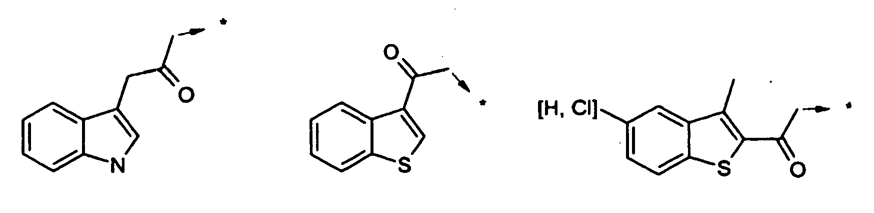

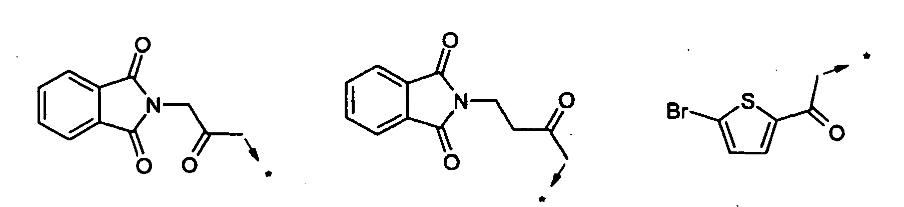

L'invention a également pour objet les procédés de préparation des composés I selon l'invention, en phase solide ou liquide, tels que décrits précédemment.

L'invention a plus particulièrement pour objet un procédé de préparation, en phase liquide, de composés de formule I telle que définie ci-dessus, caractérisé en ce qu'il comprend
l'amination réductrice de là pipéridone N-substituée suivante 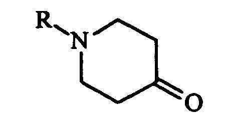 dans laquelle R représente le radical méthyle ou Boc, en présence d'une amine de formule R₁NH₂ dans laquelle R₁ a la signification indiquée ci-dessus, pour obtenir le composé de formule 1 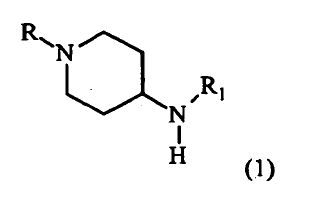 composé de formule (1) que l'on fait réagir avec
A) soit un composé de formule X₁NC(Y) dans laquelle X₁ et Y ont la signification indiquée ci-dessus, pour obtenir un composé de formule (2) 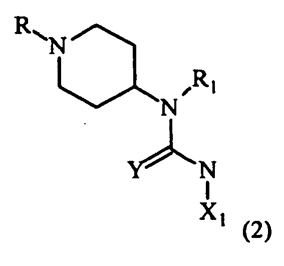 composé de formule (2) qui représente le composé de formule (I) correspondant dans lequel R₃ représente Me ou Boc et qui, lorsque R₃ représente Boc, peut être soumis à un traitement acide pour obtenir le composé de formule (I) correspondant dans laquelle R₃ représente l'atome d'hydrogène, composé de formule (I) ainsi obtenu que l'on peut faire réagir avec un composé de formule X₁NC(Y), X₂CO₂H ou bien X₃SO₂Cl dans laquelle X₁, Y, X₂ et X₃ ont la signification indiquée ci-dessus, pour obtenir le composé de formule I correspondant dans laquelle R₂ représente un radical de formule -C(Y)NHX₁ et R₃ le radical-C(Y)-NHX₁, -C(O)X₂ ou SO₂X₃ respectivement ;
B) soit un composé de formule X₂CO₂H dans laquelle X₂ a la signification indiquée ci-dessus, pour obtenir un composé de formule (3) 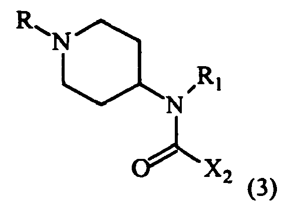 composé de formule (3) qui représente le composé de formule (I) correspondant dans lequel R₃ représente Me ou Boc et qui, lorsque R₃ représente Boc, peut être soumis à un traitement acide pour obtenir le composé de formule (I) correspondant dans laquelle R₃ représente l'atome d'hydrogène,
   composé de formule (I) ainsi obtenu que l'on peut faire réagir avec un composé de formule X₁NC(Y), X₂CO₂H ou bien X₃SO₂Cl dans laquelle X₁, Y, X₂ et X₃ ont la signification indiquée ci-dessus, pour obtenir le composé de formule I correspondant dans laquelle R₂ représente un radical de formule -C(O)X₂ et R₃ le radical-C(Y)-NHX₁, -C(O)X₂ ou SO₂X₃ respectivement.

L'invention a plus particulièrement pour objet également un procédé de préparation, en phase solide, de composés de formule I telle que définie ci-dessus, caractérisé en ce qu'il comprend
l'amination réductrice de la résine cétonique 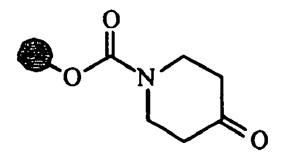 en présence d'une amine de formule R₁NH₂ dans laquelle R₁ a la signification indiquée ci-dessus, pour obtenir le composé de formule (4) 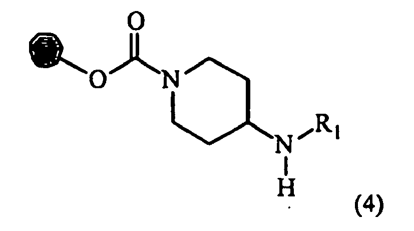 composé de formule (4) que l'on fait réagir avec
A) soit un composé de formule X₁NC(Y) dans laquelle X₁ et Y ont la signification indiquée ci-dessus, pour obtenir un composé de formule (5) 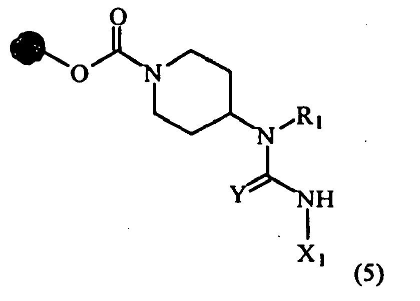 suivi du clivage de la résine pour obtenir le composé de formule (I) correspondant dans laquelle R₃ représente l'atome d'hydrogène,
B) soit un composé de formule X₃SO₂Cl dans laquelle X₃ a la signification indiquée ci-dessus, pour obtenir un composé de formule (6) 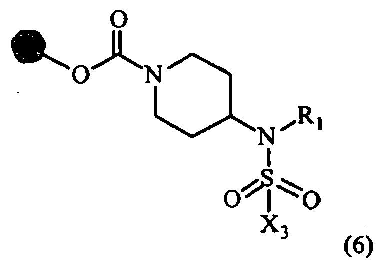 suivi du clivage de la résine pour obtenir le composé de formule (I) correspondant dans laquelle R₃ représente l'atome d'hydrogène, .
C) soit un composé de formule X₂CO₂Cl dans laquelle X₂ a la signification indiquée ci-dessus, pour obtenir un composé de formule (7) 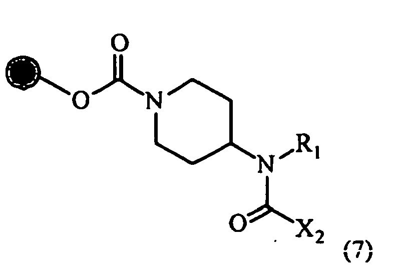 suivi du clivage de la résine pour obtenir le composé de formule (I) correspondant dans laquelle R₃ représente l'atome d'hydrogène ;
D) soit un composé de formule X₂CO₂H dans laquelle X₂ a la signification indiquée ci-dessus, pour obtenir un composé de formule (7) tel que défini ci-dessus, suivi du clivage de la résine pour obtenir le composé de formule (I) correspondant dans laquelle R₃ représente l'atome d'hydrogène.

Enfin l'invention a plus particulièrement pour objet un procédé de préparation, en phase solide, de composés de formule I telle que définie ci-dessus, caractérisé en ce qu'il comprend
l'amination réductrice de la résine cétonique 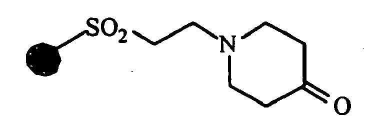 en présence d'une amine de formule R₁NH₂ dans laquelle R₁ a la signification indiquée ci-dessus, pour obtenir le composé de formule (8) 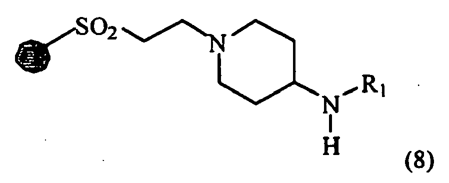 composé de formule (8) que l'on fait réagir avec
A) soit un composé de formule X₁NC(O) dans laquelle X₁ a la signification indiquée ci-dessus, pour obtenir un composé de formule (9) 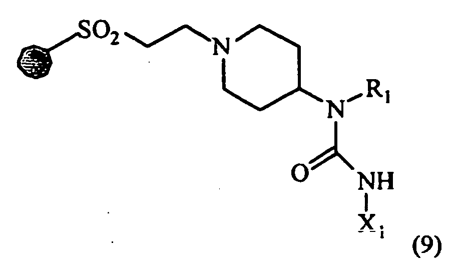 composé (9) ainsi formé que l'on fait réagir avec un composé de formule R₃X dans laquelle R₃ est tel que défini ci-dessus et X représente Br ou I, suivi du clivage de la résine pour obtenir le composé de formule (I) correspondant ;
B) soit un composé de formule X₃SO₂Cl dans laquelle X₃ a la signification indiquée ci-dessus, pour obtenir un composé de formule (10) 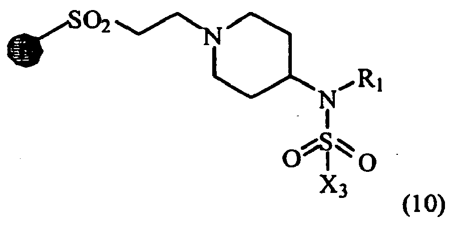 composé (10) ainsi formé que l'on fait réagir avec un composé de formule R₃X dans laquelle R₃ est tel que défini ci-dessus et X représente Br ou I, suivi du clivage de la résine pour obtenir le composé de formule (I) correspondant ;
C) soit un composé de formule X₂CO₂Cl dans laquelle X₂ a la signification indiquée ci-dessus, pour obtenir un composé de formule (11) 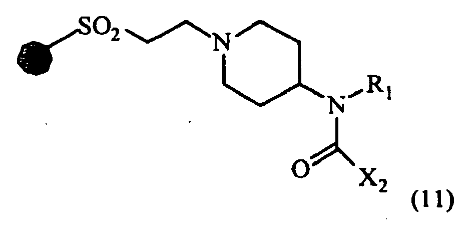 composé (11) ainsi formé que l'on fait réagir avec un composé de formule R₃X dans laquelle R₃ est tel que défini ci-dessus et X représente Br ou I, suivi du clivage de la résine pour obtenir le composé de formule (I) correspondant ;
D) soit un composé de formule X₂CO₂H dans laquelle X₂ a la signification indiquée ci-dessus, pour obtenir un composé de formule (11) tel que défini ci-dessus,
   composé (11) ainsi formé que l'on fait réagir avec un composé de formule R₃X dans laquelle R₃ est tel que défini ci-dessus et X représente Br ou I, suivi du clivage de la résine pour obtenir le composé de formule (I) correspondant.

Les composés I de la présente invention possèdent d'intéressantes propriétés pharmacologiques. C'est ainsi que l'on a découvert que les composés I de la présente invention ont une haute affinité pour un (ou plusieurs) des récepteurs de la somatostatine. Ils peuvent être utilisés comme agonistes ou antagonistes non-peptidiques de la somatostatine de manière sélective ou non.

Les composés de la présente invention peuvent ainsi être utilisés dans différentes applications thérapeutiques. Ils peuvent avantageusement être utilisés pour traiter les états pathologiques ou les maladies tels que présentés ci-dessus et dans lesquels un (ou plusieurs) des récepteurs de la somatostatine est(sont) impliqué(s).

On trouvera ci-après, dans la partie expérimentale, une illustration des propriétés pharmacologiques des composés de l'invention.

La présente demande a également pour objet, à titre de médicaments, les produits de formule 1 telle que définie ci-dessus, ainsi que les sels d'addition avec les acides minéraux ou organiques pharmaceutiquement acceptables desdits produits de formule I, ainsi que les compositions pharmaceutiques contenant, à titre de principe actif, un au moins des médicaments tels que définis ci-dessus, en association avec un support pharmaceutiquement acceptable.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrée par intraveineuse.

Certains composés de la formule générale I précédemment décrite sont couverts par la demande DE 2751138. Cette demande DE décrit des composés qui antagonisent les effets de la dopamine et des agents dopaminergiques endogènes ou exogènes et activent le mécanisme sérotoninergique, activités très différentes de celle décrite dans la présente demande.

La présente invention a donc également pour objet l'utilisation des composés de formule générale Iₐ 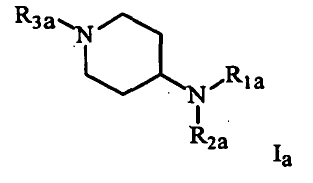 sous forme racémique, d'énantiomère ou toutes combinaisons de ces formes, dans laquelle :
R₁ₐ représente un radical (C₁-C₆)alkyle linéaire ou ramifié, le radical -(CH₂)ₘ-Y-Z₁₁ ou -(CH₂)ₘ -Z₁₂ dans lequel
   Z₁₁ représente un (C₁-C₆)alkyle,
   Z₁₂ représente *bis*-phényle, (C₃-C₇)cycloalkyle, (C₃-C₇)hétérocycloalkyle éventuellement substitué par un ou plusieurs substituants choisis indépendamment parmi les radicaux oxy et alkyle, ou aryle ou hétéroaryle éventuellement substitué par un ou plusieurs substituants choisis indépendamment parmi les radicaux fluoro, chloro, bromo, iodo, alkyle, alkoxy,
   ou bien Z₁₂ représente 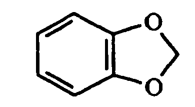 Y représente l'atome d'oxygène,
   ou bien R₁ₐ représente un radical de formule 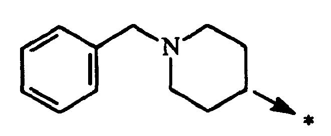
R₂ₐ représente un radical de formule -C(Y)NHX₁, -C(O)X₂ ou SO₂X₃ dans laquelle
   Y représente un atome d'oxygène ou de soufre ;
   X₁ représente un radical (C₁-C₁₅)alkyle linéaire ou ramifié, ou -(CH₂)ₚZ₂₂ dans lequel
   Z₂₂ représente cyclohexényle, *bis*-phényle, (C₃-C₇)cycloalkyle, (C₃-C₇)hétérocycloalkyle, mono- ou di-alkylamino, -C(O)-O-alkyle, ou aryle ou hétéroaryle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle, ou phényle,
   ou bien Z₂₂ représente un radical de formule 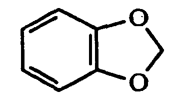 X₂ représente un radical (C₁-C₁₀)alkyle linéaire ou ramifié, alkynyle, -(CH₂)ₘ-W-(CH₂)_{q}-Z₂₃ ou -(CH₂)ₚ-U-Z₂₄ dans lequel
   W représente SO₂,
   U représente une liaison covalente,
   Z₂₃ représente un radical aryle ;
   Z₂₄ représente cyclohexényle, *bis*-phényle, (C₃-C₇)cycloalkyle éventuellement substitué par un radical aminoalkyle, ou aryle ou hétéroaryle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, mono- ou di-alkylamino, amino
   ou Z₂₄ représente un radical de formule 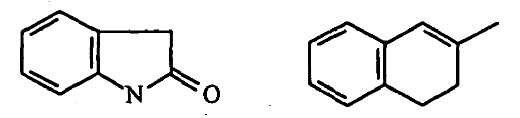 ou bien X₂ représente 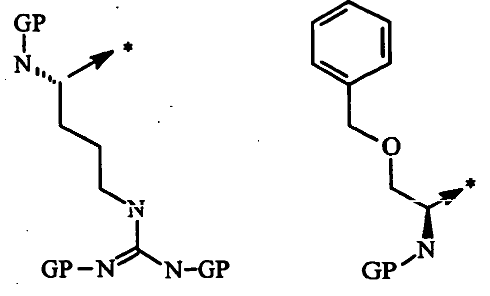 X₃ représente un radical -(CH₂)ₚZ₂₅ dans lequel Z₂₅ représente un radical aryle éventuellement substitué par un ou plusieurs radicaux identiques ou différents choisis parmi alkoxy et CF₃ ;
R₃ₐ représente l'atome d'hydrogène, un radical alkyle, alkényle, hétéroarylalkyle éventuellement substitué ou un radical de formule -C(Y)-NHX₁, -C(O)X₂ ou SO₂X₃ dans laquelle
   Y représente un atome d'oxygène ou de soufre ;
   X₁ représente un radical -(CH₂)ₚZ₂₂ dans lequel
   Z₂₂ représente un radical aryle éventuellement substitué par un ou plusieurs radicaux choisis indépendamment parmi les radicaux fluoro, chloro, bromo, iodo, alkyle, alkoxy, CF₃, nitro, phénoxy ;
   X₂ représente le radical vinyle substitué par un phényle, le radical phényle étant lui-même éventuellement substitué par un ou plusieurs radicaux halo, ou -(CH₂)ₚ-U-Z₂₄ dans lequel
   U représente une liaison covalente ou l'atome d'oxygène ;
   Z₂₄ représente alkyle, (C₃-C₇)cycloalkyle, (C₃-C₇)hétérocycloalkyle, *bis*-phényle, amino, mono ou di-alkylamino, ou aryle ou hétéroaryle éventuellement substitué par un ou plusieurs radicaux choisis parmi alkoxy, bromo, chloro, fluoro, hydroxy, CF₃, nitro, amino, mono- et di-alkylamino, pyrrolyle,
   ou bien X₂ représente un radical de formule 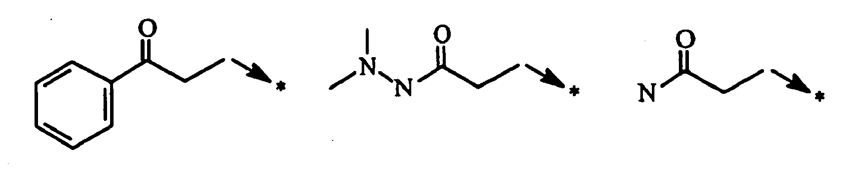 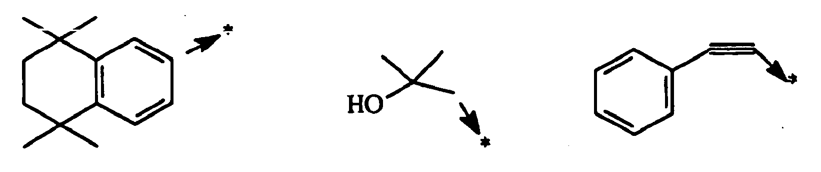 X₃ représente un radical (C₁-C₁₀)alkyle linéaire ou ramifié, le radical vinyle substitué par un radical (le radical phényle étant lui-même éventuellement substitué), CF₃, ou -(CH₂)ₚZ₂₅ dans lequel
   Z₂₅ représente aryle ou hétéroaryle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino ;
n est un entier de 0 à 4 ;
m est un entier de 1 à 6 ;
p est un entier de 0 à 6 ;
q est un entier de 0 à 2,
ou leurs sels d'addition avec les acides minéraux ou organiques pharmaceutiquement acceptables, pour la préparation d'un médicament destiné à traiter les états pathologiques ou les maladies dans lesquels un (ou plusieurs) des récepteurs de la somatostatine est(sont) impliqué(s).

De manière préférentielle, R₁ₐ représente un radical (C₁-C₆)alkyle linéaire ou ramifié, le radical-(CH₂)ₘ-Y-Z₁₁ ou -(CH₂)ₘ-Z₁₂ dans lequel
Z₁₁ représente un (C₁-C₆)alkyle,
Z₁₂ représente naphtyle, morpholino, *bis*-phényle, pyrrolidinyle substitué par le radical oxy, ou bien les radicaux phényle, pipérazinyle, pyridinyle et indolyle qui sont éventuellement substitués par un ou plusieurs substituants choisis indépendamment parmi les radicaux bromo, fluoro, chloro, alkyle, alkoxy, -CF₃, -OCF₃ ;
ou bien Z₁₂ représente 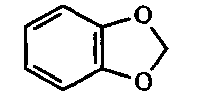 Y représente l'atome d'oxygène,
ou bien R₁ₐ représente un radical de formule ci-dessous :

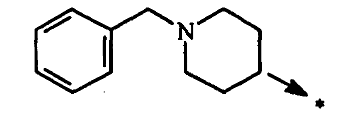

De manière préférentielle, R₂ₐ représente un radical de formule -C(Y)NHX₁, -C(O)X₂ ou SO₂X₃ dans laquelle
X₁ représente un radical (C₁-C₁₀)alkyle linéaire ou ramifié, ou -(CH₂)ₚZ₂₂ dans lequel
Z₂₂ représente cyclohexyle, cyclohexényle, *bis*-phényle, morpholino, pipéridino, mono- ou di-alkylamino, -C(O)-O-alkyle, ou phényle, naphtyle ou furyle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle ou phényle,
ou bien Z₂₂ représente un radical de formule 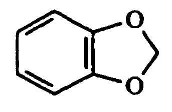 X₂ représente un radical alkyle, alkynyle, -(CH₂)ₘ-W-(CH₂)_{q}-Z₂₃ ou -(CH₂)ₚZ₂₄ dans lequel
W représente SO₂ ;
Z₂₃ représente le radical phényle ;
Z₂₄ représente cyclohexényle, *bis*-phényle, cyclohexyle éventuellement substitué par un radical aminoalkyle, ou phényle, naphtyle, benzothiényle, thiényle ou indolyle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, -NH-C(O)-alkyle, mono- ou di-alkylamino, amino, ou
Z₂₄ représente un radical de formule 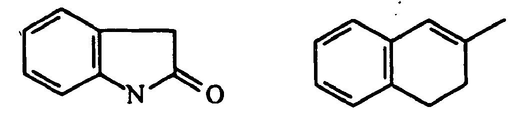 ou bien X₂ représente 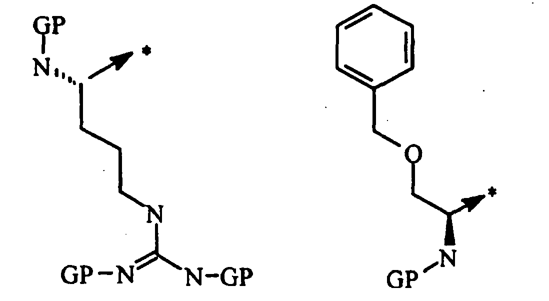 X₃ représente un radical -(CH₂)ₚZ₂₅ dans lequel Z₂₅ représente le radical phényle éventuellement substitué par un ou plusieurs radicaux identiques ou différents choisis parmi alkoxy et CF₃,

De manière préférentielle, R₃ₐ représente l'atome d'hydrogène, un radical alkyle, alkényle ou furyl-méthyl susbtitué par un ou plusieurs radicaux nitro, ou un radical de formule -C(Y)-NHX₁, -C(O)X₂ ou SO₂X₃ dans laquelle
X₁ représente un radical -(CH₂)ₚZ₂₂ dans lequel
Z₂₂ représente le radical phényle ou naphtyle éventuellement substitué par un ou plusieurs radicaux choisis indépendamment parmi les radicaux fluoro, chloro, bromo, iodo, alkyle, alkoxy, CF₃, nitro, phénoxy,
X₂ représente le radical vinyle substitué par un radical phényle lui-même éventuellement substitué par un ou plusieurs radicaux halo, ou -(CH₂)ₚ-U-Z₂₄ dans lequel
Z₂₄ représente alkyle, cyclohexyle, tétrahydrofuryle, *bis*-phényle, amino, mono ou di-alkylamino, ou phényle, indolyle, thiényle, pyridinyle, benzothiényle et furyle éventuellement substitué par un ou plusieurs radicaux choisis parmi alkoxy, bromo, chloro, fluoro, amino, mono- et di-alkylamino, nitro, hydroxy, pyrrolyle
ou bien X₂ représente un radical de formule 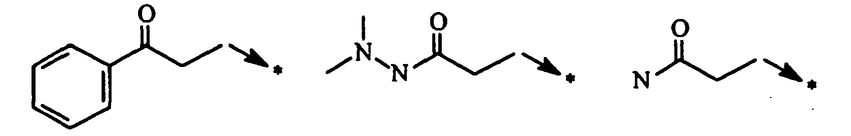 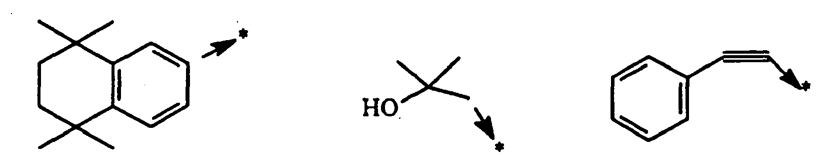 X₃ représente un radical (C₁-C₁₀)alkyle linéaire ou ramifié, le radical vinyle substitué par un radical phényle, CF₃, ou -(CH₂)ₚZ₂₅ dans lequel
Z₂₅ représente un radical phényle, napthyle, thiényle, pyrazolyle ou thiazolyle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino ;

De manière très préférentielle, R₁ₐ représente le radical -(CH₂)ₘZ₁₂ dans lequel m = 2 et Z₁₂ représente *bis*-phényle ou bien le radical indolyle substitués par un ou plusieurs substituants choisis indépendamment parmi les radicaux alkyle et alkoxy.

De manière très préférentielle, R₂ₐ représente les radicaux de formule -C(Y)NHX₁ et -C(O)X₂ dans laquelle
Y représente S ;
X₁ représente un radical phényle éventuellement substitué par un ou plusieurs radicaux azido,
X₂ représente -(CH₂)ₚZ₂₄ dans lequel
p est égal à 1, 2 ou 3,
Z₂₄ représente cyclohexyle, ou phényle ou benzothiényle éventuellement substitué par un ou plusieurs radicaux choisis parmi fluoro, chloro, bromo, iodo ou -CF₃.

De manière très préférentielle, R₃ₐ représente l'atome d'hydrogène ou le radical méthyle.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

D'autres composés selon l'invention obtenus selon les procédures des exemples A, B, C et D précédemment décrites, sont rassemblés dans le tableau ci-dessous.

Les composés sont caractérisés par leur temps de rétention (tr), exprimé en minute, et leur pic moléculaire (M+H+) déterminé par la spectroscopie de masse (SM).

Pour la spectroscopie de masse, un spectromètre de masse simple quadripôle (Micromass, modèle Platform) équipé d'une source électrospray est utilisé avec une résolution de 0,8 Da à 50 % de vallée. Les conditions pour les exemples 1 à 778 ci-dessous, sont les suivantes :

### Conditions C1 et C2

Eluant : A : Eau + 0,02 % acide trifluoracétique ; B : acétonitrile

| T (min) | *A %* | *B %* |
|---|---|---|
| 0 | 100 | 0 |
| 1 | 100 | 0 |
| 10 | 15 | 85 |
| 12 | 15 | 85 |

| Condition C1 | Condition C2 |
|---|---|
| Débit : 1,1 ml/min | Débit : 1,1 ml/min |
| Injection : 5 µl | Injection : 20 µl |
| Temp : 40° C | Temp : 40° C |
| Longueur d'ondes (% UV) : 210 nm | Longueur d'ondes (% UV) : 210 nm |
| Colonne : Uptisphere ODS 3 µm | Colonne : Kromasyl ODS 3,5 µm |
| 33 * 4,6 mm i.d | 50* 4,6 mm i.d |

### Conditions C3

Eluant : A : Eau + 0,02 % acide trifluoracétique ; B : acétonitrile

| T (min) | *A %* | *B %* |
|---|---|---|
| 0 | 90 | 10 |
| 6 | 15 | 85 |
| 10 | 15 | 85 |

Débit : 1 ml/min
Injection : 5 µl
Colonne : Uptisphere ODS 3 µm 50 * 4,6 mm i.d
Temp : 40° C
Longueur d'ondes (% UV) : 220 nm
Les conditions suivant les exemples, sont les suivantes :

| Exemples | Conditions |
|---|---|
| 1 à 29 | C2 |
| 30 à 263 | C1 |
| 264 à 425 | C3 |
| 426 à 456 | C2 |
| 457 à 503 | C3 |
| 504 à 586 | C1 |
| 587 à 778 | C3 |

Ces exemples sont présentés pour illustrer les procédures ci-dessus et ne doivent en aucun cas être considérés comme une limite à la portée de l'invention.

| Ex | R₁ | R₂ | R₃ | Pureté (en %) | tr | M+H+ |
|---|---|---|---|---|---|---|
| 1 | 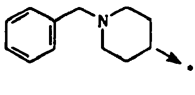 | 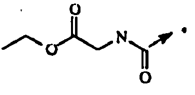 | 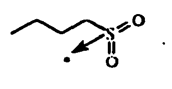 | 66 | 7.6 | 523,3 |
| 2 | " | " | 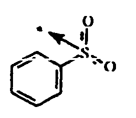 | 94 | 7.7 | 543.2 |
| 3 | " | " | 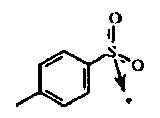 | 96 | 8.1 | 557.2 |
| 4 | " | " | 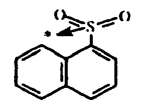 | 98 | 8.5 | 593.2 |
| 5 | " | " | 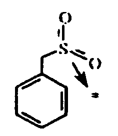 | 95 | 7.8 | 557.2 |
| 6 | " | " | 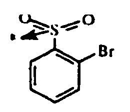 | 97 | 8.1 | 623.1 |
| 7 | " | " | 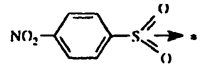 | 95 | 8.1 | 588.2 |
| 8 | " | " | 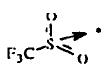 | 19 | 8.1 | 535.2 |
| 9 | " | " | 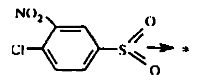 | 99 | 8,5 | 622.2 |
| 10 | " | " | 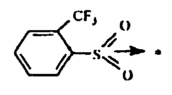 | 80 | 8.4 | 611.2 |
| 11 | " | " | 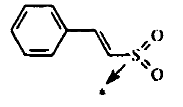 | 99 | 8.2 | 569.2 |
| 12 | " | " | 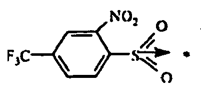 | 93 | 8.9 | 656.2 |
| 13 | " | " | 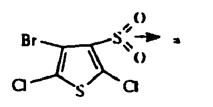 | 85 | 9.1 | 697.0 |
| 14 | " | " | 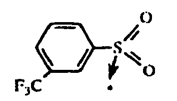 | 95 | 8.7 | 611.2 |
| 15 | " | " | 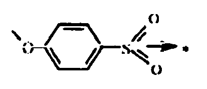 | 87 | 7.8 | 573.2 |
| 16 | " | " | 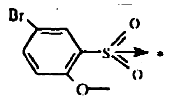 | 100 | 8.4 | 653.2 |
| 17 | " | " | 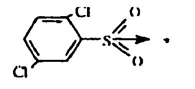 | 97 | 8.6 | 611.1 |
| 18 | " | " | 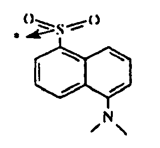 | 99 | 8.7 | 636.3 |
| 19 | " | " | 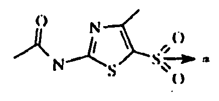 | 83 | 7.2 | 621.2 |
| 20 | " | " | 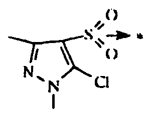 | 98 | 7.4 | 595.2 |
| 21 | " | " | 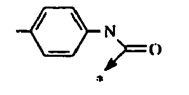 | 84 | 7.4 | 536.3 |
| 22 | " | " | 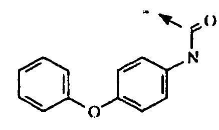 | 99 | 8.4 | 614.3 |
| 23 | " | " | 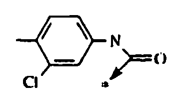 | 63 | 8.2 | 570.2 |
| 24 | " | " | 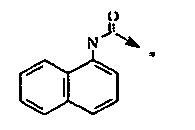 | 92 | 7.5 | 572.3 |
| 25 | " | " | 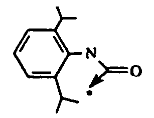 | 93 | 8.4 | 606.4 |
| 26 | " | " | 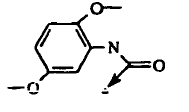 | 96 | 7.4 | 582.3 |
| 27 | " | " | 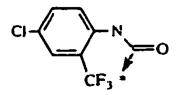 | 93 | 8.1 | 624.2 |
| 28 | " | " | 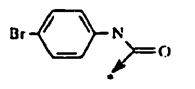 | 93 | 7.8 | 602.2 |
| 29 | " | " | 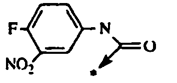 | 95 | 7.4 | 585.2 |
| 30 | " | " | 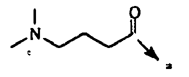 | 87.39 | 4.0 | 516.4 |
| 31 | " | " | 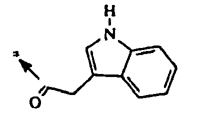 | 92 | 5.5 | 560.3 |
| 32 | " | " | 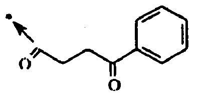 | 90 | 5.7 | 563.3 |
| 33 | " | " | 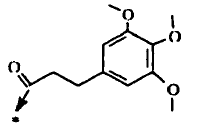 | 87.73 | 5.6 | 625.4 |
| 34 | " | " | 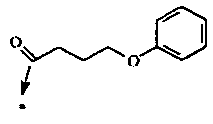 | 85.41 | 6.0 | 565.4 |
| 35 | " | " | 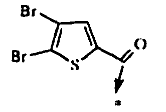 | 98.4 | 6.4 | 671.1 |
| 36 | " | " | 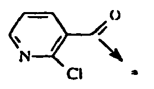 | 86 | 4.9 | 542.3 |
| 37 | " | " | 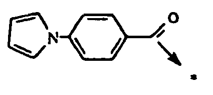 | 89 | 6.1 | 572.3 |
| 38 | " | " | 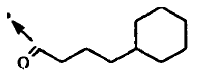 | 77.61 | 6.8 | 555.4 |
| 39 | " | " | 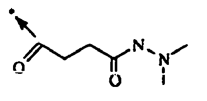 | 89.16 | 4.2 | 545.4 |
| 40 | " | " | 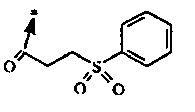 | 92.32 | 5.3 | 599.3 |
| 41 | " | " | 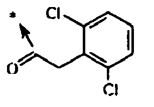 | 83 | 6.0 | 589.2 |
| 42 | " | " | 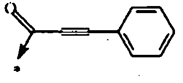 | 36.3 | 5.9 | 531.2 |
| 43 | " | " | 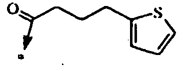 | 83.27 | 5.9 | 555.3 |
| 44 | " | " | 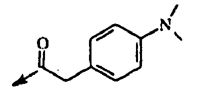 | 82 | 4.5 | 564.4 |
| 45 | " | " | 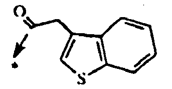 | 86.75 | 6.0 | 577.3 |
| 46 | " | " | 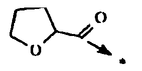 | 91.95 | 4.7 | 501.4 |
| 47 | " | " | 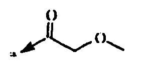 | 88.94 | 4.5 | 475.3 |
| 48 | " | " | 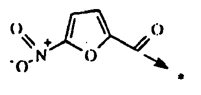 | 73 | 5.3 | 542.3 |
| 49 | " | 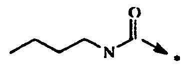 | 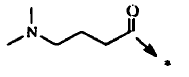 | 90.96 | 4.4 | 486.4 |
| 50 | " | " | 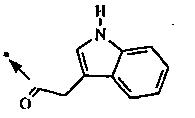 | 95.5 | 5.9 | 530.4 |
| 51 | " | " | 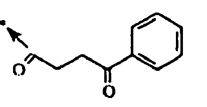 | 94.51 | 6.1 | 533.4 |
| 52 | " | " | 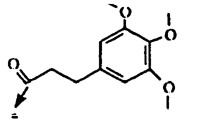 | 93.64 | 6.0 | 595.4 |
| 53 | " | " | 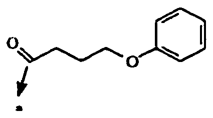 | 96.05 | 6.5 | 535.4 |
| 54 | " | " | 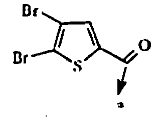 | 94.68 | 6.9 | 641.1 |
| 55 | " | " | 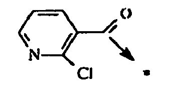 | 86 | 5.5 | 512,3 |
| 56 | " | " | 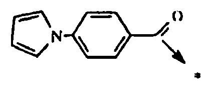 | 92 | 6.5 | 542.4 |
| 57 | " | " | 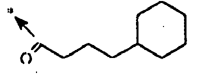 | 91.29 | 7.2 | 525.5 |
| 58 | " | " | 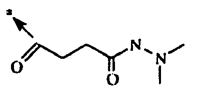 | 94.7 | 4.7 | 515.4 |
| 59 | " | " | 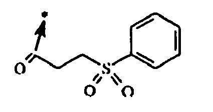 | 94 | 5.8 | 569.3 |
| 60 | " | " | 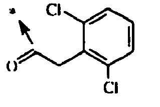 | 89.43 | 6.6 | 559.3 |
| 61 | " | " | 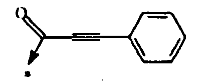 | 32 | 6.9 | 501.5 |
| 62 | " | " | 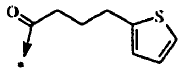 | 93.53 | 6.4 | 525.4 |
| 63 | " | " | 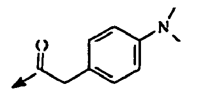 | 94.7 | 4.9 | 534.4 |
| 64 | " | " | 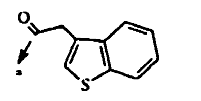 | 94.32 | 6.4 | 547.3 |
| 65 | " | " | 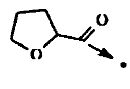 | 91.71 | 5.2 | 471.4 |
| 66 | " | " | 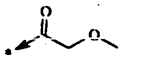 | 92.47 | 5.0 | 445.4 |
| 67 | " | " | 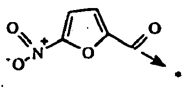 | 58 | 5.9 | 512.3 |
| 68 | " | 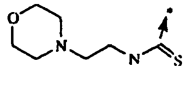 | 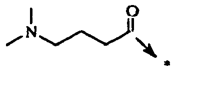 | 84.55 | 3.6 | 559.4 |
| 69 | " | " | 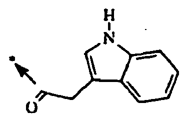 | 87.7 | 4.7 | 603.4 |
| 70 | " | " | 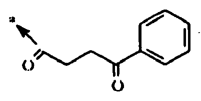 | 90.77 | 4.8 | 606.4 |
| 71 | " | " | 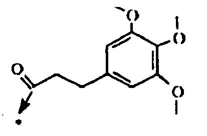 | 72.34 | 4.8 | 668.4 |
| 72 | " | " | 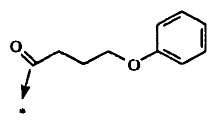 | 87.18 | 5.1 | 608.4 |
| 73 | " | " | 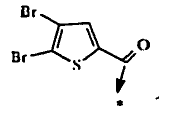 | 69.52 | 5.4 | 714.1 |
| 74 | " | " | 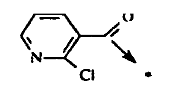 | 63.39 | 4.2 | 585,3 |
| 75 | " | " | 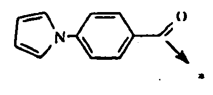 | 54.46 | 5.1 | 615.4 |
| 76 | " | " | 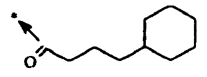 | 87.3 | 5.7 | 598.4 |
| 77 | " | " | 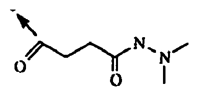 | 96.1 | 3.8 | 588.4 |
| 78 | " | " | 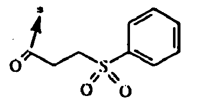 | 89.9 | 4.5 | 642.3 |
| 79 | " | " | 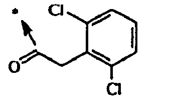 | 61.5 | 5.1 | 632.3 |
| 80 | " | " | 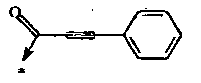 | 43.65 | 5.0 | 574.3 |
| 81 | " | " | 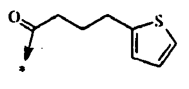 | 88.18 | 5.0 | 598.3 |
| 82 | " | " | 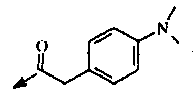 | 88.6 | 4.0 | 607.4 |
| 83 | " | " | 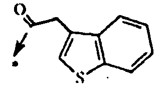 | 90.08 | 5.1 | 620.3 |
| 84 | " | " | 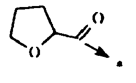 | 85.57 | 4.0 | 544.3 |
| 85 | " | " | 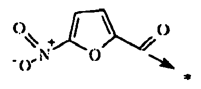 | 48.41 | 4.5 | 585.3 |
| 86 | " | 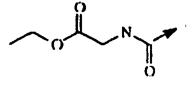 | 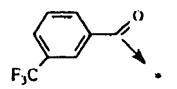 | 82.68 | 6.1 | 589.3 |
| 87 | " | " | 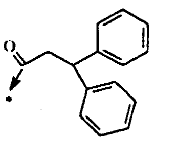 | 79.99 | 6.5 | 611.4 |
| 88 | " | " | 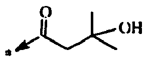 | 86.07 | 4.8 | 503.4 |
| 89 | " | " | 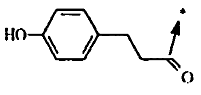 | 82 | 5.1 | 551.4 |
| 90 | " | " | 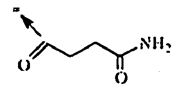 | 19.44 | 4.4 | 502.4 |
| 91 | " | " | 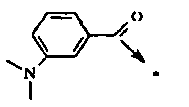 | 86.48 | 5.1 | 550.4 |
| 92 | " | " | 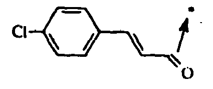 | 80 | 6.3 | 567.3 |
| 93 | " | 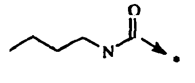 | 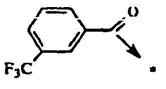 | 94.62 | 6.6 | 559.3 |
| 94 | " | " | 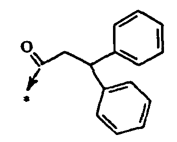 | 57.01 | 6.9 | 581.4 |
| 95 | " | " | 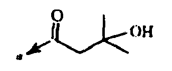 | 92 | 5.2 | 473.4 |
| 96 | " | " | 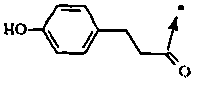 | 87.4 | 5.6 | 521.4 |
| 97 | " | " | 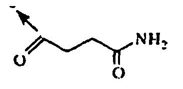 | 20.99 | 5.0 | 472.4 |
| 98 | " | " | 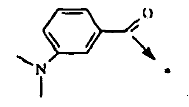 | 88.63 | 5.7 | 520.4 |
| 99 | " | " | 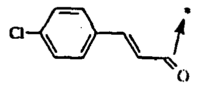 | 84 | 6.7 | 537.3 |
| 100 | " | 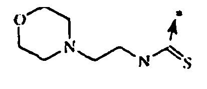 | 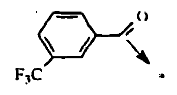 | 89.71 | 5.2 | 632.3 |
| 101 | " | " | 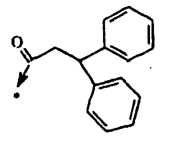 | 90.25 | 5.5 | 654.4 |
| 102 | " | " | 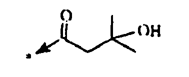 | 90.09 | 4.0 | 546.4 |
| 103 | " | " | 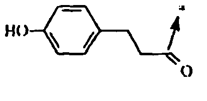 | 71 | 4.4 | 594.3 |
| 104 | " | " | 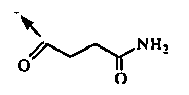 | 37.19 | 3.8 | 545.3 |
| 105 | " | | 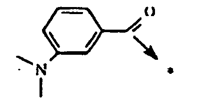 | 76.55 | 4.5 | 593.4 |
| 106 | 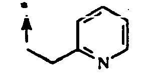 | 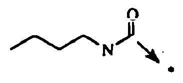 | 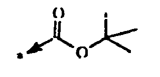 | 69.62 | 59 | 405.2 |
| 107 | 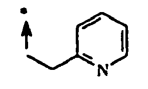 | 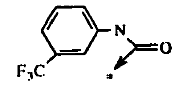 | " | 98 | 7.1 | 493.2 |
| 108 | 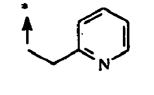 | 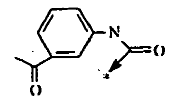 | " | 80 | 6.0 | 467.3 |
| 109 | 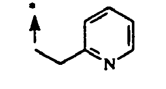 | 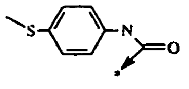 | " | 88 | 6.5 | 471.2 |
| 110 | 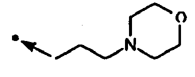 | 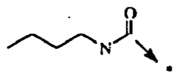 | | 60.04 | 5.7 | 427.3 |
| 111 | 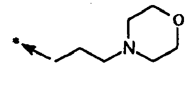 | 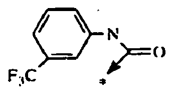 | " | 78 | 6.5 | 515.2 |
| 112 | 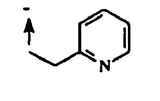 | 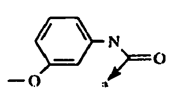 | " | 97 | 6.2 | 455.2 |
| 113 | 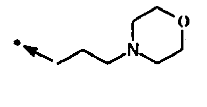 | 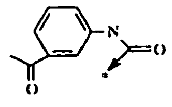 | | 70 | 5.7 | 489.3 |
| 114 | 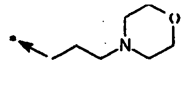 | 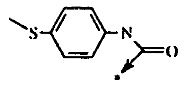 | " | 90 | 6.2 | 493.3 |
| 115 | 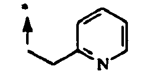 | 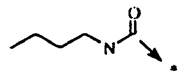 | 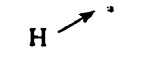 | 62.88 | 3.6 | 305.3 |
| 116 | 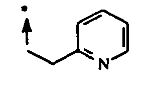 | 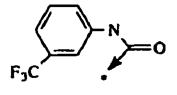 | " | 82,99 | 4.7 | 393.2 |
| 117 | 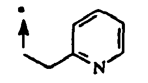 | 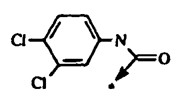 | " | 74.42 | 5.0 | 393.1 |
| 118 | 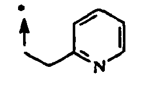 | 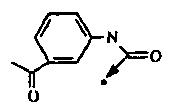 | " | 10.53 | 5.4 | 367.3 |
| 119 | 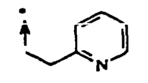 | 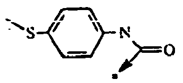 | " | 74.79 | 4.3 | 371.2 |
| 120 | 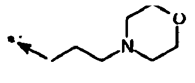 | 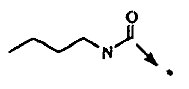 | " | 50.14 | 3.4 | 327.3 |
| 121 | 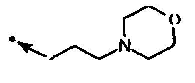 | 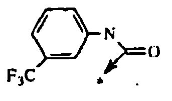 | " | 70 | 4.3 | 415.2 |
| 122 | 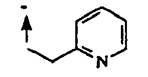 | 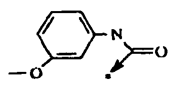 | " | 84 | 3.9 | 355.3 |
| 123 | 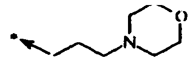 | 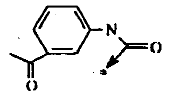 | " | 66 | 3.5 | 389.3 |
| 124 | 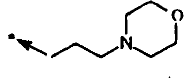 | 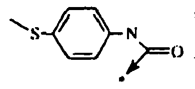 | " | 94.61 | 3.9 | 393.2 |
| 125 | 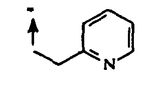 | 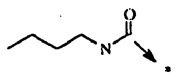 | 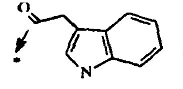 | 71 | 5.5 | 462.3 |
| 126 | 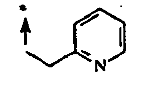 | 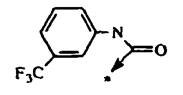 | " | 52 | 6.6 | 550.2 |
| 127 | 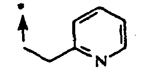 | 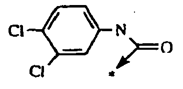 | " | 57 | 6.8 | 550.1 |
| 128 | 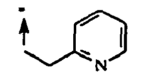 | 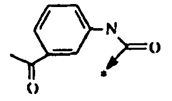 | " | 60 | 5.6 | 524.2 |
| 129 | 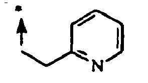 | 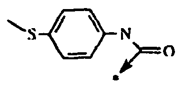 | " | 64 | 6.1 | 528.2 |
| 130 | 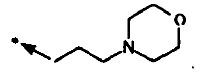 | 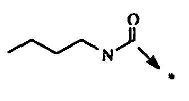 | " | 27 | 5.4 | 484.3 |
| 131 | 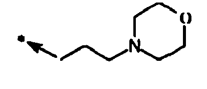 | 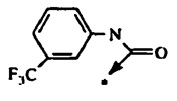 | " | 51 | 6.2 | 572.2 |
| 132 | 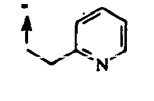 | 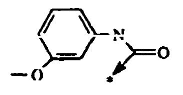 | " | 73 | 5.7 | 512.2 |
| 133 | 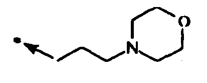 | 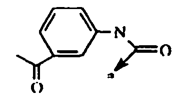 | " | 61 | 5.4 | 546.2 |
| 134 | 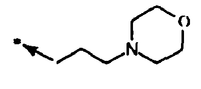 | 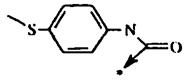 | " | 43 | 5.8 | 550.2 |
| 135 | 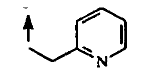 | 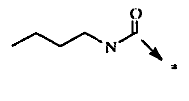 | 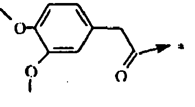 | 76 | 5.3 | 483.3 |
| 136 | 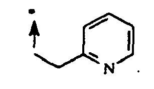 | 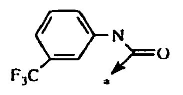 | " | 49 | 6.4 | 571.2 |
| 137 | 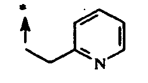 | 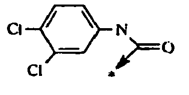 | " | 63 | 6.6 | 571.1 |
| 138 | 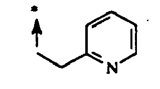 | 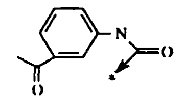 | " | 79 | 5.4 | 545.2 |
| 139 | 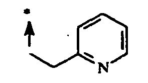 | 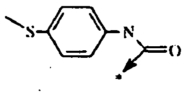 | " | 57 | 5.9 | 549.2 |
| 140 | 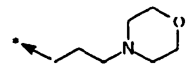 | 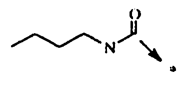 | " | 66.58 | 5.2 | 505.3 |
| 141 | 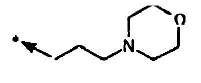 | 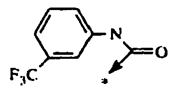 | " | 61 | 6.0 | 593.2 |
| 142 | 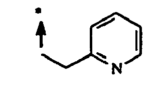 | 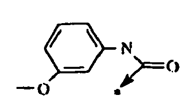 | " | 67 | 5.5 | 533.2 |
| 143 | 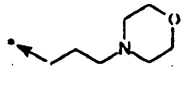 | 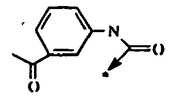 | " | 61 | 5.2 | 567.3 |
| 144 | 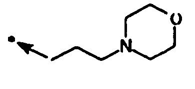 | 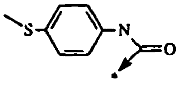 | " | 51 | 5.6 | 571.2 |
| 145 | 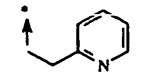 | 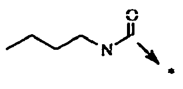 | 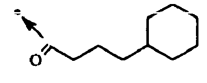 | 56 | 7.0 | 457.3 |
| 146 | 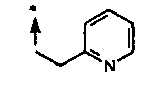 | 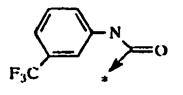 | " | 64 | 8.1 | 545.2 |
| 147 | 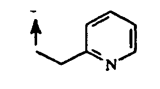 | 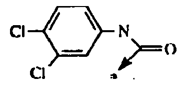 | " | 52 | 8.3 | 545.2 |
| 148 | 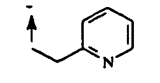 | 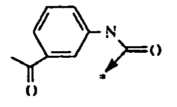 | " | 69 | 7.1 | 519.3 |
| 149 | 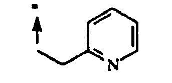 | 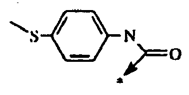 | " | 70 | 7.6 | 523.3 |
| 150 | 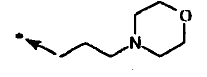 | 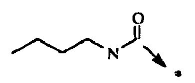 | " | 63.77 | 6.7 | 479.4 |
| 151 | 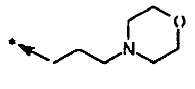 | 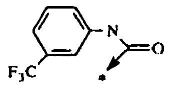 | " | 50 | 7.3 | 567.3 |
| 152 | 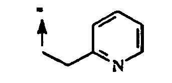 | 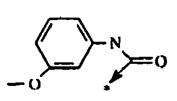 | " | 46 | 7.3 | 507.3 |
| 153 | 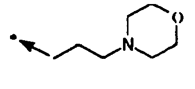 | 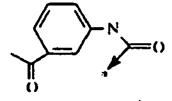 | " | 78 | 6.7 | 541.3 |
| 154 | 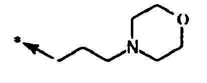 | 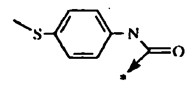 | " | 66 | 7.0 | 545.3 |
| 155 | 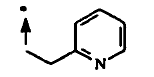 | 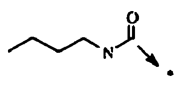 | 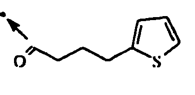 | 68 | 6.0 | 457.2 |
| 156 | 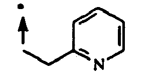 | 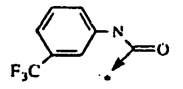 | " | 65 | 7.1 | 545.2 |
| 157 | 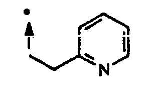 | 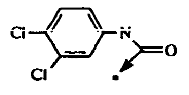 | " | 67 | 7.3 | 545.1 |
| 158 | 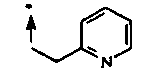 | 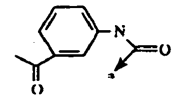 | " | 66 | 6.1 | 519.2 |
| 159 | 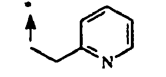 | 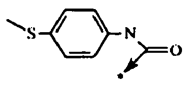 | " | 77 | 6.6 | 523,2 |
| 160 | 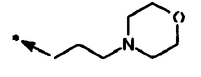 | 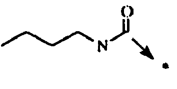 | " | 60.49 | 5.8 | 479.3 |
| 161 | 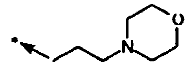 | 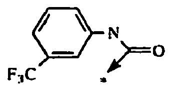 | " | 60 | 6.6 | 567.3 |
| 162 | 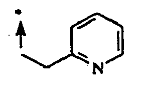 | 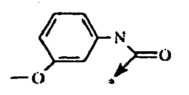 | " | 69 | 6.2 | 507.2 |
| 163 | 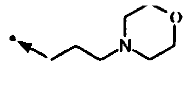 | 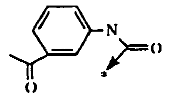 | " | 50 | 5.8 | 541.2 |
| 164 | 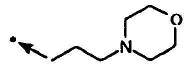 | 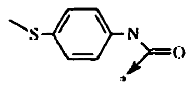 | " | 49 | 6.2 | 545.2 |
| 165 | 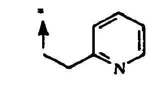 | 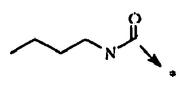 | 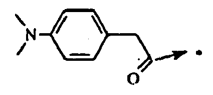 | 67 | 4.4 | 466.3 |
| 166 | 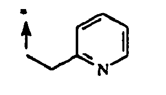 | 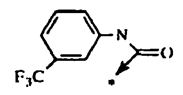 | " | 45 | 5.5 | 554.2 |
| 167 | 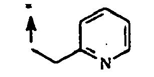 | 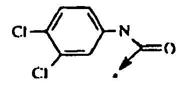 | " | 65.89 | 5.7 | 554.1 |
| 168 | 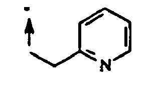 | 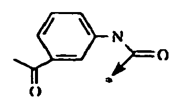 | " | 5 | 5.4 | 528.2 |
| 169 | 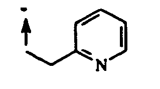 | 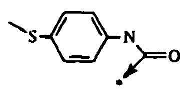 | " | 64.08 | 5.0 | 532.2 |
| 170 | 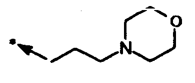 | 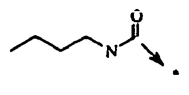 | " | 62.51 | 4.3 | 488.3 |
| 171 | 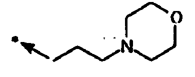 | 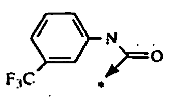 | " | 55 | 5.2 | 576.3 |
| 172 | 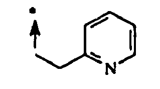 | 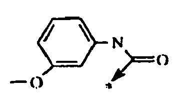 | " | 50.35 | 4.7 | 516.3 |
| 173 | 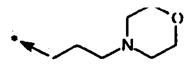 | 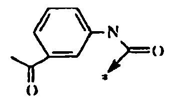 | " | 7 | 5.2 | 550.3 |
| 174 | 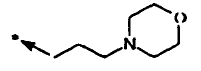 | 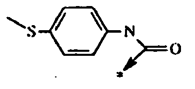 | " | 48.63 | 4.8 | 554.3 |
| 175 | 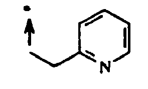 | 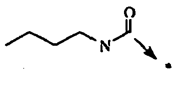 | 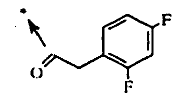 | 53 | 5.7 | 459.2 |
| 176 | 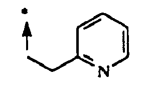 | 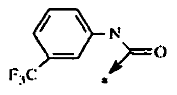 | " | 49 | 6.9 | 547.2 |
| 177 | 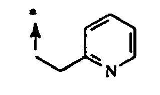 | 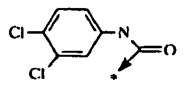 | " | 61 | 7.1 | 547.1 |
| 178 | 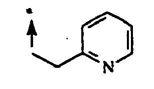 | 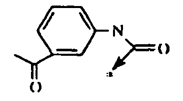 | " | 57 | 5.9 | 521.2 |
| 179 | 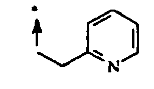 | 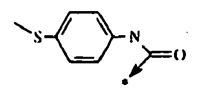 | " | 65 | 6.4 | 525.2 |
| 180 | 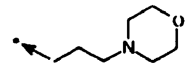 | 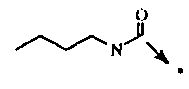 | | 88.99 | 5.6 | 481.3 |
| 181 | 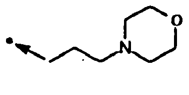 | 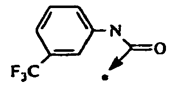 | " | 58 | 6.4 | 569.2 |
| 182 | 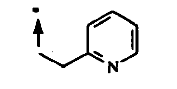 | 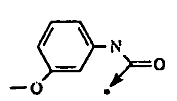 | " | 64 | 6.0 | 509.2 |
| 183 | 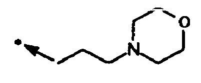 | 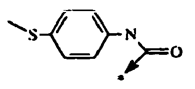 | " | 63 | 6.0 | 547.2 |
| 184 | 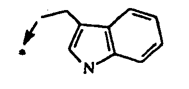 | 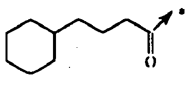 | 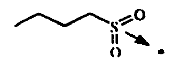 | 67.83 | 10.1 | 516.3 |
| 185 | 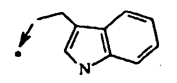 | 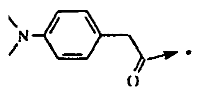 | " | 61.66 | 6.7 | 525.3 |
| 186 | 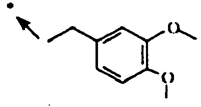 | 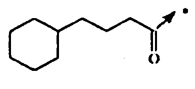 | " | 40,48 | 9.9 | 537.3 |
| 187 | 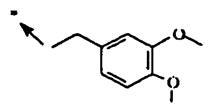 | 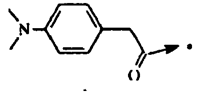 | " | 50 | 6.4 | 546.3 |
| 188 | 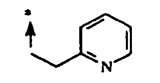 | 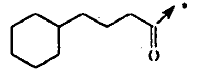 | " | 42.57 | 7.4 | 478.4 |
| 189 | 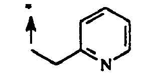 | 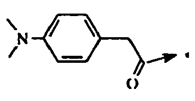 | " | 29 | 4.8 | 487.3 |
| 190 | 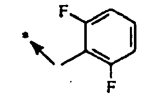 | 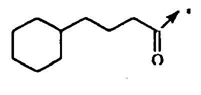 | " | 55 | 10.3 | 499.3 |
| 191 | 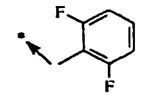 | 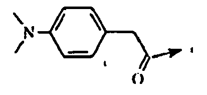 | " | 19.39 | 6.7 | 508.3 |
| 192 | 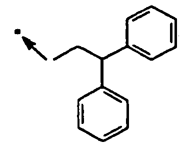 | 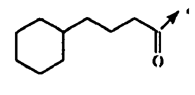 | " | 67 | 11.1 | 567.3 |
| 193 | 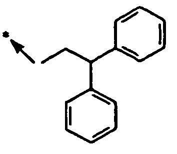 | 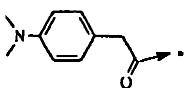 | " | 64,73 | 7.9 | 576.3 |
| 194 | 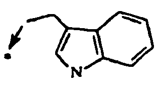 | 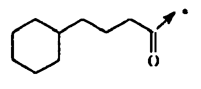 | 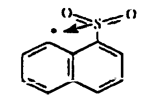 | 92 | 10.6 | 586.3 |
| 195 | 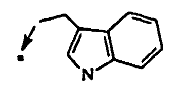 | 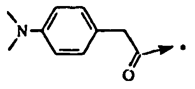 | " | 85 | 7.3 | 595.3 |
| 196 | 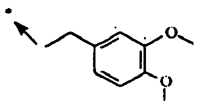 | 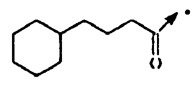 | " | 96 | 10.5 | 607.3 |
| 197 | 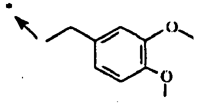 | 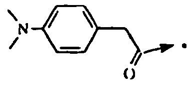 | " | 89.25 | 7.2 | 616.3 |
| 198 | 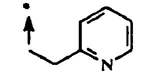 | 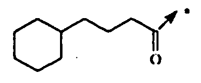 | " | 98.24 | 7.9 | 548.3 |
| 199 | 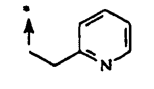 | 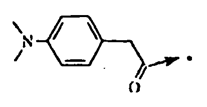 | " | 94 | 5.6 | 557.3 |
| 200 | 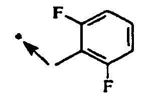 | 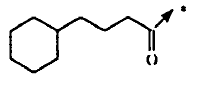 | " | 98 | 10.8 | 569.2 |
| 201 | 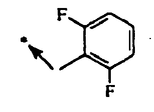 | 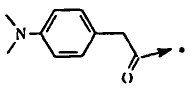 | " | 93,17 | 7,3 | 578.2 |
| 202 | 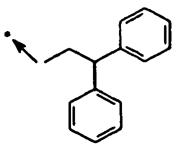 | 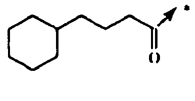 | " | 97.82 | 11.7 | 637.3 |
| 203 | 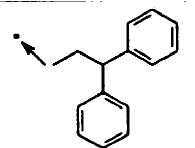 | 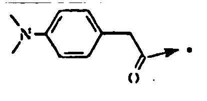 | " | 88.11 | 8.5 | 646.3 |
| 204 | 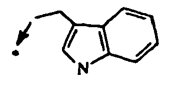 | 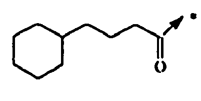 | 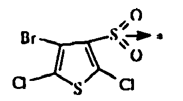 | 73 | 11.2 | 690.0 |
| 205 | 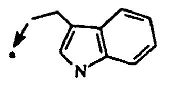 | 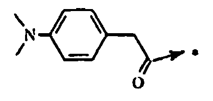 | " | 60.44 | 7.9 | 699.0 |
| 206 | 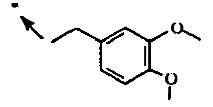 | 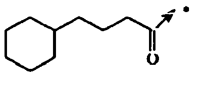 | " | 76 | 11.1 | 711.0 |
| 207 | 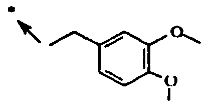 | 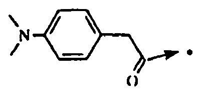 | " | 72.2 | 7.8 | 720.0 |
| 208 | 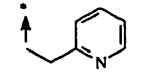 | 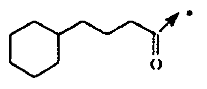 | " | 89.42 | 8.5 | 652 |
| 209 | 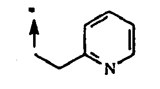 | 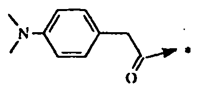 | " | 48 | 6.2 | 659.0 |
| 210 | 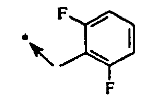 | 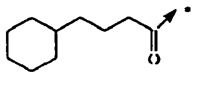 | " | 78,2 | 11.6 | 673.0 |
| 211 | 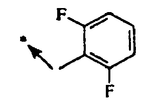 | 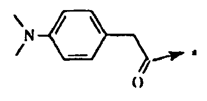 | " | 66.1 | 7.9 | 682.0 |
| 212 | 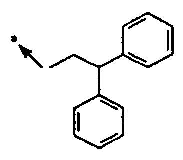 | 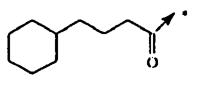 | " | 78 | 12.6 | 739.1 |
| 213 | 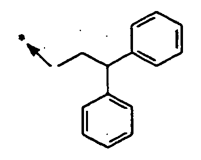 | 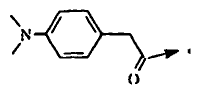 | " | 88.77 | 9.1 | 750.0 |
| 214 | 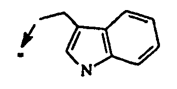 | 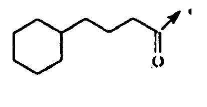 | 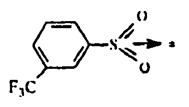 | 73 | 10.6 | 604.3 |
| 215 | 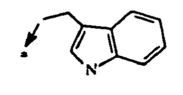 | 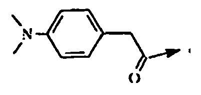 | " | 67 | 7.5 | 613.2 |
| 216 | 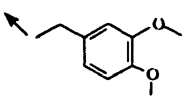 | 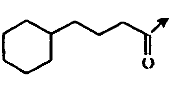 | " | 73 | 10.5 | 625.3 |
| 217 | 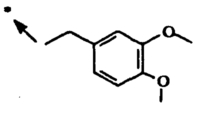 | 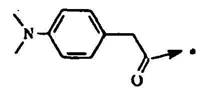 | " | 83 | 7.3 | 634.2 |
| 218 | 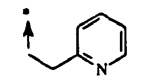 | 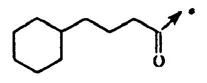 | " | 87.32 | 7.9 | 566.3 |
| 219 | 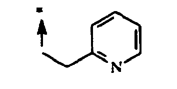 | 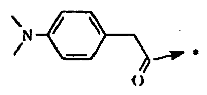 | | 79 | 5.7 | 575.2 |
| 220 | 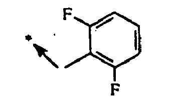 | 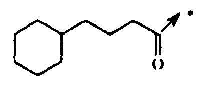 | " | 89 | 10.7 | 587.2 |
| 221 | 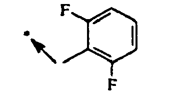 | 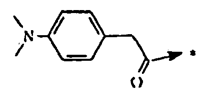 | " | 78.75 | 7.4 | 596.2 |
| 222 | 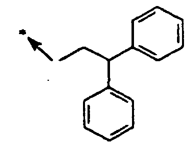 | 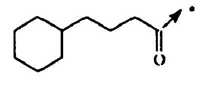 | " | 95 | 11.6 | 655.3 |
| 223 | 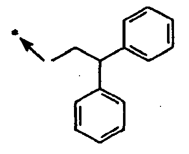 | 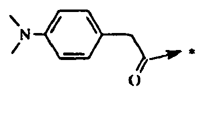 | " | 79 | 8.6 | 664.3 |
| 224 | 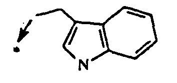 | 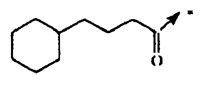 | 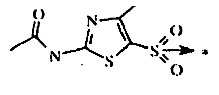 | 58 | 9.4 | 614,2 |
| 225 | 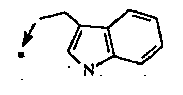 | 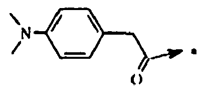 | " | 78 | 6.4 | 623,2 |
| 226 | 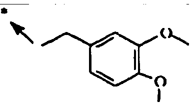 | 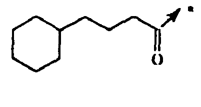 | " | 75 | 9.2 | 635.3 |
| 227 | 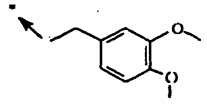 | 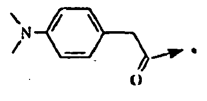 | " | 88 | 6.1 | 644.3 |
| 228 | 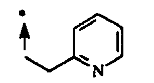 | 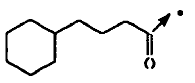 | " | 86 | 6.7 | 576.3 |
| 229 | 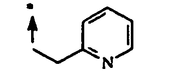 | 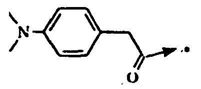 | " | 80 | 4.6 | 585.2 |
| 230 | 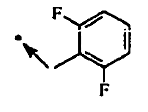 | 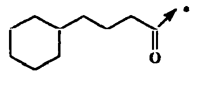 | " | 73 | 9.5 | 597.2 |
| 231 | 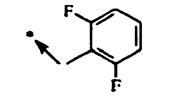 | 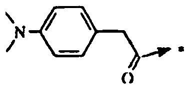 | " | 66 | 6.2 | 606.2 |
| 232 | 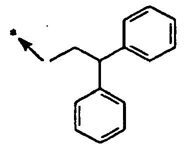 | 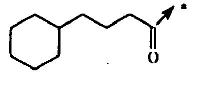 | " | 62 | 10.5 | 665.3 |
| 233 | 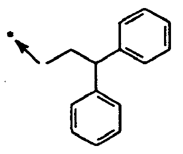 | 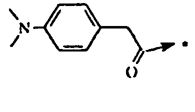 | " | 81 | 7.5 | 674.3 |
| 234 | 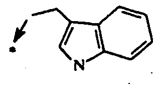 | 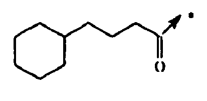 | 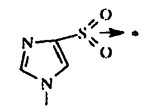 | 92 | 8.9 | 540.3 |
| 235 | 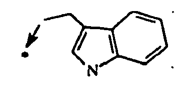 | 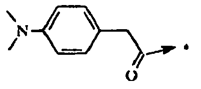 | " | 86 | 5.6 | 549.2 |
| 236 | 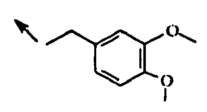 | 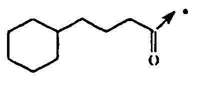 | " | 91 | 8.7 | 561.3 |
| 237 | 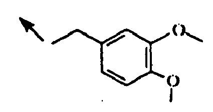 | 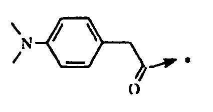 | " | 94.51 | 5.4 | 570.2 |
| 238 | 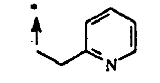 | 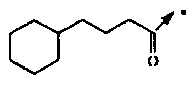 | " | 93.36 | 6.2 | 502.3 |
| 239 | 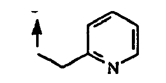 | 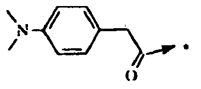 | " | 97 | 3.8 | 511.3 |
| 240 | 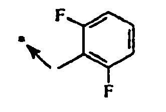 | 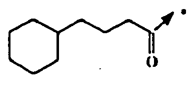 | " | 98.13 | 9.0 | 523.3 |
| 241 | 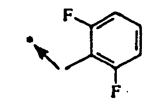 | 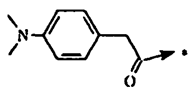 | " | 82 | 5.4 | 532.2 |
| 242 | 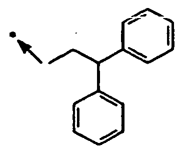 | 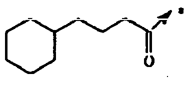 | " | 99 | 10.1 | 591.3 |
| 243 | 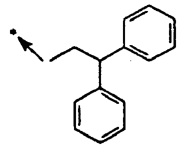 | 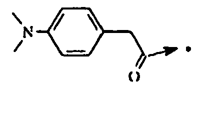 | " | 94.74 | 6.8 | 600.3 |
| 244 | 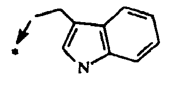 | 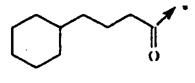 | 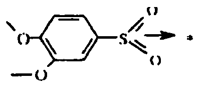 | 89 | 9.8 | 596.3 |
| 245 | 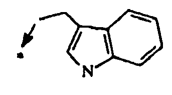 | 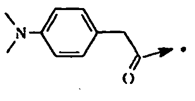 | " | 81 | 6.6 | 605.3 |
| 246 | 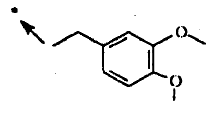 | 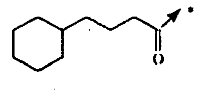 | " | 96 | 9.7 | 617.3 |
| 247 | 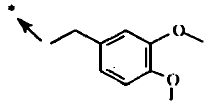 | 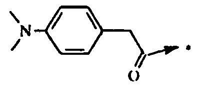 | " | 85.68 | 6.4 | 626.3 |
| 248 | 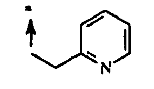 | 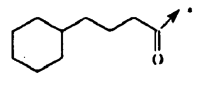 | " | 98.65 | 7.1 | 558.3 |
| 249 | 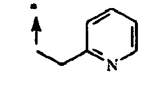 | 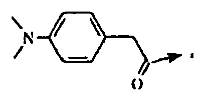 | " | 92 | 4.8 | 567.2 |
| 250 | 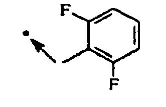 | 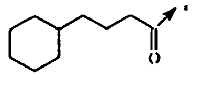 | " | 96 | 10.0 | 579.2 |
| 251 | 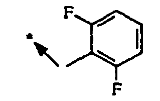 | 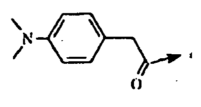 | " | 88.12 | 6.5 | 588.2 |
| 252 | 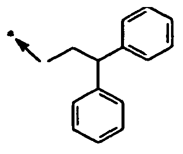 | 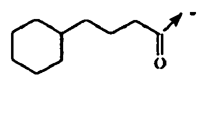 | " | 97 | 10.9 | 647.3 |
| 253 | 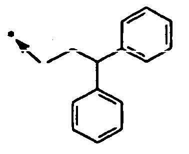 | 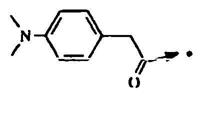 | " | 86 | 7.8 | 656.3 |
| 254 | 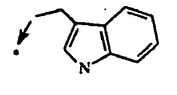 | 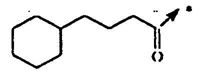 | 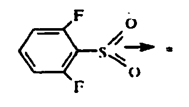 | 79 | 10.1 | 572.2 |
| 255 | 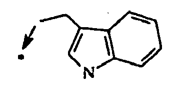 | 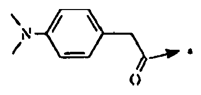 | " | 79 | 7.0 | 581.2 |
| 256 | 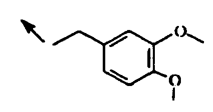 | 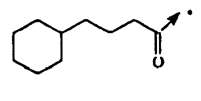 | " | 71 | 10.0 | 593.3 |
| 257 | 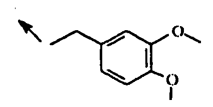 | 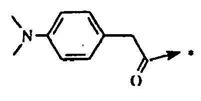 | " | 72.74 | 6.6 | 602.2 |
| 258 | 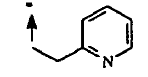 | 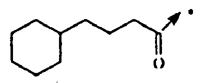 | " | 79.1 | 7.4 | 534.3 |
| 259 | 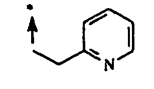 | 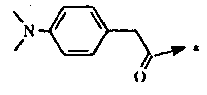 | " | 74 | 4.9 | 543.2 |
| 260 | 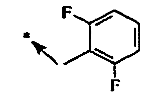 | 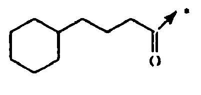 | " | 84.17 | 10.3 | 555.2 |
| 261 | 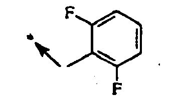 | 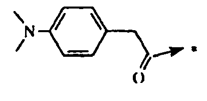 | " | 76.16 | 6.7 | 564.2 |
| 262 | 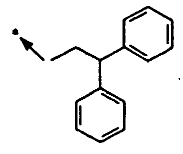 | 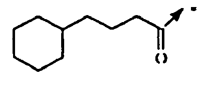 | " | 95 | 11.1 | 623.3 |
| 263 | 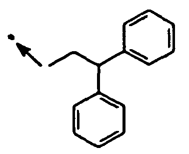 | 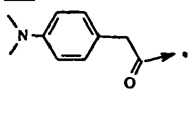 | " | 78.91 | 8.0 | 632.3 |
| 264 | 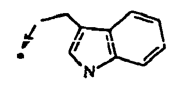 | 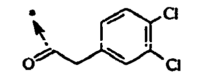 | 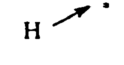 | 75.26 | 5.1 | 430.2 |
| 265 | " | 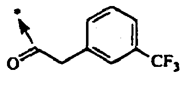 | " | 90.43 | 5.0 | 430.3 |
| 266 | " | 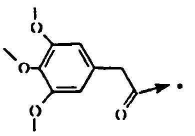 | " | 74.93 | 4.3 | 452.3 |
| 267 | " | 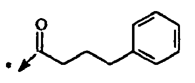 | " | 79.62 | 4.9 | 390.3 |
| 268 | " | 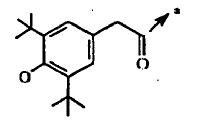 | " | 92.82 | 5.6 | 490.4 |
| 269 | " | 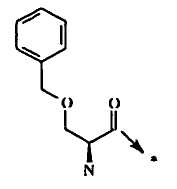 | " | 68.87 | 3.6 | 421.3 |
| 270 | " | 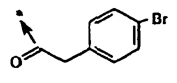 | " | 79.07 | 4.9 | 440.2 |
| 271 | " | 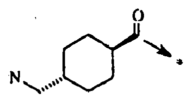 | " | 84.22 | 3.0 | 392.3 |
| 272 | " | 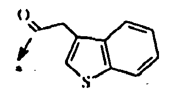 | " | 67.34 | 4.9 | 418.2 |
| 273 | " | 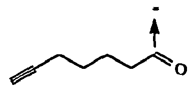 | " | 81.63 | 4.4 | 352.3 |
| 274 | " | 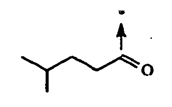 | " | 90.11 | 4.7 | 342.3 |
| 275 | " | 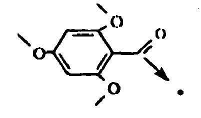 | " | 54.36 | 4.3 | 439.3 |
| 276 | " | 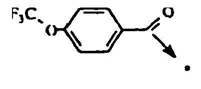 | " | 81.69 | 4.9 | 432.2 |
| 277 | " | 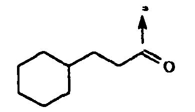 | " | 85.62 | 5.2 | 382.3 |
| 278 | " | 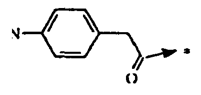 | " | 86.19 | 3.2 | 377.3 |
| 279 | 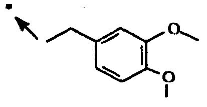 | 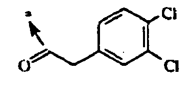 | " | 94.76 | 4.9 | 451,2 |
| 280 | " | 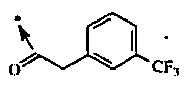 | " | 99.42 | 4.7 | 451.3 |
| 281 | " | 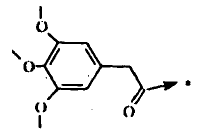 | " | 90.55 | 4.0 | 473.3 |
| 282 | " | 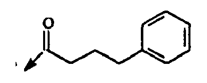 | " | 93,80 | 4.6 | 411.3 |
| 283 | " | 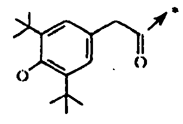 | " | 82.71 | 5.4 | 511.4 |
| 284 | " | 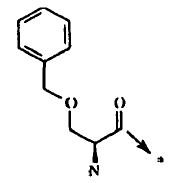 | " | 90.85 | 3,4 | 442.3 |
| 285 | " | 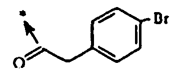 | " | 98.65 | 4.6 | 461.2 |
| 286 | " | 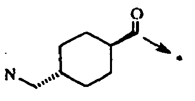 | " | 98.80 | 2.8 | 404.3 |
| 287 | " | 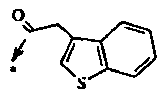 | " | 86.02 | 4.6 | 439.3 |
| 288 | " | 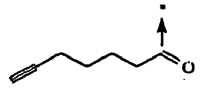 | " | 97.47 | 4.1 | 373.3 |
| 289 | " | 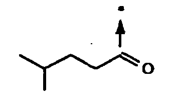 | " | 99.31 | 4.4 | 363.3 |
| 290 | " | 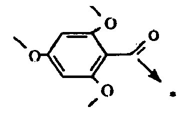 | " | 45.77 | 4.1 | 459.3 |
| 291 | " | 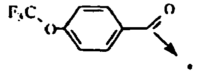 | " | 94.07 | 4.6 | 453.3 |
| 292 | " | 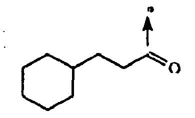 | " | 95.65 | 5.0 | 403.4 |
| 293 | " | 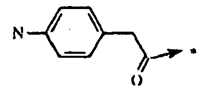 | " | 94.30 | 2.9 | 398.3 |
| 294 | 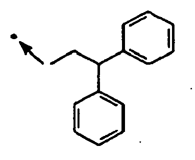 | 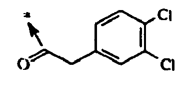 | " | 80.64 | 5.9 | 481.2 |
| 295 | " | 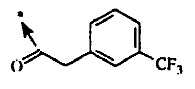 | " | 98.05 | 5.7 | 481.3 |
| 296 | " | 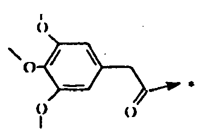 | " | 94.93 | 5.0 | 503.4 |
| 297 | " | 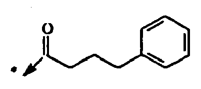 | " | 96.81 | 5.6 | 441.3 |
| 298 | " | 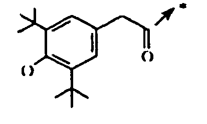 | " | 95.00 | 6.3 | 541.4 |
| 299 | " | 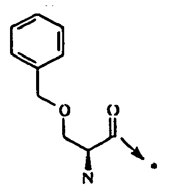 | " | 95.13 | 4.2 | 472.4 |
| 300 | " | 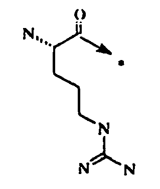 | " | 52.68 | 3.2 | 452.4 |
| 301 | " | 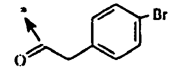 | " | 98.03 | 5.6 | 491.2 |
| 302 | " | 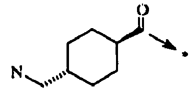 | " | 96.44 | 3.7 | 217.9 |
| 303 | " | 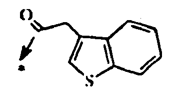 | " | 97.22 | 5.6 | 469.3 |
| 304 | " | 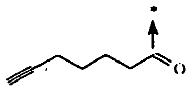 | " | 96.97 | 5.2 | 403.3 |
| 305 | " | 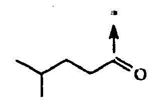 | " | 99.05 | 5.4 | 393.4 |
| 306 | " | 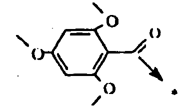 | " | 32.67 | 5.1 | 489.3 |
| 307 | " | 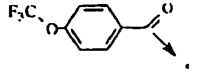 | " | 84.51 | 5.6 | 483.3 |
| 308 | " | 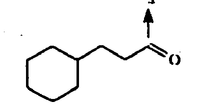 | " | 98.44 | 6.0 | 433.4 |
| 309 | " | 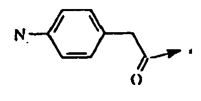 | " | 97.78 | 4.0 | 428.3 |
| 310 | 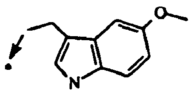 | 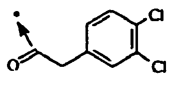 | " | 79.54 | 5.0 | 460.2 |
| 311 | " | 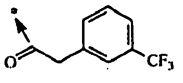 | " | 78.59 | 4.9 | 460.3 |
| 312 | " | 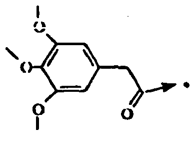 | " | 66.24 | 4.2 | 482.3 |
| 313 | " | 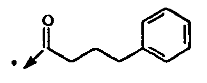 | " | 70.15 | 4.8 | 420.3 |
| 314 | " | 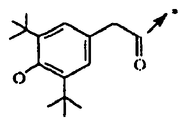 | " | 57,87 | 5.5 | 520.4 |
| 315 | " | 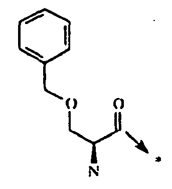 | " | 71.26 | 3.6 | 451.3 |
| 316 | " | 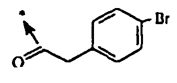 | " | 81.16 | 4.8 | 470.2 |
| 317 | " | 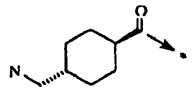 | " | 74.96 | 2.9 | 413.3 |
| 318 | " | 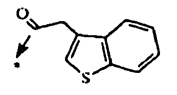 | " | 53.47 | 4.8 | 448.3 |
| 319 | " | 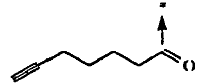 | " | 87.88 | 4.3 | 382.3 |
| 320 | " | 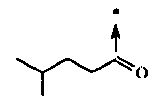 | " | 91,41 | 4.6 | 372.3 |
| 321 | " | 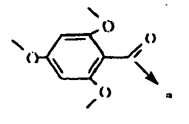 | " | 1.59 | 5.0 | 468.3 |
| 322 | " | 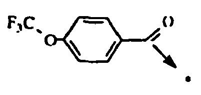 | " | 77.81 | 4.8 | 462.3 |
| 323 | " | 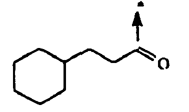 | " | 76.59 | 5.1 | 412.3 |
| 324 | " | 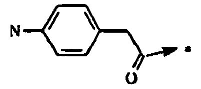 | " | 83.35 | 3.1 | 407.3 |
| 325 | 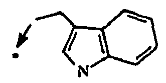 | 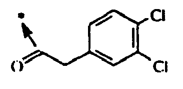 | 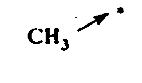 | 87.42 | 5.2 | 444.2 |
| 326 | " | 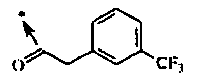 | " | 98.89 | 5.1 | 444.3 |
| 327 | " | 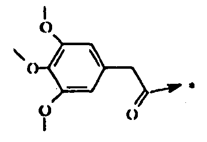 | " | 95.68 | 4.3 | 466.3 |
| 328 | " | 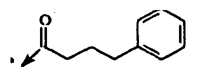 | " | 97.27 | 4.9 | 404.3 |
| 329 | " | 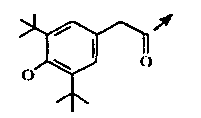 | " | 95.73 | 5.7 | 504.4 |
| 330 | | 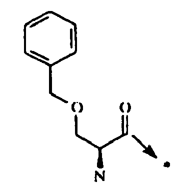 | " | 83.37 | 3.7 | 435.3 |
| 331 | " | 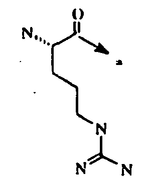 | " | 71.88 | 3.2 | 413.3 |
| 332 | " | 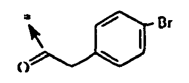 | " | 98.33 | 5.0 | 454.2 |
| 333 | " | 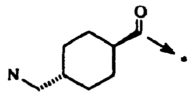 | " | 83.73 | 3.0 | 397.3 |
| 334 | " | 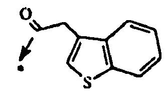 | " | 94.77 | 5.0 | 432.3 |
| 335 | " | 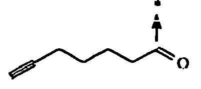 | " | 95.88 | 4.5 | 366.3 |
| 336 | " | 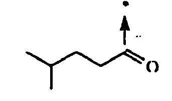 | " | 98.9 | 4.7 | 356.3 |
| 337 | " | 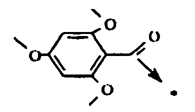 | " | 50,74 | 4.4 | 452.3 |
| 338 | " | 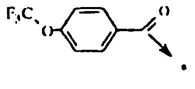 | " | 95.39 | 5.0 | 446.3 |
| 339 | " | 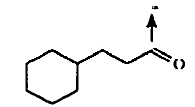 | " | 98.2 | 5.3 | 396.3 |
| 340 | " | 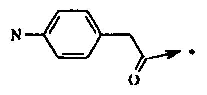 | " | 92.35 | 3.2 | 391.3 |
| 341 | 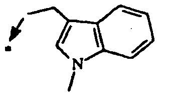 | 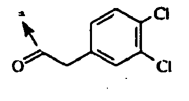 | 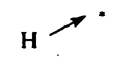 | 90.41 | 5.1 | 444.2 |
| 342 | " | 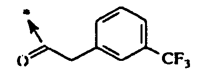 | " | 87.41 | 5.0 | 444.3 |
| 343 | " | 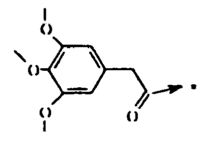 | " | 87,37 | 4.3 | 466.3 |
| 344 | " | 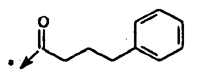 | " | 83.01 | 4.9 | 404.3 |
| 345 | " | 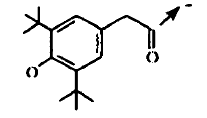 | " | 89.47 | 5.6 | 504.4 |
| 346 | " | 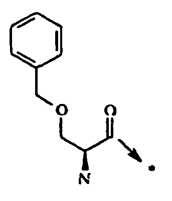 | " | 77.55 | 3.6 4 | 35.3 |
| 347 | " | 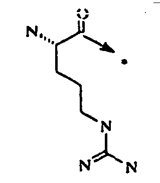 | " | 49.49 | 2.4 | 414.3 |
| 348 | " | 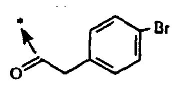 | " | 85.63 | 4,9 | 454.2 |
| 349 | " | 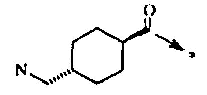 | " | 88.12 | 2.9 | 397.3 |
| 350 | " | 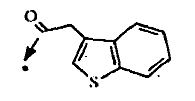 | " | 87.73 | 4.9 | 432.3 |
| 351 | " | 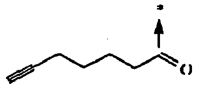 | " | 84.48 | 4.4 | 366.3 |
| 352 | " | 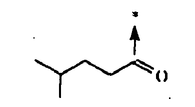 | " | 82.03 | 4.7 | 356.3 |
| 353 | " | 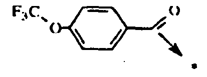 | " | 82.93 | 4.9 | 446.3 |
| 354 | " | 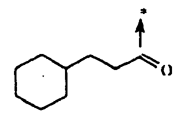 | " | 72.6 | 5.3 | 396.3 |
| 355 | " | 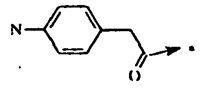 | " | 86.75 | 3.2 | 391.3 |
| 356 | 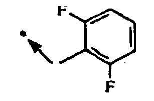 | 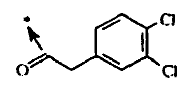 | " | 93.75 | 4.7 | 413.1 |
| 357 | " | 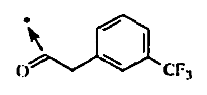 | " | 96.13 | 4.6 | 413.2 |
| 358 | " | 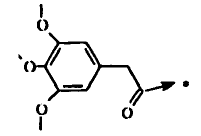 | " | 98.3 | 3.8 | 435.2 |
| 359 | " | 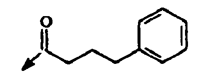 | " | 96.45 | 4.5 | 373.2 |
| 360 | " | 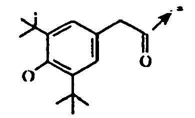 | " | 97.9 | 5.3 | 473.4 |
| 361 | " | 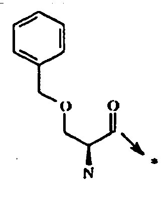 | " | 97.57 | 3.0 | 404.3 |
| 362 | " | 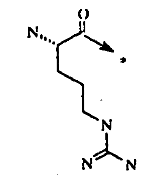 | " | 78.0 | 2.5 | 383.2 |
| 363 | " | 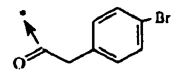 | " | 98.96 | 4.5 | 423.1 |
| 364 | " | 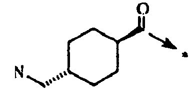 | " | 93.98 | 2.4 | 366.3 |
| 365 | " | 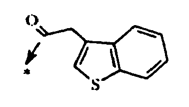 | " | 97.98 | 4.5 | 401.2 |
| 366 | " | 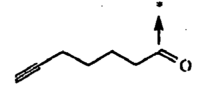 | " | 93.33 | 4.0 | 335.2 |
| 367 | " | 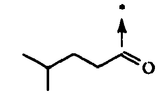 | " | 95.73 | 4.3 | 325.3 |
| 368 | " | 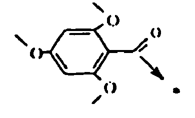 | " | 1.21 | 3.9 | 421.3 |
| 369 | " | 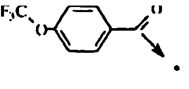 | " | 88.55 | 4.6 | 415.2 |
| 370 | " | 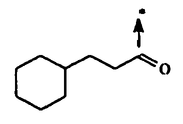 | " | 95.93 | 4.9 | 365.3 |
| 371 | " | 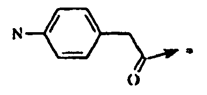 | " | 99.1 | 2.6 | 360.2 |
| 372 | 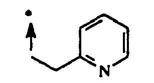 | 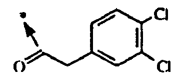 | " | 90.59 | 3,4 | 392.1 |
| 373 | " | 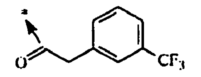 | " | 93.57 | 3.3 | 392.2 |
| 374 | " | 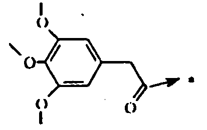 | " | 97.23 | 2.6 | 414.2 |
| 375 | " | 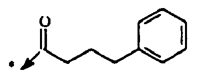 | " | 93.83 | 3.1 | 352.3 |
| 376 | " | 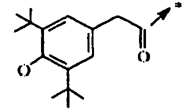 | " | 96.81 | 4.0 | 452.4 |
| 377 | " | 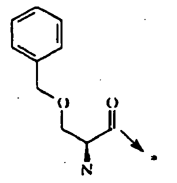 | " | 97.7 | 2.2 | 383.3 |
| 378 | " | 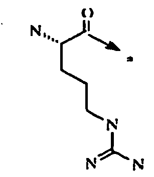 | " | 53,69 | 2.3 | 362.2 |
| 379 | " | 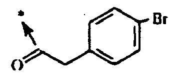 | " | 97.1 | 3.1 | 402.1 |
| 380 | " | 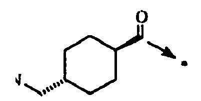 | " | 70.3 | 2.5 | 345.3 |
| 381 | " | 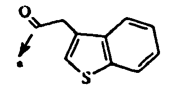 | " | 97.59 | 3.1 | 380.2 |
| 382 | " | 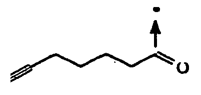 | " | 86,74 | 2.4 | 314.2 |
| 383 | " | 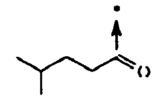 | " | 87.28 | 2.6 | 304.3 |
| 384 | " | 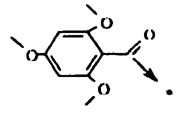 | " | 10.27 | 3.1 | 400.2 |
| 385 | " | 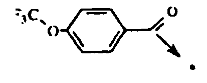 | " | 93.38 | 3.1 | 394.2 |
| 386 | " | 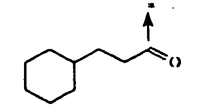 | " | 88.99 | 3.4 | 344.3 |
| 387 | " | 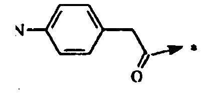 | " | 89.43 | 2.5 | 339.3 |
| 388 | 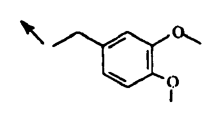 | 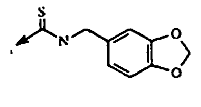 | 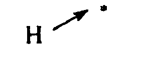 | 86.18 | 4.2 | 458.3 |
| 389 | " | 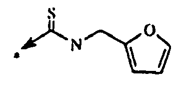 | " | 37.01 | 3.9 | 404.3 |
| 390 | " | 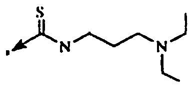 | " | 57.02 | 2.7 | 437.4 |
| 391 | " | 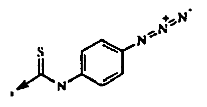 | " | 78.70 | 4.3 | 441.3 |
| 392 | " | 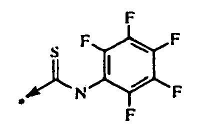 | " | 67.94 | 4.6 | 490.3 |
| 393 | " | 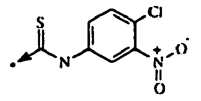 | " | 39.75 | 4.5 | 479.3 |
| 394 | " | 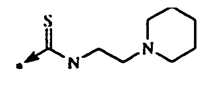 | " | 94.48 | 2.8 | 435.4 |
| 395 | " | 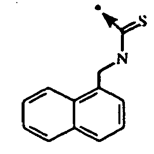 | " | 83.7 | 3.4 | 432.3 |
| 396 | " | 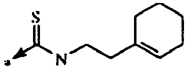 | " | 96.5 | 4.7 | 464.4 |
| 397 | " | 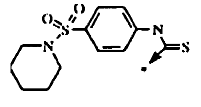 | " | 43.75 | 4.5 | 547.3 |
| 398 | 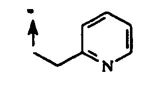 | 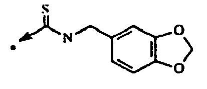 | " | 86.87 | 3.3 | 399.3 |
| 399 | " | 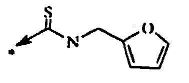 | " | 47.77 | 2.9 | 345.3 |
| 400 | " | 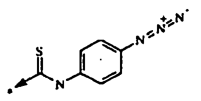 | " | 82 | 3.4 | 382.3 |
| 401 | " | 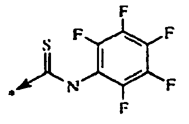 | " | 97.10 | 3.8 | 431.2 |
| 402 | " | 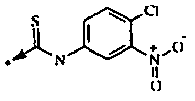 | " | 70.92 | 3.8 | 420.2 |
| 403 | " | 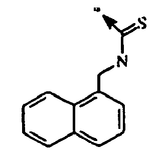 | " | 97.3 | 2.8 | 373.3 |
| 404 | " | 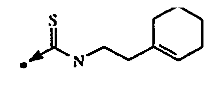 | " | 95.9 | 4.0 | 405.3 |
| 405 | " | 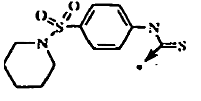 | " | 69.50 | 3.7 | 488.3 |
| 406 | 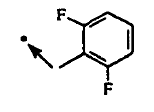 | 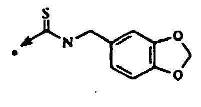 | " | 90.79 | 4.1 | 420.3 |
| 407 | " | 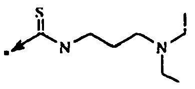 | " | 86.38 | 2.5 | 399.3 |
| 408 | " | 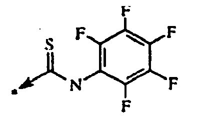 | " | 67.52 | 4.6 | 452.2 |
| 409 | " | 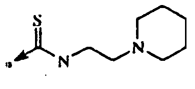 | " | 99.8 | 2.7 | 397.3 |
| 410 | " | 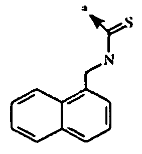 | " | 97.7 | 3.3 | 394.3 |
| 411 | 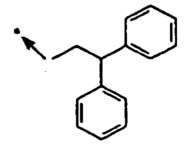 | 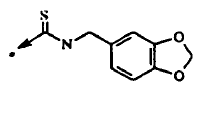 | " | 87.97 | 5.0 | 488.3 |
| 412 | " | 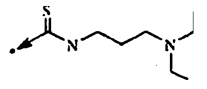 | " | 97.23 | 3.6 | 467.4 |
| 413 | " | 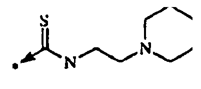 | " | 99.29 | 3.7 | 465.4 |
| 414 | " | 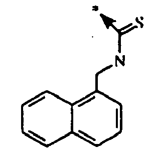 | " | 96.2 | 4.2 | 462.4 |
| 415 | " | 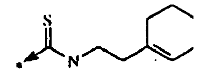 | " | 72.0 | 5.5 | 494.3 |
| 416 | 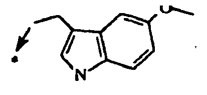 | 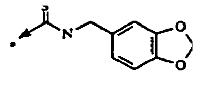 | " | 85.09 | 4.3 | 467.3 |
| 417 | " | 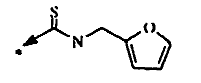 | " | 68.52 | 4.1 | 413.3 |
| 418 | " | 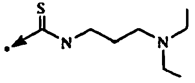 | " | 98.76 | 2.8 | 446.4 |
| 419 | " | 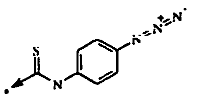 | " | 73.21 | 4.4 | 450.3 |
| 420 | " | 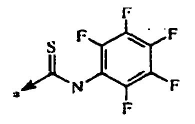 | " | 76.94 | 4.7 | 499.2 |
| 421 | " | 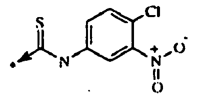 | " | 85.12 | 4.6 | 488.2 |
| 422 | " | 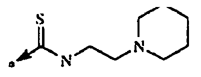 | " | 98.15 | 2.9 | 444.4 |
| 423 | " | 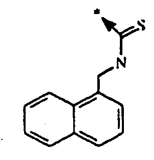 | " | 58 | 5.1 | 477.3 |
| 424 | " | 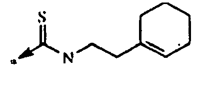 | " | 25 | 3.6 | 410.3 |
| 425 | " | 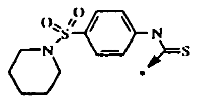 | " | 69.90 | 4.6 | 556.3 |
| 426 | 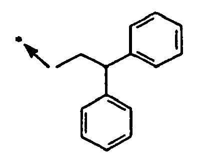 | 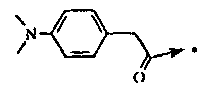 | 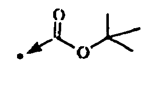 | 90.11 | 8.2 | 556.3 |
| 427 | " | 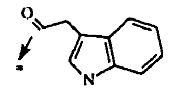 | " | 95.30 | 9.7 | 552.3 |
| 428 | " | 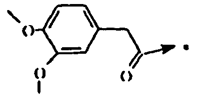 | " | 89.35 | 9.6 | 573.3 |
| 429 | " | 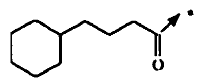 | " | 97.48 | 11.8 | 547.4 |
| 430 | " | 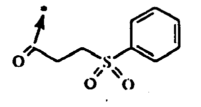 | " | 91.35 | 9.6 | 591.3 |
| 431 | " | 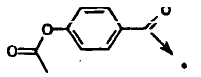 | " | 66.60 | 9.7 | 557.3 |
| 432 | " | 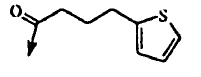 | " | 97.25 | 10.5 | 547.3 |
| 433 | " | 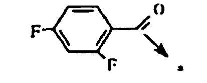 | " | 98.20 | 10.2 | 549.3 |
| 434 | 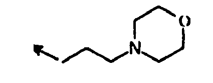 | 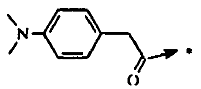 | " | 88.28 | 4.7 | 489.3 |
| 435 | " | 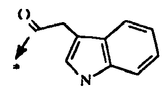 | " | 94.30 | 5.8 | 485.3 |
| 436 | " | 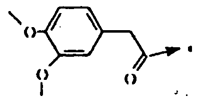 | " | 92.92 | 5.6 | 506.3 |
| 437 | " | 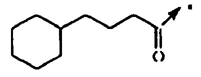 | " | 95.73 | 7.1 | 480.4 |
| 438 | " | 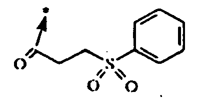 | " | 89.80 | 5.6 | 524.3 |
| 439 | " | 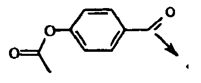 | " | 69.38 | 5.6 | 490.3 |
| 440 | " | 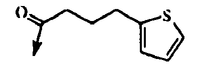 | " | 95.21 | 6.2 | 480.3 |
| 441 | " | 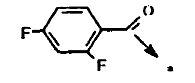 | " | 96,98 | 6.0 | 482.3 |
| 442 | 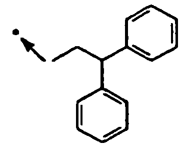 | 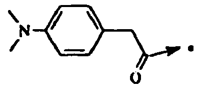 | 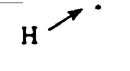 | 85.00 | 5.4 | 456.3 |
| 443 | " | 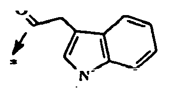 | " | 94.40 | 6.5 | 452.3 |
| 444 | " | 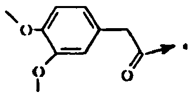 | " | 91.10 | 6.3 | 473.3 |
| 445 | " | 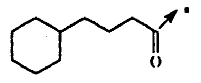 | " | 96.60 | 7.7 | 447.3 |
| 446 | " | 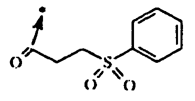 | " | 92.80 | 6.3 | 491.2 |
| 447 | " | 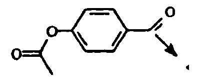 | " | 85.40 | 6.3 | 457.2 |
| 448 | " | 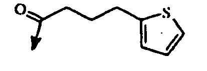 | " | 96.70 | 6.9 | 447.2 |
| 449 | " | 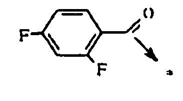 | " | 98 | 6.7 | 449.2 |
| 450 | 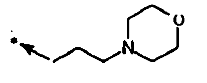 | 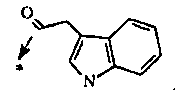 | " | 38.17 | 3.6 | 385.2 |
| 451 | " | 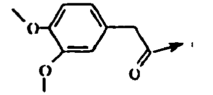 | " | 92.70 | 3.4 | 406.2 |
| 452 | " | 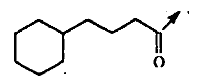 | " | 89.50 | 4.7 | 380.3 |
| 453 | " | 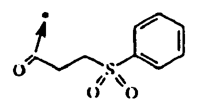 | " | 86.24 | 3.4 | 424.2 |
| 454 | " | 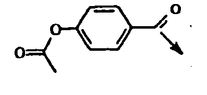 | " | 71.20 | 3.3 | 390.2 |
| 455 | " | 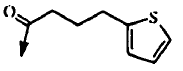 | " | 88.60 | 3.8 | 380.2 |
| 456 | " | 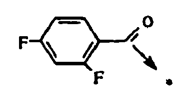 | " | 89.26 | 3.5 | 382.2 |
| 457 | 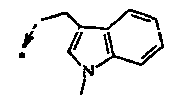 | 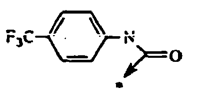 | " | 96.55 | 4,9 | 445.3 |
| 458 | " | 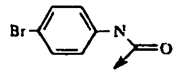 | " | 94.46 | 4.8 | 455.2 |
| 459 | " | 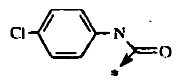 | " | 95.6 | 4.7 | 411.3 |
| 460 | " | 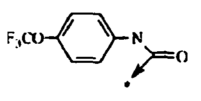 | " | 98.1 | 5.0 | 461.3 |
| 461 | " | 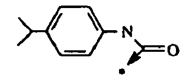 | " | 93.31 | 5.1 | 419.4 |
| 462 | " | 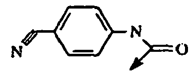 | " | 97.08 | 4.2 | 402.3 |
| 463 | " | 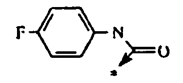 | " | 94.61 | 4.4 | 395.3 |
| 464 | " | 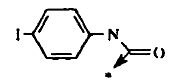 | " | 97.05 | 4.9 | 503.2 |
| 465 | " | 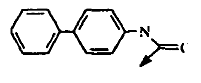 | " | 95.13 | 5.1 | 453.4 |
| 466 | 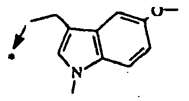 | 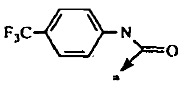 | " | 93.21 | 4.8 | 475.3 |
| 467 | " | 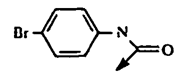 | " | 94.08 | 4.7 | 485.2 |
| 468 | " | 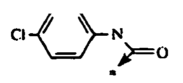 | " | 93.08 | 4.6 | 441.3 |
| 469 | " | 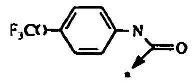 | " | 95.17 | 4.9 | 491.3 |
| 470 | " | 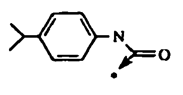 | " | 89,99 | 5.0 | 449.4 |
| 471 | " | 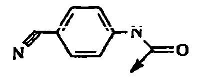 | " | 92 | 4.1 | 432.3 |
| 472 | " | 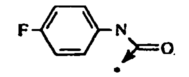 | " | 94.71 | 43 | 425.3 |
| 473 | " | 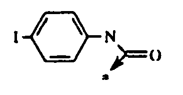 | " | 95.3 | 4.8 | 533.2 |
| 474 | " | 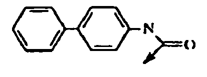 | " | 94.13 | 5.0 | 483.4 |
| 475 | 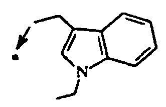 | 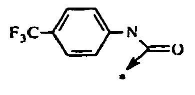 | " | 95 | 5.1 | 459.3 |
| 476 | " | 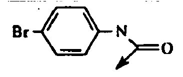 | " | 94.69 | 5.0 | 469.2 |
| 477 | " | 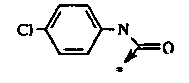 | " | 94.44 | 4.9 | 425.3 |
| 478 | " | 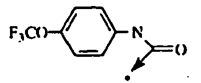 | " | 98 | 5.2 | 475.3 |
| 479 | " | 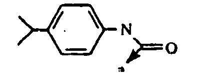 | " | 96.2 | 5.3 | 433.4 |
| 480 | " | 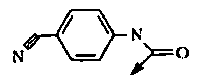 | " | 93 | 4.4 | 416.3 |
| 481 | " | 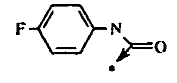 | " | 94.59 | 4.6 | 409.3 |
| 482 | " | 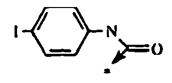 | " | 95.22 | 5.1 | 517.2 |
| 483 | " | 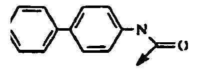 | " | 95.7 | 5.3 | 467.4 |
| 484 | 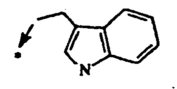 | 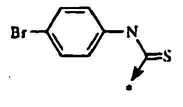 | " | 94.8 | 4.6 | 457.2 |
| 485 | " | 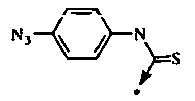 | " | 86.7 | 4,5 | 420.3 |
| 486 | " | 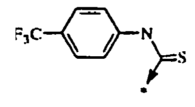 | " | 88.5 | 4.8 | 447.3 |
| 487 | " | 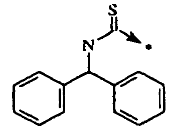 | " | 96.9 | 5.1 | 483,4 |
| 488 | " | 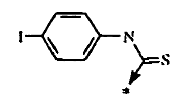 | " | 92.3 | 4.7 | 505.2 |
| 489 | 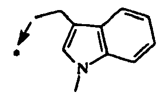 | 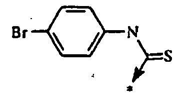 | " | 65.4 | 4.9 | 471.2 |
| 490 | " | 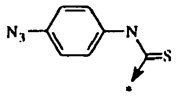 | " | 62.6 | 4.7 | 434.3 |
| 491 | " | 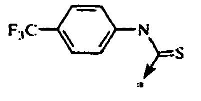 | " | 57.9 | 5.0 | 461.3 |
| 492 | " | 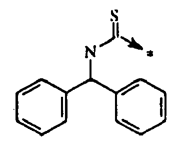 | " | 94.2 | 5.3 | 497.4 |
| 493 | " | 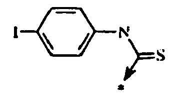 | " | 54.0 | 5.0 | 519.2 |
| 494 | 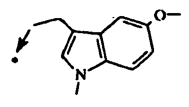 | 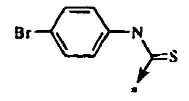 | " | 54.6 | 4.8 | 501.3 |
| 495 | " | 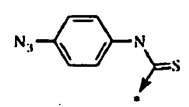 | " | 64.9 | 4.7 | 464.3 |
| 496 | " | 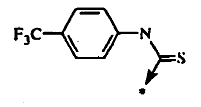 | " | 70.4 | 4.9 | 491.3 |
| 497 | " | 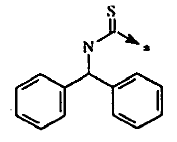 | " | 96.5 | 5.2 | 527.4 |
| 498 | " | 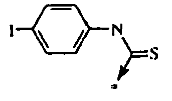 | " | 55.7 | 4.9 | 549.2 |
| 499 | 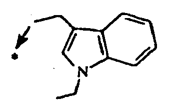 | 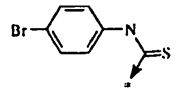 | " | 57.4 | 5.1 | 485.3 |
| 500 | " | 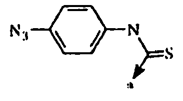 | " | 59.3 | 4.9 | 448.4 |
| 501 | " | 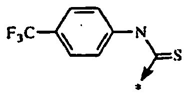 | " | 53.6 | 5.2 | 475.3 |
| 502 | " | 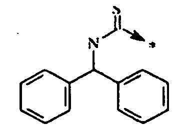 | " | 97.8 | 5.4 | 511.4 |
| 503 | " | 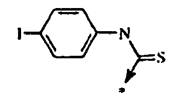 | " | 10 +36.87 | 5.2 | 533.2 |
| 504 | 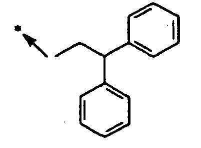 | 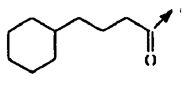 | 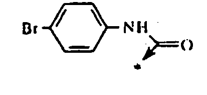 | 96.33 | 11.2 | 646.3 |
| 505 | " | 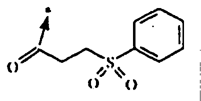 | " | 92.67 | 9.4 | 690.1 |
| 506 | " | 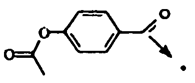 | " | 41.11 | 9.5 | 656.2 |
| 507 | " | 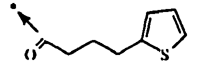 | " | 97.65 | 10.1 | 646.2 |
| 508 | " | 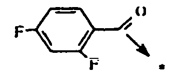 | " | 96.29 | 9.9 | 648.2 |
| 509 | 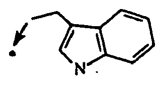 | 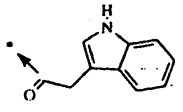 | 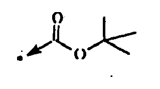 | 90.89 | 8.5 | 501.3 |
| 510 | | " | 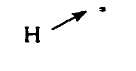 | 61.04 | 5.8 | 401.2 |
| 511 | " | 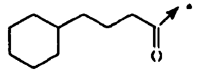 | 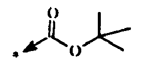 | 99.16 | 10.5 | 496.4 |
| 512 | " | " | 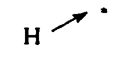 | 95.73 | 7.1 | 396.3 |
| 513 | " | 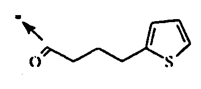 | 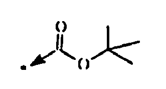 | 66 | 9.3 | 496.3 |
| 514 | " | " | 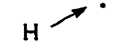 | 95.00 | 8.9 | 396.2 |
| 515 | " | 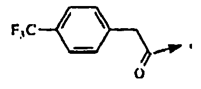 | 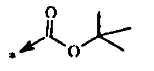 | 96.61 | 9.5 | 530.3 |
| 516 | " | " | 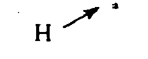 | 94.05 | 6.4 | 430.3 |
| 517 | " | 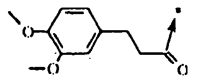 | 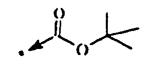 | 87 | 8.6 | 536.3 |
| 518 | " | " | 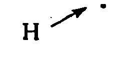 | 91.59 | 5.6 | 436.3 |
| 519 | 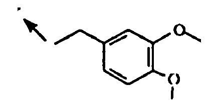 | 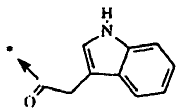 | 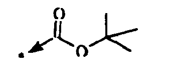 | 86,84 | 8.4 | 522.3 |
| 520 | | " | 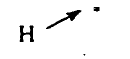 | 94.18 | 5.4 | 422.3 |
| 521 | " | 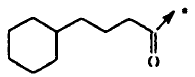 | 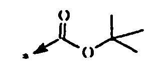 | 99.75 | 10.4 | 517.4 |
| 522 | " | " | 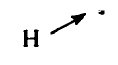 | 96.8 | 6.8 | 417.4 |
| 523 | " | 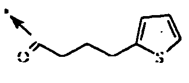 | 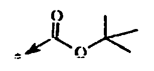 | 70.34 | 9.1 | 517.3 |
| 524 | " | " | 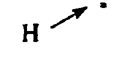 | 93.49 | 5.8 | 417.3 |
| 525 | " | 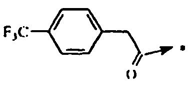 | 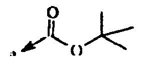 | 93.03 | 9.3 | 551.3 |
| 526 | " | " | 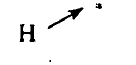 | 97.13 | 6.1 | 451.3 |
| 527 | " | 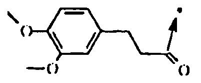 | 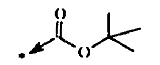 | 74.37 | 8.4 | 557.3 |
| 528 | " | " | 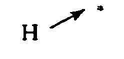 | 92.92 | 5.3 | 457.3 |
| 529 | 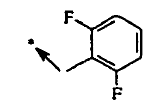 | 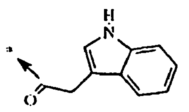 | 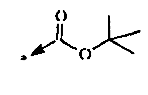 | 92.92 | 8.8 | 484.3 |
| 530 | " | " | 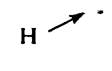 | 92.68 | 5.5 | 384.2 |
| 531 | " | 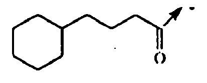 | 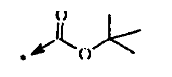 | 98.29 | 10.8 | 479.3 |
| 532 | " | " | 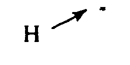 | 96.39 | 7.0 | 379.3 |
| 533 | " | 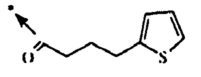 | 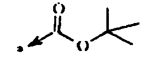 | 99 | 9.5 | 479.2 |
| 534 | " | " | 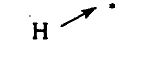 | 99.76 | 6.0 | 379.2 |
| 535 | " | 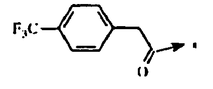 | 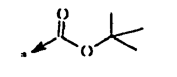 | 99.17 | 9.7 | 513.2 |
| 536 | " | " | 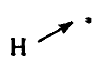 | 99.74 | 6.3 | 413.2 |
| 537 | " | 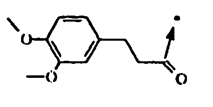 | 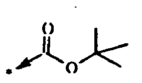 | 68.71 | 8.7 | 519.3 |
| 538 | " | " | 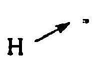 | 90.09 | 5.4 | 419.3 |
| 539 | 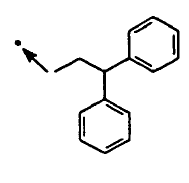 | 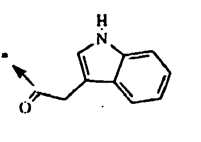 | 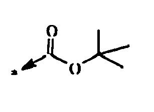 | 91.37 | 9.8 | 552.3 |
| 540 | " | " | 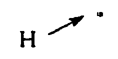 | 95.39 | 6.6 | 452.3 |
| 541 | " | 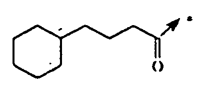 | 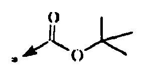 | 98.71 | 11.7 | 547.4 |
| 542 | " | " | 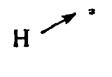 | 99.02 | 7.9 | 447.4 |
| 543 | " | 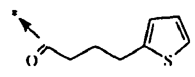 | 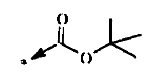 | 79.38 | 10.5 | 547,3 |
| 544 | " | " | 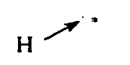 | 95.46 | 7.1 | 447.3 |
| 545 | " | 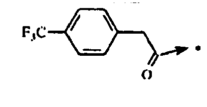 | 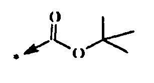 | 95.3 | 10.6 | 581.3 |
| 546 | " | " | 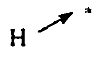 | 95.45 | 7.3 | 481,3 |
| 547 | " | 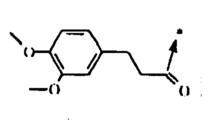 | 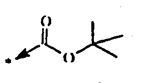 | 80.92 | 9.8 | 587.3 |
| 548 | " | " | 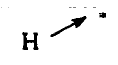 | 92.06 | 6.5 | 487.3 |
| 549 | 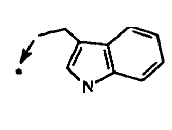 | 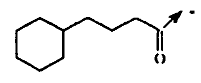 | 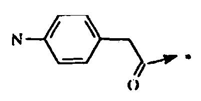 | 63 | 7.7 | 529.4 |
| 550 | " | " | 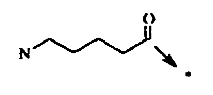 | 79 | 7.1 | 495.4 |
| 551 | " | 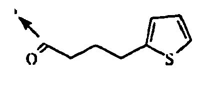 | 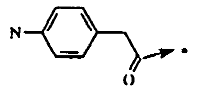 | 70 | 6.7 | 529.3 |
| 552 | " | " | 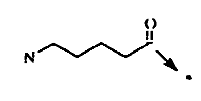 | 77 | 6.3 | 495.3 |
| 553 | " | 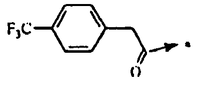 | 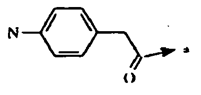 | 61 | 6.9 | 563.3 |
| 554 | " | " | 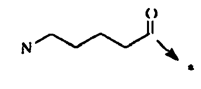 | 69 | 6.5 | 529.3 |
| 555 | " | 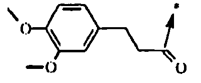 | 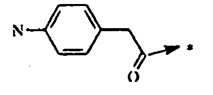 | 69 | 6.1 | 569.3 |
| 556 | " | " | 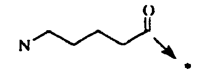 | 76 | 5.8 | 535.3 |
| 557 | 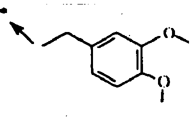 | 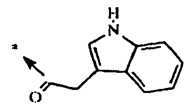 | 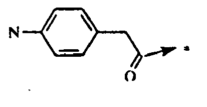 | 79 | 5.9 | 555.3 |
| 558 | " | " | 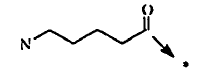 | 88 | 5.6 | 521.3 |
| 559 | " | 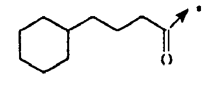 | 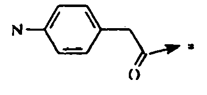 | 90.81 | 7.4 | 550.4 |
| 560 | " | " | 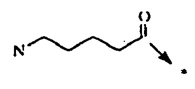 | 95.6 | 6.9 | 516.4 |
| 561 | " | 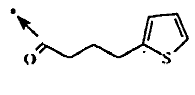 | 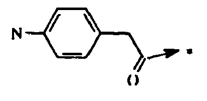 | 80.85 | 6.4 | 550.3 |
| 562 | " | " | 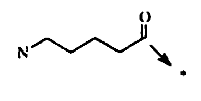 | 85.8 | 6.0 | 516.3 |
| 563 | " | 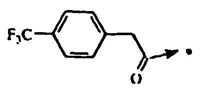 | 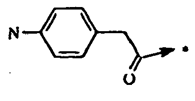 | 92.92 | 6.6 | 584.3 |
| 564 | " | " | 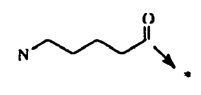 | 97.26 | 6.3 | 550.3 |
| 565 | " | 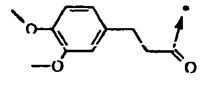 | 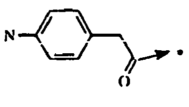 | 82.91 | 5.8 | 590.3 |
| 566 | " | " | 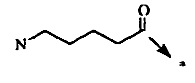 | 87.77 | 5.5 | 556.3 |
| 567 | 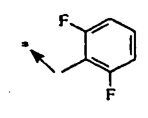 | 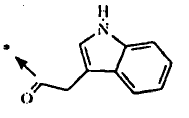 | 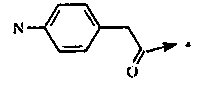 | 86 | 6.0 | 517.3 |
| 568 | " | " | 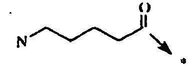 | 83.41 | 5.7 | 483.3 |
| 569 | " | 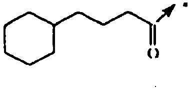 | 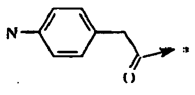 | 95 | 7.6 | 512,3 |
| 570 | " | " | 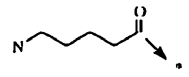 | 94.08 | 7.1 | 478.4 |
| 571 | " | 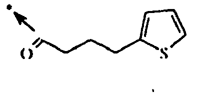 | 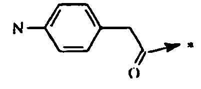 | 87.39 | 6.5 | 512.3 |
| 572 | " | " | 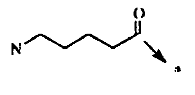 | 90.06 | 6.1 | 478.3 |
| 573 | " | 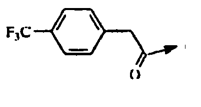 | 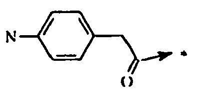 | 85.61 | 6.8 | 546.2 |
| 574 | " | " | 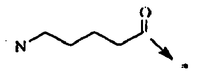 | 83.51 | 6.4 | 512.3 |
| 575 | " | 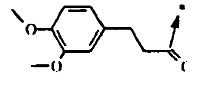 | 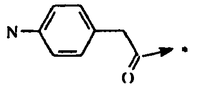 | 78.63 | 5.9 | 552.3 |
| 576 | " | " | 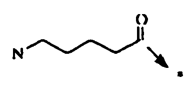 | 79.58 | 5.6 | 518.3 |
| 577 | 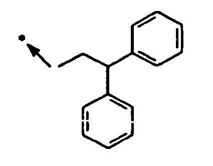 | 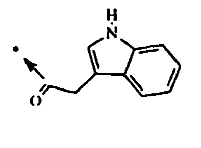 | 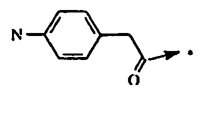 | 84 | 7.1 | 585.3 |
| 578 | " | " | 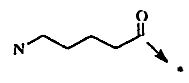 | 91 | 6.7 | 551.3 |
| 579 | " | 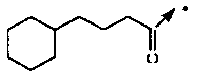 | 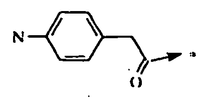 | 89.59 | 8.6 | 580.4 |
| 580 | " | " | 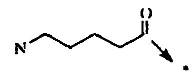 | 97.13 | 7.9 | 546.4 |
| 581 | " | 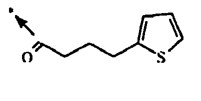 | 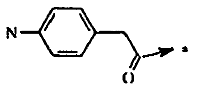 | 83 | 7.6 | 580.3 |
| 582 | " | " | 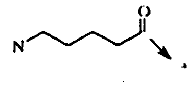 | 92.05 | 7.1 | 546.3 |
| 583 | " | 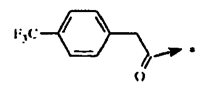 | 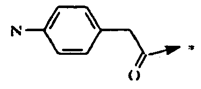 | 86 | 7.8 | 614.3 |
| 584 | " | " | 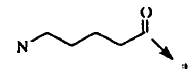 | 95.49 | 7.3 | 580.3 |
| 585 | " | 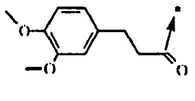 | 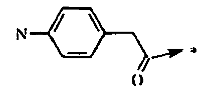 | 77 | 7.0 | 620.3 |
| 586 | " | " | 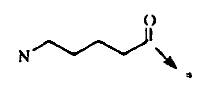 | 91.1 | 6.6 | 586.4 |
| 587 | 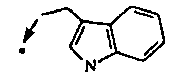 | 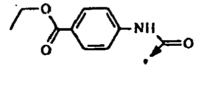 | 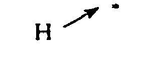 | 95 | 4.6 | 435 |
| 588 | " | 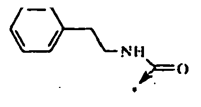 | " | 90 | 4.4 | 391.3 |
| 589 | " | 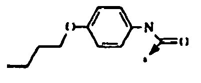 | " | 88 | 5.1 | 435.3 |
| 590 | " | 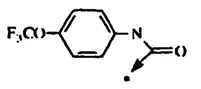 | " | 92 | 4.9 | 447.3 |
| 591 | " | 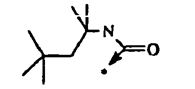 | " | 20.32 | 5.1 | 399.4 |
| 592 | 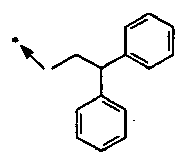 | 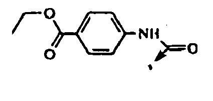 | " | 85 | 5.3 | 486.3 |
| 593 | " | 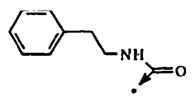 | " | 97 | 5.1 | 442,3 |
| 594 | " | 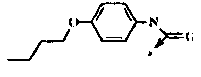 | " | 92 | 5.7 | 486.4 |
| 595 | " | 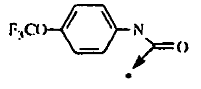 | " | 79 | 5.5 | 498.3 |
| 596 | 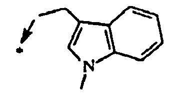 | 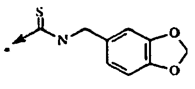 | " | 93.4 | 4.68 | 451.29 |
| 597 | " | 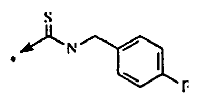 | " | 94.9 | 4.86 | 425.27 |
| 598 | " | 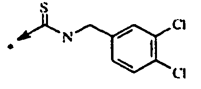 | " | 97.9 | 5.37 | 475.22 |
| 599 | " | 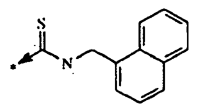 | " | 97.1 | 5.20 | 457.32 |
| 600 | " | 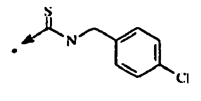 | " | 95.1 | 5.10 | 441.24 |
| 601 | 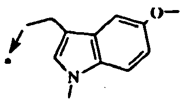 | 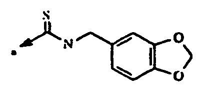 | " | 91.1 | 4.61 | 481.29 |
| 602 | " | 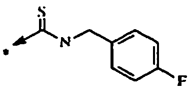 | " | 97.5 | 4.78 | 455.29 |
| 603 | " | 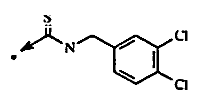 | " | 98.0 | 5.28 | 505.22 |
| 604 | " | 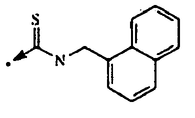 | " | 95.4 | 5.12 | 487.33 |
| 605 | " | 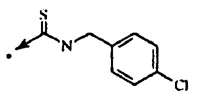 | " | 94.0 | 5.03 | 471.27 |
| 606 | 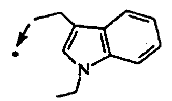 | 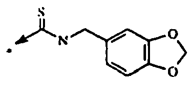 | " | 89.8 | 4.86 | 465.29 |
| 607 | " | 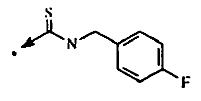 | " | 98.2 | 5.03 | 439.29 |
| 608 | " | 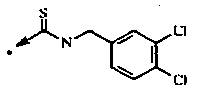 | " | 97,6 | 5.53 | 489.24 |
| 609 | " | 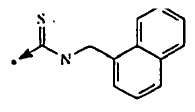 | " | 93.3 | 5.36 | 471.34 |
| 610 | " | 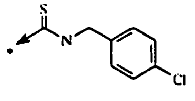 | " | 91.4 | 5.27 | 455.26 |
| 611 | 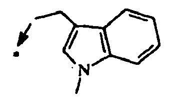 | 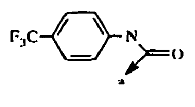 | 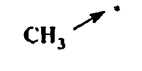 | 94 | 4.9 | 459.3 |
| 612 | " | 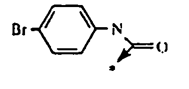 | " | 92.95 | 4.8 | 469.2 |
| 613 | " | 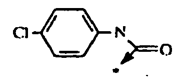 | " | 91.61 | 4.7 | 425.3 |
| 614 | " | 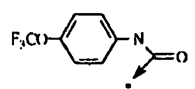 | " | 92 | 5.0 | 475.3 |
| 615 | " | 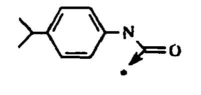 | " | 85.2 | 5.1 | 433.4 |
| 616 | " | 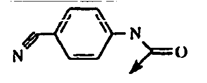 | " | 83 | 4.2 | 416.3 |
| 617 | " | 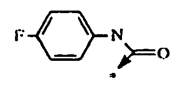 | " | 94.11 | 4.4 | 409.3 |
| 618 | " | 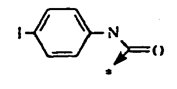 | " | 93.85 | 5.0 | 517.2 |
| 619 | " | 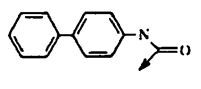 | " | 92.74 | 5.1 | 467.4 |
| 620 | 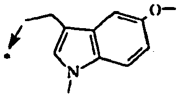 | 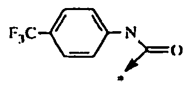 | " | 91 | 4.8 | 489.3 |
| 621 | " | 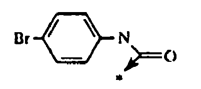 | " | 91.9 | 4.7 | 499.3 |
| 622 | " | 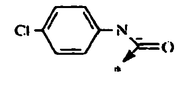 | " | 89.71 | 4.6 | 455.3 |
| 623 | " | 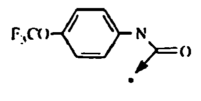 | " | 90 | 4.9 | 505.3 |
| 624 | " | 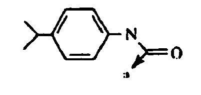 | " | 83.96 | 5.0 | 463.4 |
| 625 | " | 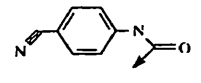 | " | 87 | 4.1 | 446.3 |
| 626 | " | 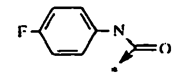 | " | 93.1 | 4.3 | 439.3 |
| 627 | " | 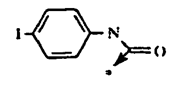 | " | 93.21 | 4.8 | 547.2 |
| 628 | " | 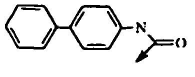 | " | 90.67 | 5.0 | 497.4 |
| 629 | 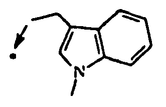 | 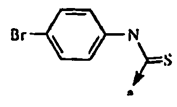 | " | 79.6 | 4.9 | 485.2 |
| 630 | " | 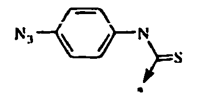 | " | 72.8 | 4.8 | 448.3 |
| 631 | " | 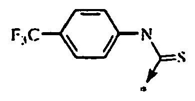 | " | 78.7 | 5.1 | 475.3 |
| 632 | " | 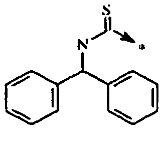 | " | 97.3 | 5.4 | 511.4 |
| 633 | " | 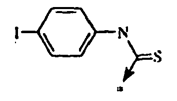 | " | 51.5 | 5.1 | 533.2 |
| 634 | 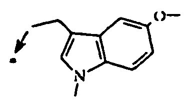 | 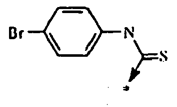 | " | 76.1 | 4.9 | 515.3 |
| 635 | " | 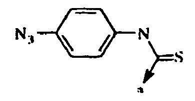 | " | 74.2 | 4.7 | 478.3 |
| 636 | " | 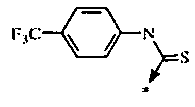 | " | 76.5 | 5.0 | 505.3 |
| 637 | " | 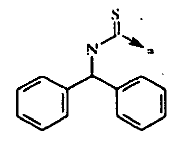 | " | 97.7 | 5.3 | 541.4 |
| 638 | " | 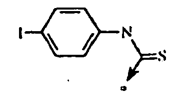 | " | 71.4 | 5.0 | 563.2 |
| 639 | 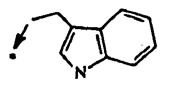 | 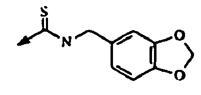 | " | 82.54 | 4.4 | 451.3 |
| 640 | " | 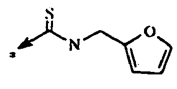 | " | 93.42 | 4.2 | 397.3 |
| 641 | " | 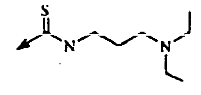 | " | 98.93 | 2.9 | 430.4 |
| 642 | " | 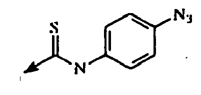 | " | 81.46 | 4.5 | 434.3 |
| 643 | " | 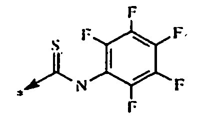 | " | 96.41 | 4.9 | 483.3 |
| 644 | " | 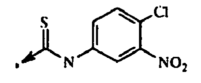 | " | 91.55 | 4.7 | 472.3 |
| 645 | " | 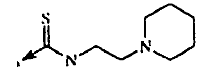 | " | 97.96 | 2.9 | 428.4 |
| 646 | " | 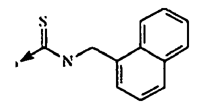 | " | 96.9 | 5.0 | 425.3 |
| 647 | " | 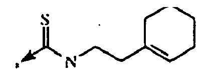 | " | 95.8 | 4.9 | 457.3 |
| 648 | " | 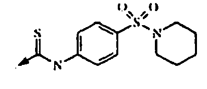 | " | 91.41 | 4.6 | 540.3 |
| 649 | 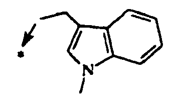 | 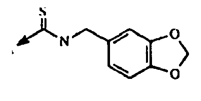 | " | 88.0 | 4.75 | 465.3 |
| 650 | " | 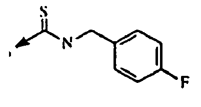 | " | 99.0 | 4.89 | 439.3 |
| 651 | " | 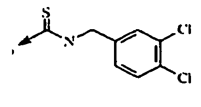 | " | 98.5 | 5.42 | 489.2 |
| 652 | " | 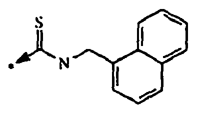 | " | 93.3 | 5.24 | 471.3 |
| 653 | " | 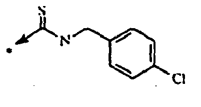 | " | 87.6 | 5.14 | 455.3 |
| 654 | 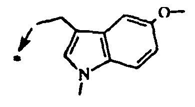 | 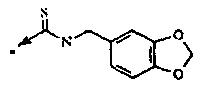 | " | 88.3 | 4.66 | 495.3 |
| 655 | " | 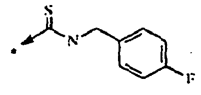 | " | 98.1 | 4.82 | 469.3 |
| 656 | " | 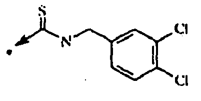 | " | 98.4 | 5.34 | 519.2 |
| 657 | " | 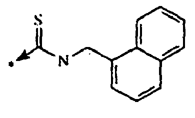 | " | 95.4 | 5.16 | 501.3 |
| 658 | " | 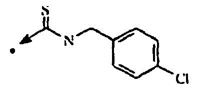 | " | 89.8 | 5.08 | 485.3 |
| 659 | 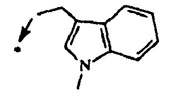 | 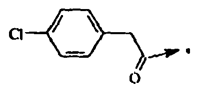 | 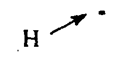 | 80.76 | 4.84 | 410.2 |
| 660 | " | 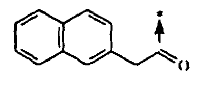 | " | 61.69 | 4.97 | 426.2 |
| 661 | " | 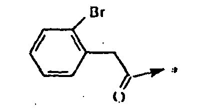 | " | 90.93 | 4.79 | 454.1 |
| 662 | " | 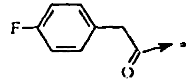 | " | 91.55 | 4.58 | 394.2 |
| 663 | " | 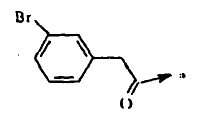 | " | 91.99 | 4.88 | 454.1 |
| 664 | " | 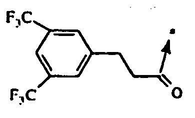 | " | 92.79 | 5.55 | 526.2 |
| 665 | " | 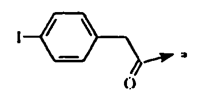 | " | 93.78 | 5.02 | 502.1 |
| 666 | " | 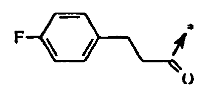 | " | 96.3 | 4.75 | 408.2 |
| 667 | " | 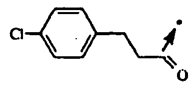 | " | 81.2 | 5.02 | 408.2 |
| 668 | 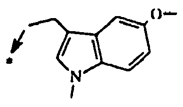 | 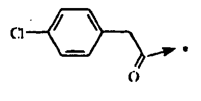 | " | 90.79 | 4.74 | 440.2 |
| 669 | " | 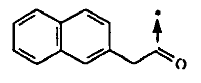 | " | 78.93 | 4.88 | 456.3 |
| 670 | " | 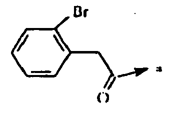 | " | 91.87 | 4.69 | 484.2 |
| 671 | " | 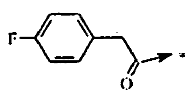 | " | 91,19 | 4.51 | 424.2 |
| 672 | " | 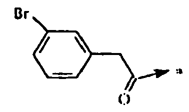 | " | 95.27 | 4.79 | 484.2 |
| 673 | " | 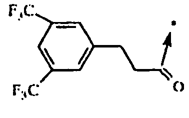 | " | 89.5 | 5.46 | 542.2 |
| 674 | " | 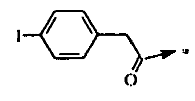 | " | 90.77 | 4.92 | 532.1 |
| 675 | " | 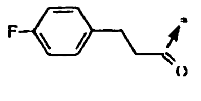 | " | 95.1 | 4.66 | 438.2 |
| 676 | " | 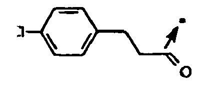 | " | 88.7 | 4.92 | 524.2 |
| 677 | 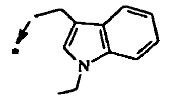 | 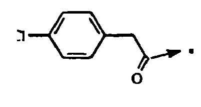 | " | 81.65 | 4.99 | 424.2 |
| 678 | " | 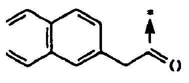 | " | 70.32 | 5.11 | 440.3 |
| 679 | " | 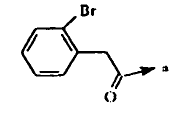 | " | 90.06 | 4.96 | 468.2 |
| 680 | " | 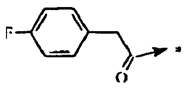 | " | 94.11 | 4.74 | 408.2 |
| 681 | " | 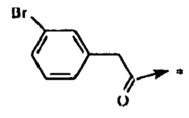 | " | 93.96 | 5.04 | 468.2 |
| 682 | " | 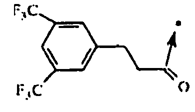 | " | 93.3 | 5.66 | 540.2 |
| 683 | " | 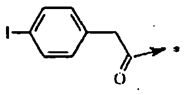 | " | 94.79 | 5.16 | 516.1 |
| 684 | " | 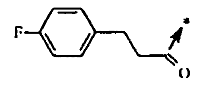 | " | 96.5 | 4.9 | 422.3 |
| 685 | " | 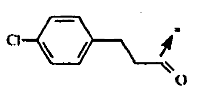 | " | 88.2 | 5.19 | 438.2 |
| 686 | 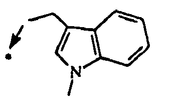 | 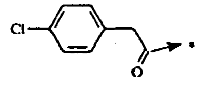 | 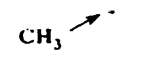 | 87.93 | 4.86 | 424.2 |
| 687 | " | 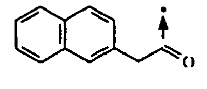 | " | 84.74 | 5 | 440.2 |
| 688 | " | 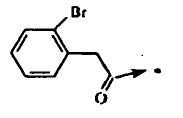 | " | 95.34 | 4.82 | 468,2 |
| 689 | " | 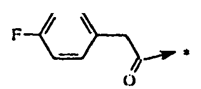 | " | 89.78 | 4.6 | 408.2 |
| 690 | " | 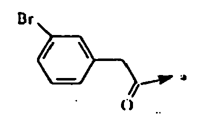 | " | 95.16 | 4.9 | 468.163 3 |
| 691 | " | 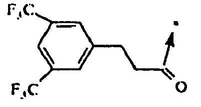 | " | 95.6 | 5.56 | 540.2 |
| 692 | " | 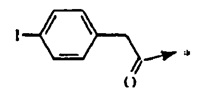 | " | 95.24 | 5.05 | 516.3 |
| 693 | " | 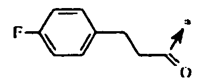 | " | 96.6 | 4.8 | 422.2 |
| 694 | " | 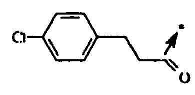 | " | 90.4 | 5.04 | 438.2 |
| 695 | 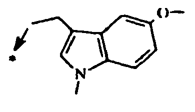 | 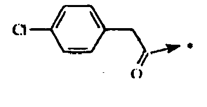 | " | 93.12 | 4.78 | 454.2 |
| 696 | " | 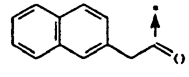 | " | 86.11 | 4.92 | 470.3 |
| 697 | " | 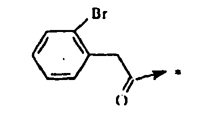 | " | 94.89 | 4.73 | 498.2 |
| 698 | " | 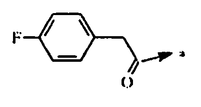 | " | 94.1 | 4.54 | 438.3 |
| 699 | " | 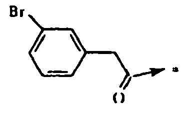 | " | 95.66 | 4.81 | 498.2 |
| 700 | " | 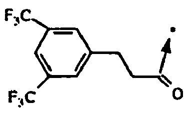 | " | 94.8 | 5.48 | 570.2 |
| 701 | " | 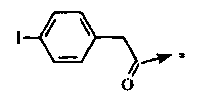 | " | 93.63 | 4.96 | 546.1 |
| 702 | " | 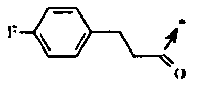 | " | 96.7 | 4.7 | 452.3 |
| 703 | " | 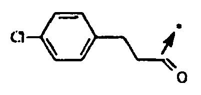 | " | 85.6 | 4.96 | 468,2 |
| 704 | 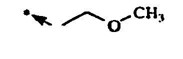 | 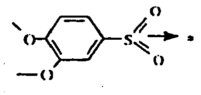 | 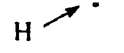 | 78.36 | 3.14 | 359.1 |
| 705 | 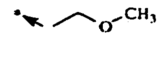 | 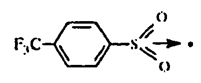 | " | 47.4 | 3.9 | 367.1 |
| 706 | 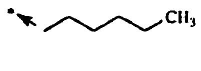 | 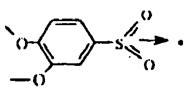 | " | 69.72 | 4.28 | 385.2 |
| 707 | 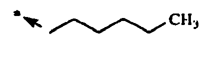 | 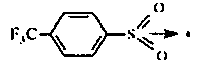 | " | 34.86 | 4.96 | 393.2 |
| 708 | 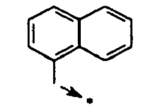 | 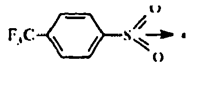 | " | 37.54 | 4.91 | 449.2 |
| 709 | 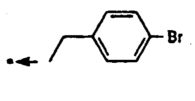 | 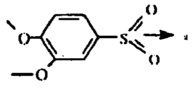 | " | 81.57 | 4.46 | 483.1 |
| 710 | 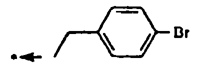 | 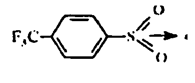 | " | 55.98 | 5.12 | 491.1 |
| 711 | 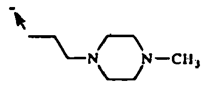 | 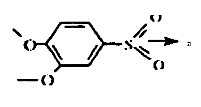 | " | 73.74 | 3.09 | 441.2 |
| 712 | 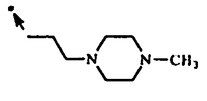 | 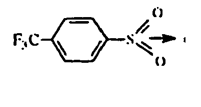 | " | 40.19 | 2.85 | 449.2 |
| 713 | 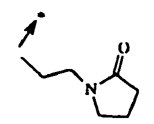 | 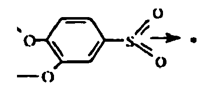 | " | 90.07 | 3.18 | 426.2 |
| 714 | 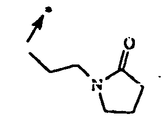 | 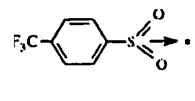 | " | 74.98 | 3.84 | 434.2 |
| 715 | 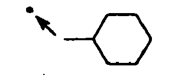 | 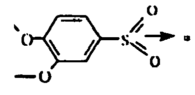 | " | 78.14 | 4.24 | 397.2 |
| 716 | 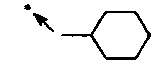 | 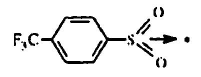 | " | 39.87 | 4.92 | 405.2 |
| 717 | 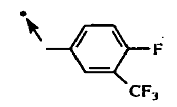 | 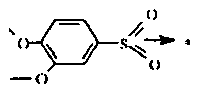 | " | 57.34 | 4.45 | 477.2 |
| 718 | 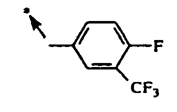 | 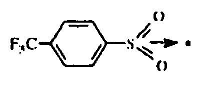 | " | 37.75 | 5.01 | 485.1 |
| 719 | 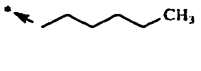 | 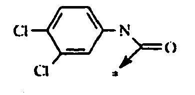 | 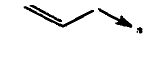 | 70.3 | 5.2 | 412.1 |
| 720 | " | " | 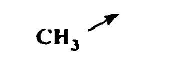 | 70.7 | 5.0 | 386.1 |
| 721 | " | " | 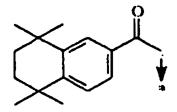 | 61.9 | 6.3 | 600.3 |
| 722 | " | 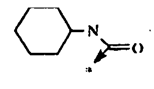 | " | 49.3 | 6.1 | 538.4 |
| 723 | 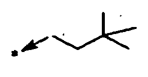 | 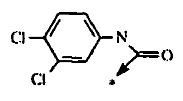 | 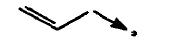 | 65.0 | 5.1 | 412.2 |
| 724 | " | " | 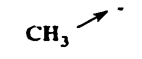 | 44.3 | 4.9 | 386.2 |
| 725 | " | " | 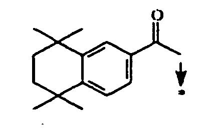 | 49.2 | 6.2 | 600.3 |
| 726 | " | 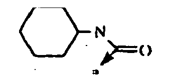 | " | 37.5 | 6.0 | 538.4 |
| 727 | 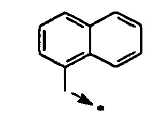 | 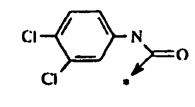 | 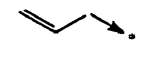 | 87.1 | 5.1 | 468.1 |
| 728 | " | " | 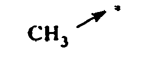 | 84.4 | 4.9 | 442.1 |
| 729 | " | " | 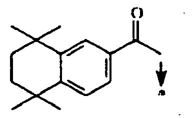 | 82.3 | 6.2 | 656.3 |
| 730 | " | 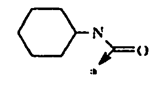 | 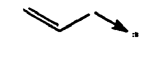 | 93.8 | 4.7 | 406.3 |
| 731 | " | " | 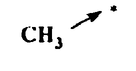 | 80.7 | 4.6 | 380.3 |
| 732 | " | " | 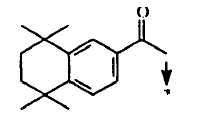 | 84.1 | 5.9 | 594.3 |
| 733 | 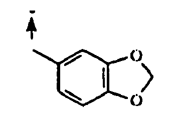 | 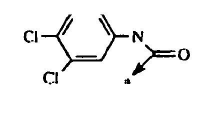 | 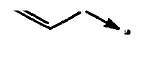 | 67.9 | 4.7 | 462.1 |
| 734 | " | " | 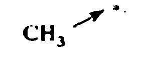 | 66.9 | 4.6 | 436.1 |
| 735 | " | " | 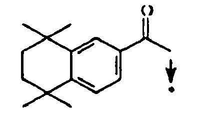 | 56.8 | 5.9 | 650.2 |
| 736 | " | 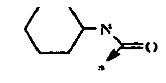 | 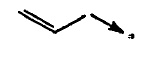 | 88.1 | 4.3 | 400.3 |
| 737 | " | " | 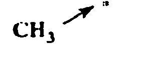 | 82.8 | 4.1 | 374.3 |
| 738 | " | " | 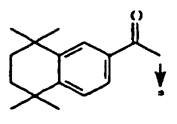 | 51.4 | 5.6 | 588.3 |
| 739 | 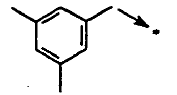 | 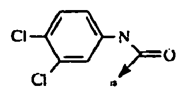 | 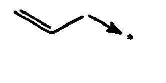 | 77.7 | 5.1 | 446.2 |
| 740 | " | " | 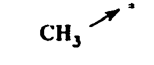 | 76.1 | 4.9 | 420.2 |
| 741 | " | " | 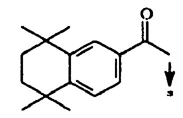 | 67.1 | 6.2 | 634.3 |
| 742 | " | 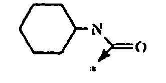 | 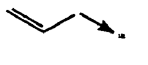 | 88.9 | 4.7 | 384.3 |
| 743 | " | " | 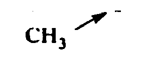 | 79.3 | 4.5 | 358.3 |
| 744 | " | " | 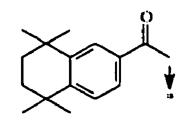 | 65.1 | 5.9 | 572.4 |
| 745 | 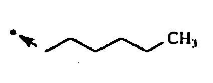 | 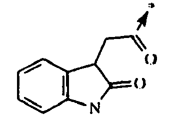 | 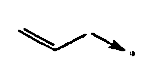 | 80.0 | 4.0 | 398.3 |
| 746 | " | " | 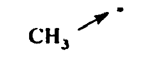 | 76.9 | 3.8 | 372.3 |
| 747 | " | " | 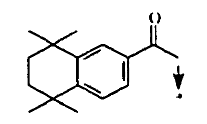 | 42.7 | 5.8 | 586.4 |
| 748 | " | " | 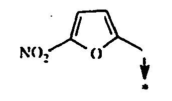 | 64.6 | 4.4 | 483.3 |
| 749 | " | 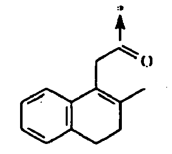 | 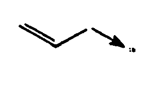 | 87.4 | 5.3 | 409.3 |
| 750 | " | " | 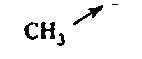 | 1.0 | 5.1 | 383.3 |
| 751 | " | " | 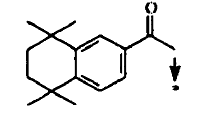 | 9.8 | 6.7 | 597.4 |
| 752 | " | " | 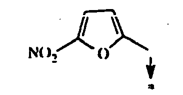 | 4.4 | 5.6 | 493.3 |
| 753 | 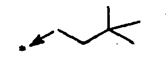 | 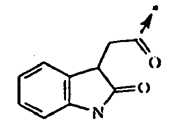 | 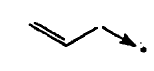 | 0.1 | 3.9 | 398.3 |
| 754 | " | " | 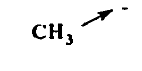 | 3.1 | 3.7 | 372.3 |
| 755 | " | " | 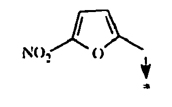 | 4.4 | 4.3 | 483.3 |
| 756 | " | 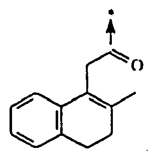 | 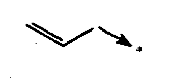 | 4.6 | 5.3 | 409.3 |
| 757 | " | " | 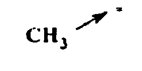 | 9.6 | 5.0 | 383.3 |
| 758 | " | " | 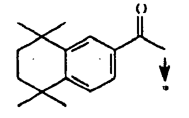 | 2.9 | 6.6 | 597.4 |
| 759 | " | " | 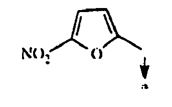 | 81.6 | 5.5 | 494.3 |
| 760 | 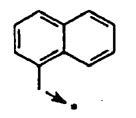 | " | 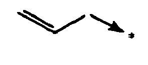 | 85.3 | 5.3 | 465.3 |
| 761 | " | " | 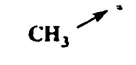 | 60.3 | 5.1 | 439.3 |
| 762 | " | " | 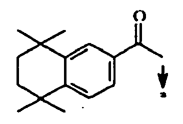 | 61.8 | 6.6 | 653.4 |
| 763 | " | " | 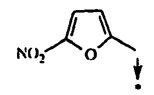 | 74.4 | 5.6 | 550.3 |
| 764 | 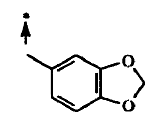 | 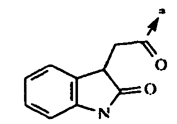 | 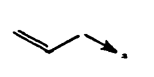 | 74.5 | 3.6 | 448.2 |
| 765 | " | " | 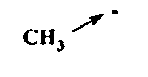 | 51.3 | 3.4 | 422.2 |
| 766 | " | " | 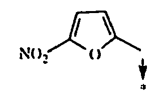 | 58.8 | 3.9 | 533.2 |
| 767 | " | 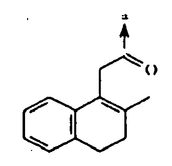 | 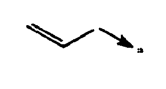 | 86.2 | 4.8 | 459.3 |
| 768 | " | " | 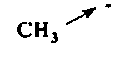 | 63.2 | 4.6 | 433.3 |
| 769 | " | " | 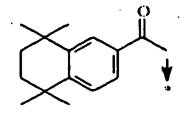 | 60.1 | 6.2 | 647.4 |
| 770 | " | " | 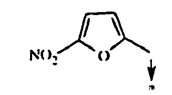 | 83.5 | 5.1 | 544.2 |
| 771 | 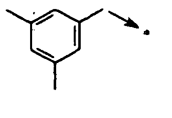 | 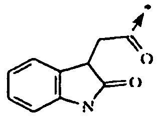 | 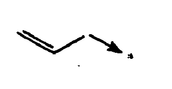 | 68.1 | 4.1 | 432.3 |
| 772 | " | " | 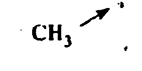 | 63.8 | 3.9 | 406.2 |
| 773 | " | " | 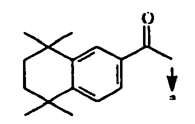 | 41.1 | 5.8 | 620.4 |
| 774 | " | " | 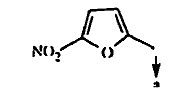 | 62.8 | 4.4 | 517.2 |
| 775 | " | 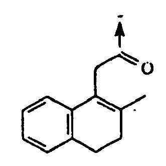 | 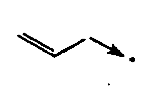 | 85.5 | 5.4 | 443.3 |
| 776 | " | " | 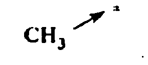 | 62.5 | 5.2 | 417.3 |
| 777 | " | " | 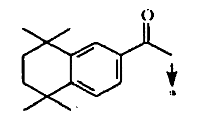 | 66.0 | 6.7 | 631.4 |
| 778 | " | " | 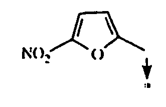 | 87.7 | 5.6 | 528.3 |

### Etude pharmacologique

Les composés de la présente invention ont été testés en ce qui concerne leur affinité pour différents sous-types de récepteurs de la somatostatine selon les procédures décrites ci-après.

### Etude de l'affinité pour les sous-types de récepteurs de la somatostatine humaine :

L'affinité d'un composé de l'invention pour les sous-types de récepteurs de la somatostatine 1 à 5 (sst₁, sst₂, sst₃, sst₄ et sst₅, respectivement) est déterminée par la mesure de l'inhibition de la liaison de [¹²⁵I-Tyr¹¹]SRIF-14 à des cellules transfectées CHO-K1.

Le gène du récepteur sst₁ de la somatostatine humaine a été cloné sous forme d'un fragment génomique. Un segment *Pst*I-*Xmn*I de 1,5 Kb contenant 100 pb de la région 5' non transcrite, 1,17 Kb de la région codante en totalité, et 230 bp de la région 3' non transcrite est modifié par l'addition du linker BglII. Le fragment d'ADN résultant est souscloné dans le site *BamH*I d'un pCMV-81 pour donner le plasmide d'expression chez les mammifères (fourni par Dr. Graeme Bell, Univ. Chicago). Une lignée de cellules clonées exprimant de façon stable le récepteur sst₁ est obtenue par transfection dans des cellules CHO-K1 (ATCC) grâce à la méthode de co-précipitation calcium phosphate. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène du récepteur sst₂ de la somatostatine humaine, isolé sous forme d'un fragment génomique d'ADN de 1,7 Kb *Bam*HI-*Hind*III et souscloné dans un vecteur plasmidique pGEM3Z (Promega), a été fourni par le Dr. G. Bell (Univ. of Chicago). Le vecteur d'expression des cellules de mammifères est construit en insérant le fragment *Bam*HI-*Hind*II de 1,7 Kb dans des sites de restriction endonucléase compatibles du plasmide pCMV5. Une lignée de cellules clonées est obtenue par transfection dans des cellules CHO-K1 grâce à la méthode de co-précipitation calcium phosphate. Le plasmide pRSV-neo est inclus comme marqueur de sélection.

Le récepteur sst₃ est isolé comme fragment génomique, et la séquence codante complète est contenue dans un fragment *Bam*HI/*Hind*III de 2,4 Kb. Le plasmide d'expression chez les mammifères, pCMV-h3, est construit par insertion du fragment *Nco*I-*Hind*III de 2,0 Kb dans le site EcoR1 du vecteur pCMV après modification des terminaisons et addition de linkers EcoR1. Une lignée de cellules clonées exprimant de façon stable le récepteur sst₃ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le plasmide d'expression du récepteur sst₄ humain, pCMV-HX, a été fourni par le Dr. Graeme Bell (Univ. Chicago). Ce vecteur contient le fragment génomique codant pour le récepteur sst₄ humain de 1,4 Kb *Nhe*I-*Nhe*I, 456 pb de la région 5' non transcrite, et 200 pb de la région 3' non transcrite, cloné dans les sites *Xha*I/*Eco*R1 de PCMV-HX. Une lignée de cellules clonées exprimant de façon stable le récepteur sst₄ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène correpondant au récepteur sst₅ humain, obtenu par la méthode PCR en utilisant un clone génomique λ comme sonde, a été fourni par le Dr. Graeme Bell (Univ. Chicago). Le fragment PCR résultant de 1,2 Kb contient 21 paires de bases de la région 5' non transcrites, la région codante en totalité, et 55 pb de la région 3' non transcrite. Le clone est inséré dans un site EcoR1 du plasmide pBSSK(+). L'insert est récupéré sous la forme d'un fragment *Hind*III-*Xba*I de 1,2 Kb pour sous-clonage dans un vecteur d'expression chez les mammifères, pCVM5. Une lignée de cellules clonées exprimant de façon stable le récepteur sst₅ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Les cellules CHO-K1 exprimant de façon stable l'un des récepteurs sst humain sont cultivées dans un milieu RPMI 1640 contenant 10% de sérum foetal de veau et 0,4 mg/ml de généticine. Les cellules sont collectées avec de l'EDTA 0,5 mM et centrifugées à 500 g pendant environ 5 min à environ 4° C. Le centrifugat est re-suspendu dans un milieu tampon 50 mM Tris à pH 7,4 et centrifugé deux fois à 500 g pendant environ 5 min à environ 4° C. Les cellules sont lysées par sonication et centrifugées à 39 000 g pendant environ 10 min à 4° C. Le centrifugat est re-suspendu dans le même milieu tampon et centrifugé à 50 000 g pendant 10 min à environ 4° C et les membranes dans le centrifugat obtenu sont stockées à -80° C.

Des tests d'inhibition compétitive de liaison avec [¹²⁵I-Tyr¹¹]SRIF-14 sont effectués en double à l'aide de plaques en polypropylène de 96 puits. Les membranes cellulaires (10 µg protéine/puits) sont incubées avec [¹²⁵I-Tyr¹¹]SRIF-14 (0,05 nM) pendant environ 60 min à environ 37° C dans un milieu tampon 50 mM HEPES (pH 7,4) comprenant 0,2 % BSA, 5 mM de MgCl₂, 200 KIU/ml de Trasylol, 0,02 mg/ml de bacitracine et 0,02 mg/ml de fluorure de phénylméthylsulphonyle.

La [¹²⁵I-Tyr¹¹]SRIF-14 liée est séparée de la [¹²⁵I-Tyr¹¹]SRIF-14 libre par filtration immédiate à travers des plaques filtres en fibre de verre GF/C (Unifilter, Packard) préimprégné avec 0,1 % de polyéthylènimine (P.E.I.), en utilisant un Filtermate 196 (Packard). Les filtres sont lavés avec du tampon 50 mM HEPES à environ 0-4° C pendant environ 4 secondes et leur radioactivité est déterminée à l'aide d'un compteur (Packard Top Count).

La liaison spécifique est obtenue en soustrayant la liaison non spécifique (déterminée en présence de 0,1 µM SRIF-14) de la liaison totale. Les données relatives à la liaison sont analysées par analyse en régression non-linéaire assistée par ordinateur (MDL) et les valeurs des constantes d'inhibition (Ki) values sont déterminées.

La détermination du caractère agoniste ou antagoniste d'un composé de la présente invention est effectuée à l'aide du test décrit ci-après.

### Test fonctionnel : Inhibition de la production d'AMPc intracellulaire :

Des cellules CHO-K1 exprimant les sous-types de récepteurs de la somatostatine humaine (SRIF-14) sont cultivées dans des plaques à 24 puits dans un milieu RPMI 1640 avec 10 % de sérum foetal de veau et 0,4 mg/ml de généticine. Le milieu est changé le jour précédant l'expérience.

Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 min à environ 37° C.
. la production d'AMP cyclique est stimulée par l'addition de 1 mM de forskoline (FSK) pendant 15-30 minutes à environ 37° C.
. l'effet inhibiteur de la somatostatine d'un composé agoniste est mesuré par l'addition simultanée de FSK (1 µM), SRIF-14 (10⁻¹² M to 10⁻⁶ M) et du composé à tester (10⁻¹⁰ M à 10⁻⁵ M).
. l'effet antagoniste d'un composé est mesuré par l'addition simultanée de FSK (1 µM), SRIF-14 (1 to 10 nM) et du composé à tester (10⁻¹⁰ M to 10⁻⁵ M).

Le milieu réactionnel est éliminé et 200 ml de HCI 0,1 N sont ajoutés. La quantité d'AMPc est mesurée par un test radioimmunologique (Kit FlashPlate SMP001 A, New England Nuclear).

### Résultats :

Les tests effectués selon les protocoles décrits ci-dessus ont permis de montrer que les produits de formule générale (I) définie dans la présente demande ont une bonne affinité pour au moins l'un des sous-types de récepteurs de la somatostatine, la constante d'inhibition Kᵢ étant inférieure au micromolaire pour certains des composés exemplifiés.

## Revendications

1. Composés de formule générale I 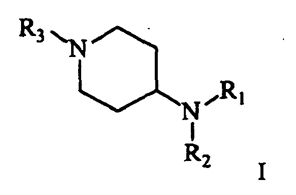 sous forme racémique, d'énantiomère ou toutes combinaisons de ces formes, dans laquelle
soit
R₁ représente un radical (C₁-C₆)alkyle linéaire ou ramifié, le radical -(CH₂)ₘ-Y-Z₁₁ ou -(CH₂)ₘ -Z₁₂ dans lequel
Z₁₁ représente un (C₁-C₆)alkyle,
Z₁₂ représente *bis*-phényle, (C₃-C₇)cycloalkyle, (C₃-C₇)hétérocycloalkyle éventuellement substitué par un ou plusieurs substituants choisis indépendamment parmi les radicaux oxy et alkyle, ou aryle ou hétéroaryle éventuellement substitué par un ou plusieurs substituants choisis indépendamment parmi les radicaux fluoro, chloro, bromo, iodo, alkyle, alkoxy,
ou bien Z₁₂ représente 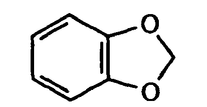 Y représente l'atome d'oxygène,
ou bien R₁ représente un radical de formule 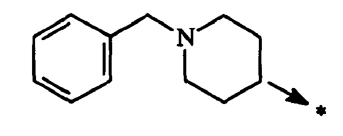 R₂ représente un radical de formule -C(Y)NHX₁, -C(O)X₂ ou SO₂X₃ dans laquelle
Y représente un atome d'oxygène ou de soufre ;
X₁ représente un radical (C₁-C₁₅)alkyle linéaire ou ramifié, ou-(CH₂)ₚZ₂₂ dans lequel
Z₂₂ représente cyclohexényle, *bis*-phényle, (C₃-C₇)cycloalkyle, (C₃-C₇)hétérocycloalkyle, mono- ou di-alkylamino, -C(O)-O-alkyle, ou aryle ou hétéroaryle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle, ou phényle,
ou bien Z₂₂ représente un radical de formule 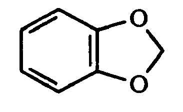 X₂ représente un radical alkynyle, -(CH₂)ₘ-W-(CH₂)_{q}-Z₂₃ ou -(CH₂)ₚ-U-Z₂₄ dans lequel
W représente SO₂,
U représente une liaison covalente,
Z₂₃ représente un radical aryle ;
Z₂₄ représente cyclohexényle, *bis*-phényle, (C₃-C₇)cycloalkyle éventuellement substitué par un radical aminoalkyle, ou aryle ou hétéroaryle éventuellement substitué par un ou plusieurs radicaux choisis parmi fluoro, chloro, bromo, iodo, alkyle, alkoxy, -CF₃, -OCF₃, -SCF₃, hydroxy, -O-C(O)-alkyle, mono- ou di-alkylamino, amino
ou Z₂₄ représente un radical de formule 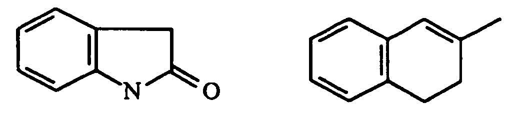 ou bien X₂ représente 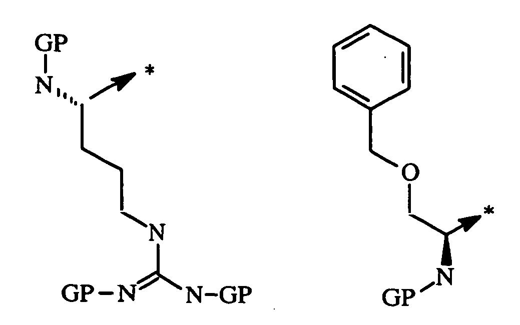 où le groupe protecteur (GP) représente H ou le *tert*-butyloxycarbonyle ;
X₃ représente un radical -(CH₂)ₚZ₂₅ dans lequel Z₂₅ représente un radical aryle éventuellement substitué par un ou plusieurs radicaux identiques ou différents choisis parmi alkoxy et CF₃,
R₃ représente l'atome d'hydrogène, un radical alkyle, alkényle, hétéroarylalkyle éventuellement substitué ou un radical de formule -C(Y)-NHX₁, -C(O)X₂ ou SO₂X₃ dans laquelle
Y représente un atome d'oxygène ou de soufre ;
X₁ représente un radical -(CH₂)ₚZ₂₂ dans lequel
Z₂₂ représente un radical aryle éventuellement substitué par un ou plusieurs radicaux choisis indépendamment parmi les radicaux fluoro, chloro, bromo, iodo, alkyle, alkoxy, CF₃, nitro, phénoxy ;
X₂ représente le radical vinyle substitué par un phényle, le radical phényle étant lui-même éventuellement substitué par un ou plusieurs radicaux halo, ou -(CH₂)ₚ-U-Z₂₄ dans lequel
U représente une liaison covalente ou l'atome d'oxygène ;
Z₂₄ représente alkyle, (C₃-C₇)cycloalkyle, (C₃-C₇)hétérocycloalkyle, *bis*-phényle, amino, mono ou di-alkylamino, ou aryle ou hétéroaryle éventuellement substitué par un ou plusieurs radicaux choisis parmi alkoxy, bromo, chloro, fluoro, hydroxy, CF₃, nitro, amino, mono- et di-alkylamino, pyrrolyle,
ou bien X₂ représente un radical de formule 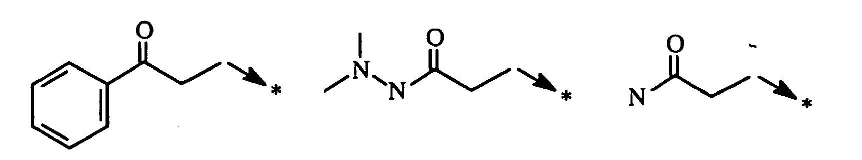 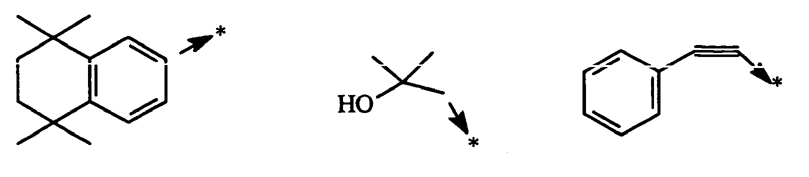 X₃ représente un radical (C₁-C₁₀)alkyle linéaire ou ramifié, le radical vinyle substitué par un radical (le radical phényle étant lui-même éventuellement substitué), CF₃, ou -(CH₂)ₚZ₂₅ dans lequel
Z₂₅ représente aryle ou hétéroaryle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino;
m est un entier de 1 à 6 ;
p est un entier de 0 à 6 ;
q est un entier de 0 à 2,
soit
les radicaux R₁, R₂ et R₃ représentent respectivement les radicaux suivants :
| R1 | R2 | R3 |
|---|---|---|
| 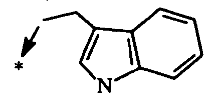 | 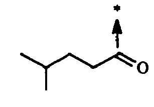 | 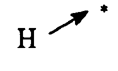 |
| 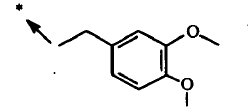 | 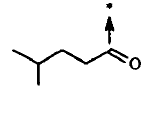 | 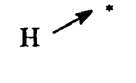 |
| 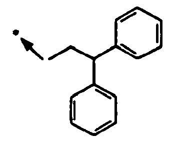 | 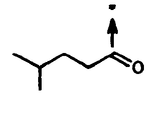 | 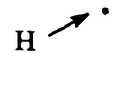 |
| 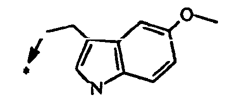 | 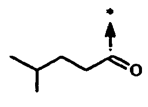 | 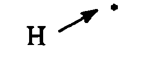 |
| 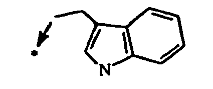 | 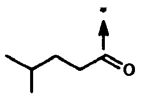 | 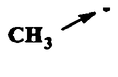 |
| 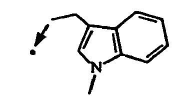 | 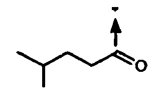 | 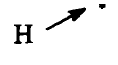 |
| 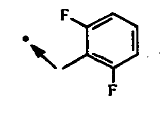 | 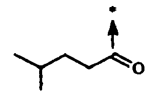 | 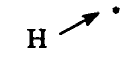 |
| 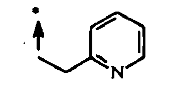 | 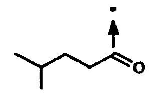 | 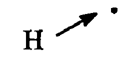 |
ou leurs sels d'addition avec les acides minéraux ou organiques pharmaceutiquement acceptables.

2. Composés de formule I telle que définie à la revendication 1, **caractérisée en ce que** R₁ représente un radical (C₁-C₆)alkyle linéaire ou ramifié, le radical -(CH₂)ₘ-Y-Z₁₁ ou -(CH₂)ₘ-Z₁₂ dans lequel
Z₁₁ représente un (C₁-C₆)alkyle,
Z₁₂ représente naphtyle, morpholino, *bis*-phényle, pyrrolidinyle substitué par le radical oxy, ou bien les radicaux phényle, pipérazinyle, pyridinyle et indolyle qui sont éventuellement substitués par un ou plusieurs substituants choisis indépendamment parmi les radicaux bromo, fluoro, chloro, alkyle, alkoxy, -CF₃, -OCF₃ ;
ou bien Z₁₂ représente 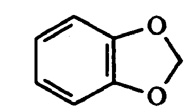 Y représente l'atome d'oxygène,
ou bien R₁ représente un radical de formule :

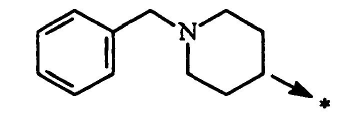

3. Composés de formule I telle que définie à l'une des revendications 1 à 2, **caractérisée en ce que** R₂ représente un radical de formule -C(Y)NHX₁, -C(O)X₂ ou SO₂X₃ dans laquelle
X₁ représente un radical (C₁-C₁₀)alkyle linéaire ou ramifié, ou -(CH₂)ₚZ₂₂ dans lequel
Z₂₂ représente cyclohexyle, cyclohexényle, *bis*-phényle, morpholino, pipéridino, mono- ou di-alkylamino, -C(O)-O-alkyle, ou phényle, naphtyle ou furyle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle ou phényle,
ou bien Z₂₂ représente un radical de formule 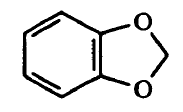 X₂ représente un radical alkynyle, -(CH₂)ₘ-W-(CH₂)_{q}-Z₂₃ ou -(CH₂)ₚZ₂₄ dans lequel
W représente SO₂;
Z₂₃ représente le radical phényle ;
Z₂₄ représente cyclohexényle, *bis*-phényle, cyclohexyle éventuellement substitué par un radical aminoalkyle, ou phényle, naphtyle, benzothiényle, thiényle ou indolyle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, -NH-C(O)-alkyle, mono- ou di-alkylamino, amino, ou
Z₂₄ représente un radical de formule 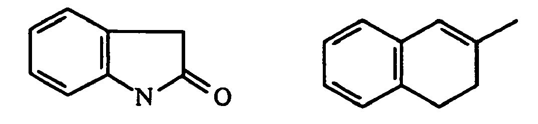 ou bien X₂ représente 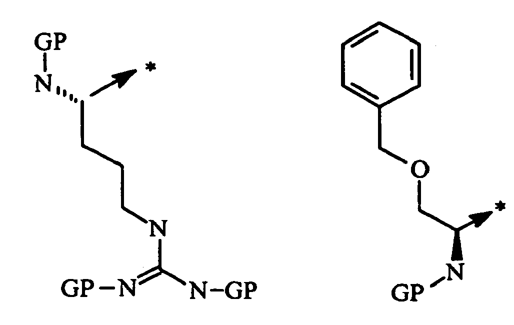 X₃ représente un radical -(CH₂)ₚZ₂₅ dans lequel Z₂₅ représente le radical phényle éventuellement substitué par un ou plusieurs radicaux identiques ou différents choisis parmi alkoxy et CF₃.

4. Composés de formule I telle que définie à l'une des revendications 1 à 3, **caractérisée en ce que** R₃ représente l'atome d'hydrogène, un radical alkyle, alkényle
ou furyl-méthyl substitué par un ou plusieurs radicaux nitro, ou un radical de formule -C(Y)-NHX₁, -C(O)X₂ ou SO₂X₃ dans laquelle
X₁ représente un radical -(CH₂)ₚZ₂₂ dans lequel
Z₂₂ représente le radical phényle ou naphtyle éventuellement substitué par un ou plusieurs radicaux choisis indépendamment parmi les radicaux fluoro, chloro, bromo, iodo, alkyle, alkoxy, CF₃, nitro, phénoxy,
X₂ représente le radical vinyle substitué par un radical phényle lui-même éventuellement substitué par un ou plusieurs radicaux halo, ou -(CH₂)ₚ-U-Z₂₄ dans lequel
Z₂₄ représente alkyle, cyclohexyle, tétrahydrofuryle, *bis*-phényle, amino, mono ou di-alkylamino, ou phényle, indolyle, thiényle, pyridinyle, benzothiényle et furyle éventuellement substitué par un ou plusieurs radicaux choisis parmi alkoxy, bromo, chloro, fluoro, amino, mono- et di-alkylamino, nitro, hydroxy, pyrrolyle
ou bien X₂ représente un radical de formule 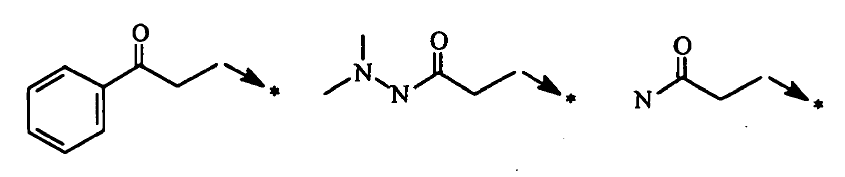 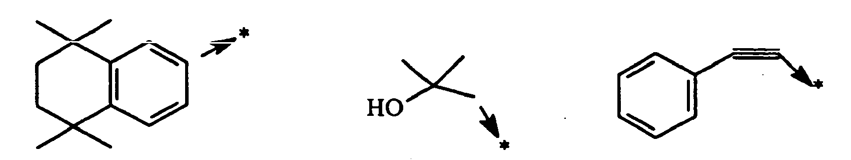 X₃ représente un radical (C₁-C₁₀)alkyle linéaire ou ramifié, le radical vinyle substitué par un radical phényle, CF₃, ou -(CH₂)ₚZ₂₅ dans lequel
Z₂₅ représente un radical phényle, napthyle, thiényle, pyrazolyle ou thiazolyle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino.

5. Composés de formule I telle que définie à l'une des revendications 1 à 4, **caractérisée en ce que** R₁ représente le radical -(CH₂)ₘZ₁₂ dans lequel m = 2 et Z₁₂ représente *bis*-phényle ou bien le radical indolyle substitués par un ou plusieurs substituants choisis indépendamment parmi les radicaux alkyle et alkoxy.

6. Composés de formule I telle que définie à l'une des revendications 1 à 5, **caractérisée en ce que** R₂ représente les radicaux de formule -C(Y)NHX₁ et -C(O)X₂ dans laquelle
Y représente S ;
X₁ représente un radical phényle éventuellement substitué par un ou plusieurs radicaux azido,
X₂ représente -(CH₂)ₚZ₂₄ dans lequel
p est égal à 1, 2 ou 3,
Z₂₄ représente cyclohexyle, ou phényle ou benzothiényle éventuellement substitué par un ou plusieurs radicaux choisis parmi fluoro, chloro, bromo, iodo ou -CF₃.

7. Composés de formule 1 telle que définie à l'une des revendications 1 à 6, **caractérisée en ce que** R₃ représente l'atome d'hydrogène ou le radical méthyle.

8. Procédé de préparation, en phase liquide, de composés de formule I telle que définie à la revendication 1, **caractérisé en ce qu'**il comprend
l'amination réductrice de la pipéridone N-substituée suivante 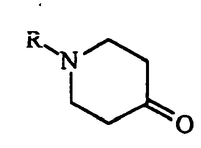 dans laquelle R représente le radical méthyle ou Boc, en présence d'une amine de formule R₁NH₂ dans laquelle R₁ a la signification indiquée à la revendication 1, pour obtenir le composé de formule 1 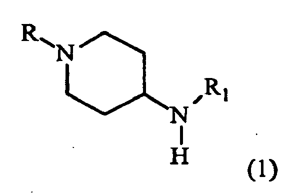 composé de formule (1) que l'on fait réagir avec
A) soit un composé de formule X₁NC(Y) dans laquelle X₁ et Y ont la signification indiquée à la revendication 1, pour obtenir un composé de formule (2) 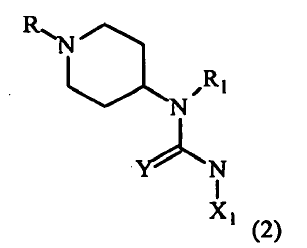 composé de formule (2) qui représente le composé de formule (I) correspondant dans lequel R₃ représente Me ou Boc et qui, lorsque R₃ représente Boc, peut être soumis à un traitement acide pour obtenir le composé de formule (I) correspondant dans laquelle R₃ représente l'atome d'hydrogène,
composé de formule (I) ainsi obtenu que l'on peut faire réagir avec un composé de formule X₁NC(Y), X₂CO₂H ou bien X₃SO₂Cl dans laquelle X₁, Y, X₂ et X₃ ont la signification indiquée à la revendication 1, pour obtenir le composé de formule I correspondant dans laquelle R₂ représente un radical de formule -C(Y)NHX₁ et R₃ le radical -C(Y)-NHX₁, -C(O)X₂ ou SO₂X₃ respectivement ;
B) soit un composé de formule X₂CO₂H dans laquelle X₂ a la signification indiquée à la revendication 1, pour obtenir un composé de formule (3) 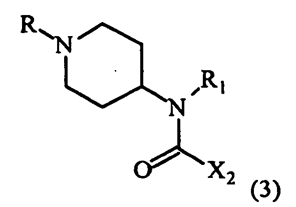 composé de formule (3) qui représente le composé de formule (I) correspondant dans lequel R₃ représente Me ou Boc et qui, lorsque R₃ représente Boc, peut être soumis à un traitement acide pour obtenir le composé de formule (I) correspondant dans laquelle R₃ représente l'atome d'hydrogène,
composé de formule (I) ainsi obtenu que l'on peut faire réagir avec un composé de formule X₁NC(Y), X₂CO₂H ou bien X₃SO₂Cl dans laquelle X₁, Y, X₂ et X₃ ont la signification indiquée à la revendication 1, pour obtenir le composé de formule I correspondant dans laquelle R₂ représente un radical de formule -C(O)X₂ et R₃ le radical -C(Y)-NHX₁, -C(O)X₂ ou SO₂X₃ respectivement.

9. Procédé de préparation, en phase solide, de composés de formule I telle que définie à la revendication 1, **caractérisé en ce qu'**il comprend
l'amination réductrice de la résine cétonique 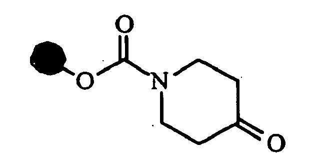 en présence d'une amine de formule R₁NH₂ dans laquelle R₁ a la signification indiquée à la revendication 1, pour obtenir le composé de formule (4) 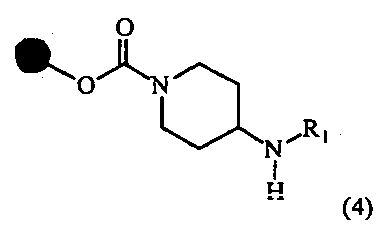 composé de formule (4) que l'on fait réagir avec
A) soit un composé de formule X₁NC(Y) dans laquelle X₁ et Y ont la signification indiquée à la revendication 1, pour obtenir un composé de formule (5) 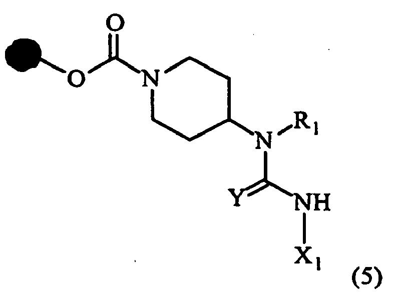 suivi du clivage de la résine pour obtenir le composé de formule (I) correspondant dans laquelle R₃ représente l'atome d'hydrogène,
B) soit un composé de formule X₃SO₂Cl dans laquelle X₃ a la signification indiquée à la revendication 1, pour obtenir un composé de formule (6) 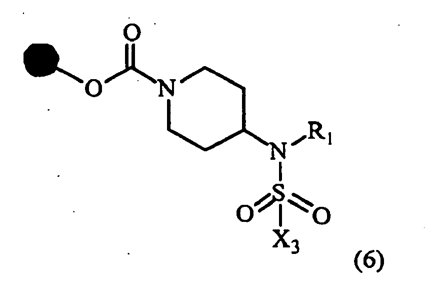 suivi du clivage de la résine pour obtenir le composé de formule (I) correspondant dans laquelle R₃ représente l'atome d'hydrogène,
C) soit un composé de formule X₂CO₂Cl dans laquelle X₂ a la signification indiquée à la revendication 1, pour obtenir un composé de formule (7) 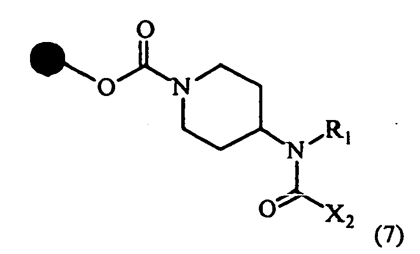 suivi du clivage de la résine pour obtenir le composé de formule (I) correspondant dans laquelle R₃ représente l'atome d'hydrogène ;
D) soit un composé de formule X₂CO₂H dans laquelle X₂ a la signification indiquée à la revendication 1, pour obtenir un composé de formule (7) tel que défini ci-dessus, suivi du clivage de la résine pour obtenir le composé de formule (I) correspondant dans laquelle R₃ représente l'atome d'hydrogène.

10. Procédé de préparation, en phase solide, de composés de formule I telle que définie à la revendication 1, **caractérisé en ce qu'**il comprend
l'amination réductrice de la résine cétonique 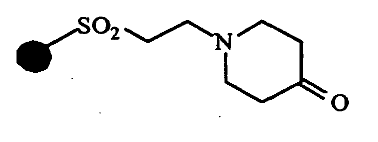 en présence d'une amine de formule R₁NH₂ dans laquelle R₁ a la signification indiquée à la revendication 1, pour obtenir le composé de formule (8) 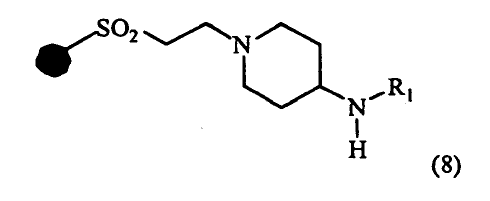 composé de formule (8) que l'on fait réagir avec
A) soit un composé de formule X₁NC(O) dans laquelle X₁ a la signification indiquée à la revendication 1, pour obtenir un composé de formule (9) 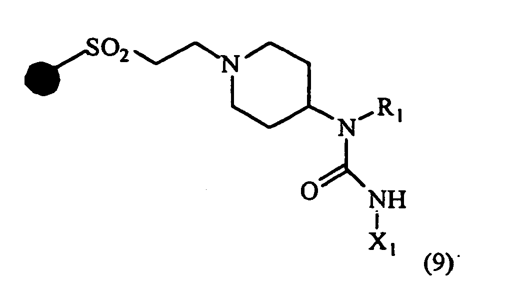 composé (9) ainsi formé que l'on fait réagir avec un composé de formule R₃X dans laquelle R₃ est tel que défini à la revendication 1 et X représente Br ou I, suivi du clivage de la résine pour obtenir le composé de formule (I) correspondant ;
B) soit un composé de formule X₃SO₂Cl dans laquelle X₃ a la signification indiquée à la revendication 1, pour obtenir un composé de formule (10) 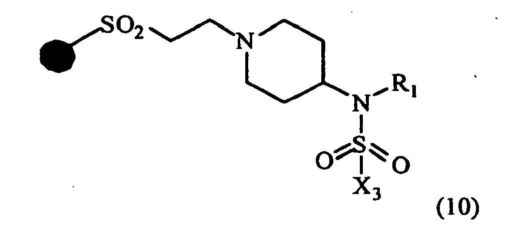 composé (10) ainsi formé que l'on fait réagir avec un composé de formule R₃X dans laquelle R₃ est tel que défini à la revendication 1 et X représente Br ou I, suivi du clivage de la résine pour obtenir le composé de formule (I) correspondant ;
C) soit un composé de formule X₂CO₂Cl dans laquelle X₂ a la signification indiquée à la revendication 1, pour obtenir un composé de formule (11) 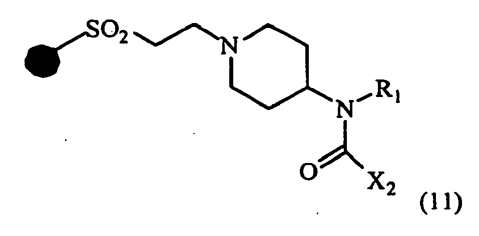 composé (11) ainsi formé que l'on fait réagir avec un composé de formule R₃X dans laquelle R₃ est tel que défini à la revendication 1 et X représente Br ou I, suivi du clivage de la résine pour obtenir le composé de formule (I) correspondant ;
D) soit un composé de formule X₂CO₂H dans laquelle X₂ a la signification indiquée à la revendication 1, pour obtenir un composé de formule (11) tel que défini ci-dessus,
composé (11) ainsi formé que l'on fait réagir avec un composé de formule R₃X dans laquelle R₃ est tel que défini à la revendication 1 et X représente Br ou I, suivi du clivage de la résine pour obtenir le composé de formule (I) correspondant.

11. A titre de médicaments, les produits de formule I telle que définie à l'une des revendications 1 à 7, ainsi que les sels d'addition avec les acides minéraux ou organiques pharmaceutiquement acceptables desdits produits de formule I.

12. Compositions pharmaceutiques contenant, à titre de principe actif, un au moins des médicaments tels que définis à la revendication 11, en association avec un support pharmaceutiquement acceptable.

13. Utilisation d'un composé de formule générale Iₐ 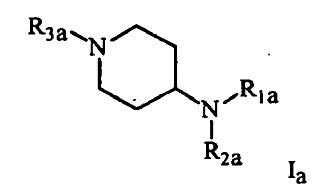 sous forme racémique, d'énantiomère ou toutes combinaisons de ces formes, dans laquelle :
R₁ₐ représente un radical (C₁-C₆)alkyle linéaire ou ramifié, le radical -(CH₂)ₘ-Y-Z₁₁
ou -(CH₂)ₘ -Z₁₂ dans lequel
Z₁₁ représente un (C₁-C₆)alkyle,
Z₁₂ représente *bis*-phényle, (C₃-C₇)cycloalkyle, (C₃-C₇)hétérocycloalkyle éventuellement substitué par un ou plusieurs substituants choisis indépendamment parmi les radicaux oxy et alkyle ;
ou aryle ou hétéroaryle éventuellement substitué par un ou plusieurs substituants choisis indépendamment parmi les radicaux fluoro, chloro, bromo, iodo, alkyle, alkoxy,
ou bien Z₁₂ représente 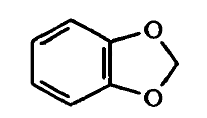 Y représente l'atome d'oxygène,
ou bien R₁ₐ représente un radical de formule 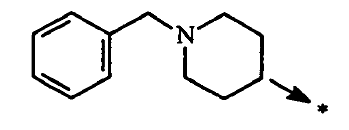
R₂ₐ représente un radical de formule -C(Y)NHX₁, -C(O)X₂ ou SO₂X₃ dans laquelle
Y représente un atome d'oxygène ou de soufre ;
X₁ représente un radical (C₁-C₁₅)alkyle linéaire ou ramifié, ou -(CH₂)ₚZ₂₂ dans lequel
Z₂₂ représente cyclohexényle, *bis*-phényle, (C₃-C₇)cycloalkyle, (C₃-C₇)hétérocycloalkyle, mono- ou di-alkylamino, -C(O)-O-alkyle, ou aryle ou hétéroaryle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle, ou phényle,
ou bien Z₂₂ représente un radical de formule 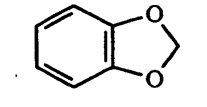 X₂ représente un radical (C₁-C₁₀)alkyle linéaire ou ramifié, alkynyle, -(CH₂)ₘ-W-(CH₂)_{q}-Z₂₃ ou -(CH₂)ₚ-U-Z₂₄ dans lequel
W représente SO₂,
U représente une liaison covalente,
Z₂₃ représente un radical aryle ;
Z₂₄ représente cyclohexényle, *bis*-phényle, (C₃-C₇)cycloalkyle éventuellement substitué par un radical aminoalkyle, ou aryle ou hétéroaryle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, mono- ou di-alkylamino, amino
ou Z₂₄ représente un radical de formule 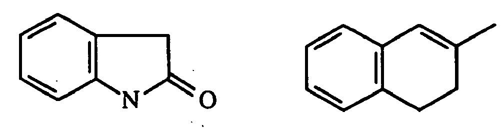 ou bien X₂ représente 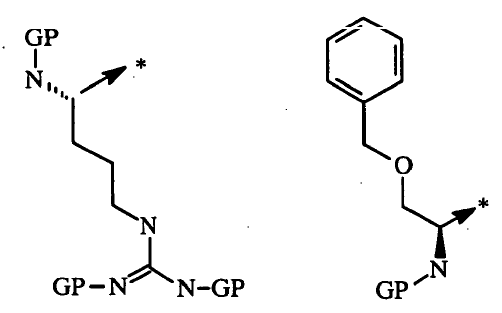 où le groupe protecteur (GP) représente H ou le *tert*-butyloxycarbonyle ;
X₃ représente un radical -(CH₂)ₚZ₂₅ dans lequel Z₂₅ représente un radical aryle éventuellement substitué par un ou plusieurs radicaux identiques ou différents choisis parmi alkoxy et CF₃ ;
R₃ₐ représente l'atome d'hydrogène, un radical alkyle, alkényle, hétéroarylalkyle éventuellement substitué ou un radical de formule -C(Y)-NHX₁, -C(O)X₂ ou SO₂X₃ dans laquelle
Y représente un atome d'oxygène ou de soufre ;
X₁ représente un radical -(CH₂)ₚZ₂₂ dans lequel
Z₂₂ représente un radical aryle éventuellement substitué par un ou plusieurs radicaux choisis indépendamment parmi les radicaux fluoro, chloro, bromo, iodo, alkyle, alkoxy, CF₃, nitro, phénoxy ;
X₂ représente le radical vinyle substitué par un phényle, le radical phényle étant lui-même éventuellement substitué par un ou plusieurs radicaux halo, ou -(CH₂)ₚ-U-Z₂₄ dans lequel
U représente une liaison covalente ou l'atome d'oxygène ;
Z₂₄ représente alkyle, (C₃-C₇)cycloalkyle, (C₃-C₇)hétérocycloalkyle, *bis*-phényle, amino, mono ou di-alkylamino, ou aryle ou hétéroaryle éventuellement substitué par un ou plusieurs radicaux choisis parmi alkoxy, bromo, chloro, fluoro, hydroxy, CF₃, nitro, amino, mono- et di-alkylamino, pyrrolyle,
ou bien X₂ représente un radical de formule 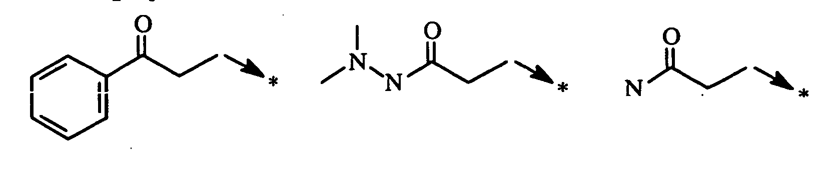 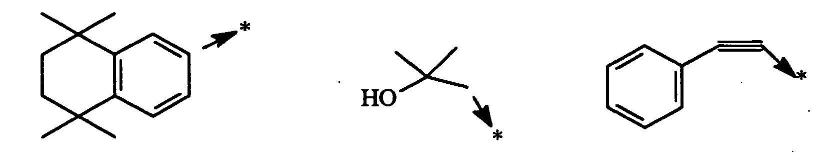 X₃ représente un radical (C₁-C₁₀)alkyle linéaire ou ramifié, le radical vinyle substitué par un radical (le radical phényle étant lui-même éventuellement substitué), CF₃, ou -(CH₂)ₚZ₂₅ dans lequel
Z₂₅ représente aryle ou hétéroaryle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino
m est un entier de 1 à 6 ;
p est un entier de 0 à 6 ;
q est un entier de 0 à 2,
ou leurs sels d'addition avec les acides minéraux ou organiques pharmaceutiquement acceptables, pour la préparation d'un médicament destiné à traiter les états pathologiques ou les maladies dans lesquels un (ou plusieurs) des récepteurs de la somatostatine est(sont) impliqué(s)

14. Utilisation des produits de formule générale Iₐ selon la revendication 13, **caractérisée en ce que** R₁ₐ représente un radical (C₁-C₆)alkyle linéaire ou ramifié, le radical -(CH₂)ₘ-Y-Z₁₁ ou -(CH₂)ₘ-Z₁₂ dans lequel
Z₁₁ représente un (C₁-C₆)alkyle,
Z₁₂ représente naphtyle, morpholino, *bis*-phényle, pyrrolidinyle substitué par le radical oxy, ou bien les radicaux phényle, pipérazinyle, pyridinyle et indolyle qui sont éventuellement substitués par un ou plusieurs substituants choisis indépendamment parmi les radicaux bromo, fluoro, chloro, alkyle, alkoxy, -CF₃, -OCF₃;
ou bien Z₁₂ représente 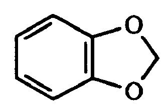 Y représente l'atome d'oxygène,
ou bien R₁ₐ représente un radical de formule ci-dessous :

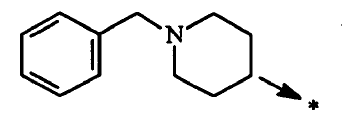

15. Utilisation des produits de formule générale Iₐ selon l'une des revendications 13 à 14, **caractérisée en ce que** R₂ₐ représente un radical de formule -C(Y)NHX₁, -C(O)X₂ ou SO₂X₃ dans laquelle
X₁ représente un radical (C₁-C₁₀)alkyle linéaire ou ramifié, ou -(CH₂)ₚZ₂₂ dans lequel
Z₂₂ représente cyclohexyle, cyclohexényle, *bis*-phényle, morpholino, pipéridino, mono- ou di-alkylamino, -C(O)-O-alkyle, ou phényle, naphtyle ou furyle éventuellement substitué par un ou plusieurs radicaux choisis indépendamment parmi les radicaux fluoro, chloro, bromo, iodo, alkyle, alkoxy, alkylthio, CF₃, OCF₃, nitro, cyano, azido, pipéridinosulfonyle, -C(O)-O-alkyle, -C(O)-alkyle ou phényle,
ou bien Z₂₂ représente un radical de formule 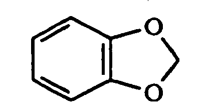 X₂ représente un radical alkyle, alkynyle, -(CH₂)ₘ-W-(CH₂)_{q}-Z₂₃ ou -(CH₂)ₚZ₂₄ dans lequel
W représente SO₂ ;
Z₂₃ représente le radical phényle ;
Z₂₄ représente cyclohexényle, *bis*-phényle, cyclohexyle éventuellement substitué par un radical aminoalkyle, ou phényle, naphtyle, benzothiényle, thiényle ou indolyle éventuellement substitué par un ou plusieurs radicaux choisis parmi fluoro, chloro, bromo, iodo, alkyle, alkoxy, -CF₃, -OCF₃, SCF₃, hydroxy, -O-C(O)-alkyle, -NH-C(O)-alkyle, mono- ou di-alkylamino, amino, ou
Z₂₄ représente un radical de formule 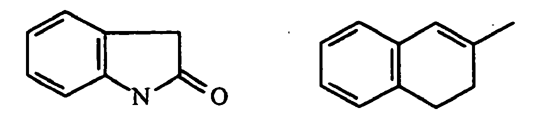 ou bien X₂ représente 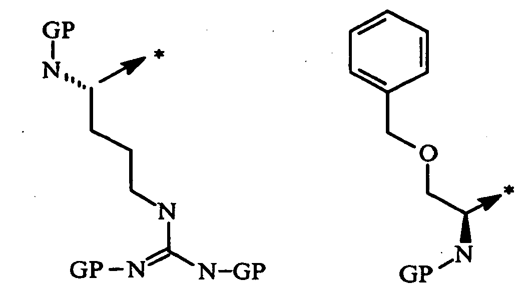 X₃ représente un radical -(CH₂)ₚZ₂₅ dans lequel Z₂₅ représente le radical phényle éventuellement substitué par un ou plusieurs radicaux identiques ou différents choisis parmi alkoxy et CF₃.

16. Utilisation des produits de formule générale la selon l'une des revendications 13 à 15, **caractérisée en ce que** R₃ₐ représente l'atome d'hydrogène, un radical alkyle, alkényle ou furyl-méthyl substitué par un ou plusieurs radicaux nitro, ou un radical de formule -C(Y)-NHX₁, -C(O)X₂ ou SO₂X₃ dans laquelle
X₁ représente un radical -(CH₂)ₚZ₂₂ dans lequel
Z₂₂ représente le radical phényle ou naphtyle éventuellement substitué par un ou plusieurs radicaux choisis indépendamment parmi les radicaux fluoro, chloro, bromo, iodo, alkyle, alkoxy, CF₃, nitro, phénoxy,
X₂ représente le radical vinyle substitué par un radical phényle lui-même éventuellement substitué par un ou plusieurs radicaux halo, ou -(CH₂)ₚ-U-Z₂₄ dans lequel
Z₂₄ représente alkyle, cyclohexyle, tétrahydrofuryle, *bis*-phényle, amino, mono ou di-alkylamino, ou phényle, indolyle, thiényle, pyridinyle, benzothiényle et furyle éventuellement substitué par un ou plusieurs radicaux choisis parmi alkoxy, bromo, chloro, fluoro, amino, mono- et di-alkylamino, nitro, hydroxy, pyrrolyle
ou bien X₂ représente un radical de formule 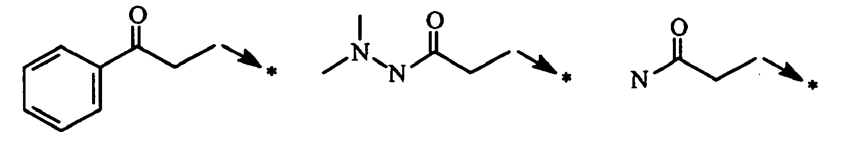 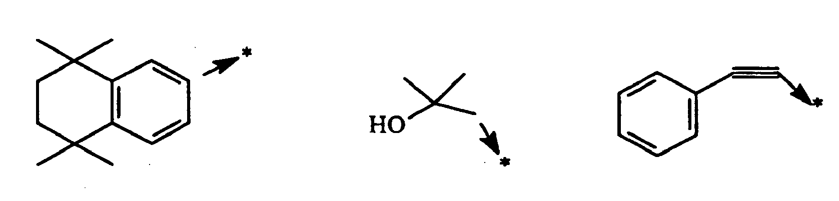 X₃ représente un radical (C₁-C₁₀)alkyle linéaire ou ramifié, le radical vinyle substitué par un radical phényle, CF₃, ou -(CH₂)ₚZ₂₅ dans lequel
Z₂₅ représente un radical phényle, napthyle, thiényle, pyrazolyle ou thiazolyle éventuellement substitué par un ou plusieurs substituants choisis indépendamment parmi les radicaux fluoro, chloro, bromo, iodo, alkyle, alkoxy, CF₃, nitro, -NH-C(O)-alkyle, mono- et di-alkylamino.

17. Utilisation des produits de formule générale Iₐ selon l'une des revendications 13 à 16, **caractérisée en ce que** R₁ₐ représente le radical -(CH₂)ₘZ₁₂ dans lequel m = 2 et Z₁₂ représente *bis*-phényle ou bien le radical indolyle substitués par un ou plusieurs substituants choisis indépendamment parmi les radicaux alkyle et alkoxy.

18. Utilisation des produits de formule générale Iₐ selon l'une des revendications 13 à 17, **caractérisée en ce que** R₂ₐ représente les radicaux de formule -C(Y)NHX₁ et -C(O)X₂ dans laquelle
Y représente S ;
X₁ représente un radical phényle éventuellement substitué par un ou plusieurs radicaux azido,
X₂ représente -(CH₂)ₚZ₂₄ dans lequel
p est égal à 1, 2 ou 3,
Z₂₄ représente cyclohexyle, ou phényle ou benzothiényle éventuellement substitué par un ou plusieurs radicaux choisis parmi fluoro, chloro, bromo, iodo ou -CF₃.

19. Utilisation des produits de formule générale Iₐ selon l'une des revendications 13 à 18, **caractérisée en ce que** R₃ₐ représente l'atome d'hydrogène ou le radical méthyle.

20. Utilisation selon l'une des revendications 13 à 19 pour la préparation d'un médicament destiné à traiter l'acromégalie, des adénomes hypophysaires ou des tumeurs gastroentéropancréatiques endocriniennes dont le syndrome carcinoïde.

## Claims

1. Compounds of general formula 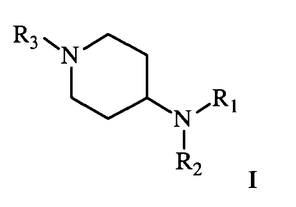 in racemic, enantiomeric form or all combinations of these forms, in which
either
R₁ represents a linear or branched (C₁-C₆)alkyl radical, the -(CH₂)ₘ-Y-Z₁₁ or -(CH₂)ₘ-Z₁₂ radical in which
Z₁₁ represents a (C₁-C₆)alkyl,
Z₁₂ represents *bis*-phenyl, (C₃-C₇)cycloalkyl, (C₃-C₇)heterocycloalkyl optionally substituted by one or more substituents chosen independently from the oxy and alkyl radicals; or aryl or heteroaryl optionally substituted by one or more substituents chosen independently from the fluoro, chloro, bromo, iodo, alkyl, alkoxy radicals,
or Z₁₂ represents 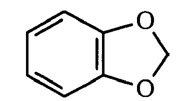 Y represents the oxygen atom,
or R₁ represents a radical of formula 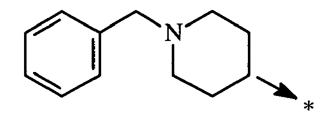 R₂ represents a radical of formula -C(Y)NHX₁, -C(O)X₂ or SO₂X₃ in which
Y represents an oxygen or sulphur atom;
X₁ represents a linear or branched (C₁-C₁₅)alkyl radical, or -(CH₂)ₚZ₂₂ in which
Z₂₂ represents cyclohexenyl, *bis*-phenyl, (C₃-C₇)cycloalkyl, (C₃-C₇)heterocycloalkyl, mono- or di-alkylamino, -C(O)-O-alkyl, or aryl or heteroaryl optionally substituted by one or more radicals chosen independently from the fluoro, chloro, bromo, iodo, alkyl, alkoxy, alkylthio, CF₃, OCF₃, nitro, cyano, azido, piperidinosulphonyl, -C(O)-O-alkyl, -C(O)-alkyl, or phenyl radicals,
or Z₂₂ represents a radical of formula 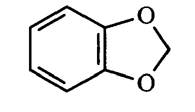 X₂ represents a alkynyl, -(CH₂)ₘ-W-(CH₂)_{q}-Z₂₃ or -(CH₂)ₚ-U-Z₂₄ radical in which
W represents SO₂,
U represents a covalent bond,
Z₂₃ represents an aryl radical;
Z₂₄ represents cyclohexenyl, *bis*-phenyl, (C₃-C₇)cycloalkyl optionally substituted by an aminoalkyl, or aryl or heteroaryl radical optionally substituted by one or more radicals chosen from fluoro, chloro, bromo, iodo, alkyl, alkoxy, -CF₃, -OCF₃, SCF₃, hydroxy, -O-C(O)-alkyl, mono- or di-alkylamino, amino
or Z₂₄ represents a radical of formula 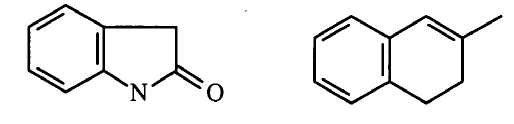 or X₂ represents 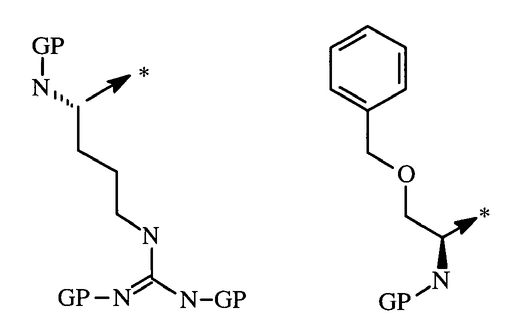 where the protective group (PG) represents H or *tert*-butyloxycarbonyl;
X₃ represents a-(CH₂)ₚZ₂₅ radical in which Z₂₅ represents an aryl radical optionally substituted by one or more identical or different radicals chosen from alkoxy and CF₃,
R₃ represents the hydrogen atom, an alkyl, alkenyl, heteroarylalkyl radical optionally substituted or a radical of formula -C(Y)-NHX₁, -C(O)X₂ or SO₂X₃ in which
Y represents an oxygen or sulphur atom;
X₁ represents a-(CH₂)ₚZ₂₂ radical in which
Z₂₂ represents an aryl radical optionally substituted by one or more radicals chosen independently from the fluoro, chloro, bromo, iodo, alkyl, alkoxy, CF₃, nitro, phenoxy radicals;
X₂ represents the vinyl radical substituted by a phenyl, the phenyl radical being itself optionally substituted by one or more halo, or -(CH₂)ₚ-U-Z₂₄ radicals in which
U represents a covalent bond or the oxygen atom;
Z₂₄ represents alkyl, (C₃-C₇)cycloalkyl, (C₃-C₇)heterocycloalkyl, *bis*-phenyl, amino, mono or di-alkylamino, or aryl or heteroaryl optionally substituted by one or more radicals chosen from alkoxy, bromo, chloro, fluoro, hydroxy, CF₃, nitro, amino, mono- and di-alkylamino, pyrrolyl,
or X₂ represents a radical of formula 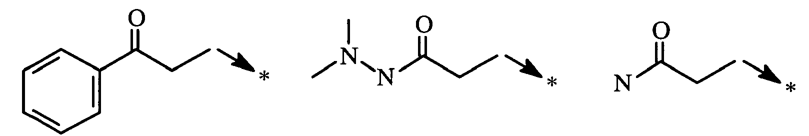 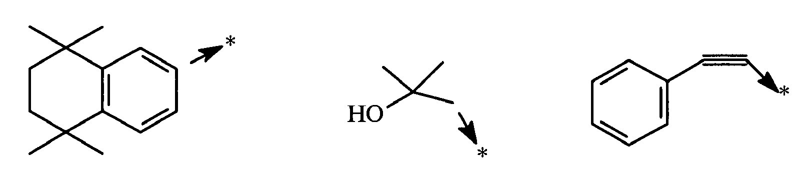 X₃ represents a linear or branched (C₁-C₁₀)alkyl radical, the vinyl radical substituted by a radical (the phenyl radical being itself optionally substituted), CF₃, or -(CH₂)ₚZ₂₅ in which
Z₂₅ represents aryl or heteroaryl optionally substituted by one or more substituents chosen independently from the fluoro, chloro, bromo, iodo, alkyl, alkoxy, CF₃, nitro, -NH-C(O)-alkyl, mono- and di-alkylamino radicals,
m is an integer from 1 to 6;
p is an integer from 0 to 6;
q is an integer from 0 to 2,
or
the radicals R₁, R₂ and R₃ represent respectively the following radicals :
| R₁ | R₂ | R3 |
|---|---|---|
| 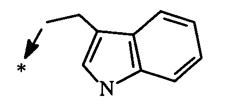 | 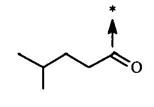 | 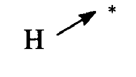 |
| 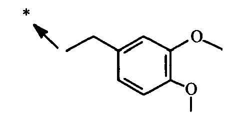 | 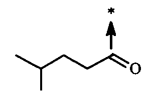 | 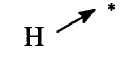 |
| 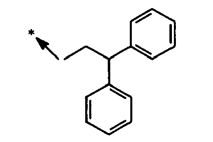 | 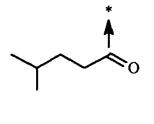 | 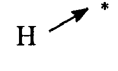 |
| 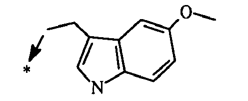 | 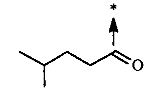 | 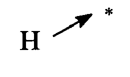 |
| 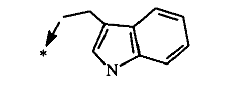 | 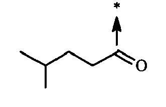 | 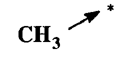 |
| 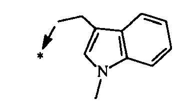 | 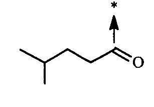 | 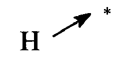 |
| 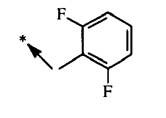 | 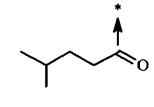 | 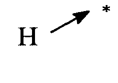 |
| 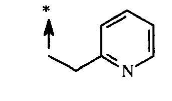 | 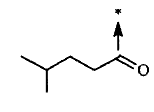 | 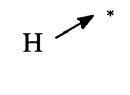 |
or their addition salts with pharmaceutically acceptable mineral or organic acids.

2. Compounds of general formula I according to claim 1, wherein R₁ represents a linear or branched (C₁-C₆)alkyl radical, the -(CH₂)ₘ-Y-Z₁₁ or -(CH₂)ₘ-Z₁₂ radical in which
Z₁₁ represents a (C₁-C₆)alkyl,
Z₁₂ represents naphthyl, morpholino, *bis*-phenyl, pyrrolidinyl substituted by the oxy radical, or the phenyl, piperazinyl, pyridinyl and indolyl radicals which are optionally substituted by one or more substituents chosen independently from the bromo, fluoro, chloro, alkyl, alkoxy, -CF₃, -OCF₃ radicals;
or Z₁₂ represents 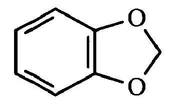 Y represents the oxygen atom,
or R₁ represents a radical of formula given below:

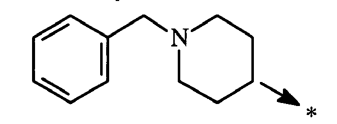

3. Compounds of general formula I according to any of claims 1 to 2, wherein R₂ represents a radical of formula -C(Y)NHX₁, -C(O)X₂ or SO₂X₃ in which
X₁ represents a linear or branched (C₁-C₁₀)alkyl, or -(CH₂)ₚZ₂₂ radical in which
Z₂₂ represents cyclohexyl, cyclohexenyl, *bis*-phenyl, morpholino, piperidino, mono- or di-alkylamino, -C(O)-O-alkyl, or phenyl, naphthyl or furyl optionally substituted by one or more radicals chosen independently from the fluoro, chloro, bromo, iodo, alkyl, alkoxy, alkylthio, CF₃, -OCF₃, nitro, cyano, azido, piperidinosulphonyl, -C(O)-O-alkyl, -C(O)-alkyl or phenyl radicals,
or Z₂₂ represents a radical of formula 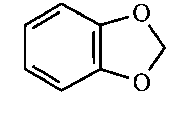 X₂ represents an alkynyl, -(CH₂)ₘ-W-(CH₂)_{q}-Z₂₃ or -(CH₂)ₚZ₂₄ radical in which
W represents SO₂;
Z₂₃ represents the phenyl radical;
Z₂₄ represents cyclohexenyl, *bis*-phenyl, cyclohexyl optionally substituted by an aminoalkyl, or phenyl, naphthyl, benzothienyl, thienyl or indolyl radical optionally substituted by one or more radicals chosen from fluoro, chloro, bromo, iodo, alkyl, alkoxy, -CF₃, -OCF₃, -SCF₃, hydroxy, -O-C(O)-alkyl, -NH-C(O)-alkyl, mono- or di-alkylamino, amino, or
Z₂₄ represents a radical of formula 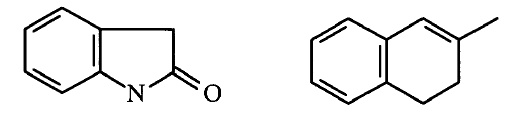 or X₂ represents 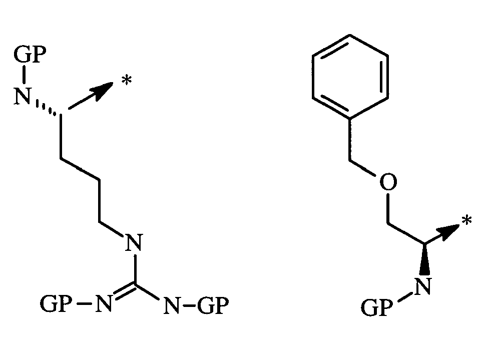 X₃ represents a -(CH₂)ₚZ₂₅ radical in which Z₂₅ represents the phenyl radical optionally substituted by one or more identical or different radicals chosen from alkoxy and CF₃,

4. Compounds of general formula I according to any of claims 1 to 3, wherein R₃ represents the hydrogen atom, an alkyl, alkenyl or furyl-methyl radical substituted by one or more nitro radicals, or a radical of formula -C(Y)-NHX₁, -C(O)X₂ or SO₂X₃ in which
X₁ represents a -(CH₂)ₚZ₂₂ radical in which
Z₂₂ represents the phenyl or naphthyl radical optionally substituted by one or more radicals chosen independently from the fluoro, chloro, bromo, iodo, alkyl, alkoxy, CF₃, nitro, phenoxy radicals,
X₂ represents the vinyl radical substituted by a phenyl radical itself optionally substituted by one or more halo, or -(CH₂)ₚ-U-Z₂₄ radicals in which
Z₂₄ represents alkyl, cyclohexyl, tetrahydrofuryl, *bis*-phenyl, amino, mono or di-alkylamino, or phenyl, indolyl, thienyl, pyridinyl, benzothienyl and furyl optionally substituted by one or more radicals chosen from alkoxy, bromo, chloro, fluoro, amino, mono- and di-alkylamino, nitro, hydroxy, pyrrolyl
or X₂ represents a radical of formula 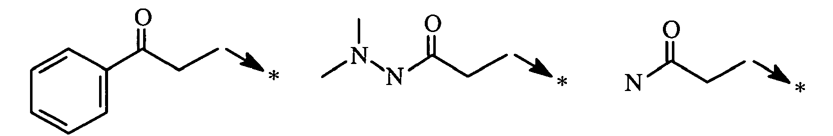 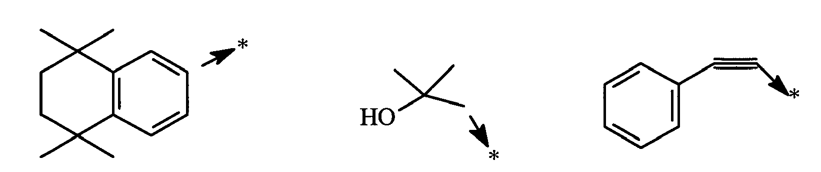 X₃ represents a linear or branched (C₁-C₁₀)alkyl radical, the vinyl radical substituted by a phenyl, CF₃, or -(CH₂)ₚZ₂₅ radical in which
Z₂₅ represents a phenyl, naphthyl, thienyl, pyrazolyl or thiazolyl radical optionally substituted by one or more substituents chosen independently from the fluoro, chloro, bromo, iodo, alkyl, alkoxy, CF₃, nitro, -NH-C(O)-alkyl, mono- and di-alkylamino radicals;

5. Compounds of general formula I according to any of claims 1 to 4, wherein R₁ represents the -(CH₂)ₘZ₁₂ radical in which m = 2 and Z₁₂ represents *bis*-phenyl or the radical indolyl substituted by one or more substituents chosen independently from the alkyl and alkoxy radicals.

6. Compounds of general formula I according to any of claims 1 to 5, wherein R₂ represents the radicals of formula -C(Y)NHX₁ and -C(O)X₂ in which
Y represents S;
X₁ represents a phenyl radical optionally substituted by one or more azido radicals,
X₂ represents -(CH₂)ₚZ₂₄ in which
p is equal to 1, 2 or 3,
Z₂₄ represents cyclohexyl, or phenyl or benzothienyl optionally substituted by one or more radicals chosen from fluoro, chloro, bromo, iodo or -CF₃.

7. Compounds of general formula I according to any of claims 1 to 6, wherein R₃ represents the hydrogen atom or the methyl radical.

8. Process for the preparation, in liquid phase, of compounds of formula I according to claim 1, **characterized in that** it comprises
the reducing amination of the following N-substituted piperidone 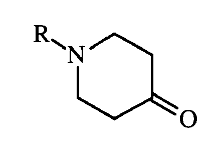 in which R represents the methyl or Boc radical, in the presence of an amine of formula R₁NH₂ in which R₁ has the meaning indicated in claim 1, in order to obtain the compound of formula 1 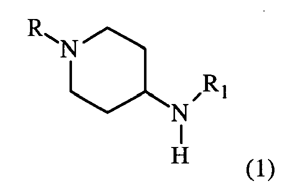 which compound of formula (1) is reacted with
A) either a compound of formula X₁NC(Y) in which X₁ and Y have the meaning indicated in claim 1, in order to obtain a compound of formula (2) 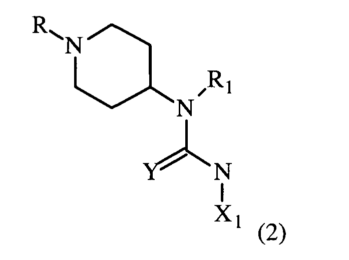 which compound of formula (2) represents the corresponding compound of formula (I) in which R₃ represents Me or Boc and which, when R₃ represents Boc, can be subjected to an acid treatment in order to obtain the corresponding compound of formula (I) in which R₃ represents the hydrogen atom,
which compound of formula (I) thus obtained can be reacted with a compound of formula X₁NC(Y), X₂CO₂H or X₃SO₂Cl in which X₁, Y, X₂ and X₃ have the meaning indicated in claim 1, in order to obtain the corresponding compound of formula I in which R₂ represents a radical of formula -C(Y)NHX₁ and R₃ the-C(Y)-NHX₁, -C(O)X₂ or SO₂X₃ radical respectively;
B) or a compound of formula X₂CO₂H in which X₂ has the meaning indicated in claim 1, in order to obtain a compound of formula (3) 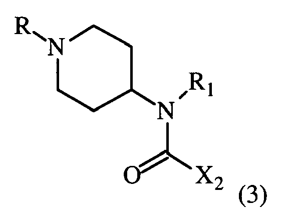 which compound of formula (3) represents the corresponding compound of formula (I) in which R₃ represents Me or Boc and which, when R₃ represents Boc, can be subjected to an acid treatment in order to obtain the corresponding compound of formula (I) in which R₃ represents the hydrogen atom, which compound of formula (I) thus obtained can be reacted with a compound of formula X₁NC(Y), X₂CO₂H or X₃SO₂Cl in which X₁, Y, X₂ and X₃ have the meaning indicated in claim 1, in order to obtain the corresponding compound of formula I in which R₂ represents a radical of formula -C(O)X₂ and R₃ the-C(Y)-NHX₁, -C(O)X₂ or SO₂X₃ radical respectively.

9. Preparation process, in solid phase, for compounds of formula I according to claim 1, **characterized in that** it comprises
the reducing amination of the ketonic resin 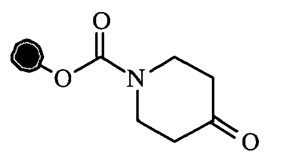 in the presence of an amine of formula R₁NH₂ in which R₁ has the meaning indicated in claim1, in order to obtain the compound of formula (4) 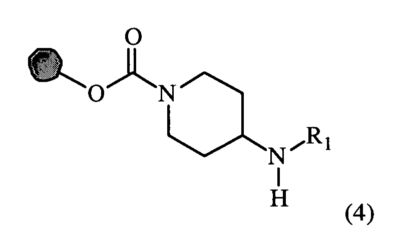 which compound of formula (4) is reacted with
A) either a compound of formula X₁NC(Y) in which X₁ and Y have the meaning indicated in claim 1, in order to obtain a compound of formula (5) 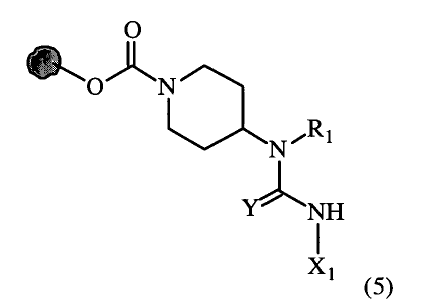 followed by cleavage of the resin in order to obtain the corresponding compound of formula (I) in which R₃ represents the hydrogen atom,
B) or a compound of formula X₃SO₂Cl in which X₃ has the meaning indicated in claim 1, in order to obtain a compound of formula (6) 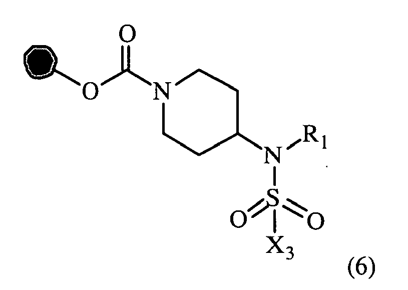 followed by cleavage of the resin in order to obtain the corresponding compound of formula (I) in which R₃ represents the hydrogen atom,
C) or a compound of formula X₂CO₂Cl in which X₂ has the meaning indicated in claim 1, in order to obtain a compound of formula (7) 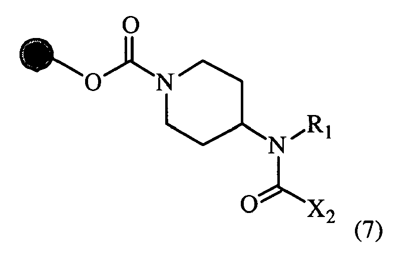 followed by cleavage of the resin in order to obtain the corresponding compound of formula (I) in which R₃ represents the hydrogen atom;
D) or a compound of formula X₂CO₂H in which X₂ has the meaning indicated in claim 1, in order to obtain a compound of formula (7) as defined above, followed by cleavage of the resin in order to obtain the corresponding compound of formula (I) in which R₃-represents the hydrogen atom.

10. Preparation process, in solid phase, for compounds of formula I according to claim 1, **characterized in that** it comprises
the reducing amination of the ketonic resin 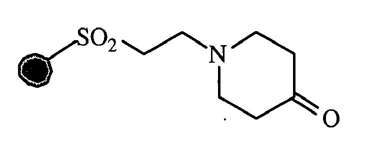 in the presence of an amine of formula R₁NH₂ in which R₁ has the meaning indicated in claim 1, in order to obtain the compound of formula (8) 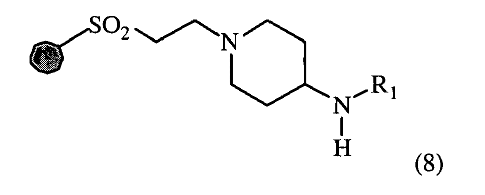 which compound of formula (8) is reacted with
A) either a compound of formula X₁NC(O) in which X₁ has the meaning indicated in claim 1, in order to obtain a compound of formula (9) 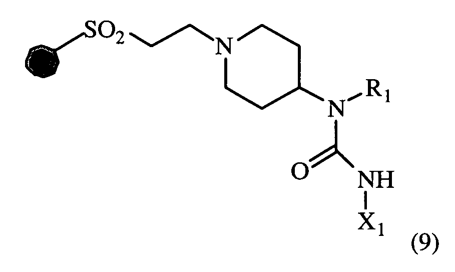 which compound (9) thus formed is reacted with a compound of formula R₃X in which R₃ is as defined in claim 1 and X represents Br or I, followed by cleavage of the resin in order to obtain the corresponding compound of formula (I);
B) or a compound of formula X₃SO₂Cl in which X₃ has the meaning indicated in claim 1, in order to obtain a compound of formula (10) 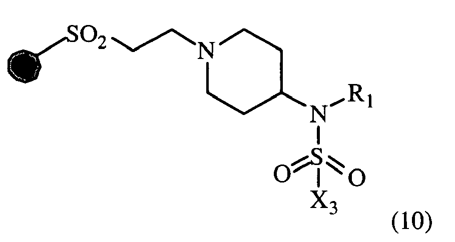 which compound (10) thus formed is reacted with a compound of formula R₃X in which R₃ is as defined in claim 1 and X represents Br or I, followed by cleavage of the resin in order to obtain the corresponding compound of formula (I);
C) or a compound of formula X₂CO₂Cl in which X₂ has the meaning indicated in claim 1, in order to obtain a compound of formula (11) 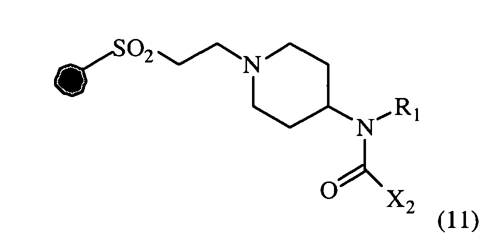 which compound (11) thus formed is reacted with a compound of formula R₃X in which R₃ is as defined in claim 1 and X represents Br or I, followed by cleavage of the resin in order to obtain the corresponding compound of formula (I);
D) or a compound of formula X₂CO₂H in which X₂ has the meaning indicated in claim 1, in order to obtain a compound of formula (11) as defined above,
which compound (11) thus formed is reacted with a compound of formula R₃X in which R₃ is as defined in claim 1 and X represents Br or I, followed by cleavage of the resin in order to obtain the corresponding compound of formula (I).

11. As medicaments, the products of formula I according to claims 1 to 7, as well as the addition salts with pharmaceutically acceptable mineral or organic acids of said products of formula I.

12. Pharmaceutical compositions containing, as active ingredient, at least one of the medicaments according to claim 11, in combination with a pharmaceutically acceptable support.

13. Use of compounds of general formula Iₐ 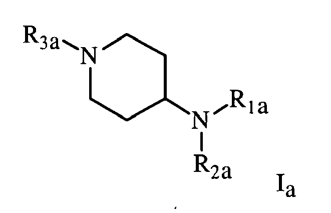 in racemic, enantiomeric form or all combinations of these forms, in which:
R₁ₐ represents a linear or branched (C₁-C₆)alkyl radical, the -(CH₂)ₘ-Y-Z₁₁ or -(CH₂)ₘ-Z₁₂ radical in which
Z₁₁ represents a (C₁-C₆)alkyl,
Z₁₂ represents *bis*-phenyl, (C₃-C₇)cycloalkyl, (C₃-C₇)heterocycloalkyl optionally substituted by one or more substituents chosen independently from the oxy and alkyl radicals; or aryl or heteroaryl optionally substituted by one or more substituents chosen independently from the fluoro, chloro, bromo, iodo, alkyl, alkoxy radicals,
or Z₁₂ represents 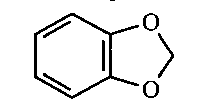 Y represents the oxygen atom,
or R₁ₐ represents a radical of formula 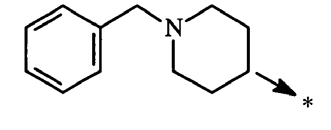
R₂ₐ represents a radical of formula -C(Y)NHX₁, -C(O)X₂ or SO₂X₃ in which
Y represents an oxygen or sulphur atom;
X₁ represents a linear or branched (C₁-C₁₅)alkyl radical, or -(CH₂)ₚZ₂₂ in which
Z₂₂ represents cyclohexenyl, *bis*-phenyl, (C₃-C₇)cycloalkyl, (C₃-C₇)heterocycloalkyl, mono- or di-alkylamino, -C(O)-O-alkyl, or aryl or heteroaryl optionally substituted by one or more radicals chosen independently from the fluoro, chloro, bromo, iodo, alkyl, alkoxy, alkylthio, CF₃, OCF₃, nitro, cyano, azido, piperidinosulphonyl, -C(O)-O-alkyl, -C(O)-alkyl, or phenyl radicals,
or Z₂₂ represents a radical of formula 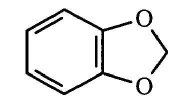 X₂ represents a linear or branched (C₁-C₁₀)alkyl, alkynyl, -(CH₂)ₘ-W-(CH₂)_{q}-Z₂₃ or -(CH₂)ₚ-U-Z₂₄ radical in which
W represents SO₂,
U represents a covalent bond,
Z₂₃ represents an aryl radical;
Z₂₄ represents cyclohexenyl, *bis*-phenyl, (C₃-C₇)cycloalkyl optionally substituted by an aminoalkyl, or aryl or heteroaryl radical optionally substituted by one or more radicals chosen from fluoro, chloro, bromo, iodo, alkyl, alkoxy, -CF₃, -OCF₃, SCF₃, hydroxy, -O-C(O)-alkyl, mono- or di-alkylamino, amino
or Z₂₄ represents a radical of formula 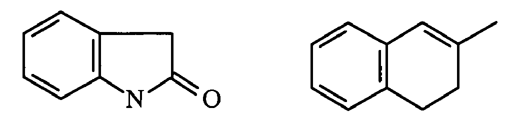 or X₂ represents 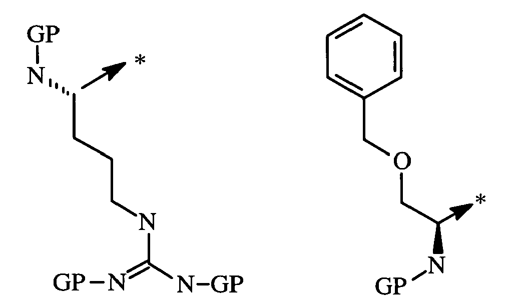 where the protective group (PG) represents H or *tert*-butyloxycarbonyl;
X₃ represents a-(CH₂)ₚZ₂₅ radical in which Z₂₅ represents an aryl radical optionally substituted by one or more identical or different radicals chosen from alkoxy and CF₃;
R₃ₐ represents the hydrogen atom, an alkyl, alkenyl, heteroarylalkyl radical optionally substituted or a radical of formula -C(Y)-NHX₁, -C(O)X₂ or SO₂X₃ in which
Y represents an oxygen or sulphur atom;
X₁ represents a-(CH₂)ₚZ₂₂ radical in which
Z₂₂ represents an aryl radical optionally substituted by one or more radicals chosen independently from the fluoro, chloro, bromo, iodo, alkyl, alkoxy, CF₃, nitro, phenoxy radicals;
X₂ represents the vinyl radical substituted by a phenyl, the phenyl radical being itself optionally substituted by one or more halo, or -(CH₂)ₚ-U-Z₂₄ radicals in which
U represents a covalent bond or the oxygen atom;
Z₂₄ represents alkyl, (C₃-C₇)cycloalkyl, (C₃-C₇)heterocycloalkyl, *bis-*phenyl, amino, mono or di-alkylamino, or aryl or heteroaryl optionally substituted by one or more radicals chosen from alkoxy, bromo, chloro, fluoro, hydroxy, CF₃, nitro, amino, mono- and di-alkylamino, pyrrolyl,
or X₂ represents a radical of formula 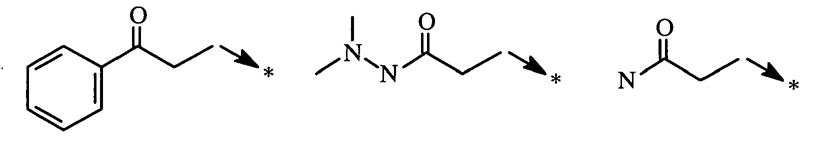 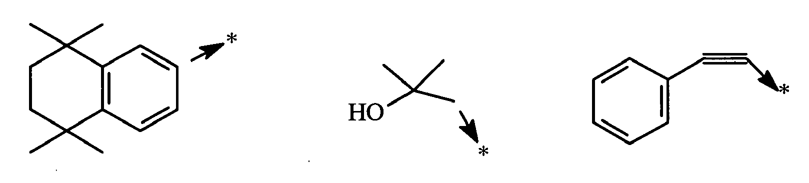 X₃ represents a linear or branched (C₁-C₁₀)alkyl radical, the vinyl radical substituted by a radical (the phenyl radical being itself optionally substituted), CF₃, or -(CH₂)ₚZ₂₅ in which
Z₂₅ represents aryl or heteroaryl optionally substituted by one or more substituents chosen independently from the fluoro, chloro, bromo, iodo, alkyl, alkoxy, CF₃, nitro, -NH-C(O)-alkyl, mono- and di-alkylamino radicals;
m is an integer from 1 to 6;
p is an integer from 0 to 6;
q is an integer from 0 to 2,
or their addition salts with pharmaceutically acceptable mineral or organic acids, for the preparation of a medicament intended to treat pathological states or diseases in which one (or more) receptor(s) of somatostatin is (are) involved.

14. Use of compounds of general formula Iₐ according to claim 13 in which R₁ₐ represents a linear or branched (C₁-C₆)alkyl radical, the -(CH₂)ₘ-Y-Z₁₁ or -(CH₂)ₘ-Z₁₂ radical in which
Z₁₁ represents a (C₁-C₆)alkyl,
Z₁₂ represents naphthyl, morpholino, *bis*-phenyl, pyrrolidinyl substituted by the oxy radical, or the phenyl, piperazinyl, pyridinyl and indolyl radicals which are optionally substituted by one or more substituents chosen independently from the bromo, fluoro, chloro, alkyl, alkoxy, -CF₃, -OCF₃ radicals;
or Z₁₂ represents 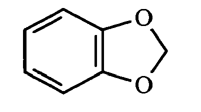 Y represents the oxygen atom,
or R₁ₐ represents a radical of formula given below:

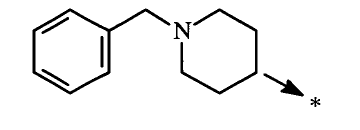

15. Use of compounds of general formula Iₐ according to any of claims 13 to 14 in which R₂ₐ represents a radical of formula -C(Y)NHX₁, -C(O)X₂ or SO₂X₃ in which
X₁ represents a linear or branched (C₁-C₁₀)alkyl, or -(CH₂)ₚZ₂₂ radical in which
Z₂₂ represents cyclohexyl, cyclohexenyl, *bis*-phenyl, morpholino, piperidino, mono- or di-alkylamino, -C(O)-O-alkyl, or phenyl, naphthyl or furyl optionally substituted by one or more radicals chosen independently from the fluoro, chloro, bromo, iodo, alkyl, alkoxy, alkylthio, CF₃, OCF₃, nitro, cyano, azido, piperidinosulphonyl, -C(O)-O-alkyl, -C(O)-alkyl or phenyl radicals,
or Z₂₂ represents a radical of formula 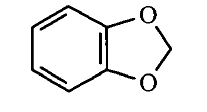 X₂ represents an alkyl, alkynyl, -(CH₂)ₘ-W-(CH₂)_{q}-Z₂₃ or -(CH₂)ₚZ₂₄ radical in which
W represents SO₂;
Z₂₃ represents the phenyl radical;
Z₂₄ represents cyclohexenyl, *bis*-phenyl, cyclohexyl optionally substituted by an aminoalkyl, or phenyl, naphthyl, benzothienyl, thienyl or indolyl radical optionally substituted by one or more radicals chosen from fluoro, chloro, bromo, iodo, alkyl, alkoxy, -CF₃, -OCF₃, SCF₃, hydroxy, -O-C(O)-alkyl, -NH-C(O)-alkyl, mono- or di-alkylamino, amino, or
Z₂₄ represents a radical of formula 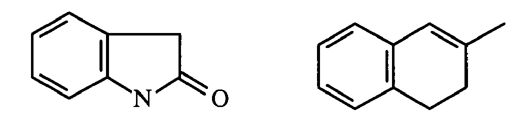 or X₂ represents 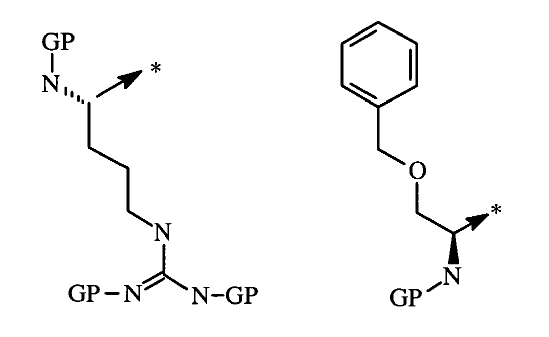 X₃ represents a -(CH₂)ₚZ₂₅ radical in which Z₂₅ represents the phenyl radical optionally substituted by one or more identical or different radicals chosen from alkoxy and CF₃,

16. Use of compounds of general formula Iₐ according to any of claims 13 to 15 in which R₃ₐ represents the hydrogen atom, an alkyl, alkenyl or furyl-methyl radical substituted by one or more nitro radicals, or a radical of formula -C(Y)-NHX₁, -C(O)X₂ or SO₂X₃ in which
X₁ represents a -(CH₂)ₚZ₂₂ radical in which
Z₂₂ represents the phenyl or naphthyl radical optionally substituted by one or more radicals chosen independently from the fluoro, chloro, bromo, iodo, alkyl, alkoxy, CF₃, nitro, phenoxy radicals,
X₂ represents the vinyl radical substituted by a phenyl radical itself optionally substituted by one or more halo, or -(CH₂)ₚ-U-Z₂₄ radicals in which
Z₂₄ represents alkyl, cyclohexyl, tetrahydrofuryl, *bis*-phenyl, amino, mono or di-alkylamino, or phenyl, indolyl, thienyl, pyridinyl, benzothienyl and furyl optionally substituted by one or more radicals chosen from alkoxy, bromo, chloro, fluoro, amino, mono- and di-alkylamino, nitro, hydroxy, pyrrolyl
or X₂ represents a radical of formula 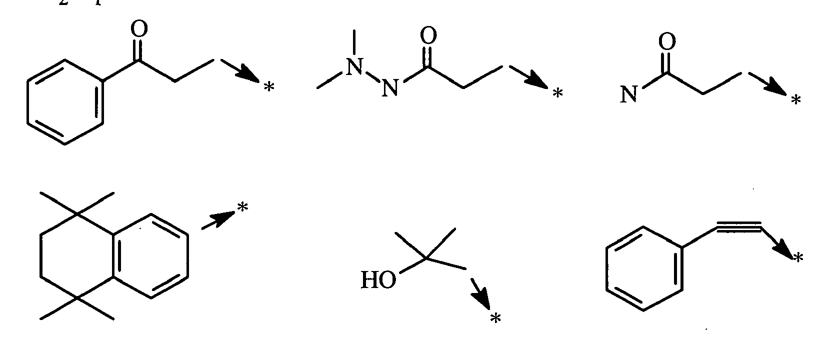 X₃ represents a linear or branched (C₁-C₁₀)alkyl radical, the vinyl radical substituted by a phenyl, CF₃, or -(CH₂)ₚZ₂₅ radical in which
Z₂₅ represents a phenyl, naphthyl, thienyl, pyrazolyl or thiazolyl radical optionally substituted by one or more substituents chosen independently from the fluoro, chloro, bromo, iodo, alkyl, alkoxy, CF₃, nitro, -NH-C(O)-alkyl, mono- and di-alkylamino radicals;

17. Use of compounds of general formula Iₐ according to any of claims 13 to 16 in which R₁ₐ represents the -(CH₂)ₘZ₁₂ radical in which m = 2 and Z₁₂ represents *bis-*phenyl or the radical indolyl substituted by one or more substituents chosen independently from the alkyl and alkoxy radicals.

18. Use of compounds of general formula Iₐ according to any of claims 13 to 17 in which R₂ₐ represents the radicals of formula -C(Y)NHX₁ and -C(O)X₂ in which
Y represents S;
X₁ represents a phenyl radical optionally substituted by one or more azido radicals,
X₂ represents -(CH₂)ₚZ₂₄ in which
p is equal to 1, 2 or 3,
Z₂₄ represents cyclohexyl, or phenyl or benzothienyl optionally substituted by one or more radicals chosen from fluoro, chloro, bromo, iodo or -CF₃.

19. Use of compounds of general formula Iₐ according to any of claims 13 to 18 in which R₃ₐ represents the hydrogen atom or the methyl radical.

20. Use according to claims 13 to 19 for the preparation of a medicament intended to treat acromegalia, hypophyseal adenomas or endocrine gastroenteropancreatic tumors including carcinoid syndrome.

## Patentansprüche

1. Verbindungen der allgemeinen Formel I 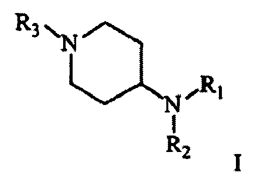 in racemischer Form, enantiomerer Form oder in allen Kombinationen dieser Formen, in der entweder R₁ einen linearen oder verzweigten (C₁-C₆)-Alkylrest, den Rest -(CH₂)ₘ-Y-Z₁₁ oder -(CH₂)ₘ-Z₁₂ darstellt, in dem
Z₁₁ ein (C₁-C₆)-Alkyl darstellt,
Z₁₂ darstellt: bis-Phenyl, (C₃-C₇)-Cycloalkyl, (C₃-C₇)-Heterocycloalkyl, das gegebenenfalls mit einem oder mehreren Substituenten substituiert ist, die unabhängig voneinander aus den Resten Oxy und Alkyl ausgewählt sind, oder Aryl oder Heteroaryl, gegebenenfalls mit einem oder mehreren Substituenten substituiert, die unabhängig voneinander aus den Resten Fluor, Chlor, Brom, Iod, Alkyl, Alkoxy ausgewählt sind,
oder Z₁₂ darstellt 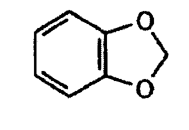 Y ein Sauerstoffatom darstellt
oder R₁ einen Rest der Formel 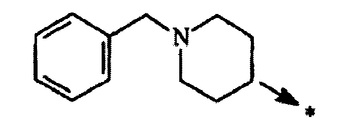 darstellt,
R₂ einen Rest der Formel -C(Y)NHX₁, -C(O)X₂ oder SO₂X₃ darstellt, in der
Y ein Sauerstoff- oder Schwefelatom darstellt;
X₁ einen linearen oder verzweigten (C₁-C₁₅)-Alkylrest oder -(CH₂)ₚZ₂₂ darstellt, in dem Z₂₂ darstellt: Cyclohexenyl, bis-Phenyl, (C₃-C₇)-Cycloalkyl, (C₃-C₇)-Heterocycloalkyl, Mono- oder Dialkylamino, -C(O)-O-Alkyl oder Aryl oder Heteroaryl, gegebenenfalls mit einem oder mehreren Resten substituiert, die unabhängig voneinander aus den Resten Fluor, Chlor, Brom, Iod, Alkyl, Alkoxy, Alkylthio, CF₃, OCF₃, Nitro, Cyano, Azido, Piperidinosulfonyl, -C(O)-O-Alkyl, -C(O)-Alkyl oder Phenyl ausgewählt sind,
oder Z₂₂ einen Rest der Formel 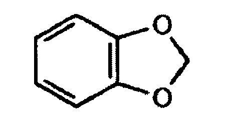 darstellt,
X₂ einen Rest Alkinyl, -(CH₂)ₘ-W-(CH₂)_{q}-Z₂₃ oder -(CH₂)ₚ-U-Z₂₄ darstellt, in dem
W SO₂ darstellt,
U eine kovalente Bindung darstellt,
Z₂₃ einen Arylrest darstellt;
Z₂₄ darstellt: Cyclohexenyl, bis-Phenyl, (C₃-C₇)-Cycloalkyl, das gegebenenfalls mit einem Aminoalkylrest substituiert ist, oder Aryl oder Heteroaryl, gegebenenfalls mit einem oder mehreren Resten substituiert, die aus Fluor, Chlor, Brom, Iod, Alkyl, Alkoxy, -CF₃, -OCF₃, -SCF₃, Hydroxy, -O-C(O)-Alkyl, Mono- oder Dialkylamino, Amino ausgewählt sind,
oder Z₂₄ einen Rest der Formel 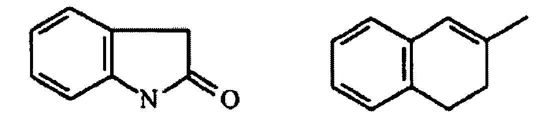 darstellt
oder X₂ darstellt 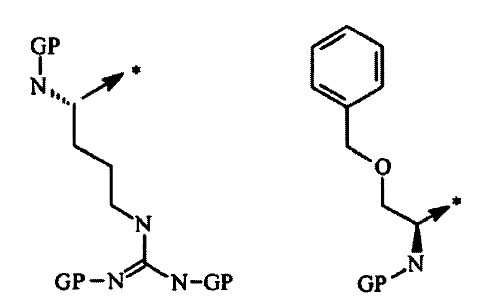 worin die Schutzgruppe (GP) H oder tert.-Butyloxycarbonyl darstellt;
X₃ einen Rest (-CH₂)ₚZ₂₅ darstellt, in dem Z₂₅ einen Arylrest darstellt, der gegebenenfalls mit einem oder mehreren gleichen oder verschiedenen Resten substituiert ist, die aus Alkoxy und CF₃ ausgewählt sind,
R₃ ein Wasserstoffatom, einen Rest Alkyl, Alkenyl, Heteroarylalkyl, der gegebenenfalls substituiert ist, oder einen Rest der Formel -C(Y)-NHX₁, -C(O)X₂ oder SO₂X₃ darstellt, in der
Y ein Sauerstoff- oder Schwefelatom darstellt;
X₁ einen Rest -(CH₂)ₚZ₂₂ darstellt, in dem
Z₂₂ einen Arylrest darstellt, der gegebenenfalls mit einem oder mehreren Resten substituiert ist, die unabhängig voneinander aus den Resten Fluor, Chlor, Brom, Iod, Alkyl, Alkoxy, CF₃, Nitro, Phenoxy ausgewählt sind;
X₂ darstellt: den Vinylrest, der mit einem Phenyl substituiert ist, wobei der Phenylrest seinerseits gegebenenfalls mit einem oder mehreren Halogenresten substituiert ist, oder -(CH₂)ₚ-U-Z₂₄, in dem
U eine kovalente Bindung oder ein Sauerstoffatom darstellt;
Z₂₄ darstellt: Alkyl, (C₃-C₇)-Cycloalkyl, (C₃-C₇)-Heterocycloalkyl, bis-Phenyl, Amino, Mono- oder Dialkylamino oder Aryl oder Heteroaryl, gegebenenfalls mit einem oder mehreren Resten substituiert, die aus Alkoxy, Brom, Chlor, Fluor, Hydroxy, CF₃, Nitro, Amino, Mono- und Dialkylamino, Pyrrolyl ausgewählt sind,
oder X₂ einen Rest der Formel 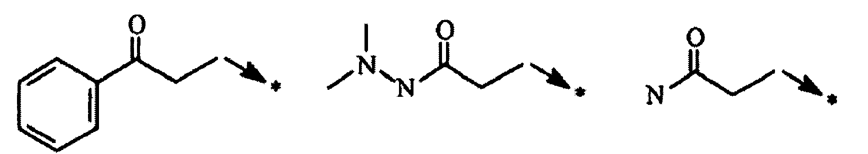 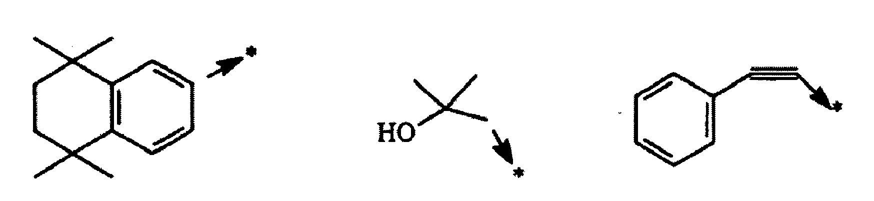 darstellt
X₃ darstellt: einen linearen oder verzweigten (C₁-C₁₀)-Alkylrest, den Vinylrest, der mit einem Rest substituiert ist (wobei der Phenylrest seinerseits gegebenenfalls substituiert ist), CF₃ oder -(CH₂)ₚZ₂₅, in dem Z₂₅ Aryl oder Heteroaryl darstellt, gegebenenfalls mit einem oder mehreren Substituenten substituiert, die unabhängig voneinander aus den Resten Fluor, Chlor, Brom, Iod, Alkyl, Alkoxy, CF₃, Nitro, -NH-C(O)-Alkyl, Mono- und Dialkylamino ausgewählt sind;
m eine ganze Zahl von 1 bis 6 ist;
p eine ganze Zahl von 0 bis 6 ist;
q eine ganze Zahl von 0 bis 2 ist,
oder
die Reste R₁, R₂ und R₃ jeweils die folgenden Reste darstellen:
| R₁ | R2 | R3 |
|---|---|---|
| 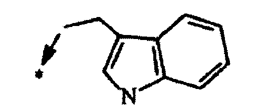 | 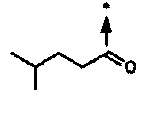 | 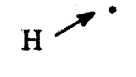 |
| 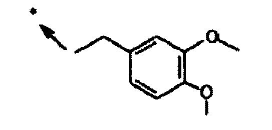 | 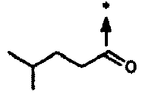 | 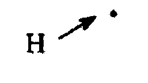 |
| 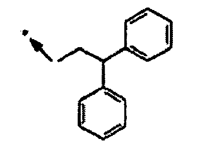 | 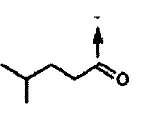 | 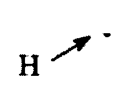 |
| 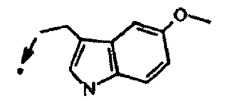 | 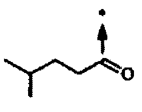 | 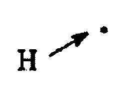 |
| 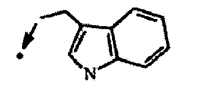 | 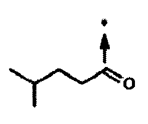 | 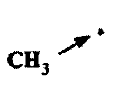 |
| 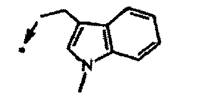 | 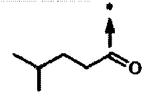 | 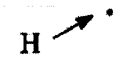 |
| 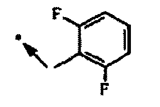 | 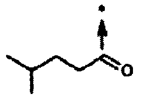 | 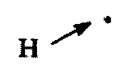 |
| 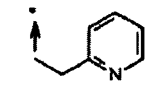 | 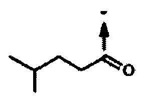 | 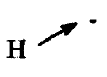 |
oder ihre Additionssalze mit den pharmazeutisch annehmbaren mineralischen oder organischen Säuren.

2. Verbindungen der Formel I, wie sie in Anspruch 1 definiert ist, **dadurch gekennzeichnet, dass** R₁ einen linearen oder verzweigten (C₁-C₆)-Alkylrest, den Rest -(CH₂)ₘ-Y-Z₁₁ oder -(CH₂)ₘ-Z₁₂ darstellt, in dem
Z₁₁ ein (C₁-C₆)-Alkyl darstellt,
Z₁₂ darstellt: Naphthyl, Morpholino, bis-Phenyl, Pyrrolidinyl, das mit dem Oxyrest substituiert ist, oder die Reste Phenyl, Piperazinyl, Pyridinyl und Indolyl, die gegebenenfalls durch einen oder mehrere Substituenten substituiert sind, die unabhängig voneinander aus den Resten Brom, Fluor, Chlor, Alkyl, Alkoxy, - CF₃, -OCF₃ ausgewählt sind;
oder Z₁₂ darstellt: 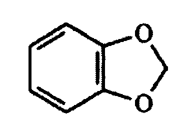 Y ein Sauerstoffatom darstellt,
oder R₁ einen Rest der Formel darstellt:

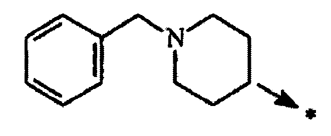

3. Verbindungen der Formel I, wie sie in einem der Ansprüche 1 bis 2 definiert ist, **dadurch gekennzeichnet, dass** R₂ einen Rest der Formel -C(Y)NHX₁, -C(O)X₂ oder SO₂X₃ darstellt, in der
X₁ einen linearen oder verzweigten (C₁-C₁₀)-Alkylrest oder -(CH₂)ₚZ₂₂ darstellt, in dem Z₂₂ darstellt: Cyclohexyl, Cyclohexenyl, bis-Phenyl, Morpholino, Piperidino, Mono- oder Dialkylamino, -C(O)-O-Alkyl, oder Phenyl, Naphthyl oder Furyl, gegebenenfalls mit einem oder mehreren Resten substituiert, die unabhängig voneinander aus den Resten Fluor, Chlor, Brom, Iod, Alkyl, Alkoxy, Alkylthio, CF₃, OCF₃, Nitro, Cyano, Azido, Piperidinosulfonyl, -C(O)-O-Alkyl, -C(O)-Alkyl oder Phenyl ausgewählt sind, oder Z₂₂ einen Rest der Formel darstellt: 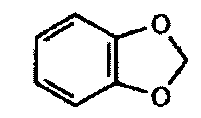 X₂ einen Rest Alkinyl, -(CH₂)ₘ-W-(CH₂)_{q}-Z₂₃ oder -(CH₂)ₚZ₂₄ darstellt, in dem
W SO₂ darstellt;
Z₂₃ den Phenylrest darstellt;
Z₂₄ darstellt: Cyclohexenyl, bis-Phenyl, Cyclohexyl, das gegebenenfalls mit einem Aminoalkylrest substituiert ist, oder Phenyl, Naphthyl, Benzothienyl, Thienyl oder Indolyl, gegebenenfalls mit einem oder mehreren Resten substituiert, die aus Fluor, Chlor, Brom, Iod, Alkyl, Alkoxy, -CF₃, -OCF₃, SCF₃, Hydroxy, -O-C(O)-Alkyl, - NH-C(O)-Alkyl, Mono- oder Dialkylamino, Amino ausgewählt sind, oder
Z₂₄ einen Rest der Formel darstellt: 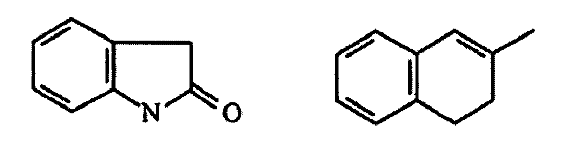 oder X₂ darstellt: 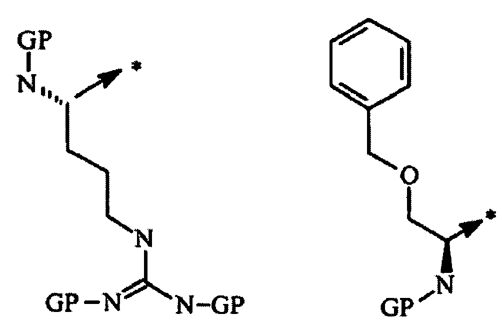 X₃ einen Rest -(CH₂)ₚZ₂₅ darstellt, in dem Z₂₅ den Phenylrest darstellt, der gegebenenfalls mit einem oder mehreren gleichen oder verschiedenen Resten substituiert ist, die aus Alkoxy und CF₃ ausgewählt sind.

4. Verbindungen der Formel I, wie sie in einem der Ansprüche 1 bis 3 definiert ist, **dadurch gekennzeichnet, dass** R₃ ein Wasserstoffatom, einen Rest Alkyl, Alkenyl oder Furylmethyl darstellt, der durch einen oder mehrere Nitroreste substituiert ist, oder einen Rest der Formel -C(Y)-NHX₁, -C(O)X₂ oder SO₂X₃, in der
X₁ einen Rest -(CH₂)ₚZ₂₂ darstellt, in dem
Z₂₂ den Rest Phenyl oder Naphthyl darstellt, der gegebenenfalls mit einem oder mehreren Resten substituiert ist, die unabhängig voneinander aus den Resten Fluor, Chlor, Brom, Iod, Alkyl, Alkoxy, CF₃, Nitro, Phenoxy ausgewählt sind,
X₂ den Vinylrest, der mit einem Phenylrest substituiert ist, der seinerseits gegebenenfalls mit einem oder mehreren Halogenresten substituiert ist, oder -(CH₂)ₚ-U-Z₂₄ darstellt, in dem
Z₂₄ darstellt: Alkyl, Cyclohexyl, Tetrahydrofuryl, bis-Phenyl, Amino, Mono- oder Dialkylamino oder Phenyl, Indolyl, Thienyl, Pyridinyl, Benzothienyl und Furyl, gegebenenfalls mit einem oder mehreren Resten substituiert, die aus Alkoxy, Brom, Chlor, Fluor, Amino, Mono- und Dialkylamino, Nitro, Hydroxy, Pyrrolyl ausgewählt sind,
oder X₂ einen Rest der Formel darstellt: 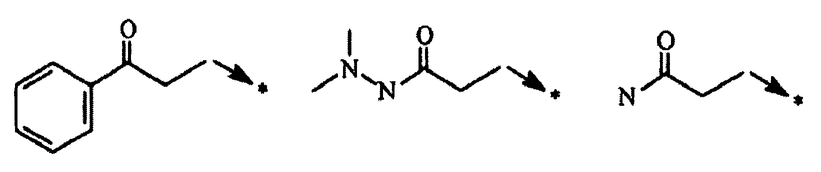 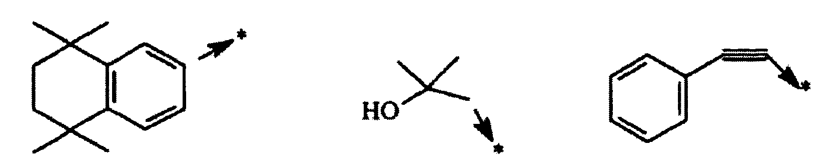 X₃ einen linearen oder verzweigten (C₁-C₁₀)-Alkylrest, den mit einem Phenylrest substituierten Vinylrest, CF₃ oder -(CH₂)ₚZ₂₅ darstellt, in dem
Z₂₅ darstellt: einen Rest Phenyl, Naphthyl, Thienyl, Pyrazolyl oder Thiazolyl, der gegebenenfalls mit einem oder mehreren Substituenten substituiert ist, die unabhängig voneinander aus den Resten Fluor, Chlor, Brom, Iod, Alkyl, Alkoxy, CF₃, Nitro, -NH-C(O)-Alkyl, Mono- und Dialkylamino ausgewählt sind.

5. Verbindungen der Formel I, wie sie in einem der Ansprüche 1 bis 4 definiert ist, **dadurch gekennzeichnet, dass** R₁ den Rest -(CH₂)ₘZ₁₂ darstellt, in dem m = 2 und Z₁₂ bis-Phenyl oder den Indolylrest darstellt, die mit einem oder mehreren Substituenten substituiert sind, die unabhängig voneinander aus den Alkyl- und Alkoxyresten ausgewählt sind.

6. Verbindungen der Formel I, wie sie in einem der Ansprüche 1 bis 5 definiert ist, **dadurch gekennzeichnet, dass** R₂ die Reste der Formel -C(Y)NHX₁ und -C(O)X₂ darstellt, in der
Y S darstellt;
X₁ einen Phenylrest darstellt, der gegebenenfalls mit einem oder mehreren Azidoresten substituiert ist,
X₂ -(CH₂)ₚZ₂₄ darstellt, in dem
p gleich 1, 2 oder 3 ist,
Z₂₄ Cyclohexyl oder Phenyl oder Benzothienyl darstellt, gegebenenfalls mit einem oder mehreren Resten substituiert, die aus Fluor, Chlor, Brom, Iod oder -CF₃ ausgewählt sind.

7. Verbindungen der Formel I, wie sie in einem der Ansprüche 1 bis 6 definiert ist, **dadurch gekennzeichnet, dass** R₃ ein Wasserstoffatom oder den Methylrest darstellt.

8. Verfahren zur Herstellung von Verbindungen der Formel I, wie sie in Anspruch 1 definiert ist, in flüssiger Phase, **dadurch gekennzeichnet, dass** es
die reduzierende Aminierung des folgenden N-substituierten Piperidons, 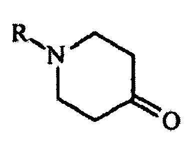 in dem R den Methylrest oder Boc darstellt, in Gegenwart eines Amins der Formel R₁NH₂ umfasst, in der R₁ die in Anspruch 1 angegebene Bedeutung hat, um die Verbindung der Formel 1 zu ergeben, 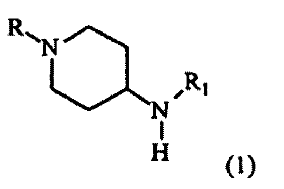 wobei man diese Verbindung der Formel (1) reagieren lässt mit
A) entweder einer Verbindung der Formel X₁NC(Y), in der X₁ und Y die in Anspruch 1 angegebene Bedeutung haben, um eine Verbindung der Formel (2) zu ergeben, 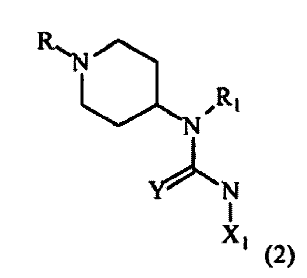 wobei diese Verbindung der Formel (2) die entsprechende Verbindung der Formel (I) darstellt, in der R₃ Me oder Boc darstellt, und, wenn R₃ Boc darstellt, einer sauren Behandlung unterzogen werden kann, um die entsprechende Verbindung der Formel (I) zu ergeben, in der R₃ ein Wasserstoffatom darstellt,
wobei man die auf diese Weise erhaltene Verbindung der Formel (I) mit einer Verbindung der Formel X₁NC(Y), X₂CO₂H oder X₃SO₂Cl, in der X₁, Y, X₂ und X₃ die in Anspruch 1 angegebene Bedeutung haben, reagieren lassen kann, um die entsprechende Verbindung der Formel I zu ergeben, in der R₂ einen Rest der Formel -C(Y)NHX₁ und R₃ den Rest -C(Y)-NHX₁, -C(O)X₂ bzw. SO₂X₃ darstellt;
B) oder mit einer Verbindung der Formel X₂CO₂H, in der X₂ die in Anspruch 1 angegebene Bedeutung hat, um eine Verbindung der Formel (3) zu ergeben, 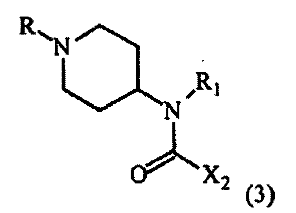
wobei die Verbindung der Formel (3) die entsprechende Verbindung der Formel (I) darstellt, in der R₃ Me oder Boc darstellt, und, wenn R₃ Boc darstellt, einer sauren Behandlung unterzogen werden kann, um die entsprechende Verbindung der Formel (I) zu ergeben, in der R₃ ein Wasserstoffatom darstellt,
wobei man die auf diese Weise erhaltene Verbindung der Formel (I) mit einer Verbindung der Formel X₁NC(Y), X₂CO₂H oder X₃SO₂Cl, in der X₁, Y, X₂ und X₃ die in Anspruch 1 angegebene Bedeutung haben, reagieren lassen kann, um die entsprechende Verbindung der Formel I zu ergeben, in der R₂ einen Rest der Formel -C(O)X₂ und R₃ den Rest -C(Y)-NHX₁, -C(O)X₂ bzw. SO₂X₃ darstellt.

9. Verfahren zur Herstellung von Verbindungen der Formel I, wie sie in Anspruch 1 definiert ist, in fester Phase, **dadurch gekennzeichnet, dass** es
die reduzierende Aminierung des Keton-Harzes 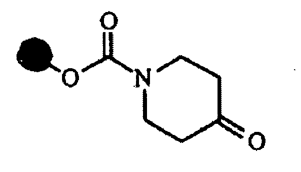 in Gegenwart eines Amins der Formel R₁NH₂, in der R₁ die in Anspruch 1 angegebene Bedeutung hat, umfasst, um die Verbindung der Formel (4) zu ergeben, 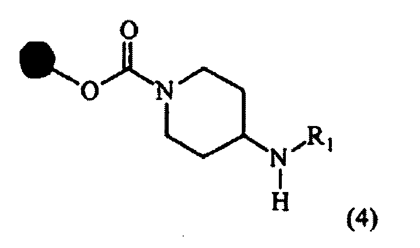 wobei man diese Verbindung der Formel (4) reagieren lässt
A) entweder mit einer Verbindung der Formel X₁NC(Y), in der X₁ und Y die in Anspruch 1 angegebene Bedeutung haben, um eine Verbindung der Formel (5) zu ergeben, 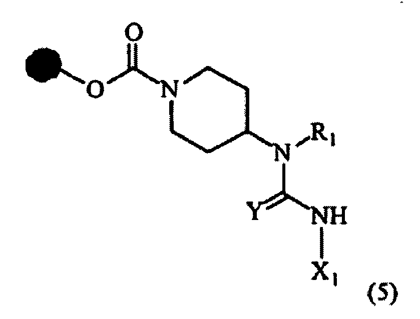 worauf eine Abspaltung des Harzes folgt, um die entsprechende Verbindung der Formel (I) zu ergeben, in der R₃ ein Wasserstoffatom darstellt,
B) oder mit einer Verbindung der Formel X₃SO₂Cl, in der X₃ die im Anspruch 1 angegebene Bedeutung hat, um eine Verbindung der Formel (6) zu ergeben, 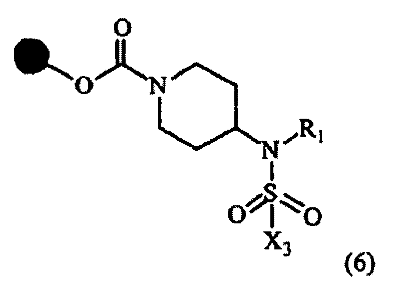 worauf die Abspaltung des Harzes folgt, um die entsprechende Verbindung der Formel (I) zu ergeben, in der R₃ ein Wasserstoffatom darstellt,
C) oder mit einer Verbindung der Formel X₂CO₂Cl, in der X₂ die in Anspruch 1 angegebene Bedeutung hat, um eine Verbindung der Formel (7) zu ergeben, 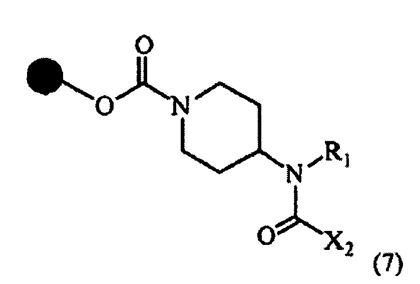 worauf die Abspaltung des Harzes folgt, um die entsprechende Verbindung der Formel (I) zu ergeben, in der R₃ ein Wasserstoffatom darstellt;
D) oder mit einer Verbindung der Formel X₂CO₂H, in der X₂ die in Anspruch 1 angegebene Bedeutung hat, um eine Verbindung der Formel (7), wie sie oben definiert ist, zu ergeben, worauf die Abspaltung des Harzes folgt, um die entsprechende Verbindung der Formel (I) zu ergeben, in der R₃ ein Wasserstoffatom darstellt.

10. Verfahren zur Herstellung von Verbindungen der Formel (I), wie sie in Anspruch 1 definiert ist, in fester Phase, **dadurch gekennzeichnet, dass** es
die reduzierende Animierung des Keton-Harzes 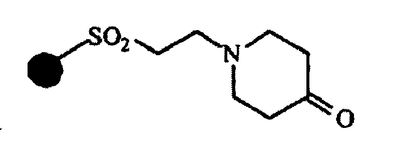 in Gegenwart eines Amins der Formel R₁NH₂ umfasst, in der R₁ die in Anspruch 1 angegebene Bedeutung hat, um die Verbindung der Formel (8) zu ergeben, 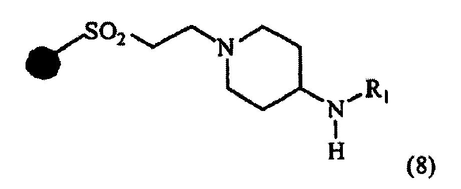 wobei man diese Verbindung der Formel (8) reagieren lässt
A) entweder mit einer Verbindung der Formel X₁NC(O), in der X₁ die in Anspruch 1 angegebene Bedeutung hat, um eine Verbindung der Formel (9) zu ergeben, 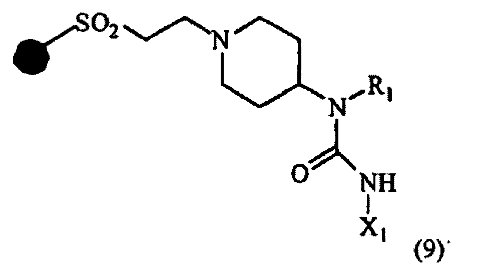 wobei man die auf diese Weise gebildete Verbindung (9) mit einer Verbindung der Formel R₃X reagieren lässt, in der R₃ wie in Anspruch 1 definiert ist, und X Br oder I darstellt, worauf die Abspaltung des Harzes folgt, um die entsprechende Verbindung der Formel (I) zu ergeben;
B) oder mit einer Verbindung der Formel X₃SO₂Cl, in der X₃ die in Anspruch 1 angegebene Bedeutung hat, um eine Verbindung der Formel (10) zu ergeben, 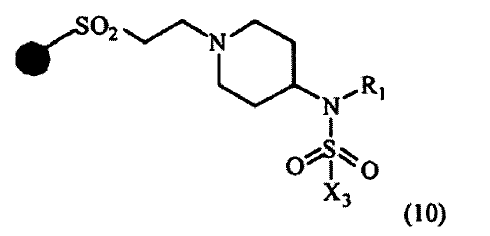 wobei man die auf diese Weise gebildete Verbindung (10) mit einer Verbindung der Formel R₃X reagieren lasst, in der R₃ wie in Anspruch 1 definiert ist, und X Br oder I darstellt, worauf die Abspaltung des Harzes folgt, um die entsprechende Verbindung der Formel (I) zu ergeben;
C) oder mit einer Verbindung der Formel X₂CO₂Cl, in der X₂ die im Anspruch 1 angegebene Bedeutung hat, um eine Verbindung der Formel (11) zu ergeben, 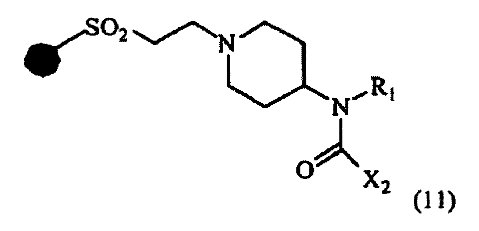 wobei man die auf diese Weise gebildete Verbindung (11) mit einer Verbindung der Formel R₃X reagieren lässt, in der R₃ wie in Anspruch 1 definiert ist, und X Br oder 1 darstellt, worauf die Abspaltung des Harzes folgt, um die entsprechende Verbindung der Formel (I) zu ergeben;
D) oder mit einer Verbindung der Formel X₂CO₂H, in der X₂ die in Anspruch 1 angegebene Bedeutung hat, um eine Verbindung der Formel (11) zu ergeben, wie sie oben definiert ist,
wobei man die auf diese Weise gebildete Verbindung (11) mit einer Verbindung der Formel R₃X reagieren lässt, in der R₃ wie in Anspruch 1 definiert ist, und X Br oder I darstellt, worauf die Abspaltung des Harzes folgt, um die entsprechende Verbindung der Formel (I) zu ergeben.

11. Die Produkte der Formel I, wie sie in einem der Ansprüche 1 bis 7 definiert ist, sowie die Additionssalze mit den pharmazeutisch annehmbaren mineralischen oder organischen Säuren dieser Produkte der Formel I als Medikamente.

12. Pharmazeutische Zusammensetzungen, die als Wirkstoff mindestens eines der Medikamente, wie sie im Anspruch 11 definiert sind, in Kombination mit einem pharmazeutisch annehmbaren Träger enthalten.

13. Verwendung einer Verbindung der allgemeinen Formel lₐ 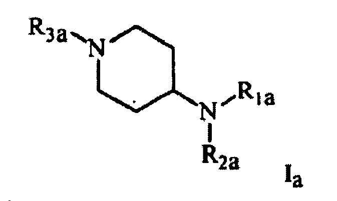 in racemischer Form, enantiomerer Form oder allen Kombinationen dieser Formen, in der:
R₁ₐ einen linearen oder verzweigten (C₁-C₆)-Alkylrest, den Rest -(CH₂)ₘ-Y-Z₁₁ oder -(CH₂)ₘ-Z₁₂ darstellt, in dem
Z₁₁ ein (C₁-C₆)-Alkyl darstellt,
Z₁₂ darstellt: *bis*-Phenyl, (C₃-C₇)-Cycloalkyl, (C₃-C₇)-Heterocycloalkyl, das gegebenenfalls mit einem oder mehreren Substituenten substituiert ist, die unabhängig voneinander aus den Resten Oxy und Alkyl ausgewählt sind; oder Aryl oder Heteroaryl, gegebenenfalls mit einem oder mehreren Substituenten substituiert, die unabhängig voneinander aus den Resten Fluor, Chlor, Brom, Iod, Alkyl, Alkoxy ausgewählt sind, oder Z₁₂ darstellt: 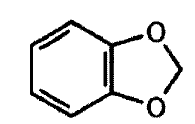
Y ein Sauerstoffatom darstellt,
oder R₁ₐ einen Rest der Formel darstellt: 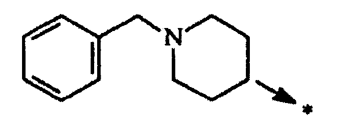
R₂ₐ einen Rest der Formel -C(Y)NHX₁, -C(O)X₂ oder SO₂X₃ darstellt, in der
Y ein Sauerstoff- oder Schwefelatom darstellt;
X₁ einen linearen oder verzweigten (C₁-C₁₅)-Alkylrest oder -(CH₂)ₚZ₂₂ darstellt, in dem Z₂₂ darstellt: Cyclohexenyl, bis-Phenyl, (C₃-C₇)-Cycloalkyl, (C₃-C₇)-Heterocycloalkyl, Mono- oder Dialkylamino, -C(O)-O-Alkyl oder Aryl oder Heteroaryl, gegebenenfalls mit einem oder mehreren Resten substituiert, die unabhängig voneinander aus den Resten Fluor, Chlor, Brom, Iod, Alkyl, Alkoxy, Alkylthio, CF₃, OCF₃, Nitro, Cyano, Azido, Piperidinosulfonyl, -C(O)-O-Alkyl, -C(O)-Alkyl oder Phenyl ausgewählt sind, oder Z₂₂ einen Rest der Formel darstellt: 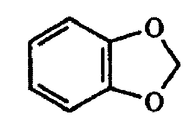
X₂ einen Rest lineares oder verzweigtes (C₁-C₁₀)-Alkyl, Alkinyl, -(CH₂)ₘ-W-(CH₂)_{q}-Z₂₃ oder -(CH₂)ₚ-U-Z₂₄ darstellt, in dem
W SO₂ darstellt,
U eine kovalente Bindung darstellt;
Z₂₃ einen Arylrest darstellt;
Z₂₄ darstellt: Cyclohexenyl, bis-Phenyl, (C₃-C₇)-Cycloalkyl, das gegebenenfalls mit einem Aminoalkylrest substituiert ist, oder Aryl oder Heteroaryl, gegebenenfalls mit einem oder mehreren Resten substituiert, die aus Fluor, Chlor, Brom, Iod, Alkyl, Alkoxy, -CF₃, -OCF₃, SCF₃, Hydroxy, -O-C(O)-Alkyl, Mono- oder Dialkylamino, Amino ausgewählt sind,
oder Z₂₄ einen Rest der Formel darstellt 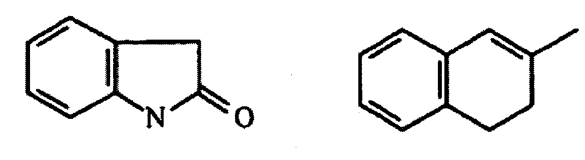 oder X₂ darstellt, 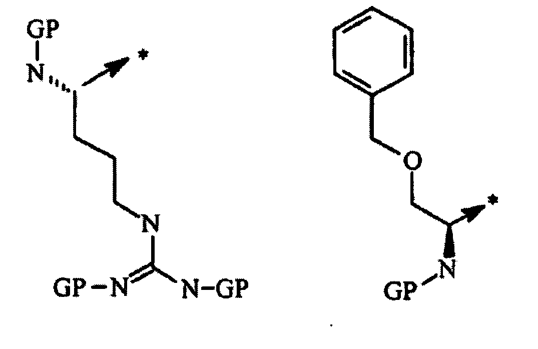
worin die Schutzgruppe (GP) H oder tert.-Butyloxycarbonyl darstellt;
X₃ einen Rest -(CH₂)ₚZ₂₅ darstellt, in dem Z₂₅ einen Arylrest darstellt, der gegebenenfalls mit einem oder mehreren gleichen oder verschiedenen Resten substituiert ist, die aus Alkoxy und CF₃ ausgewählt sind;
R₃ₐ ein Wasserstoffatom, einen Rest Alkyl, Alkenyl, Heteroarylalkyl, der gegebenenfalls substituiert ist, oder einen Rest der Formel -C(Y)-NHX₁, -C(O)X₂ oder SO₂X₃ darstellt, in der
Y ein Sauerstoff- oder Schwefelatom darstellt;
X₁ einen Rest -(CH₂)ₚZ₂₂ darstellt, in dem
Z₂₂ einen Arylrest darstellt, der gegebenenfalls mit einem oder mehreren Resten substituiert ist, die unabhängig voneinander aus den Resten Fluor, Chlor, Brom, Iod, Alkyl, Alkoxy, CF₃, Nitro, Phenoxy ausgewählt sind;
X₂ den mit einem Phenyl substituierten Vinylrest, wobei der Phenylrest seinerseits gegebenenfalls mit einem oder mehreren Halogenresten substituiert ist, oder -(CH₂)ₚ-U-Z₂₄ darstellt, in dem
U eine kovalente Bindung oder ein Sauerstoffatom darstellt;
Z₂₄ darstellt: Alkyl, (C₃-C₇)-Cycloalkyl, (C₃-C₇)-Heterocycloalkyl, bis-Phenyl, Amino, Mono- oder Dialkylamino, oder Aryl oder Heteroaryl, gegebenenfalls mit einem oder mehreren Resten substituiert, die aus Alkoxy, Brom, Chlor, Fluor, Hydroxy, CF₃, Nitro, Amino, Mono- und Dialkylamino, Pyrrolyl ausgewählt sind,
oder X₂ einen Rest der Formel darstellt 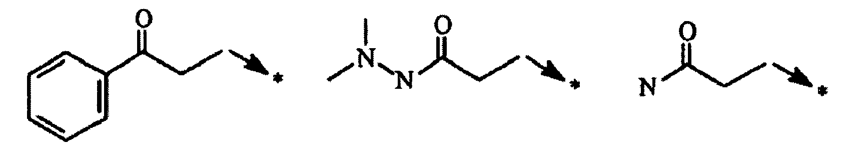 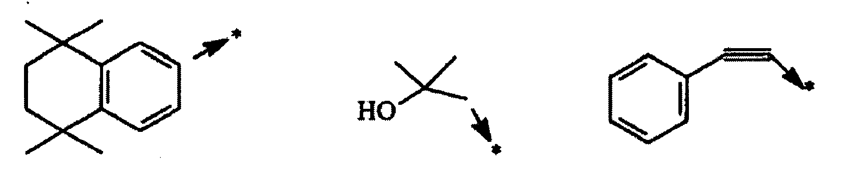 X₃ einen linearen oder verzweigten (C₁-C₁₀)-Alkylrest, den Vinylrest, der mit einem Rest substituiert ist (wobei der Phenylrest seinerseits gegebenenfalls substituiert ist), CF₃ oder -(CH₂)ₚ-Z₂₅ darstellt, in dem Z₂₅ Aryl oder Heteroaryl darstellt, gegebenenfalls mit einem oder mehreren Substituenten substituiert, die unabhängig voneinander aus den Resten Fluor, Chlor, Brom, Iod, Alkyl, Alkoxy, CF₃, Nitro, -NH-C(O)-Alkyl, Mono- und Dialkylamino ausgewählt sind,
m eine ganze Zahl von 1 bis 6 ist;
p eine ganze Zahl von 0 bis 6 ist;
q eine ganze Zahl von 0 bis 2 ist,
oder ihre Additionssalze mit den pharmazeutisch annehmbaren mineralischen oder organischen Säuren, für die Herstellung eines Medikaments, das zur Behandlung von pathologischen Zuständen oder Krankheiten bestimmt ist, an denen ein (oder mehrere) Somatostatinrezeptor(en) beteiligt ist (sind).

14. Verwendung der Produkte der allgemeinen Formel lₐ nach Anspruch 13, **dadurch gekennzeichnet, dass** R₁ₐ einen linearen oder verzweigten (C₁-C₆)-Alkylrest, den Rest -(CH₂)ₘ-Y-Z₁₁ oder -(CH₂)ₘ-Z₁₂ darstellt, in dem
Z₁₁ ein (C₁-C₆)-Alkyl darstellt,
Z₁₂ darstellt: Naphthyl, Morpholino, bis-Phenyl, Pyrrolidinyl, das mit dem Oxyrest substituiert ist, oder die Reste Phenyl, Piperazinyl, Pyridinyl und Indolyl, die gegebenenfalls mit einem oder mehreren Substituenten substituiert sind, die unabhängig voneinander aus den Resten Brom, Fluor, Chlor, Alkyl, Alkoxy, -CF₃, -OCF₃ ausgewählt sind;
oder Z₁₂ darstellt 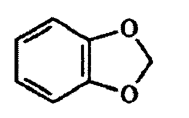 Y ein Sauerstoffatom darstellt,
oder R₁ₐ einen Rest der nachstehenden Formel darstellt:

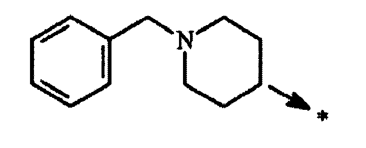

15. Verwendung der Produkte der allgemeinen Formel lₐ nach einem der Ansprüche 13 bis 14, **dadurch gekennzeichnet, dass** R₂ₐ einen Rest der Formel -C(Y)NHX₁, -C(O)X₂ oder SO₂X₃ darstellt, in der
X₁ einen linearen oder verzweigten (C₁-C₁₀)-Alkylrest oder -(CH₂)pZ₂₂ darstellt, in dem Z₂₂ darstellt: Cyclohexyl, Cyclohexenyl, bis-Phenyl, Morpholino, Piperidino, Mono- oder Dialkylamino, -C(O)-O-Alkyl oder Phenyl, Naphthyl oder Furyl, gegebenenfalls mit einem oder mehreren Resten substituiert, die unabhängig voneinander aus den Resten Fluor, Chlor, Brom, Iod, Alkyl, Alkoxy, Alkylthio, CF₃, OCF₃, Nitro, Cyano, Azido, Piperidinosulfonyl, -C(O)-O-Alkyl, -C(O)-Alkyl oder Phenyl ausgewählt sind, oder Z₂₂ einen Rest der Formel darstellt: 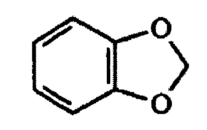 X₂ einen Rest Alkyl, Alkinyl, -(CH₂)ₘ-W-(CH₂)_{q}-Z₂₃ oder -(CH₂)ₚZ₂₄ darstellt, in dem
W SO₂ darstellt;
Z₂₃ den Phenylrest darstellt;
Z₂₄ darstellt: Cyclohexenyl, bis-Phenyl, Cyclohexyl, das gegebenenfalls mit einem Aminoalkylrest substituiert ist, oder Phenyl, Naphthyl, Benzothienyl, Thienyl oder Indolyl, gegebenenfalls mit einem oder mehreren Resten substituiert, die aus Fluor, Chlor, Brom, Iod, Alkyl, Alkoxy, -CF₃, -OCF₃, SCF₃, Hydroxy, -O-C(O)-Alkyl, - NH-C(O)-Alkyl, Mono- oder Dialkylamino, Amino ausgewählt sind, oder
Z₂₄ einen Rest der Formel darstellt 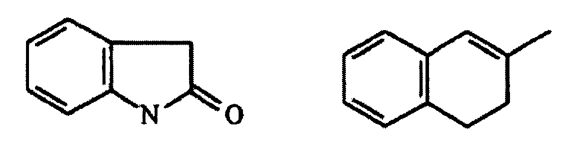 oder X₂ darstellt 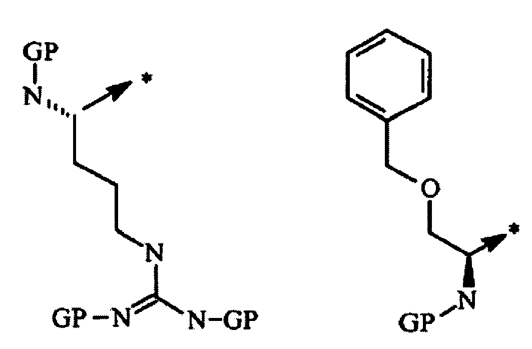 X₃ einen Rest -(CH₂)ₚZ₂₅ darstellt, in dem Z₂₅ den Phenylrest darstellt, der gegebenenfalls mit einem oder mehreren gleichen oder verschiedenen Resten substituiert ist, die aus Alkoxy und CF₃ ausgewählt sind.

16. Verwendung der Produkte der allgemeinen Formel Iₐ nach einem der Ansprüche 13 bis 15, **dadurch gekennzeichnet, dass** R₃ₐ darstellt: ein Wasserstoffatom, einen Rest Alkyl, Alkenyl oder Furylmethyl, das mit einem oder mehreren Nitroresten substituiert ist, oder einen Rest der Formel -C(Y)-NHX₁, -C(O)X₂ oder SO₂X₃, in der
X₁ einen Rest -(CH₂)ₚZ₂₂ darstellt, in dem
Z₂₂ den Phenyl- oder Naphtylrest darstellt, der gegebenenfalls mit einem oder mehreren Resten substituiert ist, die unabhängig voneinander aus den Resten Fluor, Chlor, Brom, Iod, Alkyl, Alkoxy, CF₃, Nitro, Phenoxy ausgewählt sind,
X₂ darstellt: den Vinylrest, der mit einem Phenylrest substituiert ist, der seinerseits gegebenenfalls mit einem oder mehreren Halogenresten substituiert ist, oder -(CH₂)ₚ-U-Z₂₄, in dem
Z₂₄ darstellt: Alkyl, Cyclohexyl, Tetrahydrofuryl, bis-Phenyl, Amino, Mono- oder Dialkylamino, oder Phenyl, Indolyl, Thienyl, Pyridinyl, Benzothienyl und Furyl, gegebenenfalls mit einem oder mehreren Resten substituiert, die aus Alkoxy, Brom, Chlor, Fluor, Amino, Mono- und Dialkylamino, Nitro, Hydroxy, Pyrrolyl ausgewählt sind
oder X₂ einen Rest der Formel darstellt: 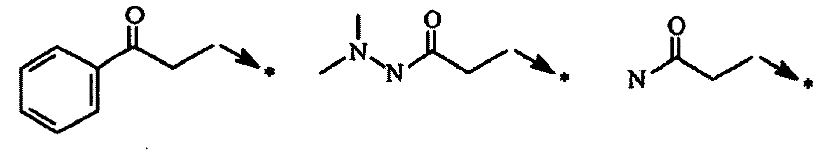 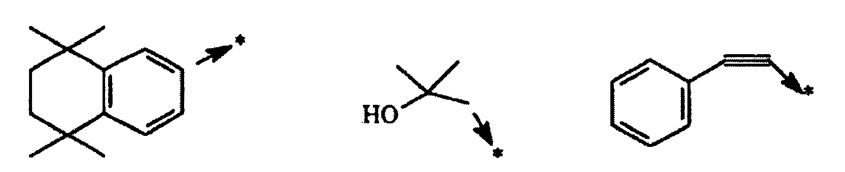 X₃ einen linearen oder verzweigten (C₁-C₁₀)-Alkylrest, den mit einem Phenylrest substituierten Vinylrest, CF₃ oder -(CH₂)ₚZ₂₅ darstellt, in dem
Z₂₅ einen Rest Phenyl, Naphthyl, Thienyl, Pyrazolyl oder Thiazolyl darstellt, der gegebenenfalls mit einem oder mehreren Substituenten substituiert ist, die unabhängig voneinander aus den Resten Fluor, Chlor, Brom, Iod, Alkyl, Alkoxy, CF₃, Nitro, -NH-C(O)-Alkyl, Mono- und Dialkylamino ausgewählt sind.

17. Verwendung der Produkte der allgemeinen Formel lₐ nach einem der Ansprüche 13 bis 16, **dadurch gekennzeichnet, dass** R₁ₐ den Rest -(CH₂)ₘZ₁₂ darstellt, in dem m = 2 und Z₁₂ bis-Phenyl oder den Indolylrest darstellt, die mit einem oder mehreren Substituenten substituiert sind, die unabhängig voneinander aus den Resten Alkyl und Alkoxy ausgewählt sind.

18. Verwendung der Produkte der allgemeinen Formel Iₐ nach einem der Ansprüche 13 bis 17, **dadurch gekennzeichnet, dass** R₂ₐ die Reste der Formel -C(Y)NHX₁ und -C(O)X₂ darstellt, in der
X S darstellt;
X₁ einen Phenylrest darstellt, der gegebenenfalls mit einem oder mehreren Azidoresten substituiert ist;
X₂ -(CH₂)ₚZ₂₄ darstellt, in dem
p gleich 1, 2 oder 3 ist,
Z₂₄ Cyclohexyl oder Phenyl oder Benzothienyl darstellt, gegebenenfalls mit einem oder mehreren Resten substituiert, die aus Fluor, Chlor, Brom, Iod oder -CF₃ ausgewählt sind.

19. Verwendung der Produkte der allgemeinen Formel Iₐ nach einem der Ansprüche 13 bis 18, **dadurch gekennzeichnet, dass** R₃ₐ ein Wasserstoffatom oder den Methylrest darstellt.

20. Verwendung nach einem der Ansprüche 13 bis 19 für die Herstellung eines Medikaments, das zur Behandlung der Akromegalie, von Hypophysenadenomen oder von gastroenteropankreatischen endokrinen Tumoren, darunter das Karzinoidsyndrom, bestimmt ist.
